# EUROPÄISCHE PATENTSCHRIFT

(11) **EP 1 625 153 B1**
(45) Veröffentlichungstag und Bekanntmachung des Hinweises auf die Patenterteilung: **26.08.2009**
(21) Anmeldenummer: 04729639.7
(22) Anmeldetag: 27.04.2004
(51) Int. Cl.: C07K 11/02, C07K 5/06, A61K 38/15

(54) **ACYLIERTE NONADEPSIPEPTIDE VOM LYSOBACTIN-TYP**
ACYLATED LYSOBACTIN-TYPE NONADEPSIPEPTIDES
NONADEPSIPEPTIDES ACYLES DU TYPE LYSOBACTINE

(30) Priorität: 09.05.2003 DE 10320781
(43) Veröffentlichungstag der Anmeldung: 15.02.2006
(73) Patentinhaber: AiCuris GmbH & Co. KG, 42117 Wuppertal (DE)
(72) Erfinder: VON NUSSBAUM, Franz, 40219 Düsseldorf (DE); BRUNNER, Nina, 45279 Essen (DE); ANLAUF, Sonja, 42115 Wuppertal (DE); ENDERMANN, Rainer, 42113 Wuppertal (DE); FÜRSTNER, Chantal, 45478 Mülheim (DE); HARTMANN, Elke, 42111 Wuppertal (DE); KÖBBERLING, Johannes, 41516 Grevenbroich (DE); RAGOT, Jacques, 40625 Düsseldorf (DE); SCHIFFER, Guido, 42111 Wuppertal (DE); SCHUHMACHER, Joachim, 42113 Wuppertal (DE); SVENSTRUP, Niels, 42553 Velbert (DE); TELSER, Joachim, 51061 Köln (DE); BRÜNING, Michael-Alexander, 13469 Berlin (DE)
(74) Vertreter: Findeisen, Marco
(86) Internationale Anmeldenummer: PCT/EP2004/004416
(87) Internationale Veröffentlichungsnummer: WO 2004/099239

(56) Entgegenhaltungen:
- PALOMO C ET AL: "A concise synthesis of alpha-amino acid N-carboxy anhydrides of (2S,3S)-beta-substituted serines" TETRAHEDRON LETTERS, ELSEVIER SCIENCE PUBLISHERS, AMSTERDAM, NL, Bd. 42, Nr. 51, 17. Dezember 2001 (2001-12-17), Seiten 8955-8957, XP004323292 ISSN: 0040-4039
- HARADA K-I ET AL: "Separation of WAP-8294A components, a novel anti-methicillin-resistant Staphylococcus aureus antibiotic, using high-speed counter-current chromatography" JOURNAL OF CHROMATOGRAPHY A, ELSEVIER SCIENCE, NL, Bd. 932, Nr. 1-2, 12. Oktober 2001 (2001-10-12), Seiten 75-81, XP004308399 ISSN: 0021-9673
- EGNER B J ET AL: "Monitoring the Solid Phase Synthesis of Analogues of Lysobactin and the Katanosins using in situ MALDI-TOF MS" TETRAHEDRON, ELSEVIER SCIENCE PUBLISHERS, AMSTERDAM, NL, Bd. 53, Nr. 41, 13. Oktober 1997 (1997-10-13), Seiten 14021-14030, XP004106274 ISSN: 0040-4020
- TYMIAK A A ET AL: "STRUCTURE DETERMINATION OF LYSOBACTIN A MACROCYCLIC PEPTIDE LACTONE ANTIBIOTIC" JOURNAL OF ORGANIC CHEMISTRY, Bd. 54, Nr. 5, 1989, Seiten 1149-1157, XP002289533 ISSN: 0022-3263 in der Anmeldung erwähnt
- TYMIAK A A ET AL: "STRUCTURE DETERMINATION OF LYSOBACTIN, A MACROCYCLIC PEPTIDE LACTONE ANTIBIOTIC" JOURNAL OF ORGANIC CHEMISTRY, AMERICAN CHEMICAL SOCIETY, BALTIMORE, MD, US, Bd. 54, Nr. 5, 1989, Seiten 1149-1157, XP002942416 ISSN: 0021-9258

## Beschreibung

Die Erfindung betrifft Nonadepsipeptide und Verfahren zu ihrer Herstellung sowie ihre Verwendung zur Herstellung von Arzneimitteln zur Behandlung und/oder Prophylaxe von Krankheiten, insbesondere bakteriellen Infektionskrankheiten.

Die bakterielle Zellwand wird durch eine Reihe von Enzymen synthetisiert (Zellwandbiosynthese) und ist essentiell für das Überleben bzw. die Vermehrung von Mikroorganismen. Die Struktur dieses Makromoleküls ebenso wie die an ihrer Synthese beteiligten Proteine sind innerhalb der Bakterien stark konserviert. Aufgrund ihrer essentiellen Natur und Einheitlichkeit ist die Zellwandbiosynthese ein idealer Angriffspunkt für neue Antibiotika (D.W.Green, The bacterial cell wall as a source of antibacterial targets, Expert Opin. Ther. Targets, 2002, 6, 1-19).

Vancomycin und Penicilline sind Inhibitoren der bakteriellen Zellwandbiosynthese und stellen erfolgreiche Beispiele für die antibiotische Potenz dieses Wirkprinzips dar. Sie werden seit mehreren Jahrzehnten in der Klinik zur Behandlung von bakteriellen Infektionen, vor allem mit Gram-positiven Erregern eingesetzt. Durch das wachsende Auftreten von resistenten Keimen, z.B. Methicillin-resistenten Staphylokokken, Penicillin-resistenten Pneumokokken und Vancomycin-resistenten Enterokokken (F. Baquero, Gram-positive resistance: challenge for the development of new antibiotics, J. Antimicrob. Chemother., 1997, 39, Suppl A:1-6; A.P. Johnson, D.M. Livermore, G.S. Tillotson, Antimicrobial susceptibility of Gram-positive bacteria: what's current, what's anticipated ?, J. Hosp. Infect., 2001, (49), Suppl A: 3-11) sowie jüngst auch erstmals Vancomycin-resistenten Staphylokokken (B. Goldrick, First reported case of VRSA in the United States, Am. J. Nurs., 2002, 102, 17) verlieren diese Substanzen zunehmend ihre therapeutische Wirksamkeit.

Die vorliegende Erfindung beschreibt eine neue Klasse von Zellwandbiosynthese-Inhibitoren ohne Kreuzresistenzen zu bekannten Antibiotika-Klassen.

Der Naturstoff Lysobactin und einige Derivate sind als antibakteriell wirksam beschrieben in US 4,754,018. Die Isolierung und antibakterielle Wirksamkeit von Lysobactin ist auch in EP-A-196 042 und JP 01132600 beschrieben.

Die antibakterielle Wirkung von Lysobactin und Katanosin A wird weiterhin beschrieben in O'Sullivan, J. et al., J. Antibiot. 1988, 41, 1740 - 1744, Bonner, D. P. et al., J. Antibiot. 1988, 41, 1745 - 1751, Shoji, J. et al., J. Antibiot. 1988, 41, 713 - 718 und Tymiak, A. A. et al., J. Org. Chem. 1989, 54, 1149 - 1157.

Eine Aufgabe der vorliegenden Erfindung ist es, alternative Verbindungen mit vergleichbarer oder verbesserter antibakterieller Wirkung und besserer Verträglichkeit, z. B. geringerer Nephrotoxizität, zur Behandlung von bakteriellen Erkrankungen bei Menschen und Tieren zur Verfügung zu stellen.

Gegenstand der Erfindung sind Verbindungen der Formel 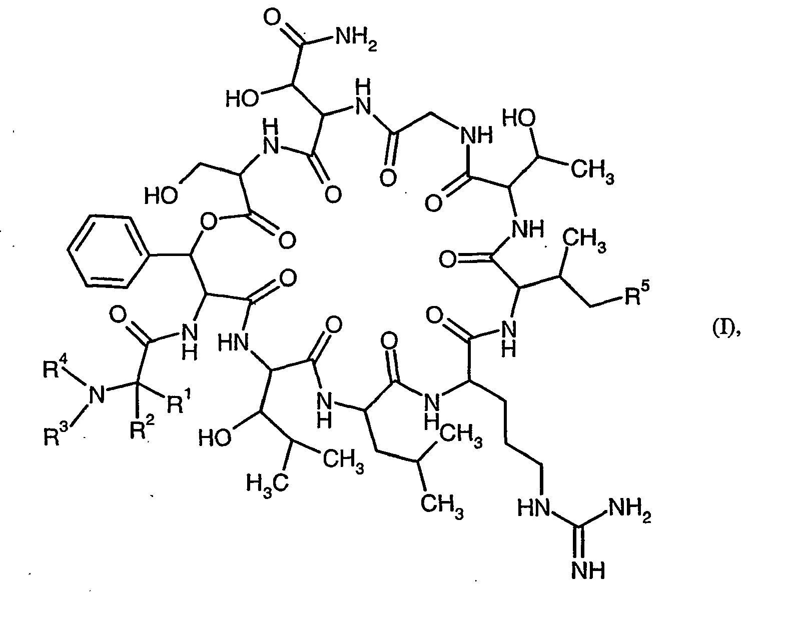 in welcher
- R¹: Wasserstoff, C₃-C₆-Cycloalkyl, C₅-C₆-Cycloalkenyl, C₃-C₆-Cycloalkylinethyl, 5- bis 7-gliedriges Heterocyclylmethyl, Methyl, Ethyl, n-Propyl, iso-Propyl, 1-Methylprop-1-yl, 2-Methylprop-1-yl, 2,2-Dimethylprop-1-yl, 1,1-Dimethylprop-1-yl, 1-Ethyl-prop-1-yl, 1-Ethyl-1-methylprop-1-yl, n-Butyl, 2-Methylbut-1-yl, 3-Methylbut-1-yl, 1-Ethylbut-1-yl, *tert*-Butyl, 4-Methylpent-1-yl, n-Hexyl, Alkenyl oder Aryl bedeutet,
wobei R¹ substituiert sein kann mit 0, 1, 2 oder 3 Substituenten unabhängig voneinander ausgewählt aus der Gruppe bestehend aus Halogen, Hydroxy, Amino, Cyano, Trimethylsilyl, Alkyl, Alkoxy, Benzyloxy, C₃-C₆-Cycloalkyl, Aryl, 5- bis 10-gliedriges Heteroaryl, Alkylamino, Arylamino, Alkylcarbonylamino, Arylcarbonylamino, Alkylcarbonyl, Alkoxycarbonyl, Arylcarbonyl und Benzyloxycarbonylamino,
worin Aryl und Heteroaryl ihrerseits substituiert sein können mit 0, 1, 2 oder 3 Substituenten unabhängig voneinander ausgewählt aus der Gruppe bestehend aus Halogen, Hydroxy, Amino, Cyano, Nitro, Alkyl, Alkoxy und Phenyl,
- R²: Wasserstoff oder C₁-C₄-Alkyl bedeutet,
- oder: und
- R¹ R²: zusammen mit dem Kohlenstoffatom, an das sie gebunden sind, einen C₃-C₆-Cycloalkyl-Ring oder einen 5- bis 7-gliedrigen Heterocyclyl-Ring bilden, wobei der Cycloalkyl-Ring und der Heterocyclyl-Ring substituiert sein können mit 0, 1, 2 oder 3 Substituenten un-abhängig voneinander ausgewählt aus der Gruppe bestehend aus Trifluormethyl, Alkyl, Alkoxy, und Alkylcarbonyl,
- R³: Alkyl, C₃-C₆-Cycloalkyl, 5- bis 7-gliedriges Heterocyclyl, Aryl, 5- oder 6-gliedriges Heteroaryl, Alkylcarbonyl, Alkoxycarbonyl, C₃-C₆-Cycloalkylcarbonyl, 5- bis 7-gliedriges Heterocyclylcarbonyl, Arylcarbonyl, 5- oder 6-gliedriges Heteroarylcarbonyl oder Alkylaminocarbonyl bedeutet,
wobei Alkyl, Cycloalkyl, Heterocyclyl, Aryl, Heteroaryl, Alkoxycarbonyl, Cyclo-alkylcarbonyl, Heterocyclylcarbonyl, Arylcarbonyl, Heteroarylcarbonyl und Alkylaminocarbonyl substituiert sein können mit 0, 1, 2 oder 3 Substituenten unabhängig voneinander ausgewählt aus der Gruppe bestehend aus Halogen, Hydroxy, Amino, Alkyl-amino und Phenyl,
und
wobei Alkylcarbonyl substituiert ist mit einem Substituenten Amino oder Alkylamino,
und
wobei Alkylcarbonyl substituiert sein kann mit weiteren 0, 1 oder 2 Substituenten unabhängig voneinander ausgewählt aus der Gruppe bestehend aus Halogen, Hydroxy, Trimethylsilyl, Alkoxy, Alkylthio, Benzyloxy, C₃-C₆-Cycloalkyl, Phenyl, Naphthyl, 5- bis 10-gliedriges Heteroaryl, Alkylcarbonylamino, Alkoxycarbonylamino, Arylcarbonylamino, Arylcarbonyloxy, Benzyloxycarbonyl und Benzyloxycarbonylamino,
worin Phenyl und Heteroaryl ihrerseits substituiert sein können mit 0, 1, 2 oder 3 Substituenten unabhängig voneinander ausgewählt aus der Gruppe bestehend aus Halogen, Hydroxy, Nitro, Alkyl, Alkoxy und Phenyl,
oder zwei Substituenten an demselben Kohlenstoffatom im Alkylcarbonyl zusammen mit dem Kohlenstoffatom, an das sie gebunden sind, einen C₃-C₆-Cyloakyl-Ring oder einen 5-bis 7-gliedrigen Heterocyclyl-Ring bilden,
wobei der Cycloalkyl-Ring und der Heterocyclyl-Ring substituiert sein können mit 0, 1, 2 oder 3 Substituenten unabhängig voneinander ausgewählt aus der Gruppe bestehend aus Trifluormethyl, Alkyl und Alkoxy,
oder
wobei der Cycloalkyl-Ring Benzo-anneliert sein kann,
- R⁴: Wasserstoff, C₁-C₄-Alkyl, Cyclopropyl oder Cyclopropylmethyl bedeutet,
- oder: und
- R³ R⁴: zusammen mit dem Stickstoffatom, an das sie gebunden sind, einen 5- bis 7-gliedrigen Heterocyclyl-Ring bilden, wobei der Heterocyclyl-Ring substituiert sein kann mit 0, 1, 2 oder 3 Substituenten unabhängig voneinander ausgewählt aus der Gruppe bestehend aus Halogen, Hydroxy, Amino, Cyano, Alkyl, Alkoxy und Alkylamino,
und
- R⁵: Wasserstoff oder Methyl bedeutet,
und ihre Salze, ihre Solvate und die Solvate ihrer Salze,
mit der Maßgabe, dass für den Fall, dass R¹ Wasserstoff, R² 2-Methylprop-1-yl, R⁴ Wasserstoff und R⁵ Methyl bedeutet und das Kohlenstoffatom, an das R¹ und R² gebunden sind, (S)-Konfiguration hat oder dass R¹ 2-Methylprop-1-yl, R² Wasserstoff, R⁴ Wasserstoff und R⁵ Methyl bedeutet und das Kohlenstoffatom, an das R¹ und R² gebunden sind, (*S*)-Konfiguration hat, R³ nicht Glycyl, D-Alanyl, L-Alanyl oder D-Leucyl bedeutet, und
mit der Maßgabe, dass für den Fall, dass R¹ Wasserstoff, R² 2-Methylprop-1-yl, R⁴ Wasserstoff und R⁵ Wasserstoff bedeutet und das Kohlenstoffatom, an das R¹ und R² gebunden sind, (S)-Konfiguration hat oder dass R¹ 2-Methylprop-1-yl, R² Wasserstoff, R⁴ Wasserstoff und R⁵ Wasserstoff bedeutet und das Kohlenstoffatom, an das R¹ und R² gebunden sind, (S)-Konfiguration hat, R³ nicht D-Leucyl bedeutet.

Erfindungsgemäße Verbindungen sind die Verbindungen der Formel (I), (Ia), (Ib) und (Ic) und deren Salze, Solvate, Solvate der Salze und Prodrugs, die von Formel (I), (Ia), (Ib) und (Ic) umfassten Verbindungen der nachfolgend genannten Formeln und deren Salze, Solvate, Solvate der Salze und Prodrugs sowie die von Formel (I), (Ia), (Ib) und (Ic) umfassten, nachfolgend als Ausführungsbeispiele genannten Verbindungen und deren Salze, Solvate, Solvate der Salze und Prodrugs, soweit es sich bei den von Formel (I), (Ia), (Ib) und (Ic) umfassten, nachfolgend genannten Verbindungen nicht bereits um Salze, Solvate, Solvate der Salze und Prodrugs handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegenden Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:

Alkyl per se und "Alk" und "Alkyl" in Alkoxy Alkylamino, Alkylcarbonyl, Alkoxycarbonyl, Alkylaminocarbonyl, Alkylcarbonylamino und Alkoxycarbonylamino steht für einen linearen oder verzweigten Alkylrest mit in der Regel 1 bis 6, vorzugsweise 1 bis 4, besonders bevorzugt 1 bis 3 Kohlenstoffatomen, beispielhaft und vorzugsweise für Methyl, Ethyl, n-Propyl, Isopropyl, *tert*-Butyl, 2,2-Dimethylprop-1-yl, n-Pentyl und n-Hexyl.

Alkoxy steht beispielhaft und vorzugsweise für Methoxy, Ethoxy, n-Propoxy, Isopropoxy, *tert-*Butoxy, n-Pentoxy und n-Hexoxy.

Alkenyl steht für einen geradkettigen oder verzweigten Alkenylrest mit 2 bis 6 Kohlenstoffatomen. Bevorzugt ist ein geradkettiger oder verzweigter Alkenylrest mit 2 bis 4, besonders bevorzugt mit 2 bis 3 Kohlenstoffatomen. Beispielsweise und vorzugsweise seien genannt: Vinyl, Allyl, n-Prop-1-en-1-yl, n-But-2-en-1-yl, 2-Methylprop-1-en-1-yl und 2-Methylprop-2-en-1-yl.

Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, *tert*-Butylamino, n-Pentylamino, n-Hexylamino, *N,N*-Dimethylamino, *N,N-*Diethylamino, *N*-Ethyl-*N*-methylamino, *N*-Methyl-*N*-n-propylamino, *N*-Isopropyl-*N*-n-propylamino, *N*-*tert*-Butyl-*N*-methylamino, *N*-Ethyl-*N*-n-pentylamino und *N*-n-Hexyl-*N-*methylamino. C₁-C₃-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.

Arylamino steht für einen über eine Aminogruppe gebundenen Arylsubstituenten, wobei an die Aminogruppe gegebenenfalls ein weiterer Substituenten, wie z.B. Aryl oder Alkyl, gebunden ist, beispielhaft und vorzugsweise für Phenylamino, Naphthylamin, Phenylmethylamino öder Diphenylamino.

Alkylcarbonyl steht beispielhaft und vorzugsweise für Methylcarbonyl, Ethylcarbonyl, n-Propylcarbonyl, Isopropylcarbonyl, *tert*-Butylcarbonyl, n-Pentylcarbonyl und n-Hexylcarbonyl.

Alkoxycarbonyl steht beispielhaft und vorzugsweise für Methoxycarbonyl, Ethoxycarbonyl, n-Propoxycarbonyl, Isopropoxycarbonyl, *tert*-Butoxycarbonyl, n-Pentoxycarbonyl und n-Hexoxycarbonyl.

Alkoxycarbonylamino steht beispielhaft und vorzugsweise für Methoxycarbonylamino, Ethoxycarbonylamino, n-Propoxy-carbonylamino, Isopropoxycarbonylamino, *tert*-Butoxycarbonylamino, n-Pentoxycarbonylamino und n-Hexoxycarbonylamino.

Cycloalkylcarbonyl steht für einen über eine Carbonylgruppe gebundenen Cycloalkylsubstituenten, beispielhaft und vorzugsweise für Cyclopropylcarbonyl, Cyclobutylcarbonyl, Cyclopentylcarbonyl und Cyclohexylcarbonyl.

Heterocyclylcarbonyl steht für einen über eine Carbonylgruppe gebundenen Heterocyclylsubstituenten, beispielhaft und vorzugsweise für Tetrahydrofuran-2-ylcarbonyl, Pyrrolidin-2-ylcarbonyl, Pyrrolidin-3-ylcarbonyl, Pyrrolinylcarbonyl, Piperidinylcarbonyl, Morpholinylcarbonyl und Perhydroazepinylcarbonyl.

Arylcarbonyl steht für einen über eine Carbonylgruppe gebundenen Arylsubstituenten, beispielhaft und vorzugsweise für Phenylcarbonyl, Naphthylcarbonyl und Phenanthrenylcarbonyl.

Heteroarylcarbonyl steht für einen über eine Carbonylgruppe gebundenen Heteroarylsubstituenten, beispielhaft und vorzugsweise für Thienylcarbonyl, Furylcarbonyl, Pyrrolylcarbonyl, Thiazolylcarbonyl, Oxazolylcarbonyl, Imidazolylcarbonyl, Pyridylcarbonyl, Pyrimidylcarbonyl, Pyridazinylcarbonyl, Indolylcarbonyl, Indazolylcarbonyl, Benzofuranylcarbonyl, Benzothiophenylcarbonyl, Chinolinylcarbonyl und Isochinolinylcarbonyl.

Alkylcarbonylamino steht beispielhaft und vorzugsweise für Methylcarbonylamino, Ethylcarbonylamino, n-Propylcarbonylamino, Isopropylcarbonylamino, *tert*-Butylcarbonylamino, n-Pentylcarbonylamino und n-Hexylcarbonylamino.

Arylcarbonylamino steht beispielhaft und vorzugsweise für Phenylcarbonylamino, Naphthylcarbonylamino und Phenanthrenyl-carbonylamino.

Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, Isopropylaminocarbonyl, *tert-*Butylaminocarbonyl, n-Pentylaminocarbonyl, n-Hexylaminocarbonyl, *N,N*-Dimethylaminocarbonyl, *N,N*-Diethylaminocarbonyl, *N*-Ethyl-*N*-methylaminocarbonyl, *N*-Methyl-*N*-n-propylaminocarbonyl, *N-*Isopropyl-*N-*n-propylaminocarbonyl, *N*-*tert*-Butyl-*N*-methylaminocarbonyl, *N*-Ethyl-*N*-n-pentylamino-carbonyl und *N*-n-Hexyl-*N*-methylaminocarbonyl. C₁-C₃-Alkylaminocarbonyl steht beispielsweise für einen Monoalkylaminocarbonylrest mit 1 bis 3 Kohlenstoffatomen oder für einen Dialkylaminocarbonylrest mit jeweils 1 bis 3 Kohlenstoffatomen pro Alkylsubstituent.

Cycloalkyl steht für eine Cycloalkylgruppe mit in der Regel 3 bis 6 Kohlenstoffatomen, beispielhaft und vorzugsweise für Cyclopropyl, Cyclobutyl, Cyclopentyl und Cyclohexyl.

Cycloalkenyl steht für eine Cycloalkenylgruppe mit in der Regel 5 bis 6 Kohlenstoffatomen und einer oder zwei Doppelbindungen, beispielhaft und vorzugsweise für Cyclopent-1-en-1-yl, Cyclopent-2-en-1-yl, Cyclopent-3-en-1-yl, Cyclohex-1-en-1-yl, Cyclohex-2-en-1-yl und Cyclohex-3-en-1-yl.

Aryl steht für einen mono- bis tricyclischen aromatischen, carbocyclischen Rest mit in der Regel 6 bis 14 Kohlenstoffatomen; beispielhaft und vorzugsweise für Phenyl, Naphthyl und Phenanthrenyl.

Heterocyclyl steht für einen mono- oder polycyclischen, vorzugsweise mono- oder bicyclischen, heterocyclischen Rest mit in der Regel 5 bis 7 Ringatomen und bis zu 3, vorzugsweise bis zu 2 Heteroatomen und/oder Heterogruppen aus der Reihe N, O, S, SO, SO₂. Die Heterocyclyl-Reste können gesättigt oder teilweise ungesättigt sein. Bevorzugt sind 5- bis 7-gliedrige, monocyclische gesättigte Heterocyclylreste mit bis zu zwei Heteroatomen aus der Reihe O, N und S, wie beispielhaft und vorzugsweise Tetrahydrofuran-2-yl, Pyrrolidin-2-yl, Pyrrolidin-3-yl, Pyrrolinyl, Piperidinyl, Morpholinyl und Perhydroazepinyl.

Heteroaryl steht für einen aromatischen, mono- oder bicyclischen Rest mit in der Regel 5 bis 10, vorzugsweise 5 bis 6 Ringatomen und bis zu 5, vorzugsweise bis zu 4 Heteroatomen aus der Reihe S, O und N, beispielhaft und vorzugsweise für Thienyl, Furyl, Pyrrolyl, Thiazolyl, Oxazolyl, Imidazolyl, Pyridyl, Pyrimidyl, Pyridazinyl, Indolyl, Indazolyl, Benzofuranyl, Benzothiophenyl, Chinolinyl und Isochinolinyl.

Carbonylgebundene Aminosäure steht für eine Aminosäure, der über die Carbonylgruppe der Aminosäure-Säurefunktion gebunden ist. Bevorzugt sind dabei α-Aminosäuren in der L- oder in der D-Konfiguration, insbesondere natürlich vorkommende α-Aminosäuren, wie z.B. Glycin, L-Alanin, L-Valin, L-Leucin, L-Isoleucin, L-Prolin, L-Phenylalanin, L-Tryptophan oder natürlich vorkommende α-Aminosäuren in der unnatürlichen D-Konfiguration, wie z.B. D-Alanin, D-Valin, D-Leucin, D-Isoleucin, D-Prolin, D-Phenylalanin, D-Tryptophan oder unnatürliche Aminosäuren, mit einer an das α-Kohlenstoffatom der Aminosäure gebundenen Seitengruppe, wie z.B. C₃-C₆-Cycloalkylmethyl, C₃-C₆-Cycloalkyl, Ethyl, n-Propyl, 2,2-Dimethylpropyl, *tert*-Butyl, 3-Methylbutyl, n-Hexyl oder Allyl, oder die Seitenkette bildet mit dem α-Kohlenstoffatom der Aminosäure einen Ring, wie z.B. Cyclopropyl (Aminosäure: 1-Amino-1-cyclopropancarbonsäure), Cyclobutyl, Cyclopentyl, Cyclohexyl oder einen 5- bis 7-gliedrigen Heterocyclus, wobei der Ring Benzo-anneliert sein kann, oder β-Aminosäuren (zur Nomenklatur vgl.: D. Seebach, M. Overhand, F. N. M. Kühnle, B. Martinoni, L. Oberer, U. Hommel, H. Widmer, Helv. Claim. Acta 1996, 79, 913-941), wie z.B. β-Alanin, β-Phenylalanin, β-Aib oder Derivate der 2,3-Diaminopropionsäure (z.B. 2,3-Diamino-3-phenylpropionsäure).

Halogen steht für Fluor, Chlor, Brom und Jod, bevorzugt Fluor und Chlor.

### Beschreibung der Abbildungen

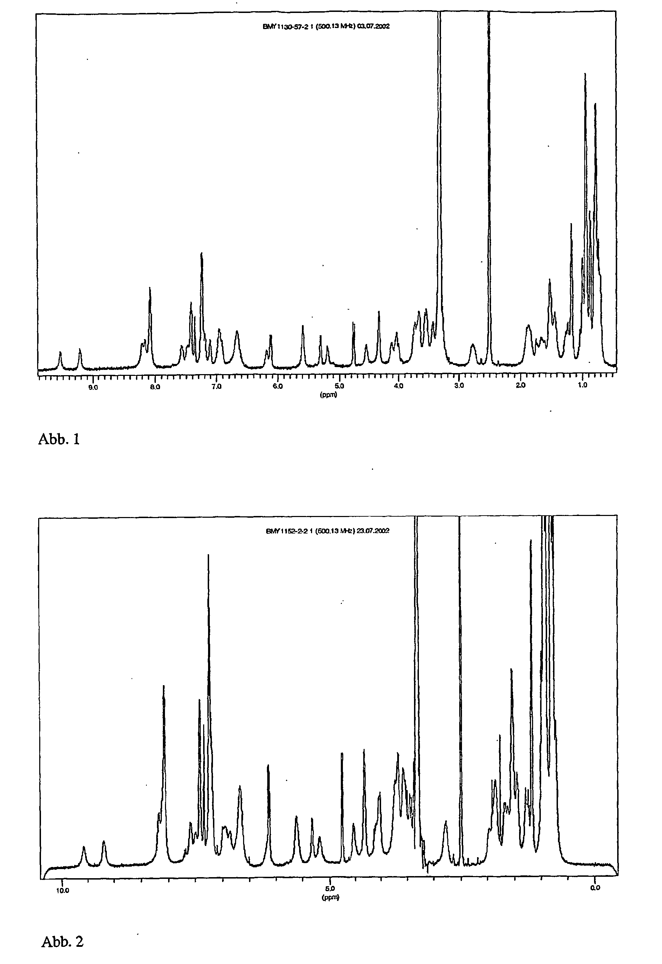

Abb.1: ¹H NMR (500 MHz, d₆-DMSO, 302 K) von Beispiel 1A
Abb.2: ¹H NMR (500 MHz, d₆-DMSO, 302 K) von Beispiel 2A

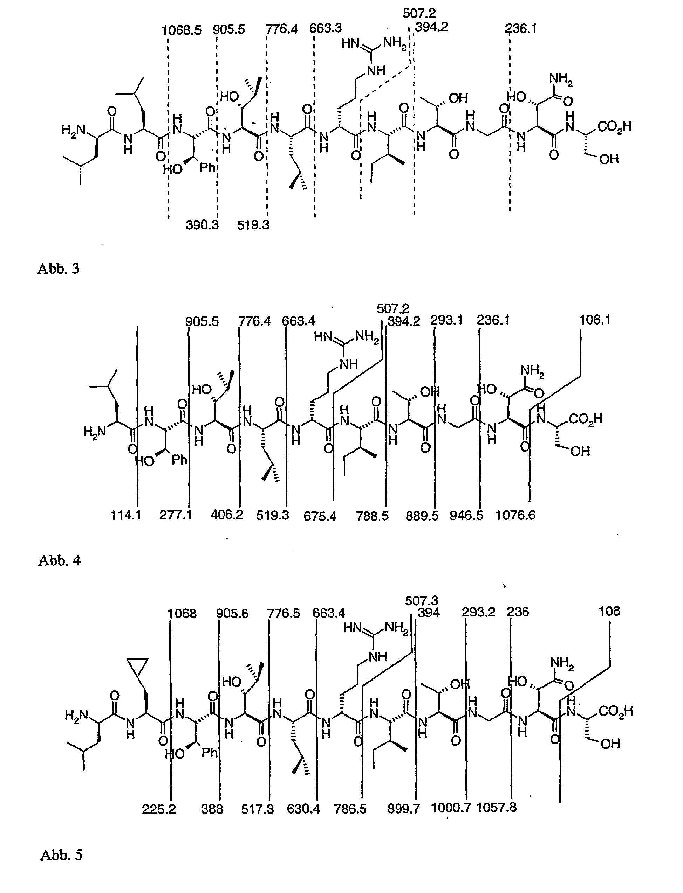

Abb.3: MALDI-MS Sequenzierung von hydrolytisch Ring-geöffnetem Lysobactin.
Abb.4: MALDI-MS Sequenzierung von hydrolytisch Ring-geöffnetem Deca-depsipeptid (Beispiel 11A).
Abb. 5: MALDI-MS Sequenzierung von hydrolytisch Ring-geöffnetem Undecadepsipeptid (Beispiel 1).

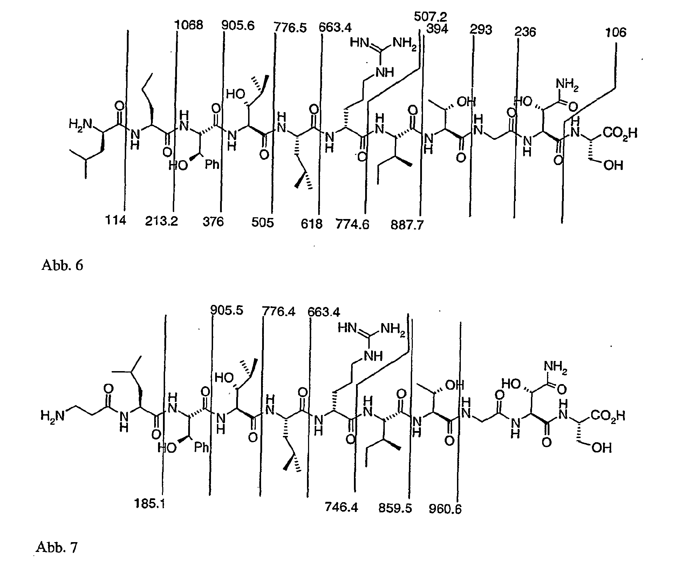

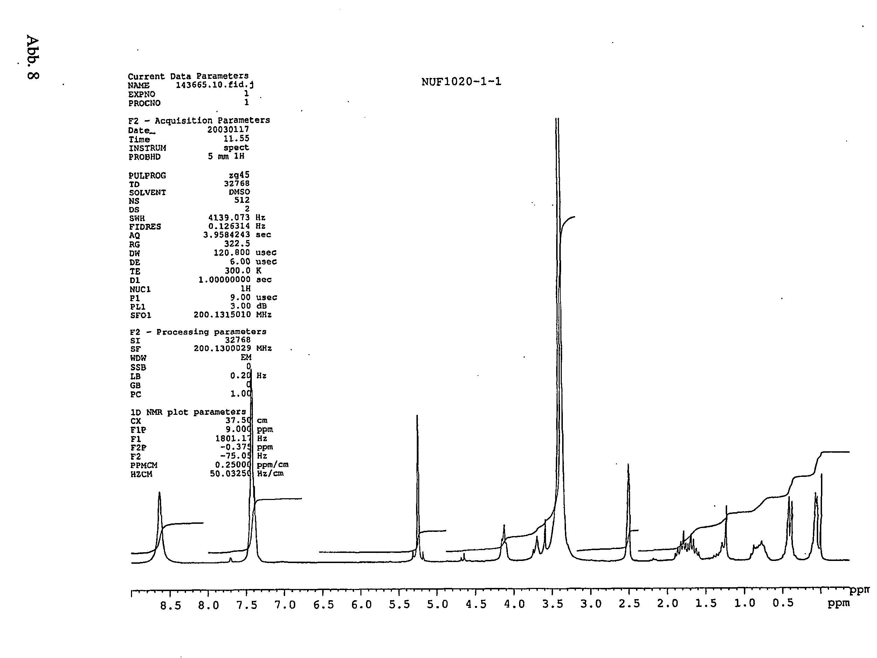

Abb.6: MALDI-MS Sequenzierung von hydrolytisch Ring-geöffnetem Undecadepsipeptid (Beispiel 2).
Abb.7: MALDI-MS Sequenzierung von hydrolytisch Ring-geöffnetem Undecadepsipeptid (Beispiel 8).
Ab.8: ¹H NMR (200 MHz, *d₆*-DMSO) von Benzyl-3-cyclopropyl-L-alaninat-hydrochlorid (Beispiel 3A).

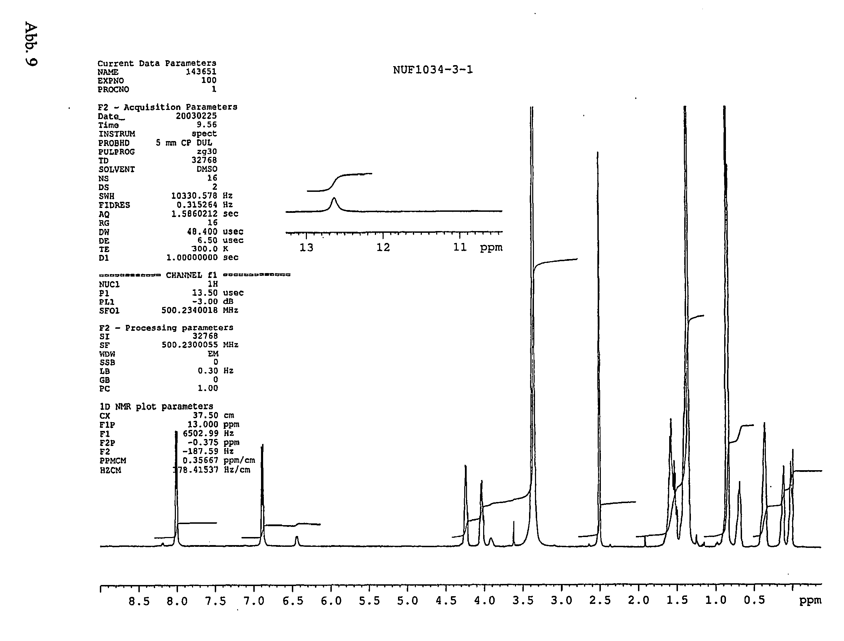

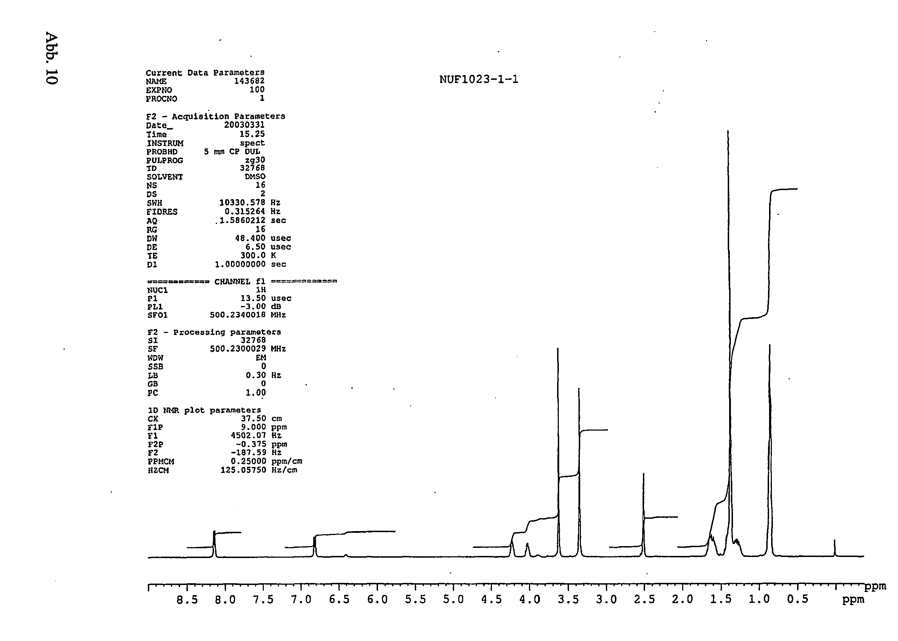

Abb.9: ¹H NMR (500 MHz, *d₆*-DMSO) von *N*-(*tert*-butoxycarbonyl)-D-leucyl-3-cyclopropyl-L-alanin (Beispiel 5A). Abb. 10: ¹H NMR (500 MHz, *d₆*-DMSO) von Methyl-*N*-(*tert-*butoxycarbonyl)-D-leucyl-L-norvalinat (Beispiel 6A).

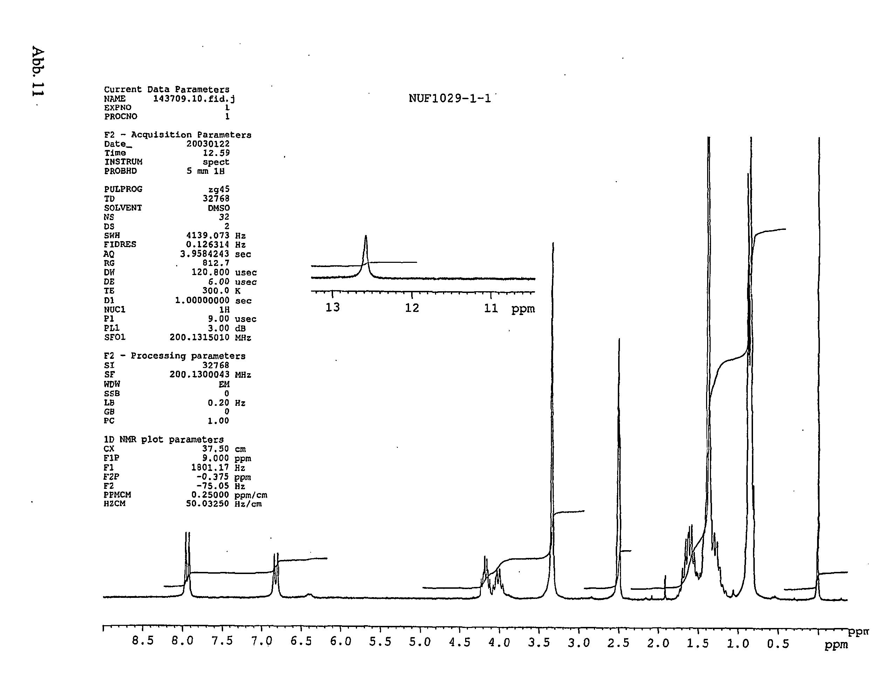

Abb.11: ¹H NMR (200 MHz, *d₆*-DMSO) von *N*-(*tert*-Butoxycarbonyl)-D-leucyl-L-norvalin (Beispiel 7A).

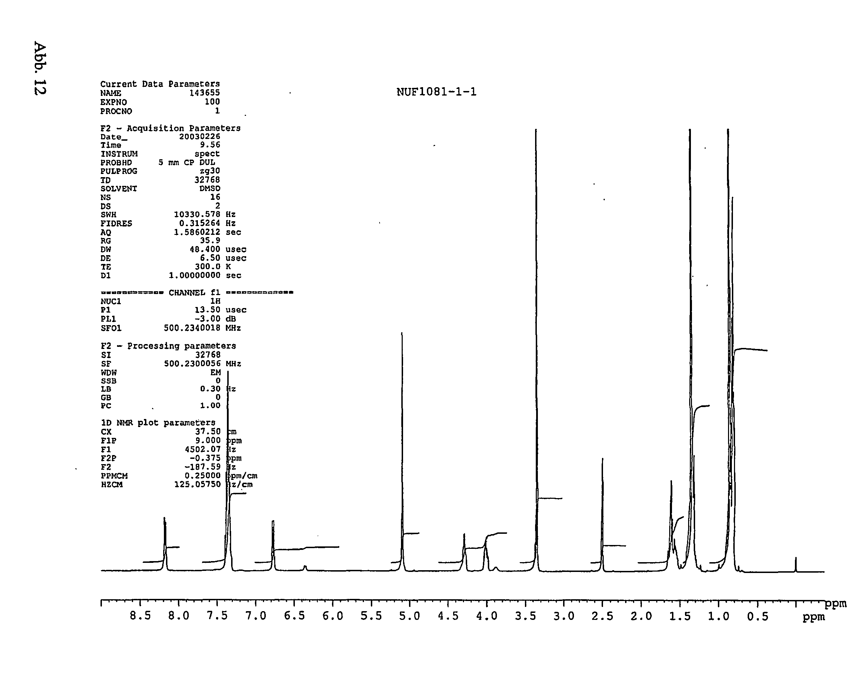

Abb.12: ¹H NMR (500 MHz, *d₆*-DMSO) von Benzyl-*N*-(*tert*-butoxycarbonyl)-D-leucyl-3-*tert-*butyl-L-alaninat (Beispiel 8A).

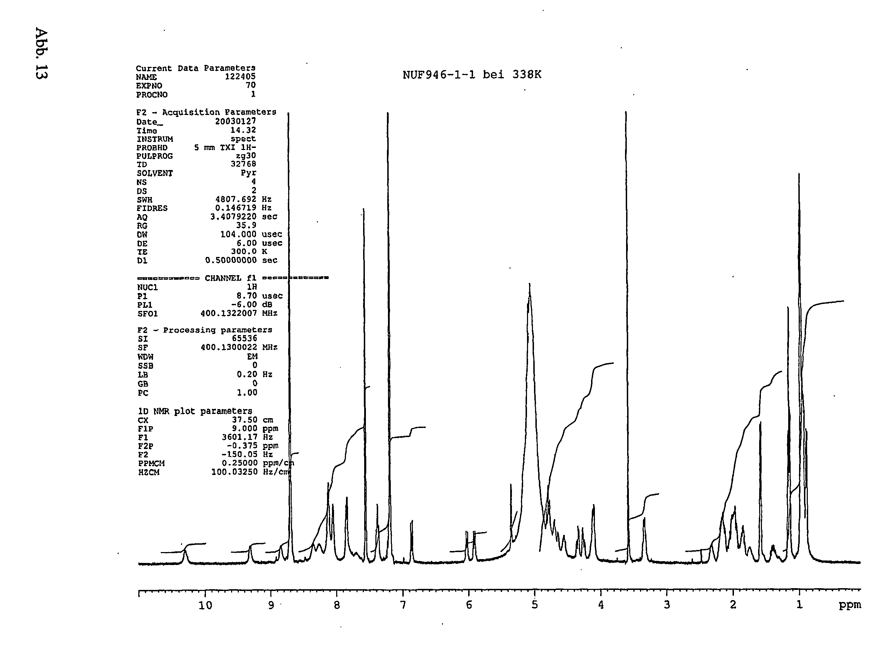

Abb.13: ¹*H* NMR (400 MHz, 338 K, *d₅*-Pyridin) von Desleucyllysobactin-bistrifluoracetat (Beispiel 11A).

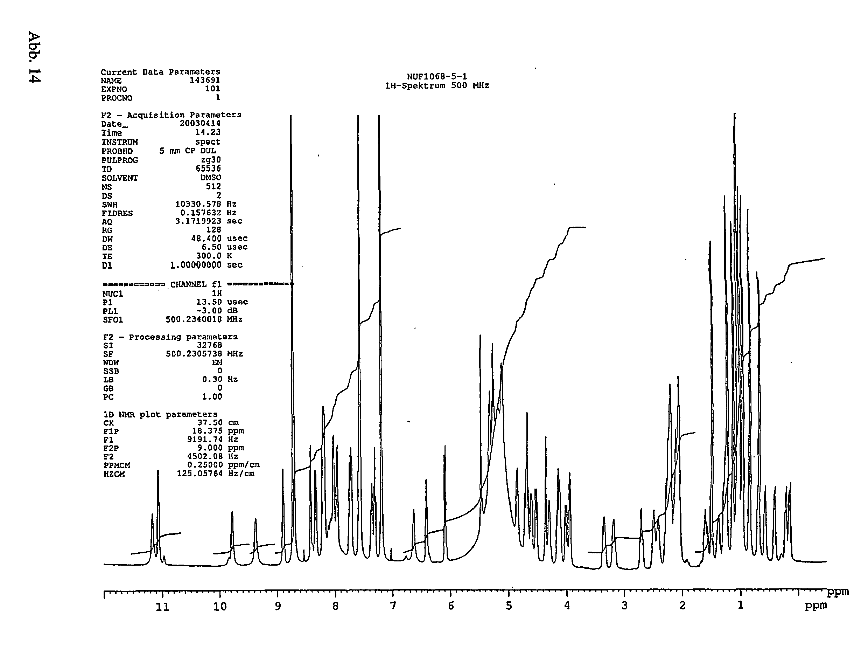

Abb. 14: ¹H NMR (500 MHz, *d₅*-Pyridin) von D-Leucyl-*N*¹-{(3*S*,6*S*,12*S*,15*S*,18*R*,21*S*,24*S*,27*S*,-28*R*)-6-[(1*S*)-2-amino-1-hydroxy-2-oxoethyl]-18-(3-{[amino(imino)methyl]amino}propyl)-12-[(1*S*)-1-hydroxyethyl]-3-(hydroxymethyl)-24-[(1*R*)-1-hydroxy-2-methylpropyl]-21-isobutyl-15-[(1*S*)-1-methylpropyl]-2,5,8,11,14,17,20,23,26-nonaoxo-28-phenyl-1-oxa-4,7,10,13,16,19,22,25-octaazacyclooctacosan-27-yl}-3-cylopropyl-L-alaninamid-bistrifluoracetat (Beispiel 1).

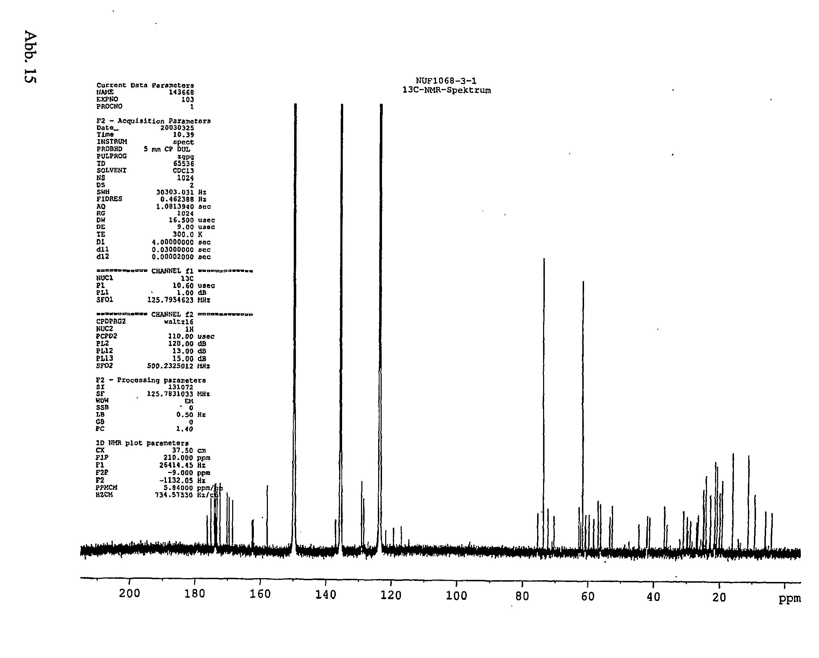

Abb.15: ¹³C NMR (126MHz, *d₅*-Pyridin) von D-Leucyl-*N*¹-{(3*S*,6*S*,12*S*,15*S*,18*R*,21*S*,24*S*,27*S*,-28*R*)-6-[(1*S*)-2-amino-1-hydroxy-2-oxoethyl]-18-(3-{[amino(imino)methyl]amino}propyl)-12-[(1*S*)-1-hydroxyethyl]-3-(hydroxymethyl)-24-[(1*R*)-1-hydroxy-2-methylpropyl]-21-isobutyl-15-[(1*S*)-1-methylpropyl]-2,5,8,11,14,17,20,23,26-nonaoxo-28-phenyl-1-oxa-4,7,10,13,16,19,22,25-octaazacyclooctacosan-27-yl}-3-cyclopropyl-L-alaninamid-bistrifluoracetat (Beispiel 1).

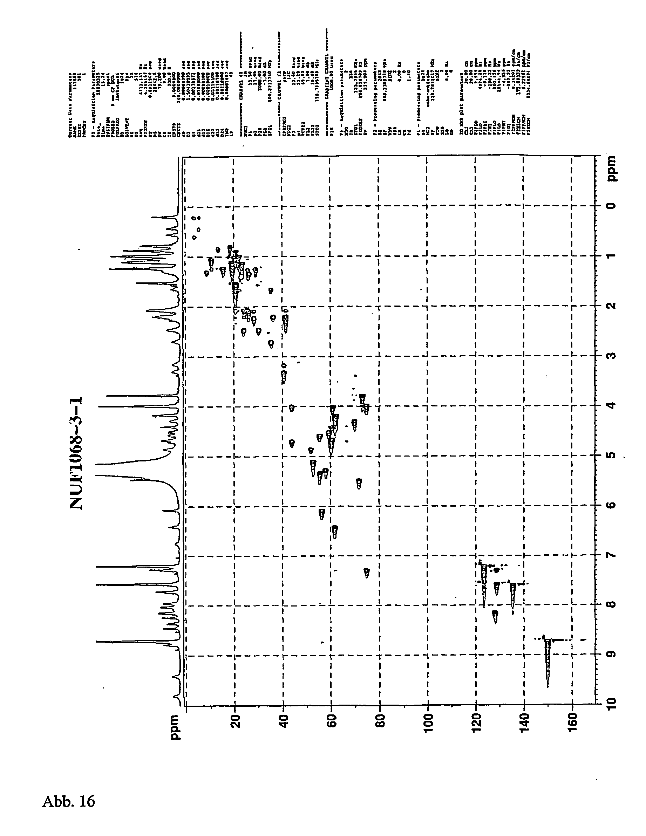

Abb.16: HSQC NMR (500 MHz, *d₅*-Pyridin) von D-Leucyl-*N*¹-{(3*S*,6*S*,12*S*,15*S*,18*R*,21*S*,24*S*,27*S*,-28*R*)-6-[(1*S*)-2-amino-1-hydroxy-2-oxoethyl]-18-(3-{[amino(imino)methyl]amino}propyl)-12-[(1*S*)-1-hydroxyethyl]-3-(hydroxymethyl)-24-[(1*R*)-1-hydroxy-2-methylpropyl]-21-isobutyl-15-[(1*S*)-1-methylpropyl]-2,5,8,11,14,17,20,23,26-nonaoxo-28-phenyl-1-oxa-4,7,10,13,16,19,22,25-octaazacyclooctacosan-27-yl}-3-cyclopropyl-L-alaninamid-bistrifluoracetat (Beispiel 1).

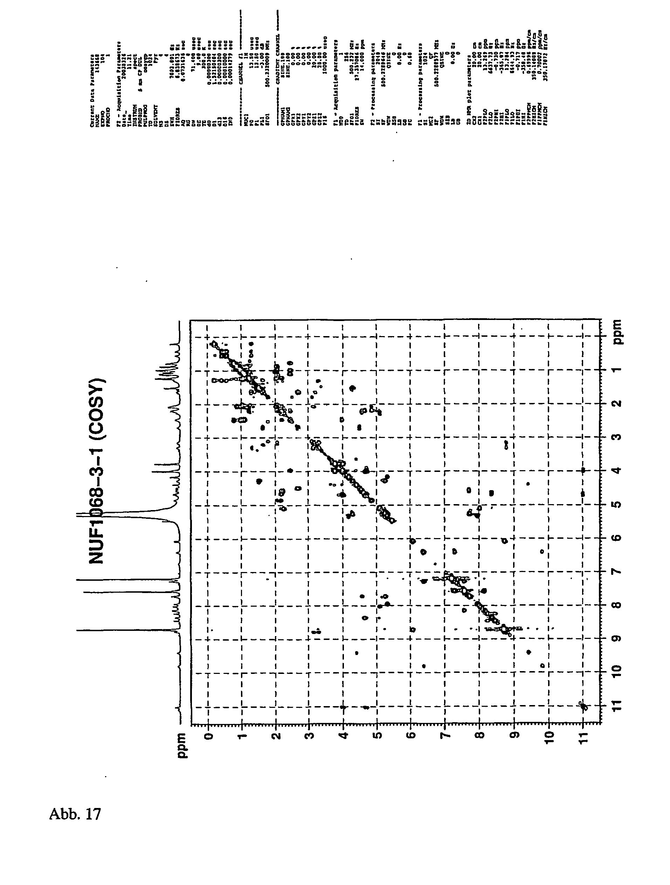

Abb.17:COSY NMR (500MHz, *d₅*-Pyridin) von D-Leucyl-*N*¹-{(3*S*,6*S*,12*S*,15*S*,18*R*,21*S*,24*S*,27*S*,-28*R*)-6-[(1*S*)-2-amino-1-hydroxy-2-oxoethyl]-18-(3-{[amino(imino)methyl]amino}propyl)-12-[(1*S*)-1-hydroxyethyl]-3-(hydroxymethyl)-24-[(1*R*)-1-hydroxy-2-methylpropyl]-21-isobutyl-15-[(1*S*)-1-methylpropyl]-2,5,8,11,14,17,20,23,26-nonaoxo-28-phenyl-1-oxa-4,7,10,13,16,19,22,25-octaazacyclooctacosan-27-yl}-3-cyclopropyl-L-alaninamid-bistrifluoracetat (Beispiel 1).

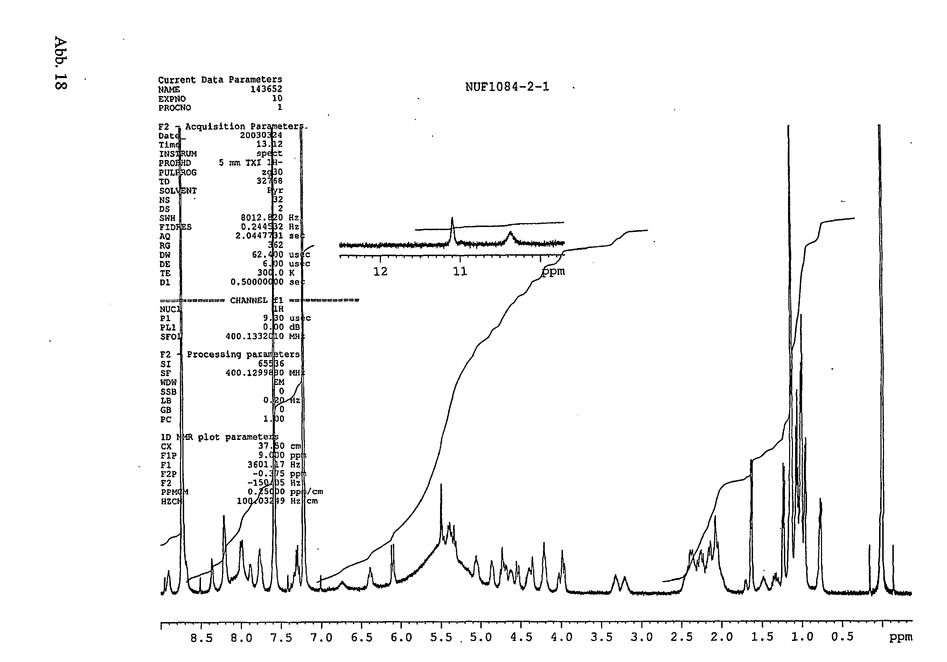

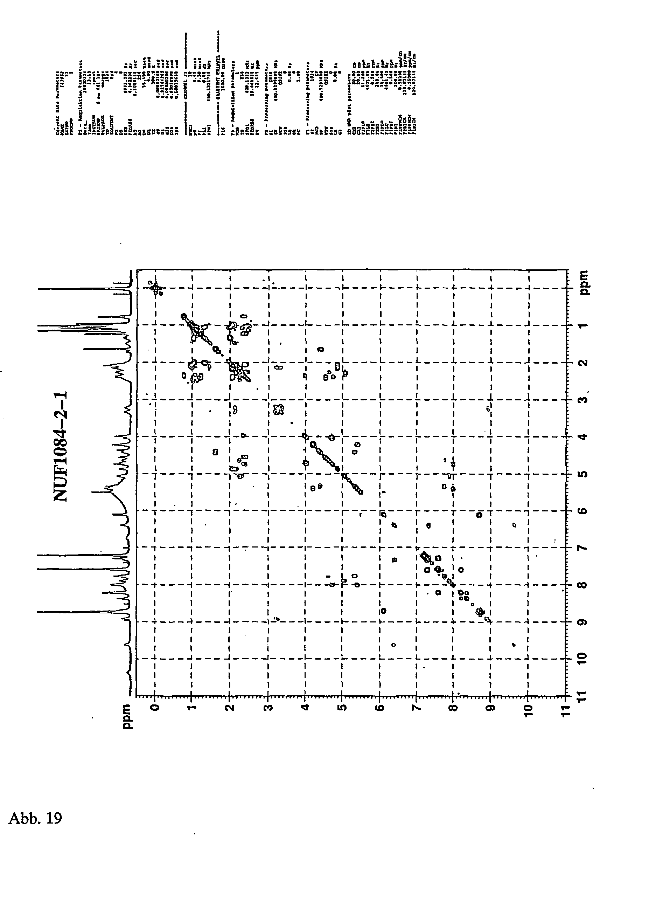

Abb. 18: ¹H NMR (400 MHz, *d₅*-Pyridin) von Undecadepsipeptid-bistrifluoracetat (Beispiel 3).
Ab.19: ¹H,¹H-COSY (400 MHz, *d₅*-Pyridin) von Undecadepsipeptid-bistrifluoracetat (Beispiel 3).

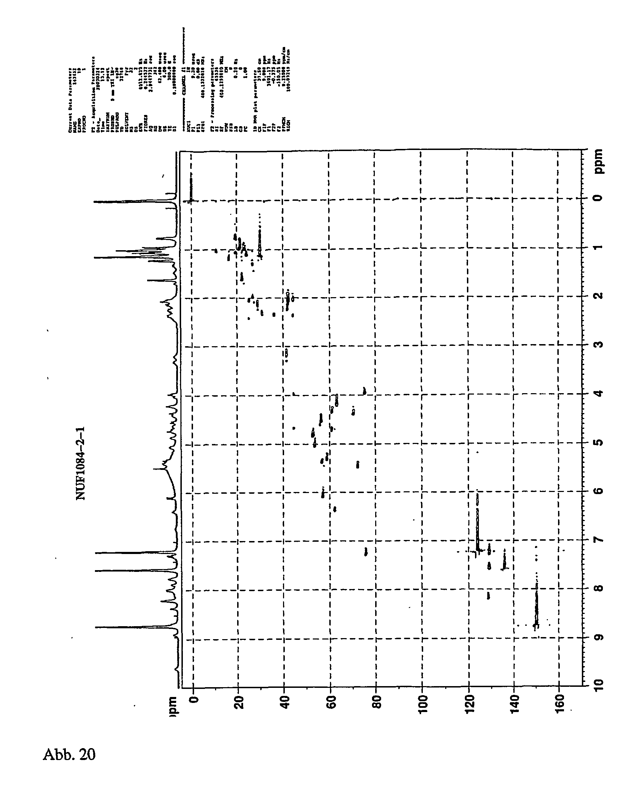

Abb.20: HSQC NMR (500 MHz, *d₅*-Pyridin) D-Leucyl-*N¹*-{(3*S*,6*S*,12*S*,15*S*,18*R*,21*S*,24*S*,27*S*,-28*R*)-6-[(1*S*)-2-amino-1-hydroxy-2-oxoethyl]-18-(3-{[amino(imino)methyl]amino}propyl)-12-[(1*S*)-1-hydroxyethyl]-3-(hydroxymethyl)-24-[(1*R*)-1-hydroxy-2-methylpropyl]-21-isobutyl-15-[(1*S*)-1-methylpropyl]-2,5,8,11,14,17,20,23,26-nonaoxo-28-phenyl-1-oxa-4,7,10,13,16,19,22,25-octaazacyclooctacosan-27-yl}-3-*tert*-butyl-L-alaninamid-bistrifluoracetat (Beispiel 3).

Gegenstand der Erfindung sind auch Verbindungen der Formel (I), welche der Formel 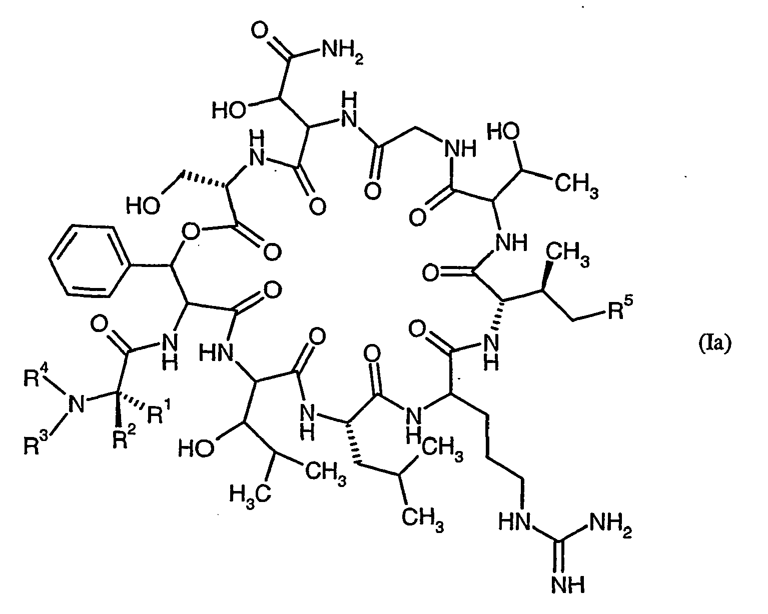 entsprechen, in welcher
R¹, R², R³, R⁴ und R⁵ die oben angegebene Bedeutung haben,
und ihre Salze, ihre Solvate und die Solvate ihrer Salze,
mit der Maßgabe, dass für den Fall, dass R¹ 2-Methylprop-1-yl, R² Wasserstoff, R⁴ Wasserstoff und R⁵ Methyl bedeutet, R³ nicht Glycyl, D-Alanyl, L-Alanyl oder D-Leucyl bedeutet, und
mit der Maßgabe, dass für den Fall, dass R¹ 2-Methylprop-1-yl, R² Wasserstoff, R⁴ Wasserstoff und R⁵ Wasserstoff bedeutet, R³ nicht D-Leucyl bedeutet.

Gegenstand der Erfindung sind auch Verbindungen der Formel (I), welche der Formel 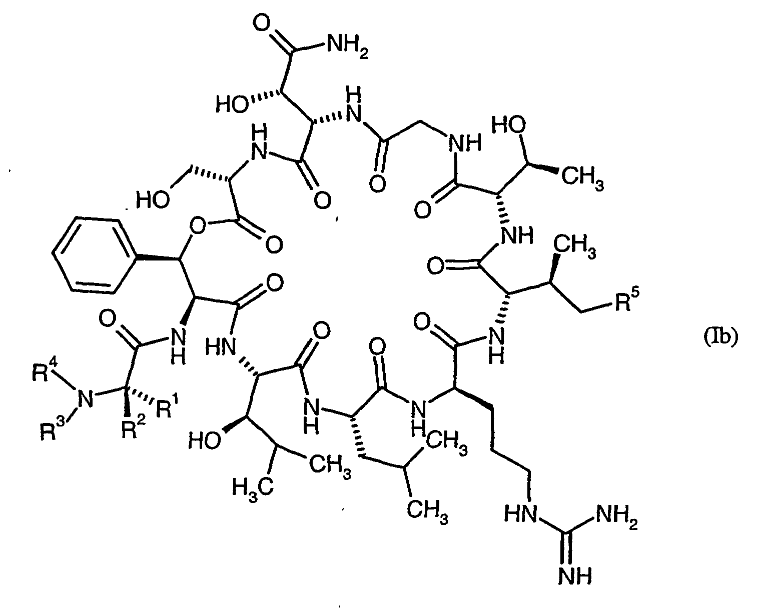 entsprechen, in welcher
R¹, R², R³, R⁴ und R⁵ die oben angegebene Bedeutung haben,
und ihre Salze, ihre Solvate und die Solvate ihrer Salze,
mit der Maßgabe, dass für den Fall, dass R¹ 2-Methylprop-1-yl, R² Wasserstoff, R⁴ Wasserstoff und R⁵ Methyl bedeutet, R³ nicht Glycyl, D-Alanyl, L-Alanyl oder D-Leucyl bedeutet, und
mit der Maßgabe, dass für den Fall, dass R¹ 2-Methylprop-1-yl, R² Wasserstoff, R⁴ Wasserstoff und R⁵ Wasserstoff bedeutet, R³ nicht D-Leucyl bedeutet.

Bevorzugt sind Verbindungen der Formel (Ia) und (Ib), in welchen
- R¹: 2-Methylprop-1-yl bedeutet,
- R²: Wasserstoff oder C₁-C₄-Alkyl bedeutet,
- R³: Alkyl, C₃-C₆-Cycloalkyl, 5- bis 7-gliedriges Heterocyclyl, Aryl, 5- oder 6-gliedriges Heteroaryl, Alkylcarbonyl, Alkoxycarbonyl, C₃-C₆-Cycloalkylcarbonyl, 5- bis 7-gliedriges Heterocyclylcarbonyl, Arylcarbonyl, 5- oder 6-gliedriges Heteroarylcarbonyl oder Alkylaminocarbonyl bedeutet,
wobei Alkyl, Cycloalkyl, Heterocyclyl, Aryl, Heteroaryl, Alkoxycarbonyl, Cyclo
alkylcarbonyl, Heterocyclylcarbonyl, Arylcarbonyl, Heteroarylcarbonyl und Alkylaminocarbonyl substituiert sein können mit 0, 1, 2 oder 3 Substituenten unabhängig voneinander ausgewählt aus der Gruppe bestehend aus Halogen, Hydroxy, Amino, Alkyl-amino und Phenyl,
und
wobei Alkylcarbonyl substituiert ist mit einem Substituenten Amino oder Alkylamino,
und
wobei Alkylcarbonyl substituiert sein kann mit weiteren 0, 1 oder 2 Substituenten unabhängig voneinander ausgewählt aus der Gruppe bestehend aus Halogen, Hydroxy, Trimethylsilyl, Alkoxy, Alkylthio, Benzyloxy, C₃-C₆-Cycloalkyl, Phenyl, Naphthyl, 5- bis 10-gliedriges Heteroaryl, Alkylcarbonylamino, Alkoxycarbonylamino, Arylcarbonylamino, Arylcarbonyloxy, Benzyloxycarbonyl und Benzyloxycarbonylamino,
worin Phenyl und Heteroaryl ihrerseits substituiert sein können mit 0, 1, 2 oder 3 Substituenten unabhängig voneinander ausgewählt aus der Gruppe bestehend aus Halogen, Hydroxy, Nitro, Alkyl, Alkoxy und Phenyl,
oder zwei Substituenten an demselben Kohlenstoffatom im Alkylcarbonyl zusammen mit dem Kohlenstoffatom, an das sie gebunden sind, einen C₃-C₆-Cycloalkyl-Ring oder einen 5-bis 7-gliedrigen Heterocyclyl-Ring bilden,
wobei der Cycloalkyl-Ring und der Heterocyclyl-Ring substituiert sein können mit 0, 1, 2 oder 3 Substituenten unabhängig voneinander ausgewählt aus der Gruppe bestehend aus Trifluormethyl, Alkyl und Alkoxy,
oder
wobei der Cycloalkyl-Ring Benzo-anneliert sein kann,
- R⁴: Wasserstoff, C₁-C₄-Alkyl, Cyclopropyl oder Cyclopropylmethyl bedeutet,
oder
- R³ und: R⁴ zusammen mit dem Stickstoffatom, an das sie gebunden sind, einen 5- bis 7-gliedrigen Heterocyclyl-Ring bilden, wobei der Heterocyclyl-Ring substituiert sein kann mit 0, 1, 2 oder 3 Substituenten unabhängig voneinander ausgewählt aus der Gruppe bestehend aus Halogen, Hydroxy, Amino, Cyano, Alkyl, Alkoxy und Alkylamino,
und
- R⁵: Wasserstoff oder Methyl bedeutet,
und ihre Salze, ihre Solvate und die Solvate ihrer Salze,
mit der Maßgabe, dass für den Fall, dass R² Wasserstoff, R⁴ Wasserstoff und R⁵ Methyl bedeutet, R³ nicht Glycyl, D-Alanyl, L-Alanyl oder D-Leucyl bedeutet,
und mit der Maßgabe, dass für den Fall, dass R² Wasserstoff, R⁴ Wasserstoff und R⁵ Wasserstoff bedeutet, R³ nicht D-Leucyl bedeutet.

Bevorzugt sind auch Verbindungen der Formel (Ia) und (Ib), in welchen
- R¹: 2-Methylprop-1-yl bedeutet,
- R²: Wasserstoff oder C₁-C₄-Alkyl bedeutet,
- R³: C₁-C₆-Alkylcarbonyl oder 5- bis 7-gliedriges Heterocyclylcarbonyl bedeutet,
wobei Alkylcarbonyl substituiert ist mit einem Substituenten Amino,
und
wobei Alkylcarbonyl substituiert sein kann mit weiteren 0, 1 oder 2 Substituenten unabhängig voneinander ausgewählt aus der Gruppe bestehend aus Halogen, Hydroxy, Trimethylsilyl, Alkoxy, Alkylthio, Benzyloxy, C₃-C₆-Cycloalkyl, Phenyl, Naphthyl, 5- bis 10-gliedriges Heteroaryl, Alkylcarbonylamino, Alkoxycarbonylamino, Arylcarbonylamino, Arylcarbonyloxy, Benzyloxycarbonyl und Benzyloxycarbonylamino,
worin Phenyl seinerseits substituiert sein kann mit 0, 1, 2 oder 3 Substituenten unabhängig voneinander ausgewählt aus der Gruppe bestehend aus Halogen, Hydroxy, Alkyl, Alkoxy und Phenyl,
oder zwei Substituenten an demselben Kohlenstoffatom im Alkylcarbonyl zusammen mit dem Kohlenstoffatom, an das sie gebunden sind, einen C₃-C₆-Cycloalkyl-Ring oder einen 5-bis 7-gliedrigen Heterocyclyl-Ring bilden,
wobei der Cycloalkyl-Ring und der Heterocyclyl-Ring substituiert sein können mit 0, 1, 2 oder 3 Substituenten unabhängig voneinander ausgewählt aus der Gruppe bestehend aus Trifluormethyl, Alkyl und Alkoxy,
oder
wobei der Cycloalkyl-Ring Benzo-anneliert sein kann,
- R⁴: Wasserstoff oder C₁-C₄-Alkyl bedeutet,
und
- R⁵: Methyl bedeutet,
und ihre Salze, ihre Solvate und die Solvate ihrer Salze,
mit der Maßgabe, dass für den Fall, dass R² Wasserstoff und R⁴ Wasserstoff bedeutet, R³ nicht Glycyl, D-Alanyl, L-Alanyl oder D-Leucyl bedeutet.

Bevorzugt sind auch Verbindungen der Formel (Ia) und (Ib), in welchen
- R¹: 2-Methylprop-1-yl bedeutet,
- R²: Wasserstoff bedeutet,
- R³: C₁-C₆-Alkylcarbonyl bedeutet,
wobei Alkylcarbonyl substituiert ist mit einem Substituenten Amino,
und
wobei Alkylcarbonyl substituiert sein kann mit weiteren 0, 1 oder 2 Substituenten unabhängig voneinander ausgewählt aus der Gruppe bestehend aus Trimethylsilyl, C₁-C₄-Alkoxy, Methylhio, Benzyloxy, C₃-C₆-Cycloalkyl, Phenyl, Naphthyl, Thienyl, Pyridyl, Indolyl, C₁-C₄-Alkoxycarbonylamino, Benzyloxycarbonyl und Benzyloxycarbonylamino,
worin Phenyl seinerseits substituiert sein kann mit 0, 1, 2 oder 3 Substituenten unabhängig voneinander ausgewählt aus der Gruppe bestehend aus Halogen, C₁-C₄-Alkyl, C₁-C₄-Alkoxy und Phenyl,
oder zwei Substituenten an demselben Kohlenstoffatom im Alkylcarbonyl zusammen mit dem Kohlenstoffatom, an das sie gebunden sind, einen C₃-C₆-Cycloalkyl-Ring bilden,
wobei der Cycloalkyl-Ring Benzo-anneliert sein kann,
- R⁴: Wasserstoff bedeutet,
und
- R⁵: Methyl bedeutet,
und ihre Salze, ihre Solvate und die Solvate ihrer Salze,
mit der Maßgabe, dass R³ nicht Glycyl, D-Alanyl, L-Alanyl oder D-Leucyl bedeutet.

Bevorzugt sind auch Verbindungen der Formel (I), welche der Formel 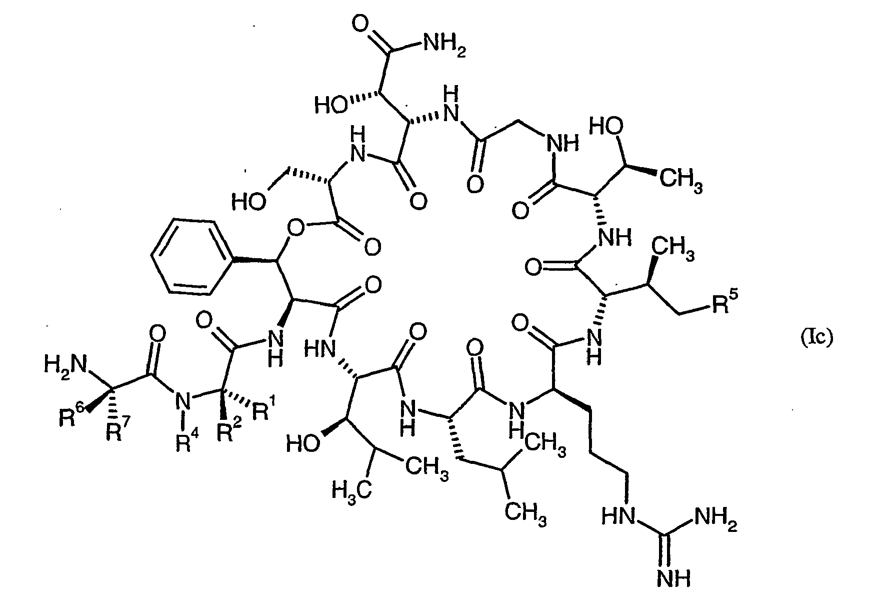 entsprechen, in welcher
- R¹: 2-Methylprop-1-yl bedeutet,
- R²: Wasserstoff bedeutet,
- R⁴: Wasserstoff bedeutet,
- R⁵: Methyl bedeutet,
- R⁶: Methyl, iso-Propyl, 1-Methylprop-1-yl, 2,2-Dimethylprop-1-yl, C₃-C₆-Cycloalkyl, Phenyl, Thienyl, *tert*-Butoxycarbonylaminopropyl, *tert*-Butoxycarbonylaminobutyl, Benzyloxy-carbonylaminopropyl oder Benzyloxycarbonylaminobutyl bedeutet,
wobei Phenyl substituiert sein kann mit 0, 1 oder 2 Substituenten unabhängig voneinander
ausgewählt aus der Gruppe bestehend aus Halogen, Methoxy und Phenyl,
und
wobei Methyl substituiert ist mit einem Substituenten ausgewählt aus der Gruppe bestehend aus Trimethylsilyl, *tert*-Butoxy, Benzyloxy, C₃-C₆-Cycloalkyl, Phenyl, Naphthyl, Pyridyl, Indolyl und Benzyloxycarbonyl,
worin Phenyl seinerseits substituiert sein kann mit 0, 1 oder 2 Substituenten unabhängig voneinander ausgewählt aus der Gruppe bestehend aus Halogen Methoxy und Phenyl,
und
- R⁷: Wasserstoff bedeutet,
oder
- R⁶ und: R⁷ zusammen mit dem Kohlenstoffatom, an das sie gebunden sind, einen C₃-C₆-Cycloalkyl-Ring bilden,
und ihre Salze, ihre Solvate und die Solvate ihrer Salze.

Bevorzugt sind auch Verbindungen der Formel (Ia) und (Ib), in welchen
- R¹: Wasserstoff, C₃-C₆-Cycloalkyl, C₅-C₆-Cycloalkenyl, C₃-C₆-Cycloalkylmethyl, 5- bis 7-gliedriges Heterocyclylmethyl, Methyl, Ethyl, n-Propyl, iso-Propyl, 1-Methylprop-1-yl, 2,2-Dimethylprop-1-yl, 1,1-Dimethylprop-1-yl, 1-Ethyl-prop-1-yl, 1-Ethyl-1-methylprop-1-yl, n-Butyl, 2-Methylbut-1-yl, 3-Methylbut-1-yl, 1-Ethylbut-1-yl, *tert*-Butyl, 4-Methylpent-1-yl, n-Hexyl, Alkenyl oder Aryl bedeutet,
wobei R¹ substituiert sein kann mit 0, 1, 2 oder 3 Substituenten unabhängig voneinander ausgewählt aus der Gruppe bestehend aus Halogen, Hydroxy, Amino, Cyano, Trimethylsilyl, Alkyl, Alkoxy, Benzyloxy, C₃-C₆-Cycloalkyl, Aryl, 5- bis 10-gliedriges Heteroaryl, Alkylamino, Arylamino, Alkylcarbonylamino, Arylcarbonylamino, Alkylcarbonyl, Alkoxycarbonyl, Arylcarbonyl und Benzyloxycarbonylamino,
worin Aryl und Heteroaryl ihrerseits substituiert sein können mit 0, 1, 2 oder 3 Substituenten unabhängig voneinander ausgewählt aus der Gruppe bestehend aus Halogen, Hydroxy, Amino, Cyano, Nitro, Alkyl, Alkoxy und Phenyl,
- R²: Wasserstoff oder C₁-C₄-Alkyl bedeutet,
oder
- R¹ und: R² zusammen mit dem Kohlenstoffatom, an das sie gebunden sind, einen C₃-C₆-Cycloallcyl-Ring oder einen 5- bis 7-gliedrigen Heterocyclyl-Ring bilden, wobei der Cycloalkyl-Ring
und der Heterocyclyl-Ring substituiert sein können mit 0, 1, 2 oder 3 Substituenten un-abhängig voneinander ausgewählt aus der Gruppe bestehend aus Trifluormethyl, Alkyl, Alkoxy und Alkylcarbonyl,
- R³: Alkyl, C₃-C₆-Cycloalkyl, 5- bis 7-gliedriges Heterocyclyl, Aryl, 5- oder 6-gliedriges Heteroaryl, Alkylcarbonyl, Alkoxycarbonyl, C₃-C₆-Cycloalkylcarbonyl, 5- bis 7-gliedriges Heterocyclylcarbonyl, Arylcarbonyl, 5- oder 6-gliedriges Heteroarylcarbonyl oder Alkylaminocarbonyl bedeutet,
wobei Alkyl, Cycloalkyl, Heterocyclyl, Aryl, Heteroaryl, Alkoxycarbonyl, Cyclo-alkylcarbonyl, Heterocyclylcarbonyl, Arylcarbonyl, Heteroarylcarbonyl und Alkylaminocarbonyl substituiert sein können mit 0, 1, 2 oder 3 Substituenten unabhängig voneinander ausgewählt aus der Gruppe bestehend aus Halogen, Hydroxy, Amino, Alkyl-amino und Phenyl,
und
wobei Alkylcarbonyl substituiert ist mit einem Substituenten Amino oder Alkylamino,
und
wobei Alkylcarbonyl substituiert sein kann mit weiteren 0, 1 oder 2 Substituenten unabhängig voneinander ausgewählt aus der Gruppe bestehend aus Halogen, Hydroxy, Trimethylsilyl, Alkoxy, Alkylthio, Benzyloxy, C₃-C₆-Cycloalkyl, Phenyl, Naphthyl, 5- bis 10-gliedriges Heteroaryl, Alkylcarbonylamino, Alkoxycarbonylamino, Arylcarbonylamino, Arylcarbonyloxy, Benzyloxycarbonyl und Benzyloxycarbonylamino,
worin Phenyl und Heteroaryl ihrerseits substituiert sein können mit 0, 1, 2 oder 3 Substituenten unabhängig voneinander ausgewählt aus der Gruppe bestehend aus Halogen, Hydroxy, Nitro, Alkyl, Alkoxy und Phenyl,
oder zwei Substituenten an demselben Kohlenstoffatom im Alkylcarbonyl zusammen mit dem Kohlenstoffatom, an das sie gebunden sind, einen C₃-C₆-Cycloalkyl-Ring oder einen 5-bis 7-gliedrigen Heterocyclyl-Ring bilden,
wobei der Cycloalkyl-Ring und der Heterocyclyl-Ring substituiert sein können mit 0, 1, 2 oder 3 Substituenten unabhängig voneinander ausgewählt aus der Gruppe bestehend aus Trifluormethyl, Alkyl und Alkoxy,
oder
wobei der Cycloalkyl-Ring Benzo-anneliert sein kann,
- R⁴: Wasserstoff, C₁-C₄-Alkyl, Cyclopropyl oder Cyclopropylmethyl bedeutet,
oder
- R³ und: R⁴ zusammen mit dem Stickstoffatom, an das sie gebunden sind, einen 5- bis 7-gliedrigen Heterocyclyl-Ring bilden, wobei der Heterocyclyl-Ring substituiert sein kann mit 0, 1, 2 oder 3 Substituenten unabhängig voneinander ausgewählt aus der Gruppe bestehend aus Halogen, Hydroxy, Amino, Cyano, Alkyl, Alkoxy und Alkylamino,
und
- R⁵: Wasserstoff oder Methyl bedeutet,
und ihre Salze, ihre Solvate und die Solvate ihrer Salze.
Bevorzugt sind auch Verbindungen der Formel (Ia) und (Ib), in welchen
R¹ 5- bis 7-gliedriges Heterocyclylmethyl, Methyl, Ethyl, n-Propyl, iso-Propyl, 1-Methylprop-1-yl, 2,2-Dimethylprop-1-yl, 1,1-Dimethylprop-1-yl, 1-Ethyl-prop-1-yl, 1-Ethyl-1-methylprop-1-yl, n-Butyl, 2-Methylbut-1-yl, 3-Methylbut-1-yl, 1-Ethylbut-1-yl, *tert*-Butyl, 4-Methylpent-1-yl oder n-Hexyl bedeutet, wobei R¹ substituiert sein kann mit 0, 1, 2 oder 3 Substituenten unabhängig voneinander ausgewählt aus der Gruppe bestehend aus Trimethylsilyl, Alkoxy, Benzyloxy, C₃-C₆-Cycloalkyl, Aryl, 5- bis 10-gliedriges Heteroaryl, Alkylamino, Alkylcarbonylamino, Alkylcarbonyl, Alkoxycarbonyl, Arylcarbonyl und Benzyloxycarbonylamino,
worin Aryl und Heteroaryl ihrerseits substituiert sein können mit 0, 1, 2 oder 3 Substituenten unabhängig voneinander ausgewählt aus der Gruppe bestehend aus Halogen, Hydroxy, Nitro, Alkyl, Alkoxy und Phenyl,
- R²: Wasserstoff oder C₁-C₄-Alkyl bedeutet,
oder
- R¹ und: R² zusammen mit dem Kohlenstoffatom, an das sie gebunden sind, einen C₃-C₆-Cycloalkyl-Ring oder einen 5- bis 7-gliedrigen Heterocyclyl-Ring bilden, wobei der Cycloalkyl-Ring und der Heterocyclyl-Ring substituiert sein können mit 0, 1, 2 oder 3 Substituenten un-abhängig voneinander ausgewählt aus der Gruppe bestehend aus Trifluormethyl, Alkyl, Alkoxy und Alkylcarbonyl,
- R³: Alkylcarbonyl oder 5- bis 7-gliedriges Heterocyclylcarbonyl bedeutet,
wobei Alkylcarbonyl substituiert ist mit einem Substituenten Amino,
und
wobei Alkylcarbonyl substituiert sein kann mit weiteren 0, 1 oder 2 Substituenten unabhängig voneinander ausgewählt aus der Gruppe bestehend aus Halogen, Hydroxy, Trimethylsilyl, Alkoxy, Alkylthio, Benzyloxy, C₃-C₆-Cycloalkyl, Phenyl, Naphthyl, 5- bis 10-gliedriges Heteroaryl, Alkylcarbonylamino, Alkoxycarbonylamino, Arylcarbonylamino, Arylcarbonyloxy, Benzyloxycarbonyl und Benzyloxycarbonylamino,
worin Phenyl seinerseits substituiert sein kann mit 0, 1, 2 oder 3 Substituenten unabhängig voneinander ausgewählt aus der Gruppe bestehend aus Halogen,
Hydroxy, Alkyl, Alkoxy und Phenyl,
oder zwei Substituenten an demselben Kohlenstoffatom im Alkylcarbonyl zusammen mit dem Kohlenstoffatom, an das sie gebunden sind, einen C₃-C₆-Cycloalkyl-Ring oder einen 5-bis 7-gliedrigen Heterocyclyl-Ring bilden,
wobei der Cycloalkyl-Ring und der Heterocyclyl-Ring substituiert sein können mit 0, 1, 2 oder 3 Substituenten unabhängig voneinander ausgewählt aus der Gruppe bestehend aus Trifluormethyl, Alkyl und Alkoxy,
oder
wobei der Cycloalkyl-Ring Benzo-anneliert sein kann,
- R⁴: Wasserstoff oder C₁-C₄-Alkyl bedeutet,
und
- R⁵: Methyl bedeutet,
und ihre Salze, ihre Solvate und die Solvate ihrer Salze.

Bevorzugt sind auch Verbindungen der Formel (Ia) und (Ib), in welchen
- R¹: Methyl, Ethyl, n-Propyl, iso-Propyl, 1-Methylprop-1-yl, 2,2-Dimethylprop-1-yl, 1,1-Dimethylprop-1-yl, 1-Ethyl-prop-1-yl, 1-Ethyl-methylprop-1-yl, n-Butyl; 2-Methylbut-1-yl, 3-Methylbut-1-yl, 1-Ethylbut-1-yl, *tert*-Butyl, 4-Methylpent-1-yl oder n-Hexyl bedeutet,
wobei R¹ substituiert sein kann mit 0 oder 1 Substituenten ausgewählt aus der Gruppe bestehend aus Trimethylsilyl, C₁-C₄-Alkoxy, Benzyloxy, C₃-C₆-Cycloalkyl, Phenyl, Naphthyl, Pyridyl, Indolyl, C₁-C₄-Alkoxycarbonyl und Benzyloxycarbonylamino,
worin Phenyl und Pyridyl ihrerseits substituiert sein können mit 0, 1, 2 oder 3 Substituenten unabhängig voneinander ausgewählt aus der Gruppe bestehend aus Halogen, Hydroxy, Nitro, C₁-C₄-Alkyl, C₁-C₄-Alkoxy und Phenyl,
- R²: Wasserstoff bedeutet,
oder
- R¹ und: R² zusammen mit dem Kohlenstoffatom, an das sie gebunden sind, einen C₃-C₆-Cycloalkyl-Ring bilden, wobei der Cycloalkyl-Ring substituiert sein kann mit 0 oder 1 Substituenten ausgewählt aus der Gruppe bestehend aus Trifluormethyl und C₁-C₄-Alkoxy,
- R³: C₁-C₆-Alkylcarbonyl bedeutet,
wobei Alkylcarbonyl substituiert ist mit einem Substituenten Amino,
und
wobei Alkylcarbonyl substituiert sein kann mit weiteren 0, 1 oder 2 Substituenten unabhängig voneinander ausgewählt aus der Gruppe bestehend aus Trimethylsilyl, C₁-C₄-Alkoxy, Methylhio, Benzyloxy, C₃-C₆-Cycloalkyl, Phenyl, Naphthyl, Thienyl, Pyridyl, Indolyl, C₁-C₄-Alkoxycarbonylamino, Benzyloxycarbonyl und Benzyloxycarbonylamino,
worin Phenyl seinerseits substituiert sein kann mit 0, 1, 2 oder 3 Substituenten unabhängig voneinander ausgewählt aus der Gruppe bestehend aus Halogen, C₁-C₄-Alkyl, C₁-C₄-Alkoxy und Phenyl,
oder zwei Substituenten an demselben Kohlenstoffatom im Alkylcarbonyl zusammen mit dem Kohlenstoffatom, an das sie gebunden sind, einen C₃-C₆-Cycloalkyl-Ring bilden,
wobei der Cycloalkyl-Ring Benzo-anneliert sein kann,
- R⁴: Wasserstoff bedeutet,
und
- R⁵: Methyl bedeutet,
und ihre Salze, ihre Solvate und die Solvate ihrer Salze.
Bevorzugt sind auch Verbindungen der Formel (Ic), in welcher
- R¹: Methyl, Phenylethyl, n-Propyl, 1-Methylprop-1-yl, 2,2-Dimethylprop-1-yl, Benzyloxy-carbonylaminopropyl oder Benzyloxycarbonylaminobutyl bedeutet,
wobei Methyl substituiert ist mit einem Substituenten ausgewählt aus der Gruppe bestehend aus *tert*-Butoxy, Benzyloxy, C₃-C₆-Cycloalkyl, Phenyl, Naphthyl, Pyridyl, Indolyl und *tert*-Butoxycarbonyl,
worin Phenyl seinerseits substituiert sein kann mit 0, 1 oder 2 Substituenten unabhängig voneinander ausgewählt aus der Gruppe bestehend aus Halogen, Nitro, Methoxy und Phenyl,
- R²: Wasserstoff bedeutet,
oder
- R¹ und R²: zusammen mit dem Kohlenstoffatom, an das sie gebunden sind, einen C₃-C₆-Cycloalkyl Ring bilden,
- R⁴: Wasserstoff bedeutet,
- R⁵: Methyl bedeutet,
- R⁶: Methyl, iso-Propyl, 1-Methylprop-1-yl, 2-Methylprop-1-yl, 2,2-Dimethylprop-l-yl, C₃-C₆-Cycloalkyl, Phenyl, Thienyl, *tert*-Butoxycarbonylaminopropyl, *tert*-Butoxycarbonyl-aminobutyl, Benzyloxycarbonylaminopropyl oder Benzyloxycarbonylaminobutyl bedeutet,
wobei Phenyl substituiert sein kann mit 0, 1 oder 2 Substituenten unabhängig voneinander ausgewählt aus der Gruppe bestehend aus Halogen, Methoxy und Phenyl,
und
wobei Methyl substituiert ist mit einem Substituenten ausgewählt aus der Gruppe bestehend aus Trimethylsilyl, *tert*-Butoxy, Benzyloxy, C₃-C₆-Cycloalkyl, Phenyl, Naphthyl, Pyridyl, Indolyl und Benzyloxycarbonyl,
worin Phenyl seinerseits substituiert sein kann mit 0, 1 oder 2 Substituenten unabhängig voneinander ausgewählt aus der Gruppe bestehend aus Halogen Methoxy und Phenyl,
und
- R⁷: Wasserstoff bedeutet,
oder
- R⁶ und: R⁷ zusammen mit dem Kohlenstoffatom, an das sie gebunden sind, einen C₃-C₆-Cycloalkyl-Ring bilden,
und ihre Salze, ihre Solvate und die Solvate ihrer Salze.
Gegenstand der Erfindung sind auch solche Verbindungen der Formel (I), (Ia) und (Ib), in welchen
- R¹: Wasserstoff, C₃-C₆-Cycloalkyl, C₅-C₆-Cycloalkenyl, C₃-C₆-Cycloalkylmethyl, 5- bis 7-gliedriges Heterocyclylmethyl, Methyl, Ethyl, n-Propyl, iso-Propyl, 1-Methylprop-1-yl, 2,2-Dimethylprop-1-yl, 1,1-Dimethylprop-1-yl, 1-Ethyl-prop-1-yl, 1-Ethyl-1-methylprop-1-yl, 2-Methylbut-1-yl, 3-Methylbut-1-yl, 1-Ethylbut-1-yl, tert-Butyl, 4-Methylpent-1-yl, n-Hexyl, Alkenyl oder Aryl bedeutet,
wobei R¹ substituiert sein kann mit 0, 1, 2 oder 3 Substituenten unabhängig voneinander ausgewählt aus der Gruppe bestehend aus Halogen, Hydroxy, Amino, Cyano, Alkyl, Alkoxy, C₃-C₆-Cycloalkyl, Aryl, Alkylamino, Arylamino, Alkylcarbonylamino, Arylcarbonylamino, Alkylcarbonyl und Arylcarbonyl,
- R²: Wasserstoff oder C₁-C₄-Alkyl bedeutet,
oder
- R¹ und: R² zusammen mit dem Kohlenstoffatom, an das sie gebunden sind, einen C₃-C₆-Cycloalkyl-Ring oder einen C₅-C₇-Heterocyclyl-Ring bilden, wobei der Cycloalkyl-Ring und der Heterocyclyl-Ring substituiert sein können mit 0, 1, 2 oder 3 Substituenten unabhängig voneinander ausgewählt aus der Gruppe bestehend aus Alkyl und Alkylcarbonyl,
- R³: eine carbonylgebundene Aminosäure bedeutet, wobei die Aminofunktion der Aminosäure
substituiert sein kann mit 0, 1 oder 2 Substituenten C₁-C₄-Alkyl,
oder
- R³: Alkyl, C₃-C₆-Cycloalkyl, 5- bis 7-gliedriges Heterocyclyl, Aryl, 5- oder 6-gliedriges Heteroaryl, Alkylcarbonyl, Alkoxycarbonyl, C₃-C₆-Cycloalkylcarbonyl, 5- bis 7-gliedriges Heterocyclylcarbonyl, Arylcarbonyl, 5- oder 6-gliedriges Heteroarylcarbonyl oder Alkylaminocarbonyl bedeutet,
wobei Alkyl, C₃-C₆-Cycloalkyl, 5- bis 7-gliedriges Heterocyclyl, Aryl, 5- oder 6-gliedriges Heteroaryl, Alkylcarbonyl, Alkoxycarbonyl, C₃-C₆-Cycloalkylcarbonyl, 5- bis 7-gliedriges Heterocyclylcarbonyl, Arylcarbonyl, 5- oder 6-gliedriges Heteroarylcarbonyl und Alkylaminocarbonyl substituiert sein können mit 0, 1, 2 oder 3 Substituenten unabhängig voneinander ausgewählt aus der Gruppe bestehend aus Halogen, Hydroxy, Amino und Alkylamino,
- R⁴: Wasserstoff, C₁-C₄-Alkyl, Cyclopropyl oder Cyclopropylmethyl bedeutet,
oder
- R³ und: R⁴ zusammen mit dem Stickstoffatom, an das sie gebunden sind, einen C₅-C₇-Heterocyclyl-Ring bilden, wobei der Heterocyclyl-Ring substituiert sein kann mit 0, 1, 2 oder 3 Substituenten unabhängig voneinander ausgewählt aus der Gruppe bestehend aus Halogen, Hydroxy, Amino, Cyano, Alkyl, Alkoxy und Alkylamino,
und
- R⁵: Wasserstoff oder Methyl bedeutet.

Bevorzugt sind auch solche Verbindungen der Formeln (I), (Ia) und (Ib), in welchen
- R¹: Cyclopropylmethyl, Cyclopentylmethyl, Cyclohexylmethyl, Ethyl, n-Propyl, iso-Propyl, 2,2-Dimethylprop-1-yl oder 2-Methylbut-1-yl bedeutet,
- R²: Wasserstoff bedeutet,
- R³: eine carbonylgebundene Aminosäure bedeutet,
- R⁴: Wasserstoff bedeutet,
und
- R⁵: Wasserstoff oder Methyl bedeutet.

Bevorzugt sind auch solche Verbindungen der Formeln (I), (Ia) und (Ib), in welchen
- R¹: Cyclopropylmethyl oder n-Propyl bedeutet,
- R²: Wasserstoff bedeutet,
- R³: eine carbonylgebundene Aminosäure bedeutet,
- R⁴: Wasserstoff bedeutet,
und
- R⁵: Methyl bedeutet.

Bevorzugt sind auch solche Verbindungen der Formeln (I), (Ia) und (Ib), in welchen R¹ Cyclopropylmethyl bedeutet.

Bevorzugt sind auch solche Verbindungen der Formeln (I), (Ia) und (Ib), in welchen R² Wasserstoff bedeutet.

Bevorzugt sind auch solche Verbindungen der Formeln (I), (Ia) und (Ib), in welchen R³ eine carbonylgebundene Aminosäure bedeutet.

Bevorzugt sind auch solche Verbindungen der Formeln (I), (Ia) und (Ib), in welchen R⁴ Wasserstoff bedeutet.

Bevorzugt sind auch solche Verbindungen der Formeln (I), (Ia) und (Ib), in welchen R⁵ Methyl bedeutet.

Die in den jeweiligen Kombinationen bzw. bevorzugten Kombinationen von Resten im einzelnen angegebenen Restedefinitionen werden unabhängig von den jeweiligen angegebenen Kombinationen der Reste beliebig auch durch Restedefinitionen anderer Kombination ersetzt.

Ganz besonders bevorzugt sind auch Kombinationen von zwei oder mehreren der oben genannten Vorzugsbereiche.

Gegenstand der Erfindung ist weiterhin ein Verfahren zur Herstellung der Verbindungen der Formeln (I), wobei Verbindungen der Formel 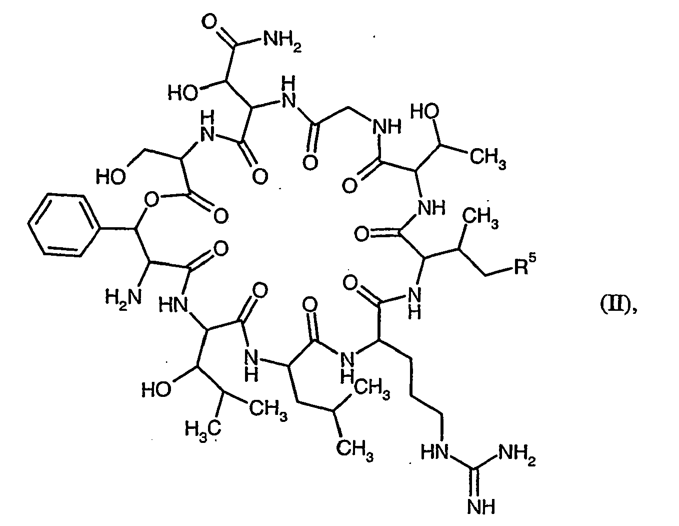 in welcher
- R⁵: die oben angegebene Bedeutung hat,
mit Verbindungen der Formel 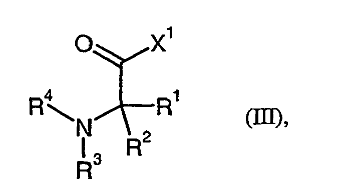 in welcher
- R¹, R²,R³ und R⁴: die oben angegebene Bedeutung haben, und
- X¹: Halogen, bevorzugt Brom, Chlor oder Fluor, oder Hydroxy bedeutet,
umgesetzt werden.

Falls X¹ für Halogen steht, erfolgt die Umsetzung im Allgemeinen in inerten Lösungsmitteln, gegebenenfalls in Gegenwart einer Base, bevorzugt in einem Temperaturbereich von -30°C bis 50°C bei Normaldruck.

Inerte Lösungsmittel sind beispielsweise Tetrahydrofuran, Methylenchlorid, Pyridin, Dioxan oder Dimethylformamid, bevorzugt ist Pyridin oder Dimethylformamid.

Als inerte Lösungsmittel sind Tetrahydrofuran oder Methylenchlorid bevorzugt.

Basen sind beispielsweise Triethylamin, Diisopropylethylamin oder N-Methylmorpholin, bevorzugt ist Diisopropylethylamin.

Falls X¹ für Hydroxy steht, erfolgt die Umsetzung im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Als Dehydratisierungsreagenzien eignen sich hierbei beispielsweise Carbodiimide wie z.B. *N,N'-*Diethyl-, *N,N*,'-Dipropyl-, *N,N*'-Diisopropyl-, *N,N*'-Dicyclohexylcarbodiimid, *N*-(3-Di-methylaminoisopropyl)-*N*'-ethylcarbodiimid-hydrochlorid (EDC), *N*-Cyclohexylcarbodiimid-*N*'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2*-tert-*Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)-phosphoniumhexafluorophosphat, oder *O*-(Benzotriazol-1-yl)-*N,N,N;N*'-tetra-methyluronium-hexafluorophosphat (HBTU), 2-(2-Oxo-1-(2H)-pyridyl)-1,1,3,3-tetramethyluroniumtetrafluoro-borat (TPTU) oder *O*-(7-Azabenzotriazol-1-yl)-*N,N,N',N*'-tetramethyl-uroniumhexafluorophosphat (HATU), oder 1-Hydroxybenztriazol (HOBt), oder Benzotriazol-1-yloxytris(dimethylamino)-phos-phoniumhexafluorophosphat (BOP), oder *N*-Hydroxysuccinimid, oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, *N*-Methylpiperidin, 4-Dimethylaminopyridin oder Diisopropylethylamin.

Vorzugsweise wird die Kondensation mit HATU oder mit EDC in Gegenwart von HOBt durchgeführt.

Die Verbindungen der Formel (III) tragen gegebenenfalls Schutzgruppen, so dass sich in diesen Fällen der Umsetzung von Verbindungen der Formel (II) mit Verbindungen der Formel (III) eine Abspaltung der Schutzgruppen mit Trifluoressigsäure nach dem Fachmann bekannten Methoden anschließt.

Die Verbindungen der Formel (II) lassen sich durch doppelten Edmann-Abbau aus Lysobactin (Beispiel 1A) bzw. Katanosin (Beispiel 2A) synthetisieren, wie im experimentellen Teil unter Beispiel 10A bis 13A beschrieben.

Die Verbindungen der Formel (III) sind bekannt oder lassen sich nach bekannten Verfahren aus den entsprechenden Edukten synthetisieren.

Die Herstellung der erfindungsgemäßen Verbindungen kann durch folgendes Syntheseschemata verdeutlicht werden.

### Syntheseschema:

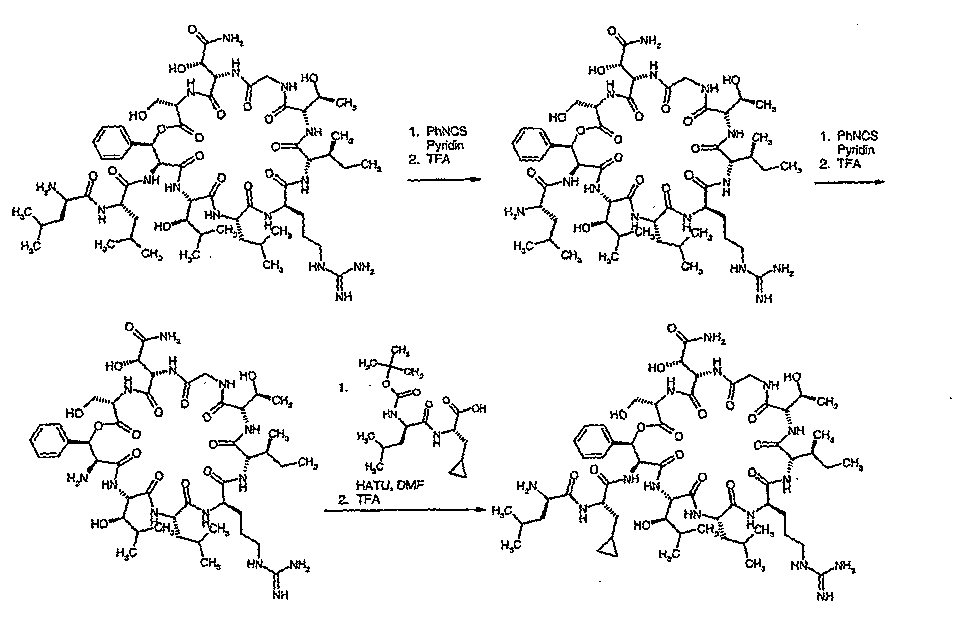

Die erfindungsgemäßen Verbindungen zeigen ein nicht vorhersehbares, wertvolles pharmakologisches Wirkspektrum. Sie zeigen eine antibakterielle Wirkung.

Sie eignen sich daher zur Verwendung als Arzneimittel zur Behandlung und/oder Prophylaxe von Krankheiten bei Menschen und Tieren.

Die erfmdungsgemäßen Verbindungen zeichnen sich durch eine geringere Nephrotoxizität gegenüber Lysobactin aus.

Die beschriebenen Nonadepsipeptide wirken als Inhibitoren der bakteriellen Zellwandbiosynthese.

Besonders wirksam sind die erfindungsgemäßen Zubereitungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Grundsätzlich können die erfindungsgemäßen Zubereitungen gegen alle Bakterien und bakterienähnlichen Mikroorganismen verwendet werden, die im Besitz einer bakteriellen Zellwand (Murein sacculus) bzw. den dazugehörigen Enzymsystemen sind, beispielsweise durch die folgenden Erreger oder durch Mischungen der folgenden Erreger:
Gram-negative Kokken (*Neisseria gonorrhoeae*) sowie Gram-negative Stäbchen wie Enterobakteriaceen, z.B. *Escherichia coli, Hämophilus influenzae,* Pseudomonas, Klebsiella, Citrobacter (*C*. *freundii, C. divernis),* Salmonella und Shigella; ferner Enterobacter (*E. aerogenes, E. agglomerans*)*,* Hafnia, Serratia (*S. marcescens*)*,* Providencia, Yersinia, sowie die Gattung Acinetobacter, Branhamella und Chlamydia. Darüber hinaus umfasst das antibakterielle Spektrum strikt anaerobe Bakterien wie z.B. *Bacteroides fragilis,* Vertreter der Gattung Peptococcus, Peptostreptococcus sowie die Gattung Clostridium; ferner Mykobakterien, z.B. *M. tuberculosus.* Besonders ausgeprägte Wirkung zeigen die erfindungsgemäßen Verbindungen gegen Gram-positive Kokken, z.B. Staphylokokken (*S. aureus, S. epidermidis, S. haemolyticus, S. carmosus*)*,* Enterokokken *(E. faecalis, E. faecium)* und Streptokokken *(S. agalactiae, S. pneumoniae, S. pyogenes).*

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Zubereitungen verhindert, gebessert oder geheilt werden können, seien beispielsweise genannt:
Infektionskrankheiten beim Menschen wie z.B. unkomplizierte und komplizierte Harnwegsinfekte, unkomplizierte Haut- und Oberflächeninfektionen, komplizierte Haut- und Weichteilinfektionen, im Hospital und ambulant erwoibene Lungenentzündung, nosokomiale Pneumonien, akute Exazerbationen und sekundäre bakterielle Infektionen der chronischen Bronchitis, akute Otitis media, akute Sinusitis, streptokokkale Pharyngitis, bakterielle Meningitis, unkomplizierte gonokokkale und nicht-gonokokkale Urethritis/Cervizitis, akute Prostatitis, Endocarditis, unkomplizierte und komplizierte intra-abdominale Infektionen, gynäkologische Infektionen, pelvische Entzündungskrankheit, bakterielle Vaginose, akute und chronische Osteomyelitis, akute bakterielle Arthritis, empirische Therapie in febrilen neutropenischen Patienten, desweiteren Bakterämien, MRSA Infektionen, akute infektiöse Diarrhoe, *Helicobacter pylori* Infektionen, postoperative Infektionen, odontogene Infektionen, ophthalmologische Infektionen, postoperative Infektionen (inkl. periproktaler Abszess, Wundinfektionen, Galleninfektionen, Mastitis und akute Appendizitis), zystische Fibrose und Bronchiectasis.

Außer beim Menschen können bakterielle Infektionen auch bei anderen Spezies behandelt werden. Beispielhaft seien genannt:
Schwein: Diarrhoe, Enterotoxamie, Sepsis, Dysenterie, Salmonellose, Metritis-Mastitis-Agalaktiae-Syndrom, Mastitis;

Wiederkäuer (Rind, Schaf, Ziege): Diarrhoe, Sepsis, Bronchopneumonie, Salmonellose, Pasteurellose, Genitalinfektionen;

Pferd: Bronchopneumonien, Fohlenlähme, puerperale und postpuerperale Infektionen, Salmonellose;

Hund und Katze: Bronchopneumonie, Diarrhoe, Dermatitis, Otitis, Harnwegsinfekte, Prostatitis;

Geflügel (Huhn, Pute, Wachtel, Taube, Ziervögel und andere): *E. coli-*Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie z.B. Pasteurella, Brucella, Campylobacter, Listeria, Erysipelothris, Corynebakterien, Borellia, Treponema, Nocardia, Rikettsia, Yersinia, erweitert.

Weiterer Gegenstand der vorliegenden Erfindung ist der Einsatz der erfindungsgemäßen Verbindungen zur Behandlung und/oder Prophylaxe von Erkrankungen, insbesondere von bakteriellen Infektionskrankheiten.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Bevorzugt werden die erfindungsgemäßen Verbindungen zur Herstellung von Arzneimitteln verwendet, die zur Prophylaxe und/oder Behandlung von bakteriellen Erkrankungen geeignet sind.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer antibakteriell wirksamen Menge der erfindungsgemäßen Verbindungen.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen. Bevorzugte Kombinationswirkstoffe sind antibakteriell wirkende Verbindungen, die ein anderes Wirkspektrum, insbesondere ergänzendes Wirkspektrum, haben und/oder synergistisch zu den erfindungsgemäßen Verbindungen sind.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a.

Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfmdungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei intravenöser Applikation Mengen von etwa 0.001 bis 100 mg/kg, vorzugsweise etwa 0.1 bis 10 mg/kg Körpergewicht zur Erzielung wirksamer Ergebnisse zu verabreichen, und bei oraler Applikation beträgt die Dosierung etwa 0.01 bis 50 mg/kg, vorzugsweise 0.5 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen

- Allg.: Allgemein(e)
- Area: (Peak)fläche
- ber.: berechnet
- BHI: Brain Heart Infusion
- Boc: *tert*-Butyloxycarbonyl
- br.: breites Signal (bei NMR-Spektren)
- Bsp.: Beispiel
- d: Dublett (bei NMR-Spektren)
- DC: Dünnschichtchromatographie
- DCI: direkte chemische Ionisation (bei MS)
- DCM: Dichlormethan
- DIEA: *N*,*N*-Diisopropylethylamin
- DMSO: Dimethylsulfoxid
- DMF: *N*,*N-*Dimethylformamid
- d. Th.: der Theorie
- EDC: 1-Ethyl-3-(3-dimethylaminopropyl)carbodiimide (auch EDCI)
- EDCxHCl: 1-Ethyl-3-(3-dimethylaminopropyl)carbodiimide-hydrochlorid
- EE: Ethylacetat (Essigsäureethylester)
- EI: Elektronenstoß-Ionisation (bei MS)
- ESI: Elektrospray-Ionisation (bei MS)
- Fp.: Schmelzpunkt
- gef.: gefunden
- ges.: Gesättigt
- h: Stunde
- HATU: *O*-(7-Azabenzotriazol-1-yl)-*N*,*N*,*N*',*N*'-tetramethyluronium-hexa-fluorphosphat
- HOBt: 1-Hydroxybenzotriazol
- HPLC: Hochdruck-, Hochleistungsflüssigchromatographie
- HR: High Resolution (Hochauflösung)
- i. V.: im Vakuum
- konz.: Konzentriert
- LC-MS: Flüssigchromatographie-gekoppelte Massenspektroskopie
- LDA: Lithium-Diisopropylamid
- m: middle (mittel) (bei UV- und IR-Spektren)
- m: Multiplett (bei NMR-Spektren)
- MALDI: Matrix-Assisted Laser Desorption/Ionization
- MHK: Minimale Hemmkonzentration
- min: Minute/Minuten
- MRSA: Methicillin-resistenter *Staphylococcus aureus*
- MS: Massenspektroskopie
- NCCLS: National Committee for Clinical Laboratory Standards
- neg.: negativ
- NMM: *N*-Methylmorpholin
- NMR: Kernresonanzspektroskopie (nuclear magnetic resonance)
- p.a.: pro analysi
- Pd-C: Palladium auf Kohle
- pos.: positiv
- proz.: Prozentig
- quant.: quantitativ
- RP-HPLC: Reverse Phase HPLC
- RT: Raumtemperatur
- Rₜ: Retentionszeit (bei HPLC)
- s: strong (stark) (bei UV- und IR-Spektren)
- s: Singulett (bei NMR-Spektren)
- TBTU: *O*-(Benzotriazol-1-yl)-*N*,*N*,*N*',*N*'-tetramethyluronium-Tetrafluoroborat
- TCTU: *O*-(1H-6-Chlorbenzotriazol-1-yl)-1,1,3,3-tetramethyluronium-Tetrafluoroborat
- TFA: Trifluoressigsäure
- TFE: 2,2,2-Trifluorethanol
- THF: Tetrahydrofuran
- TOF: Flugzeit (time of flight)
- UV: Ultraviolett
- Vis: sichtbar (visible)
- VRSA: Vancomycin-resistenter *Stapylococcus aureus*
- w: weak (schwach) (bei UV- und IR-Spektren)
- Z, Cbz: Benzyloxycarbonyl

### Literatur

Zur Nomenklatur der Peptide und Cyclodepsipeptide vergleiche:
1. A Guide to IUPAC Nomenclature of Organic Compounds (Recommendations 1993), 1993, Blackwell Scientific publications.
2. Nomenclature and symbolism for amino acids and peptides. Recommendations 1983. IUPAC-IUB Joint Commission on Biochemical Nomenclature, UK. Biochemical Journal 1984, 219, 345-373. Sowie zitierte Literatur.

### Allgemeine Methoden LC-MS, HR-MS, HPLC und Gelchromatographie

**Methode 1 (HPLC):** Gerät: HP1100 mit DAD (G1315A) und Autosampler (G1329A), Autosamplertherme (G1330A, 5°C), Degaser (G1322A) und Binäre Pumpe (G1312A); Vorsäule: Waters Symmetry C-18, 10 x 2.1 mm, 3.5 µm; Analytiksäule: Waters Symmetry C-18, 50 x 2.1 mm, 3.5 µm; Säulenofen: 45°C; Eluent A: Wasser/0.05% Trifluoressigsäure; Eluent B: Acetonitril/0.05% Trifluoressigsäure; Fluss: 0.4 ml/min.; Gradient 0-100% B in 9 min, hiernach 3 min bei 100% B, hiernach Regeneration der Säule.

**Methode 2 (LC-MS):** Gerät: Micromass LCT; Ionisation: ESI positiv/negativ; HP1100 mit DAD und Autosampler; Ofen 40°C; Säule: Waters Symmetry C-18, 50 x 2.1 mm, 3.5 µm; Eluent A: 0.1% Ameisensäure/Acetonitril, Eluent B: 0.1% Ameisensäure/Wasser; Fluss: 0.5 ml/min.; Gradient: 0-1 min 0% A, 1-6 min 90% A, 6-8 min 100% A, 8-10 min 100% A, 10-15 0% A.

**Methode 3 (HPLC):** Gerät: Gilson Abimed HPLC; UV Detektor 254 nm; binäres Pumpensystem; Säule: Nucleosil RP-18, 7 µm; 250 x 50 mm; Fluss: 30 ml/min; Eluent A: Wasser/0.1% Trifluoressigsäure, Eluent B: Acetonitril/0.1% Trifluoressigsäure; Gradient: 0-40 min 20-25% B, 40-60 min 25% B, 60-110 min 25-50% B, 110-120 min 50% B, 120-130 min 50-100% B, 130-160 min 100% B, anschließend Regeneration der Chromatographiesäule.

**Methode 4 (HPLC):** Gerät: Gilson Abimed HPLC; UV Detektor 254 nm; binäres Pumpensystem; Säule: Nucleosil RP-18, 7 µm; 250 x 50 mm; Fluss: 40 ml/min; Eluent A: Wasser/0.05% Trifluoressigsäure, Eluent B: Acetonitril/0.05% Trifluoressigsäure; Gradient: 0-105 min 20-25% B, 105-111 min 25% B, 111-131 min 25-27% B, 131-157 min 27-35% B, 157-192 min 35-40% B, 192-207 min 40-45% B, anschließend Regeneration der Chromatographiesäule.

**Methode 5 (HPLC):** Gerät: Gilson Abimed HPLC; UV Detektor 254 nm; binäres Pumpensystem; Säule: Nucleosil RP-18, 7 µm; 250 x 50 mm; Fluss: 40 ml/min; Eluent A: Wasser/0.05% Trifluoressigsäure, Eluent B: Acetonitril/0.05% Trifluoressigsäure; Gradient: 0-40 min 20-25% B, 40-105 min 25% B, 105-130 min 25-27% B, 130-170 min 27-40% B, 170-190 min 40% B, 190-210 min 40-45% B, anschließend Regeneration der Chromatographiesäule.

**Methode 6 (Gelchromatographie an Sephadex LH-20):** Gelchromatographie wird ohne Druck an Sephadex LH-20 (Firma Pharmacia) durchgeführt. Es wird nach UV-Aktivität (UV Detektor für 254 nm, Firma Knauer) fraktioniert (Fraktionssammler ISCO Foxy 200). Säulendimensionen: 32 x 7 cm (1000-100 µmol Maßstab); 30 x 4 cm (100-10 µmol Maßstab); 25 x 2 cm (10-1 µmol Maßstab).

**Methode 7 (präparative HPLC; Symmetry; Essigsäure):** Gerät: Gilson Abimed HPLC; UV Detektor 210 nm; binäres Pumpensystem; Säule: SymmetryPrep^{™}C₁₈, Firma Waters, 7 µm; 300 x 19 mm; Fluss: 7 ml/min; Eluent A: Wasser/0.5-0.25% Essigsäure, Eluent B: Acetonitril; Gradient: 0-2 min 5% B, 2-60 min 5-90% B, 60-80 min 100% B, anschließend Regeneration der Chromatographiesäule.

**Methode 8 (präparative HPLC; Symmetry; TFA):** Gerät: Gilson Abimed HPLC; UV Detektor 210 nm; binäres Pumpensystem; Säule: SymmetryPrep^{™}C₁₈, Firma Waters, 7 µm; 300 x 19 mm; Fluss: 7 ml/min; Eluent A: Wasser/0.1-0.25% Trifluoressigsäure, Eluent B: Acetonitril; Gradient: 0-8 min 5% B, 8-40 min 5-60% B, 40-60 min 60% B, 60-75 min 60-100% B, 75-80 min 100% B, anschließend Regeneration der Chromatographiesäule.

**Methode 9 (präparative HPLC; Kromasil, Essigsäure):** Gerät: Gilson Abimed HPLC; UV Detektor 210 nm; binäres Pumpensystem; Säule: Kromasil-100A C₁₈, 5 µm; 250 x 20 mm; Fluss: 25 ml/min; Eluent A: Wasser/0.25-0.5% Essigsäure, Eluent B: Acetonitril; Gradient: 0-3 min 5% B, 3-30 min 5-100% B, 30-38 min 100% B, anschließend Regeneration der Chromatographiesäule.

**Methode 10 (präparative HPLC; Kromasil, TFA):** Gerät: Gilson Abimed HPLC; UV Detektor 210 nm; binäres Pumpensystem; Säule: Kromasil-100A C₁₈, 5 µm; 250 x 20 mm; Fluss: 25 ml/min; Eluent A: Wasser/0.1-0.25% Trifluoressigsäure, Eluent B: Acetonitril; Gradient: 0-3 min 5% B, 3-30 min 5-100% B, 30-38 min 100% B, anschließend Regeneration der Chromatographiesäule.

**Methode 11 (LC-MS):** Gerätetyp MS: Micromass ZQ; Gerätetyp HPLC: HP 1100 Series; UV DAD; Säule: Grom-Sil 120 ODS-4 HE 50 x 2 mm, 3.0 µm; Eluent A: Wasser/0.025% Ameisensäure/l, Eluent B: Acetonitril/0.025% Ameisensäure; Gradient: 0-2.9 min 0-70% B, 2.9-3.1 min 70-90% B, 3.1-4.5 min 70-90% B; Ofen: 50°C, Fluss: 0.8 ml/min, UV-Detektion: 210 nm.

**Methode 12 (LC-MS):** Gerätetyp MS: Micromass LCT (ESI pos./neg.); Gerätetyp HPLC: HP 1100 Series; UV DAD 1100 Series; Säule SymmetryPrep^{™}C₁₈, Firma Waters, 50 x 2.1 mm, 3.5 µm; Eluent A: Wasser/0.1% Ameisensäure, Eluent B: Acetonitril/0.1% Ameisensäure; Gradient: 0-1 min 0% B, 1-5.5 min 0-95% B, 5.5-8 min 95% B, 8-8.1 min 95-0% B, 8.1-10 min 0% B, anschließend Regeneration der Chromatographiesäule. Ofen: 40°C, Fluss: 0.5 ml/min (bei 8.1-10 min kurzfristig auf 1 ml/min), UV-Detektion: 210 nm.

**Methode 13 (HPLC):** Gerätetyp HPLC: HP 1050 Series; UV DAD 1100 Series; Säule Symmetry-Prep^{™}C₁₈, Firma Waters, 50 x 2.1 mm, 3.5 µm; Eluent A: Wasser/0.05% Trifluoressigsäure, Eluent B: Acetonitril; Gradient: 0-9 min 0-100% B, 9-11 min 100% B, 11-12 min 100-0% B, anschließend Regeneration der Chromatographiesäule. Ofen: 40°C, Fluss: 0.4 ml/min, UV-Detektion: 210 nm.

**Methode 14 (präparative HPLC; Nucleodur C₁₈, Essigsäure):** Gerät: Gilson Abimed HPLC; UV Detektor 210 nm; binäres Pumpensystem; Säule: Nucleodur C₁₈ Gravity, Firma Macherey-Nagel, 5 µm; 250 x 21 mm; Fluss: 20 ml/min; Eluent A: Wasser/0.25-0.5% Essigsäure, Eluent B: Acetonitril; Gradient: 0-3 min 5% B, 3-30 min 5-100% B, 30-38 min 100% B, anschließend Regeneration der Chromatographiesäule.

**Methode 15 (präparative HPLC; Nucleodur C₁₈, TFA):** Gerät: Gilson Abimed HPLC; UV **Detektor 210 nm; binäres Pumpensystem; Säule:** Nucleodur C₁₈ Gravity, Firma Macherey-Nagel, 5 µm; 250 x 21 mm; Fluss: 7 ml/min; Eluent A: Wasser/0.1-0.25% Trifluoressigsäure, Eluent B: Acetonitril; Gradient: 0-8 min 5% B, 8-40 min 5-60% B, 40-60 min 60% B, 60-75 min 60-100% B, 75-80 min 100% B, anschließend Regeneration der Chromatographiesäule.

**Methode 16 (präparative HPLC; YMC Gel ODS-AQ5-5 ; Essigsäure):** Gerät: Gilson Abimed HPLC; UV Detektor 254 nm; binäres Pumpensystem; Säule: YMC Gel ODS-AQ5-5, 15 µm; 250 x 50 mm; Fluss: 25 ml/min; Eluent A: Wasser/0.5% Essigsäure, Eluent B: Acetonitril; Gradient: 0-3 min 20% B, 3-25 min 20-95% B, anschließend Regeneration der Chromatographiesäule.

**Methode 17 (LC-MS):** Gerät: Micromass Quattro LCZ, mit HPLC Agilent Serie 1100; Säule: Grom-SIL120 ODS-4 HE, 50 x 2.0 mm, 3 µm; Eluent A: Wasser/0.05% Ameisensäure, Eluent B: Acetonitril/0.05% Ameisensäure; Gradient: 0.0-0.2 min 100% A, 0.2-2.9 min 100-30% A, 2.9-3.1 min 30-10% A, 3.1-4.5 min 10% A; Ofen: 55°C, Fluss: 0.8 ml/min, UV-Detektion: 208-400 nm.

**Methode 18 (HPLC):** Gerätetyp HPLC: HP 1050 Series; UV DAD 1100 Series; Säule: Kromasil C₁₈, 60 x 2 mm, 3.5 µm; Eluent A: Wasser/0.5% HClO₄, Eluent B: Acetonitril; Gradient: 0-0.5 min 2% B, 0.5-4.5 min 2-90% B, 4.5-6.5 min 90% B, 6.5-6.7 min 90-2% B, 6.7-7.5 min 2% B; Fluss: 0.75 ml/min, Ofen: 30°C, UV-Detektion 210 nm.

**Methode 19 (LC-MS):** Gerätetyp MS: Micromass ZQ; Gerätetyp HPLC: Waters Alliance 2790; Säule: Grom-Sil 120 ODS-4 HE 50 x 2 mm, 3.0 µm; Eluent B: Acetonitril/0.05% Ameisensäure, Eluent A: Wasser/0.05% Ameisensäure; Gradient: 0.0-2.0 min 5%-40%B, 2.0-4.5 min 40-90% B, 4.5-5.5 min 90%B; Fluss: 0.0 min 0.75 ml/min, 4.5 min 0.75ml/min, 5.5 min 1.25 ml/min; Ofen: 45°C; UV-Detektion: 210 nm.

**Methode 20 (MALDI-MS):** Die *MALDI-MS*/*MS-Untersuchungen* werden auf einem 4700 Proteomics Analyzer (Applied Biosystems, Framingham, MA, USA) durchgeführt, der mit TOF/TOF Ionenoptik und 200 Hz Nd:YAG Laser (355 nm) ausgestattet ist. Die Quasimolekülionen werden in der Ionenquelle mit 8 kV beschleunigt, mit einem elektrischen Deflektor selektiert (MS1), und in einer Stoßzelle, die zwischen MS1 und MS2 angeordnet ist, mit Argonatomen gestoßen. Die entstehenden Fragmentionen werden mit 15 kV nachbeschleunigt und mit dem zweiten Flugzeit-Massenanalysator (MS2) charakterisiert.

**Methode 21 (TOF-HR-MS):** *TOF-HR-MS-ESI+* Spektren werden mit einem Micromass LCT Gerät (Kapillarspannung: 3.2 KV, Cone-Spannung: 42 V, Quellentemperatur: 120°C, Desolvations-Temperatur: 280°C) aufgenommen. Hierzu wird eine Spritzenpumpe (Firma Harvard Apparatus) für die Probenzuführung verwendet. Als Standart dient Leucin-Enkephalin (Tyr-Gly-Gly-Phe-Leu).

**Methode 22 (LC-MS):** Gerätetyp MS: Micromass ZQ; Gerätetyp HPLC: Waters Alliance 2795; Säule: Phenomenex Synergi 2 µm Hydro-RP Mercury 20 x 4 mm; Eluent A: Wasser/0.25% Ameisensäure, Eluent B: Acetonitril/0.25% Ameisensäure; Gradient: 0.0-2.5 min, 90-30% A, Fluss 1-2 ml/min, 2.5-3.0 min; 30-5% A, Fluss 2.0 min, 3.0-4.5 min, 5% A; Ofen: 50°C; UV-Detektion: 210 nm.

**Methode 23 (FT-ICR-HR-MS):** Die Massenpräzisionsmessungen werden an einem hochauflösenden Apex II Fourier-Transform Ionen-Cyclotron-Resonanz Massenspektrometer (Bruker Daltonik GmbH, Bremen) durchgeführt, das mit einem 7 Tesla Magneten, einer externen Elektrospray-Ionenquelle und einem Unix-basierten XMASS-Datensystem ausgestattet ist. Die Massenauflösung beträgt ca. 40.000 (50%-Tal-Definition).

**Methode 24 (präparative HPLC; Nucleodur C₁₈, Essigsäure):** Gerät: Gilson Abimed HPLC; UV Detektor 210 nm; binäres Pumpensystem; Säule: Nucleodur C₁₈ Gravity, Firma Macherey-Nagel, 5 µm; 250 x 40 mm; Fluß: 15-45 ml/min; Eluent A: Wasser/0.2% Essigsäure, Eluent B: Acetonitril/0,2%Essigsäure; Gradient: 0-10 min 10% B, 10-24 min 10-30% B, 24-28 min 30-50% B, 28-35 min 50% B, 35-45 min 50-60% B, 45-53 min 60-70% B, 53-60 min 60-90% B, 60-70 min 100% B, anschließend Regeneration der Chromatographiesäule. **Methode 25 (präparative HPLC; Nucleodur C₁₈, Trifluoressigsäure):** Gerät: Gilson Abimed HPLC; UV Detektor 210 nm; binäres Pumpensystem; Säule: Nucleodur C₁₈ Gravity, Firma Macherey-Nagel, 5 µm; 250 × 40 mm; Fluß: 15-45 ml/min; EluentA: Wasser/0.1% Trifluoressigsäure, Eluent B: Acetonitril; Gradient: 0-12 min 10% B, 12-20 min 10-35% B, 20-25 min 35-40% B, 25-35 min 40% B, 35-45 min 40-50% B, 45-50 min 50-60% B 100% B, 50-60 min 60-100% B, 60-75 min 100% B, anschließend Regeneration der Chromatographiesäule.

**Methode 26 (LC-MS):** Gerätetyp MS: Micromass ZQ; Gerätetyp HPLC: HP 1100 Series; UV DAD; Säule: Phenomenex Synergi 2 µ Hydro-RP Mercury 20 mm x 4 mm; Eluent A: 11 Wasser + 0.5 ml 50%ige Ameisensäure, Eluent B: 11 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.

**Methode 27 (LC-MS):** Instrument: Micromass Platform LCZ mit HPLC Agilent Serie 1100; Säule: Phenomenex Synergi 2 µ Hydro-RP Mercury 20 mm x 4 mm; Eluent A: 11 Wasser + 0.5 ml 50%ige Ameisensäure, Eluent B: 11 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

**Methode 28 (analytische HPLC):** Gerätetyp HPLC: HP 1050 Series; UV DAD 1100 Series; Säule: Kromasil C₁₈, 60 x 2 mm, 3.5 µm; . Eluent A: Wasser/0.5% Perchlorsäure, Eluent B: Acetonitril; Gradient: 0-0.5 min 2% B, 0.5-4.5 min 2-90% B, 4.5-9.0 min 90% B, 9.0-9.2 min 90-2% B, 9.2-10.0 min 2% B; Fluss: 0.75 ml/min, Ofen: 30°C, UV-Detektion 210 nm.

**Methode 29 (LC-MS):** Gerätetyp MS: Micromass LCT (ESI pos./neg.); Gerätetyp HPLC: HP 1100 Series; UV DAD 1100 Series; Säule SymmetryPrep™C₁₈, Firma Waters, 50 x 2.1 mm, 3.5 µm; Eluent A: Wasser/0.1% Ameisensäure, Eluent B: Acetonitril/0.1% Ameisensäure; Gradient: 0-1 min 0% B, 1-6 min 0-90% B, 6-8 min 90-100% B, 8-10 min 100% B, 10-10.1 min 100-0% B, 10.1-12 min 0% B, anschließend Regeneration der Chromatographiesäule. Ofen: 40°C, Fluss: 0.5 ml/min (bei 10.1 min kurzfristig auf 1 ml/min), UV-Detektion: 210 nm.

**Methode 30 (LC-MS):** Gerätetyp MS: Micromass LCT (ESI pos./neg.); Gerätetyp HPLC: HP 1100 Series; UV DAD 1100 Series; Säule SymmetryPrep™C₁₈, Firma Waters, 50 x 2.1 mm, 3.5 µm; Eluent A: Wasser/0.1% Ameisensäure, Eluent B: Acetonitril/0.1% Ameisensäure; Gradient: 0-1 min 0% B, 1-5.5 min 0-95% B, 5.5-8 min 95% B, 8-8.1 min 95-0% B, 8.1-10 min 0% B, anschließend Regeneration der Chromatographiesäule. Ofen: 40°C, Fluss: 0.5 ml/min (bei 10.1 min kurzfristig auf 1 ml/min), UV-Detektion: 210 nm.

**Methode 31 (präparative HPLC):** Gerät: Gilson Abimed HPLC; UV-Detektor 210 nm; binäres Pumpensystem; Säule: Reprosil ODS-A, 5 µm, 250 x 20 mm; Eluent A: 0.2% Trifluoressigsδure in Wasser, Eluent B: Acetonitril; Flussrate: 25 ml/min; Säulentemperatur 40°C; 0-10 min 20% B, 10-15 min 80% B.

**Methode 32 (präparative HPLC):** Gerät: Gilson Abimed HPLC; UV-Detektor 210 nm; binäres Pumpensystem; Säule: Kromasil C₁₈, 5 µm, 100 E, 250 x 20 mm; Eluent A: 0.05% Trifluoressigsäure in Wasser, Eluent B: 0.05% Trifluoressigsäure in Acetonitril: Flussrate: 20 ml/min; 0-3 min 10% B, Rampe, 30-38 min 90% B, 38-45 min 10% B. Für N-Butoxycarbonylgeschützte Substanzen wird die Trifluoressigsäure im Laufmittel grundsätzlich durch 0.05% Essigsäure ersetzt.

**Methode 33 (präparative HPLC):** Gerät: Gilson Abimed HPLC; UV-Detektor 210 nm; binäres Pumpensystem; Säule: Waters Symmetry-Prep™ C₁₈, 7 µm, 300 x 19 mm; Eluent A: 0.05% Trifluoressigsäure in Wasser, Eluent B: 0.05% Trifluoressigsäure in Acetonitril: Flussrate: 20 ml/min; 0-3 min 10% B, Rampe, 30-38 min 90% B, 38-45 min 10% B. Für N-Butoxycarbonylgeschützte Substanzen wird die Trifluoressigsäure im Laufmittel grundsätzlich durch 0.05% Essigsäure ersetzt.

**Methode 34 (Gelchromatographie, Sephadex LH-20):** Die Probe wird an Sephadex LH-20 bei Normaldruck mit einem Fließmittel aus Methanol + Aceton 9 + 1 + 0.5% Essigsäure chromatographiert. UV-Detektion bei 210 nm.

**Methode 35 (LC-MS):** Instrument: Micromass Quattro LCZ mit HPLC Agilent Serie 1100; Säule: Phenomenex Synergi 2 µm Hydro-RP Mercury 20 mm x 4 mm; Eluent A: 11 Wasser + 0.5 ml 50%ige Ameisensäure, Eluent B: 11 Acetonitril + 0.5 ml 50% Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 208-400 nm.

**Methode 36 (analytische HPLC):** Gerät: Agilent 1100 mit DAD (G1315B), binärer Pumpe (G1312A), Autosampler (G1313A), Lösemittel-Entgaser (G1379A) und Säulenthermostat (G1316A); Säule: Agilent Zorbax Eclipse XDB-C8 4.6 x 150 x 5 mm; Säulentemperatur: 30°C; Eluent A: 0.05% 70% Perchlorsäure in Wasser; EluentB: Acetonitril; Fluss: 2.00 ml/min; Gradient: 0-1 min 10% B, Rampe, 4-5 min 90% B, Rampe, 5.5 min 10% B.

**Methode 37 (LC-MS):** Instrument: Micromass Quattro LCZ mit HPLC Agilent Serie 1100; Säule: Phenomenex Synergi 2 µ Hydro-RP Mercury 20 mm x 4 mm; Eluent A: 11 Wasser + 0.5 ml 50%ige Ameisensäure, Eluent B: 11 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 208-400 nm.

### Allgemeine Arbeitsvorschriften

### Allgemeine Arbeitsvorschrift 1 (Edman^{0,5 und 1,5})

Zu einer Lösung des N-terminal freien Peptids (0.3 mmol) in trockenem Pyridin (30 ml) wird unter Argonschutzgasatmosphäre Phenylisothiocyanat (50 mmol) getropft. Das Reaktionsgemisch wird bei 37°C solange (ca. 1 h) gerührt bis die analytische HPLC-Kontrolle (Methode 13) ausreichenden Umsatz anzeigt (>95%). Das Reaktionsgemisch wird unter Temperaturkontrolle (<40°C) im Vakuum eingeengt und dann lyophilisiert.

### Allgemeine Arbeitsvorschrift 2 (Edman^{1,0 und 2,0})

Unter Argonschutzgasatomosphäre wird der Peptid-Thioharnstoff (0.2 mmol) als Feststoff bei starkem Rühren mit trockener Trifluoressigsäure versetzt und dann bei 40°C solange gerührt (ca. 20 min) bis die analytische HPLC-Kontrolle ausreichenden Umsatz (>95%) anzeigt. Das Reaktionsgemisch wird zügig bei Raumtemperatur (Temperaturkontrolle) im Vakuum eingeengt. Um das Rohprodukt von weiterer Trifluoressigsäure zu befreien, wird das Rohprodukt in Dichlormethan aufgenommen und erneut im Vakuum von Lösungsmittel befreit. Dieser Vorgang wird mehrfach mit Toluol (zweimal) und mit Dichlormethan (zweimal) wiederholt. Abschließend wird das Rohprodukt lyophilisiert.

### Allgemeine Arbeitsvorschrift 3 (Acylierung Edman^{1,0 und 2,0})

Zu einer Lösung des am *N*-Terminus freien Depsipeptids (Amin-Komponente, 1.0 Äquivalente, 4 µmol), der freien Carbonsäure (Carbonsäure-Komponente, 5-20 Äquivalente, 20-80 µmol), HOBt (10-40 Äquivalente, 40-160 µmol) und EDCxHCl (10-25 Äquivalente, 40-100 µmol) in trockenem DMF (1.0 ml) wird bei 0°C zunächst *N*-Methylmorpholin (0.3 Äquivalente, 1.3 µmol) gegeben. Das Reaktiorisgemisch wird gerührt (ca. 15 min) und erneut mit N-Methylmorpholin (0.7 Äquivalente, 2.7 µmol) versetzt. Das Reaktionsgemisch erwärmt sich langsam (3-18 h) auf Raumtemperatur, wobei nahezu vollständiger Umsatz der Aminkomponente mittels HPLC (beispielsweise Methode 13) beobachtet wird. Das Reaktionsgemisch wird im Hochvakuum eingedampft und chromatographisch aufgereinigt.

### Allgemeine Arbeitsvorschrift 4 (Acylierung Edman^{1,0 und 2,0})

Zu einer Lösung des am *N*-Terminus freien Depsipeptids (Amin-Komponente, 1.0 Äquivalente, 88 µmol), der freien Carbonsäure (Carbonsäure-Komponente, 4.0 Äquivalente, 350 µmol) und N-Methylmorpholin (4.0 Äquivalente, 350 µmol) in trockenem DMF (2.0 ml) wird bei 0°C zunächst HATU (4.1 Äquivalente, 360 µmol) gegeben. Das Reaktionsgemisch wird gerührt (ca. 15 min) und erneut mit *N*-Methylmorpholin (5.0 Äquivalente, 438 µmol) versetzt. Das Reaktionsgemisch erwärmt sich langsam (ca. 1 h) auf Raumtemperatur und wird anschließen bis zu vollständigem Umsatz der Amin-Komponente (HPLC-Kontrolle, beispielsweise Methode 13) nachgerührt (ca. 3 h). Das Reaktionsgemisch wird im Hochvakuum eingedampft und chromatographisch aufgereinigt.

### Allgemeine Arbeitsvorschrift 5 (Abspaltung der tert-Butoxycarbonyl-Schutzgruppe)

Das *N*-(*tert*-Butoxycarbonyl)-Peptid (30 µmol) wird als Suspension in wenig Dichlormethan (5 ml) vorgelegt, mit Trifluoressigsäure/Dichlormethan (1/3, 30 ml) versetzt und bei RT gerührt (ca. 40 min) bis die analytische HPLC vollständigen Umsatz anzeigt (beispielsweise Methode 13). Um das Rohprodukt von weiterer Trifluoressigsäure zu befreien wird das Rohprodukt in Dichlormethan aufgenommen und erneut im Vakuum von Lösungsmittel befreit. Dieser Vorgang wird mehrfach mit Toluol (zweimal) und mit Dichlormethan (zweimal) wiederholt. Abschließend wird das Rohprodukt lyophilisiert.

### Allgemeine Arbeitsvorschrift 6 (Peptidkupplung)

Zu einer Lösung der Amin-Komponente (1.0 Äquivalente, 2 mmol), der freien Carbonsäure (Carbonsäure-Komponente, 1.2 Äquivalente, 2.4 mmol), HOBt (4 Äquivalente, 8 mmol) und EDC (2 Äquivalente, 4 mmol) in trockenem Methylenchlorid (75 ml) wird bei -10°C langsam *N*-Methylmorpholin (3 Äquivalente, 6 mmol) gegeben. Das Reaktionsgemisch erwärmt sich langsam (ca. 12 h) auf Raumtemperatur, wobei vollständiger Umsatz der Amin-Komponente mittels HPLC (beispielsweise Methode 13) beobachtet wird. Das Reaktionsgemisch wird im Vakuum eingedampft.

Aufarbeitung Methode 1: Für eine wässrige Aufarbeitung wird das Rohprodukt in Ethylacetat (200 ml) aufgenommen. Anschließend wird dreimal mit ges. wässriger Natriumhydrogencarbonat-Lösung, einmal mit 2 N wässriger Citronensäure, erneut einmal mit ges. wässriger Natriumhydrogencarbonat-Lösung und einmal mit ges. Natriumchlorid-Lösung gewaschen. Man trocknet über Natriumsulfat und filtriert. Im Vakuum wird bis zur Trockne eingedampft und anschließend im Hochvakuum getrocknet.

Aufarbeitung Methode 2: Das Rohprodukt wird in Acetonitril (2 ml) aufgenommen und dann direkt chromatographisch aufgereinigt.

### Allgemeine Arbeitsvorschrift 7 (Hydrolytische Esterspaltung, Verseifung)

Der Carbonsäureester (3 mmol) wird unter Argonschutzgasatmosphäre in THF/Wasser/DMF 200/100/2.5 (20 ml) vorgelegt. Bei 0°C wird unter strikter Temperaturkontrolle gepulvertes Lithiumhydroxid (3.6 mmol, 1.2 Äquivalente) portionsweise in die stark gerührte Lösung gegeben. Wird nach 2 h mittels analytischer HPLC (Methode 13) kein vollständiger Umsatz beobachtet, so wird erneut festes Lithiumhydroxid zugesetzt (3.3 mmol, 1.1 Äquivalente). Dieser Vorgang wird bis zu vollständigem Umsatz wiederholt, woraufhin man das Reaktionsgemisch bei 0°C mit 0.1N wässriger Salzsäure auf pH 3-4 einstellt im Vakuum einengt und anschließend gefriertrocknet. Das Rohprodukt kann nun gelchromatographiert werden (Methode 6; Methanol/0.25% Essigsäure) und/oder mittels präparativer HPLC (Methode 9 oder Methode 14) feingereinigt werden.

### Allgemeine Arbeitsvorschrift 8 (Hydrogenolytische Esterspaltung)

Der peptidische Benzylester (1.2 mmol) wird in Methanol (60 ml) gelöst und unter Argon-schutzgasatmosphäre mit 10proz. Palladium-Kohle (100 mg) versetzt. Bei RT und Normaldruck wird so lange hydriert bis die analytische HPLC (Methode 13) vollständigen Umsatz anzeigt. Das Reaktionsgemisch wird filtriert (z. B. über Kieselgur, Celite^{®}), im Vakuum eingeengt und am Hochvakuum getrocknet.

### Allgemeine Arbeitsvorschrift 9 (Abspaltung der N-tert-Butoxycarbonyl-Schutzgruppe, Dioxan, Chlorwasserstoff)

Die *N*-(*tert*-Butoxycarbonyl)-geschützte Verbindung (1 mmol) wird in Dioxan (2-3 ml) vorgelegt. Bei RT und unter starkem Rühren wird 4 N Salzsäure in Dioxan (30 ml) zugetropft. Man rührt, bis die analytische HPLC (Methode 13) vollständigen Umsatz anzeigt (ca. 2 h). Das Reaktionsgemisch wird bei RT im Vakuum eingedampft. Das Rohprodukt wird in wenig Dichlormethan aufgenommen und erneut im Vakuum von Lösungsmittel befreit. Dieser Vorgang wird mehrfach mit Toluol (zweimal) und mit Dichlormethan (zweimal) wiederholt. Abschließend wird das Rohprodukt lyophilisiert oder direkt weiter umgesetzt.

### Allgemeine Arbeitsvorschrift 10 (Hydrolytische Probenvorbereitung für MALDI-MS)

Das zu öffnende Depsipeptid (z.B. Lysobactin, 0.05 µMol) wird in einem Microvial zunächst mit einem Borat-Salzsäure Puffer (Fa. Merck) pH 8 (250 µl) versetzt. Man lässt über Nacht stehen, versetzt mit Essigsäure (100 µl) und gefriertrocknet die Probe. Das Rohprodukt wird ohne weitere Reinigungsschritte mittels MALDI-MS-Sequenzierung untersucht.

### Allgemeine Arbeitsvorschrift 11 (Peptidkupplung mit Kaliumdihydrogenphosphat-Quench)

Zu einer Lösung des am *N*-Terminus freien Depsipeptids (Amin-Komponente, 1.0 Äquivalente, 88 µmol), der freien Carbonsäure (Carbonsäure-Komponente, 4.0 Äquivalente, 350 µmol) und *N*-Methylmorpholin (4.0 Äquivalente, 350 µmol) in trockenem Dimethylformamid (2.0 ml) wird bei 0°C zunächst HATU (4.1 Äquivalente, 360 µmol) gegeben. Das Reaktionsgemisch wird gerührt (ca. 15 min) und erneut mit *N*-Methylmorpholin (5.0 Äquivalente, 438 µmol) versetzt. Das Reaktionsgemisch erwärmt sich langsam (ca. 1 h) auf Raumtemperatur und wird anschließen bis zu vollständigem Umsatz der Amin-Komponente (HPLC-Kontrolle, Methode 13) nachgerührt (ca. 3 h). Das Reaktionsgemisch wird mit festem Kaliumdihydrogenphosphat (10 Äquivalente, 500 µmol) versetzt und dann im Hochvakuum eingedampft und chromatographisch aufgereinigt.

### Allgemeine Arbeitsvorschrift 12 (Festphasensynthese Dipeptide)

1.0 g Tritylharz (entspricht 1 mmol) in Dichlormethan vorlegen, 5 Äquivalente *N*-Fmoc-geschützte Aminosäure und 10 Äquivalente Ethyldiisopropylamin zugegeben, 20 h bei Raumtemperatur schütteln, absaugen, nachwaschen dreimal mit Dichlormethan/Methanol/Ethyldiisopropylamin (17/2/1), dreimal Dichlormethan, zweimal Dimethylformamid, dreimal Dichlormethan. Trocknen im Hochvakuum über Kaliumhydroxid.

Entschützen mit Piperidin, Dimethylformamid 1/4 (zweimal 15 min), achtmal mit Dimethylformamid nachwaschen. Kupplung von 5 Äquivalente *N*-Boc-geschützten Aminosäure mit 5 Äquivalente TBTU und 10 Äquivalente Ethyldiisopropylamin in Dimethylformamid, Raumtemperatur über Nacht. Waschen dreimal mit Dichlormethan/Methanol/Ethyldiiopropylamin (17/2/1), dreimal Dichlormethan, dreimal Dimethylformamid, zweimal Dichlormethan, einmal Diethylether.

Abspaltung mit je 2.5 ml Essigsäure/Trifluorethanol/Dichlormethan (1/1/3) 2 h Raumtemperatur, dann absaugen, dreimal mit je 500 µl Spaltlösung nachwaschen, mit 2.5 ml Cyclohexan versetzen, einengen, wiederholt mit 5 ml Cyclohexan versetzt einengen.

### Allgemeine Arbeitsvorschrift 13 (Reduktive N-Alkylierung)

Zu einer Mischung von Beispiel 1A (15 mg, 10 µmol) und aktiviertem Molsieb (3 Å, 400 mg) in trockenem Methanol (2 ml) wird unter Argon-Schutzgasatmosphäre wahlweise der entsprechende Aldehyd (0.10 mmol, 10 Äquivalente) oder aber das entsprechende Keton (0.10 mmol, 10 Äquivalente) zugegeben. Das Reaktionsgemisch wird für 30 min bei Raumtemperatur gerührt und dann mit Natriumcyanoborhydrid (6 mg, 0.1 mmol, 10 Äquivalente) versetzt. Nach weiteren 18h wird das Reaktionsgemisch durch Zugabe von 4 N Salzsäure (500 µl) gequencht und dann gefriergetrocknet, wobei man ein festes Rohprodukt erhält, das durch präparative HPLC/UV-Vis (beispielsweise Methode 15) gereinigt wird. Die Produktfraktionen werden erneut gefriergetrocknet, wobei man feste Schäume als Produkte erhält.

### Allgemeine Arbeitsvorschrift 14 (Kupplung zum Dipeptid, EDC)

Eine *N*-geschützte Aminosäure und der Ester (beispielsweise Methyl- oder Benzylester) einer Aminosäure werden in äquimolarem Verhältnis unter Argon in Dichlormethan (40 ml/mmol der Aminosäure) vorgelegt und auf -8°C gekühlt. 1-Hydroxybenzotriazol (3 Äquivalente), *N-*Methylmorpholin (3 Äquivalente), EDC (2 Äquivalente) werden in dieser Reihenfolge zugetropft. Anschließend werden weitere 2 Äquivalente *N*-Methylmorpholin zugesetzt. Man lässt auf Raumtemperatur erwärmen und über 12 h rühren. Das Dichlormethan wird am Rotationsverdampfer abdestilliert, der Rückstand wird in Ethylacetat aufgenommen und mit gesättigter Natriumhydrogencarbonatlösung gewaschen. Die wässrige Phase wird noch einmal mit Ethylacetat gewaschen, die vereinigten organischen Extrakte werden mit wässriger 5%iger Citronensäure und anschließend abermals mit gesättigter Natriumhydrogencarbonatlösung gewaschen. Anschließend wird die organische Phase über Natriumsulfat getrocknet, filtriert und im Vakuum eingeengt.

### Allgemeine Arbeitsvorschrift 15

Eine 1 M Lösung von *N*-Methylmorpholin in Dimethylformamid wird vorbereitet. Eine *N*-geschützte Aminosäure und der Ester (Methyl- oder Benzyl-) einer Aminosäure werden in äquimolarem Verhältnis unter Argon in Dimethylformamid (14 ml/mmol) gelöst und auf 0°C gekühlt. Dann wird zunächst 1 Äquivalent der *N*-Methylmorpholinlösung, dann TCTU (1.5 Äquivalente) und nach 15 min *N*-Methylmorpholinlösung (1 Äquivalent) zugegeben. Dann wird über Nacht bei Raumtemperatur weitergerührt. Der Ansatz wird direkt auf auf eine Reversed-Phase Flash-Kartusche (Biotage 40M C₁₈) aufgegeben und mit Wasser-Acetonitril-Gradienten (10-90% Acetonitril in 15 min, dann 5 min Haltezeit) gereinigt. Das Produkt wird anschließend chromatographiert (Methode 31).

### Allgemeine Arbeitsvorschrift 16

### Eine 1M Lösung von N-Methylmorpholin in Dimethylformamid wird vorbereitet

Eine *N*-geschützte Aminosäure und der Ester (beispielsweise Methyl- oder Benzyl-) einer Aminosäure werden in äquimolarem Verhältnis unter Argon in Dimethylformamid (14 ml/mmol) gelöst und auf 0°C gekühlt. Dann wird zunächst *N*-Methylmorpholin-Lösung (1 Äquivalent), dann HATU (1.5 Äquivalente) und nach 15 min *N*-Methylmorpholin-Lösung (1 Äquivalent) zugegeben. Dann wird über Nacht bei Raumtemperatur weitergerührt. Der Ansatz wird direkt auf auf eine Reversed-Phase Flash-Kartusche (Biotage 40M C₁₈) aufgegeben und mit Wasser-Acetonitril-Gradienten (10-90% Acetonitril in 15 min, dann 5 min Haltezeit) gereinigt.

### Allgemeine Arbeitsvorschrift 17

Der Ester wird in Tetrahydrofuran/Wasser 2/1 (15 ml/mmol) gelöst und die Lösung auf 0°C gekühlt. Festes Lithiumhydroxid-Monohydrat (2 Äquivalente) wird zugegeben. Das Reaktionsgemisch wird etwa 1 h bei 0°C gerührt. Wenn die Umsetzung vollständig ist (Reaktionskontrolle mit HPLC, Methode 36), wird mit Eisessig angesäuert. Das Tetrahydrofuran wird im Vakuum abdestilliert, der Rückstand wird mit Ethylacetat extrahiert und die vereinigten organischen Phasen werden über Natriumsulfat getrocknet und eingeengt. Der Rückstand wird durch Reversed-Phase-Flashchromatographie (Biotage C₁₈ 40M, Wasser + 0.05% Trifluoressigsäure -Acetonitril + 0.05% Trifluoressigsäure-Gradient 10-90% in 15 min, 10 min Haltezeit) gereinigt. Produkthaltige Fraktionen werden am Rotationsverdampfer eingeengt oder lyophilisiert.

### Allgemeine Arbeitsvorschrift 18

Der Benzylester wird in Methanol (36 ml/mmol) gelöst. Dann gibt man unter Argon 10%iges Palladium auf Aktivkohle (228 mg/mmol) zu und hydriert unter hydrostatischem Druck für 1 h. Es wird vom Katalysator abfiltriert, das Filtrat eingeengt und durch Flashchromatographie (Kieselgel, Cyclohexan/Ethylacetat 3/1 mit 0.05% Essigsäure) gereinigt.

### Allgemeine Arbeitsvorschrift 19 (Methode HATU)

Eine 1 M Lösung von N-Methylmorpholin in Dimethylformamid wird vorbereitet. Beispiel 11A (1 Äquivalent) und geschützte Aminosäure (4 Äquivalente) werden in Dimethylformamid (10 ml/mmol Beispiel 11A) vorgelegt und auf 0°C gekühlt. Dann werden zunächst 2 Äquivalente der N-Methylmorpholinlösung, dann HATU (4.1 Äquivalente), dann nach 15 min wieder 2 Äquivalente der *N*-Methylmorpholinlösung und nach 15 min die restlichen 5 Äquivalente der *N*-Methylmorpholinlösung, zugegeben. Dann wird über Nacht bei Raumtemperatur weitergerührt.
Der Ansatz wird anschließend an Sephadex LH-20 chromatographiert (Methode 34) getrennt. Produkthaltige Fraktionen werden vereinigt und am Rotationsverdampfer bei einer Badtemperatur von maximal 30°C eingeengt.

### Allgemeine Arbeitsvorschrift 20

Die *N*-(*tert*-Butoxycarbonyl)-geschützte Verbindung wird in Dichlormethan suspendiert (ca 1.6 ml/10 mg Edukt). Eine Lösung von 30%iger Trifluoressigsäure in Dichlormethan wird zugegeben (ca 0.32 ml/10 mg Edukt) und die Mischung wird 30 min bei Raumtemperatur gerührt. Dann wird das Lösungsmittel im Vakuum abdestilliert, wobei die Badtemperatur 30°C nicht übersteigen soll. Der Rückstand wird chromatographisch gereinigt (Methode 33).

### Allgemeine Arbeitsvorschrift 21

Die *N*-(*tert*-Butoxycarbonyl)-geschützte Verbindung wird in 30%iger Trifluoressigsäure in Dichlormethan (ca 1 ml/10 mg Edukt) gelöst und 30 min bei Raumtemperatur gerührt. Dann wird das Lösungsmittel im Vakuum abdestilliert, wobei die Badtemperatur 30°C nicht übersteigen soll. Der Rückstand wird chromatographisch gereinigt (Methode 33).

### Allgemeine Arbeitsvorschrift 22

Das Edukt wird in Eisessig/Wasser (1/2) aufgenommen, mit 10%igem Palladium auf Aktivkohle (ca. 30 Gewichtsprozent vom Edukt) versetzt und 1 h bei Normaldruck und Raumtemperatur hydriert. Wenn die Reaktionskontrolle mit analytischer HPLC vollständige Umsetzung anzeigt, filtriert man vom Katalysator ab und destilliert das Lösungsmittel im Vakuum ab, wobei die Badtemperatur 30°C nicht übersteigen soll. Der Rückstand wird chromatographisch gereinigt (Methode 33).

### Allgemeine Arbeitsvorschrift 23

Herstellung der Pufferlösung: eine 0.1 M Natriumacetat-Lösung wird durch Zugabe von Eisessig auf pH 5 gebracht.

Das Edukt wird in einer Mischung aus Puffer/Methanol (2/3) gelöst, mit 10%igem Palladium auf Aktivkohle (ca 20 Gewichtsprozent vom Edukt) versetzt und bis zur vollständigen Umsetzung (ca 1 h, Kontrolle mit analytischer HPLC) bei Normaldruck und Raumtemperatur hydriert. Dann filtriert man vom Katalysator ab und destilliert das Lösungsmittel im Vakuum ab, wobei die Badtemperatur 30°C nicht übersteigen soll. Der Rückstand wird chromatographisch gereinigt (Methode 33).

### Ausgangsverbindungen

### Beispiel 1A

D-Leucyl-*N*¹-{(3*S*,6*S*,12*S*,15*S*,18*R*,21*S*,24*S*,27*S*,28*R*)-6-[(1*S*)-2-amino-1-hydroxy-2-oxoethyl]-18-(3-{[amino(imino)methyl]amino}propyl)-12-[(1*S*)-1-hydroxyethyl]-3-(hydroxymethyl)-24-[(1*R*)-1-hydroxy-2-methylpropyl]-21-isobutyl-15-[(1*S*)-1-methylpropyl]-2,5,8,11,14,17,20,23,26-nonaoxo-28-phenyl-1-oxa-4,7,10,13,16,19,22,25-octaazacyclooctacosan-27-yl}-L-leucinamid-bistrifluoracetat

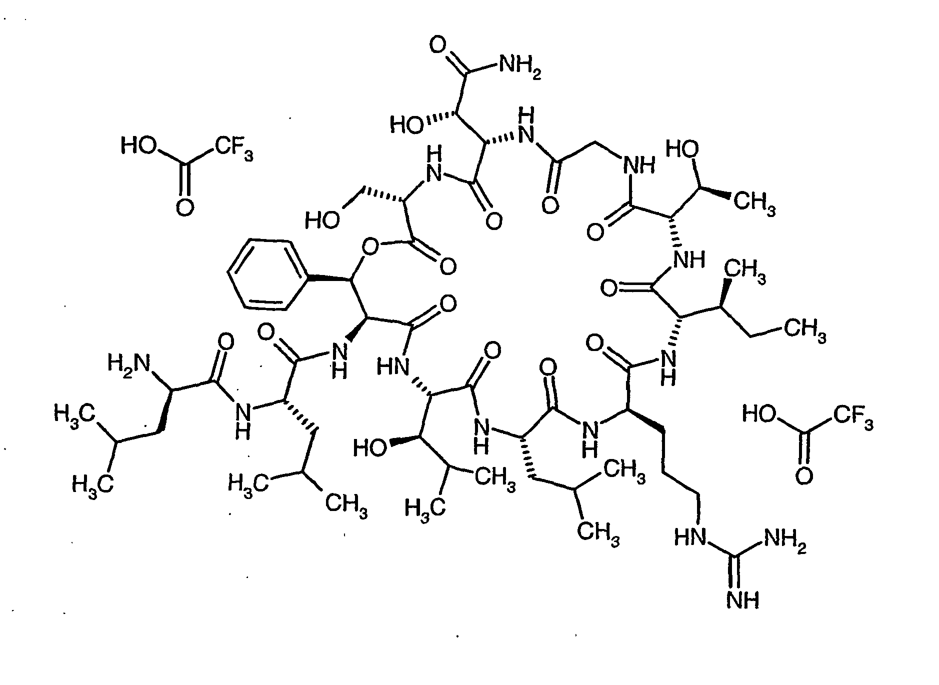

### Beispiel 2A

D-Leucyl-*N*¹-1(3*S*,6*S*,12*S*,15*S*,18*R*,21*S*,24*S*,27*S*,28*R*)-6-[(1*S*)-2-amino-1-hydroxy-2-oxoethyl]-18-(3-{[amino(imino)methyl]amino}propyl)-12-[(1*S*)-1-hydroxyethyl]-3-(hydroxymethyl)-24-[(1*R*)-1-hydroxy-2-methylpropyl]-21-isobutyl-15-isopropyl-2,5,8,11,14,17,20,23,26-nonaoxo-28-phenyl-1-oxa-4,7,10,13,16,19,22,25-octaazacyclooctacosan-27-yl}-L-leucinamid-bistrifluoracetat

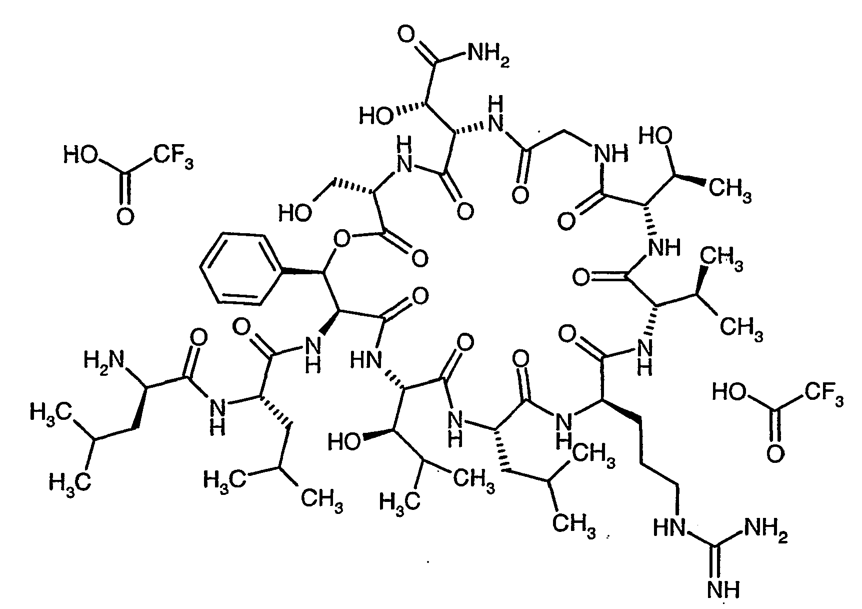

### Fermentation von Beispiel 1A und Beispiel 2A

Kultur Medium:
YM: Hefe-Malz Agar: D-Glucose (4 g/l), Hefe Extrakt (4 g/l), Malz Extrakt (10 g/l), 1 Liter Lewatit Wasser. Vor dem Sterilisieren (20 Minuten bei 121°C) wird der pH auf 7.2 eingestellt.
HPM: Mannit (5.4 g/l), Hefe Extrakt (5 g/l), Fleischpepton (3 g/l).
Arbeitskonserve: Der lyophilisierte Stamm (ATCC 53042) wird in 50 ml YM Medium angezogen.
Kolbenfermentation: 150 ml YM Medium oder 100 ml HPM Medium in einem 1 Erlenmeyerkolben werden mit 2 ml der Arbeitskonserve beimpft und für 30-48 Stunden bei 28°C auf einem Schüttler bei 240 Upm wachsen gelassen.
30 l Fermentation: 300 ml der Kolbenfermentation (HPM Medium) werden zum Animpfen einer sterilen 30 l Nährmedienlösung verwandt (1 ml Antifoam SAG 5693/1). Diese Kultur wird für 21 Stunden bei 28°C, 300 Upm und einer Belüftung mit steriler Luft von 0.3 vvm wachsen gelassen. Der pH wird mit 1 M Salzsäure auf pH = 7.2 konstant gehalten. Insgesamt werden während der Kultivierungszeit 880 ml 1M Salzsäure zugeführt.
Hauptkultur (200 l): 15 x 150 ml YM Medium in 1 l Erlenmeyerkolben werden mit 2 ml der Arbeitskonserve beimpft und bei 28°C für 48 Stunden und 240 Upm auf dem Schüttler wachsen gelassen. 2250 ml dieser Kultur werden zum Beimpfen einer sterilen 200 1 Nährmedienlösung (YM) verwandt (1 ml Antifoam SAG 5693/l) und für 18.5 Stunden bei 28°C, 150 Upm und einer Belüftung mit steriler Luft von 0.3 vvm wachsen gelassen.
Zur Kontrolle des Fermentationsverlaufs werden stündlich Proben (50 ml) entnommen. 2 ml dieser Kulturbrühe werden mit 1 ml Methanol (0.5% Trifluoressigsäure) versetzt und über einen 0.45 µm Filter filtriert. 30 µl dieser Suspension werden mittels HPLC analysiert (Methode 1 und Methode 2).
Nach 18.5 Stunden wird die Kulturbrühe der Hauptkultur bei 17000 Upm in Überstand und Sediment getrennt.

### Isolierung von Beispiel 1A und Beispiel 2A

Der Überstand (183 l) wird mit konzentrierter Trifluoressigsäure bzw. Natronlauge auf pH 6.5-7 eingestellt und auf eine Lewapolsäule (OC 1064, 60 l Inhalt) aufgetragen. Anschließend wird mit reinem Wasser, Wasser/Methanol 1:1 und anschließend mit reinem Methanol (mit 0.1% Trifluoressigsäure) eluiert. Diese organische Phase wird im Vakuum bis zu einem verbleibenden wässrigen Rest von 11.5 l eingeengt.

Die verbleibende wässrige Phase wird an Kieselgel C₁₈ gebunden und aufgetrennt (MPLC, Biotage Flash 75, 75 x 30 cm, KP-C18-WP, 15-20 µm, Fluss: 30 ml; Eluent: Acetonitril/ Wasser mit 0.1% Trifluoressigsäure; Gradient: 10%, 15% and 40% Acetonitril). Die 40% Acetonitril Phase, die die Hauptmenge von Beispiel 1A und Beispiel 2A enthält, wird im Vakuum eingeengt und anschließend lyophilisiert (∼13 g). Dieses Feststoffgemisch wird in 1.2 g Portionen zunächst an einer präparativen HPLC (Methode 3), anschließend durch Gelfiltration an Sephadex LH-20 (5 x 70 cm, Acetonitril/Wasser 1:1, jeweils mit 0.05% Trifluoressigsäure) und einer weiteren präparativen HPLC (Methode 4) getrennt.

Dieser Prozess liefert 2250 mg Beispiel 1A und 33 mg Beispiel 2A.

Das Sediment wird in 4 1 Aceton/Wasser 4:1 aufgenommen, mit 2 kg Celite versetzt, mit Trifluoressigsäure auf pH = 6 eingestellt, ausgerührt und zentrifugiert. Das Lösungsmittel wird im Vakuum eingeengt und der Rückstand gefriergetrocknet. Das erhaltene Lyophilisat (89.9 g) wird in Methanol aufgenommen, abfiltriert, eingeengt und an Kieslegel (Methode 5) in Beispiel 1A und Beispiel 2A aufgetrennt. Beispiel 1A wird danach per Gelfiltration (Sephadex LH-20, 5 x 68 cm, Wasser/Acetonitril 9:1 (mit 0.05% Trifluoressigsäure), Fluss: 2.7 ml/min, Fraktionsgröße 13.5 ml) zur Reinsubstanz aufgereinigt.

Dieser Prozess liefert 447 mg Beispiel 1A.

### Beispiel 1A:

HPLC (Methode 1): Rₜ = 6.19 min

MS (ESIpos): m/z = 1277 (M+H)⁺

¹H NMR (500.13 MHz, d₆-DMSO): δ = 0.75 (d, 3H), 0.78 (d, 6H), 0.80 (t, 3H), 0.82 (d, 3H), 0.90 (d, 3H), 0.91 (d, 3H), 0.92 (d, 3H), 0.95 (d, 3H), 0.96 (d, 3H), 1.05 (m, 1H), 1.19 (d, 3H), 1.25 (m, 2H), 1.50 (m, 4H), 1.51 (m, 2H), 1.55 (m, 1H), 1.61 (m, 1H), 1.65 (m, 1H), 1.84 (m, 1H), 1.85 (m, 1H), 1.86 (m, 1H), 1.89 (m, 1H), 1.95 (m, 1H), 2.75 (m, 2H), 3.40 (m, 1H), 3.52 (m, 2H), 3.53 (dd, 1H), 3.64 (m, 2H), 3.66 (m, 1H), 3.68 (dd, 1H), 3.73 (m, 2H), 4.00 (dd, 1H), 4.02 (br., 1H), 4.13 (br., 1H), 4.32 (dd, 1H), 4.39 (t, 1H), 4.55 (m, 1H), 4.75 (dd, 1H), 5.19 (t, 1H), 5.29 (d, 1H), 5.30 (br., 1H), 5.58 (m, 2H), 6.68 (m, 3H), 6.89 (d, 1H), 6.93 (m, 3H), 6.94 (br., 1H), 6.98 (d, 1H), 7.12 (br., 1H), 7.20 (br., 2H), 7.23 (m, 2H), 7.42 (m, 2H), 7.54 (d, 1H), 7.58 (d, 1H), 8.32 (br., 1H), 9.18 (br., 1H), 9.20 (m, 2H), 9.50 (br., 1H).

¹³C-NMR (125.77 MHz, d₆-DMSO): δ = 10.3, 15.3, 19.0, 19.2, 19.6, 20.0, 20.9, 22.0, 22.4, 23.0, 23.2, 24.3, 24.4, 25.0, 25.4, 26.0, 27.8, 30.9, 35.4, 39.5, 40.8, 40.9, 41.6, 44.1, 51.5, 52.7, 55.9, 56.2, 56.4, 57.9, 58.8, 60.2, 61.1, 62.6, 70.1, 71.6, 71.7, 75.5, 128.1, 128.6, 136.7, 156.8, 168.2, 170.1, 170.4, 171.2, 171.5, 171.9, 172.2, 172.4, 173.7.

Die Zuordnung der Signale erfolgte nach der in der Literatur beschriebenen Zuordnung (T. Kato, H. Hinoo, Y. Terui, J. Antibiot., 1988, 61, 719-725).

¹H NMR (500 MHz, d₆-DMSO, 302 K) und ¹³C-NMR (d₆-DMSO) Daten:

| **Rest** | **NH** | **CO** | **CH-α** | **CH-β** | **CH-χ** | **Weitere Reste** |
|---|---|---|---|---|---|---|
| Leu 1 | 8.32 | 171.5 | 4.13 (51.5) | ∼1.51 (41.6) | ∼1.65 (25.0) | CH₃: 0.95 (22.0) |
| | | | | | | CH₃: 0.92 (22.4) |
| Leu 2 | 9.50 | 172.4 | 3.73 (56.2) | 1.86 (39.5) | 1.89 (24.4) | CH₃: 0.91 (23.0) |
| | | | | 1.25 (39.5) | | CH₃: 0.75 (19.6) |
| Ph-Ser | 9.18 | 171.5 | 5.58 (61.1) | 6.89 (71.7) | - | Ph-o:7.42 (128.1) |
| | | | | | | Ph-m: 7.23 (128.6) |
| | | | | | | Ph-p: 7.20 (128.6) |
| | | | | | | Ph-i: 136.7 |
| Hy-Leu | 9.20 | 171.2 | 3.66 (59.8) | 3.40 (75.5) | 1.84 (30.9) | CH₃: 0.96 (19.0) |
| | | | | -OH: 5.30 | | CH₃: 0.78 (19.2) |
| Leu 3 | 7.12 | 171.5 | 4.02 (52.7) | 1.62 (40.9) | 1.95 (24.3) | CH₃: 0.82 (20.0) |
| | | | | | | CH₃: 0.78 (23.2) |
| Arg | 6.94 | 171.9 | 3.73 (56.4) | ∼1.50 (27.8) | ∼1.50 (26.0) | CH₂-δ:2.75 (40.8) |
| | | | | | ∼1.25 (26.0) | NH-δ: 7.20 |
| | | | | | | [NH-C-NH₃⁺]: |
| | | | | | | 6.68 (156.8) |
| Ile | 7.58 | 170.4 | 3.68 (60.2) | ∼1.85 (35.4) | ∼1.05 (21.4) | CH₃: 0.80 (10.3) |
| | | | | CH₃: 0.90 (15.3) | ∼1.55 (25.4) | |
| Thr | 6.98 | 171.5 | 4.39 (57.2) | 3.52 (70.1) | | CH₃: 1.19 (20.9) |
| | | | | -OH: 5.29 | | |
| Gly | 9.20 | 172.2 | 4.00 (44.1) | - | - | - |
| | | | 3.53 (44.1) | | | |
| Hy-Asn | 7.54 | 170.8 | 4.75 (56.2) | 4.32 (71.6) | - | CO-NH₂: 6.93 |
| | | | | -OH: 5.58 | | (173.7) |
| Ser | 6.93 | 168.2 | 4.55 (55.9) | 3.64 (62.6) | - | - |
| | | | | -OH: 5.19 | | |

### Beispiel 2A:

HPLC (Methode 1): Rₜ = 6.01 min

MS (ESIpos): m/z = 1263 (M+H)⁺

¹H NMR (500.13 MHz, d₆-DMSO): δ = 0.75 (d, 3H), 0.78 (d, 9H), 0.79 (d, 3H), 0.90 (d, 3H), 0.92 (d, 3H), 0.96 (d, 3H), 0.97 (d, 3H), 0.98 (d, 3H), 1.16 (d, 3H), 1.25 (m, 1H), 1.50 (m, 1H), 1.55 (m, 4H), 1.65 (m, 1H), 1.33 (m, 1H), 1.62 (m, 2H), 1.84 (m, 1H), 1.85 (m, 2H), 1.90 (m, 1H), 1.95 (m, 1H), 2.78 (m, 2H), 3.43 (m, 1H), 3.52 (m, 2H), 3.63 (m, 1H), 3.65 (m, 2H), 3.67 (dd, 1H), 3.70 (m, 1H), 3.71 (br., 1H), 4.01 (dd, 1H), 4.04 (br., 2H), 4.29 (t, 1H), 4.30 (dd, 1H), 4.54 (m, 1H), 4.73 (dd, 1H), 5.19 (t, 1H), 5.23 (d, 1H), 5.31 (br., 1H), 5.57 (br., 1H), 5.63 (d, 1H), 6.68 (m, 3H), 6.90 (m, 3H), 6.98 (d, 1H), 7.01 (br., 1H), 7.20 (br., 2M, 7.21 (br., 1H), 7.23 (m, 3H), 7.24 (br., 1H), 7.42 (m, 2H), 7.50 (br., 1H), 7.57 (d, 1H), 8.16 (br., 1H), 9.18 (br., 1H), 9.19 (d, 1H), 9.20 (br., 1H), 9.57 (br., 1H).

¹³C-NMR (125.77 MHz, d₆-DMSO): δ = 19.0, 19.2, 19.3, 19.4, 19.6, 20.9, 21.9, 22.0, 22.4, 23.0, 23.2, 23.7, 24.3, 24.5, 26.8, 27.8, 30.4, 35.4, 31.5, 39.0, 40.3, 40.9, 41.8, 44.3, 52.2, 52.4, 55.2, 55.4, 55.9, 56.5, 57.9, 60.0, 60.8, 61.1, 62.6, 71.1, 71.9, 74.8, 75.1, 127.9, 129.1, 136.1, 156.2, 167.4, 168.1, 171.0, 172.0, 172.2, 172.4, 172.5, 172.9, 173.3.

Die Zuordnung der Signale erfolgte nach der in der Literatur beschriebenen Zuordnung (T. Kato, H. Hinoo, Y. Terui, J. Antibiot.,1988, 61, 719-725).

¹H NMR (500 MHz, *d₆*-DMSO, 302 K) und ¹³C-NMR (*d₆*-DMSO) Daten:

| **Rest** | **NH** | **CO** | **CH-α** | **CH-β** | **CH-χ** | **Weitere Reste** |
|---|---|---|---|---|---|---|
| Leu 1 | 8.16 | 173.3 | 4.04 (52.4) | ∼1.55 (41.8) | ∼1.65 (24.5) | CH₃: 0.98 (22.0) |
| | | | | | | CH₃: 0.92 (22.4) |
| Leu 2 | 9.57 | 172.4 | 3.71 (55.2) | 1.84 (39.0) | 1.90 (23.7) | CH₃: 0.90 (23.0) |
| | | | | 1.33 (39.0) | | CH₃: 0.75 (19.4) |
| Ph-Ser | 9.18 | 172.9 | 5.57 (61.1) | 6.90 (71.7) | - | Ph-o:7.42 (127.9) |
| | | | | | | Ph-m: 7.23 (129.1) |
| | | | | | | Ph-p: 7.23 (129.1) |
| | | | | | | Ph-i: 136.1 |
| Hy-Leu | 9.20 | 173.3 | 3.63 (60.0) | 3.43 (74.8) | 1.85 (31.5) | CH₃: 0.96 (19.0) |
| | | | | -OH: 5.31 | | CH₃: 0.78 (19.2) |
| Leu 3 | 7.21 | 172.5 | 4.04 (52.2) | 1.62 (40.9) | 1.85 (24.3) | CH₃: 0.79 (20.9) |
| | | | | | | CH₃: 0.78 (23.2) |
| Arg | 6.90 | 172.5 | 3.70 (55.4) | ∼1.55 (27.8) | ∼1.50 (26.8) | CH₂-δ:2.78 (40.3) |
| | | | | | ∼1.25 (26.8) | NH-δ: 7.24 |
| | | | | | | [NH-C-NH₃⁺]: |
| | | | | | | 6.68 (156.2) |
| Val | 7.50 | 171.0 | 3.67 (60.8) | 1.95 (30.4) | - | CH₃: 0.97 (19.3) |
| | | | | | | CH₃: 0.78 (19.6) |
| Thr | 6.98 | 172.0 | 4.29 (57.9) | 3.52 (71.1) | | CH₃: 1.16 (21.9) |
| | | | | -OH: 5.23 | | |
| Gly | 9.19 | 172.2 | 4.01 (44.3) | - | - | - |
| | | | 3.52 (44.3) | | | |
| Hy-Asn | 7.57 | 168.1 | 4.73 (56.5) | 4.30 (71.9) | - | CO-NH₂: 7.20 |
| | | | | -OH: 5.63 | | (172.0) |
| Ser | 7.01 | 167.4 | 4.54 (55.9) | 3.65 (62.6) | - | - |
| | | | | -OH: 5.19 | | |

### Bestimmung der Stereochemie von Beispiel 1A

Zur Identifizierung der relativen Stereochemie von Beispiel 1A, wird das Depsipeptid mit Salzsäure hydrolysiert. Dazu werden 100 µg Beispiel 1A mit 20.0 µl 6M Salzsδure versetzt, das Probenröhrchen unter Vakuum zugeschmolzen und bei 166°C für 1 Stunde erhitzt. Nach der Hydrolyse wird die Probe im Hochvakuum eingeengt. Der verbliebene Rückstand wird mit 400 µl Natriumcitratpuffer auf pH = 2.2 eingestellt. Interner Standard: Homoarginin.

Nach der Derivatisierung wird das Hydrolysat an einer Permabond-L-Chirasil-Val- und FS-LipodexE-Säule analysiert. Die Retentionszeit der einzelnen Aminosäurederivate aus Beispiel 1A wird mit D- bzw. L-Aminosäuren (nach Derivatisierung) verglichen. Alle natürlichen Aminosäuren haben L-Konfiguration.

Anhand der großen Übereinstimmung von Beispiel 1A mit den Literaturdaten, wird davon ausgegangen, dass Beispiel 1A in seiner Stereochemie der in der Literatur beschriebenen Stereochemie entspricht (T. Kato, H. Hinoo, Y. Terui, J. Antibiot., 1988, 61 (6), 719-725).

Die bei der Fermentation entstandene Verbindung 1A wird in weiteren Reaktionen eingesetzt (siehe Beispiel 10A). Sollte sich die Zuordnung der Stereochemie in Verbindung 1A tatsächlich anders sein, so ist auch die Stereochemie der Folgeprodukte dementsprechend anders.

### Darstellung der Dipeptide

### Beispiel 3A

### Benzyl-3-cyclopropyl-L-alaninat-hydrochlorid

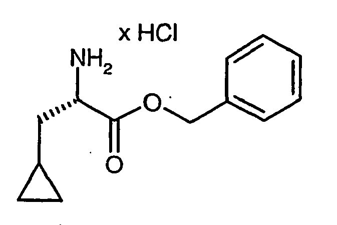

Benzyl-*N*-(*tert*-butoxycarbonyl)-3-cyclopropyl-L-alaninat (260 mg, 0.81 mmol) (Neil W. Boaz, Sheryl D. Debenham, Elaine B. Mackenzie, Shannon E. Large, Org. Lett., 2002, 14, 2421-2424) wird nach der Allgemeinen Arbeitsvorschrift 9 umgesetzt. Das Produkt erhält man in quantitativer Ausbeute.

HPLGL/UV-Vis (Methode 13): Rₜ = 5.13 min, λₘₐₓ (qualitativ) = 220 nm (s), 250-275 (w). LC-MS (Methode 12): Rₜ = 3.93 min; MS (ESIpos.): *m*/*z* (%) = 220 (100) [M + H]⁺.

### Beispiel 4A

### Benzyl-N-(tert-butoxycarbonyl)-D-leucyl-3-cyclopropyl-L-alaninat

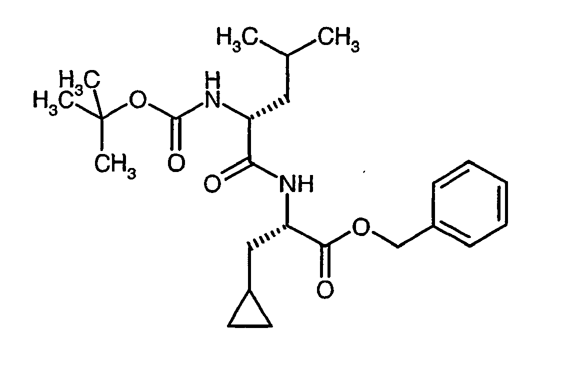

Nach der Allgemeinen Arbeitsvorschrift 6 werden *N*-(*tert*-Butoxycarbonyl)-D-Leucin (540 mg, 2.32 mmol) und Benzyl-3-cyclopropyl-L-alaninat-hydrochlorid (Beispiel 3A, 490 mg, 1.93 mmol) umgesetzt. Das Rohprodukt wird mittels präparativer HPLC (Methode 16) aufgereinigt, wobei man das Produkt in quantitativer Ausbeute erhält.

[α]²⁰_{Na} = + 30° (c = 0.1 in Methylenchlorid). HPLGL/UV-Vis (Methode 13): Rₜ = 9.00 min, λₘₐₓ (qualitativ) = 220 nm (s), 250-275 (w). LC-MS (Methode 17): Rₜ = 3.40 min; MS (ESIpos.): m/z (%) = 333 (100) [M - Boc + H]⁺, 433 (15) [M + H]⁺.

### Beispiel 5A

### N-(tert-Butoxycarbonyl)-D-leucyl-3-cyclopropyl-L-alanin

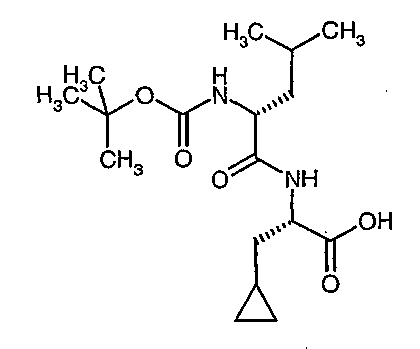

Benzyl-*N*-(*tert*-butoxycarbonyl)-D-leucyl-3-cyclopropyl-L-alaninat (Beispiel 4A, 500 mg, 1.16 mmol) wird nach der Allgemeinen Arbeitsvorschrift 8 umgesetzt. Man erhält 366 mg (92% d. Th.) Produkt.
[a]²⁰_{Na} = + 15° (*c* = 0.1 in Methylenchlorid).
HPLGLTV-Vis (Methode 13): Rₜ = 7.16 min.
LC-MS (Methode 12): Rₜ = 5.28 min;
MS (ESIpos.): m/z (%) = 343 (30) [M + H]⁺.

### Beispiel 6A

### Methyl-N-(tert-butoxycarbonyl)-D-leucyl-L-norvalinat

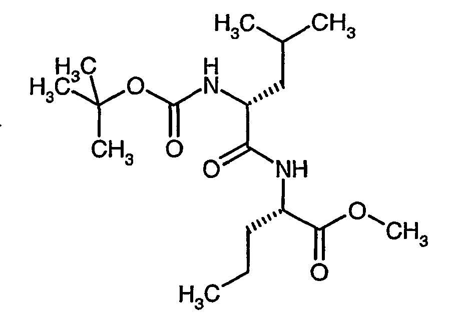

Nach der Allgemeinen Arbeitsvorschrift 6 werden *N-*(*tert*-Butoxycarbonyl)-D-Leucin (1000 mg, 4.32 mmol) und Methyl-L-norvalinat-hydrochlorid (1090 mg, 6.49 mmol) umgesetzt. Das Rohprodukt wird nach der Aufarbeitung Methode 1 gereinigt. Man erhält das Produkt in quantitativer Ausbeute.

HR-TOF-MS (Methode 21): C₁₇H₃₃N₂O₅ ber. 345.2389, gef. 345.2406;
- C₁₇H₃₂N₂O₅Na ber. 367.2209, gef. 367.2207.
LC-MS (Methode 11): Rₜ = 3.62 min;
MS (ESIpos.): m/z (%) = 245 (30) [M - Boc + H]⁺;
MS (ESIneg.): m/z (%) = 389 [M + HCO₂H - H]⁻, 343 (20) [M- H]⁻.

### Beispiel 7A

### N-(tert-Butoxycarbonyl)-D-leucyl-L-norvalin

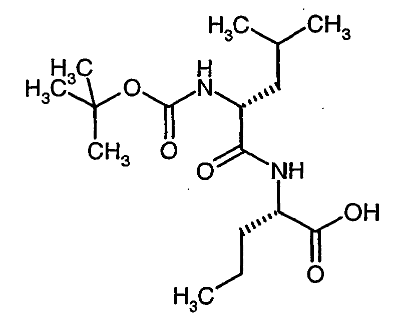

Methyl-*N-*(*tert*-butoxycarbonyl)-D-leucyl-L-norvalinat (Beispiel 6A, 1000 mg, 2.9 mmol) wird nach der Allgemeinen Arbeitsvorschrift 7 umgesetzt. Man erhält 808 mg (84% d. Th.) Produkt.
[α]²⁰_{Na} = + 24° (*c* = 0.1 in Methanol).
HPLC/UV-Vis (Methode 13): Rₜ = 6.97 min.
LC-MS (Methode 12): Rₜ = 5.33 min;
MS (ESIpos.): m/z (%) = 331 (20) [M + H]⁺;
MS (ESIneg.): m/z (%) = 329 (20) [M-H]⁻.
HR-TOF-MS (Methode 21): C₁₆H₃₁N₂O₅ ber. 331.2233, gef. 331.221.

### Beispiel 8A

### Benzyl-N-(tert-butoxycarbonyl)-D-leucyl-3-tert-butyl₋L-alaninat

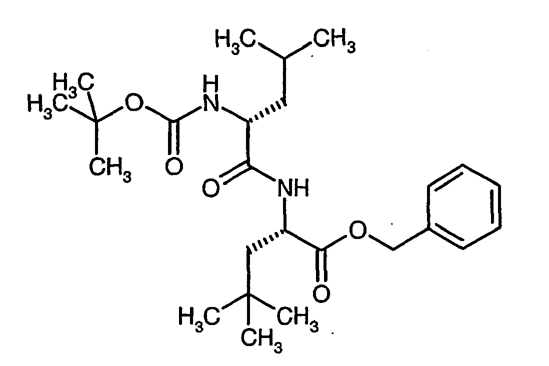

Nach der Allgemeinen Arbeitsvorschrift 6 werden *N-*(*tert*-Butoxycarbonyl)-D-Leucin (230 mg, 1.0 mmol) und Benzyl-3-*tert*-butyl-L-alaninat-hydrochlorid (300 mg, 1.10 mmol) (John X. He, Wayne L. Cody, Annette M. Doherty, J. Org. Chem., 1995, 60, 8262-8266) umgesetzt. Das Rohprodukt wird mittels präparativer HPLC (Methode 16) aufgereinigt (Aufarbeitung Methode 2), wobei man 362 mg (80% d. Th.) Produkt erhält.
[α]²⁰_{Na} = + 19° (*c* = 0.1 in Methylenchlorid).
HPLC/UV-Vis (Methode 18): Rₜ = 5.3 min.
LC-MS (Methode 22): Rₜ = 2.83 min;
MS (ESIpos.): m/z (%) = 349 (100) [M - Boc H]⁺, 449 (85) [M + H]⁺.

### Beispiel 9A

### N-(tert-Butoxycarbonyl)-D-leucyl-3-tert-butyl-L-alanin

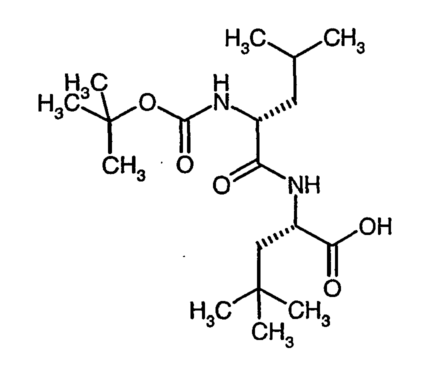

Benzyl-*N*-(*tert*-butoxycarbonyl)-D-leucyl-3-*tert*-butyl-L-alaninat (Beispiel 8A, 308 mg, 0.69 mmol) wird nach der Allgemeinen Arbeitsvorschrift 8 umgesetzt. Man erhält 245 mg (99% d. Th.) Produkt.
[α]²⁰_{Na} = - 2° (*c* = 0.1 in Methylenchlorid)
HPLC/UV-Vis (Methode 13): Rₜ = 7.70 min.
HR-TOF-MS (Methode 21): C₁₈H₃₅N₂O₅ ber. 359.2546, gef. 359.2535.

### Beispiel 10A

### N-(Anilinocarbonothioyl)-D-leucyl-N¹-{(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-L-leucinamid-monotrifluoracetat

### {N-(Anilinocarbonothioyl)lysobactin-monotrifluoracetat}

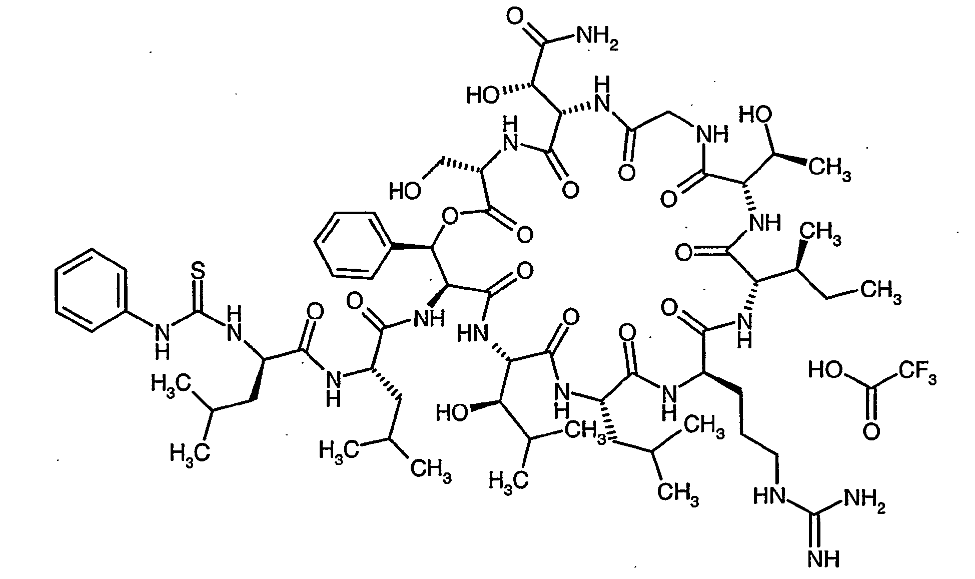

Gemäß der Allgemeinen Arbeitsvorschrift 1 wird Lysobactin-bistrifluoracetat (500 mg, 0.33 mmol) (Beispiel 1A) umgesetzt. Man erhält 600 mg (quant.) Produkt, das ungereinigt weiter umgesetzt werden kann.

Zur weiteren Aufreinigung kann das Rohprodukt gelchromatographiert (Methode 6; Methanol/0.1% Essigsäure) werden. Die Produkt-haltigen Fraktionen werden im Vakuum bei Raumtemperatur eingeengt und dann lyophilisiert. Man erhält das Produkt in 80% Ausbeute.

HPLCIUV-Vis (Methode 13): Rₜ = 6.84 min, λₘₐₓ (qualitativ) = 220 nm (s), 248 (m), 269 (m).
LC-MS (Methode 11): Rₜ = 2.64 min;
MS (ESIpos.): m/z (%) = 706.5 (50) [M + 2H]²⁺, 1412 (20) [M + H]⁺;
LC-MS (Methode 12) : Rₜ = 4.95 min;
MS (ESIpos.): m/z (%) = 1412 (100) [M + H]⁺.

### Beispiel 11A

### N¹-{(3S,6S,12S,15S,18R,21S,24S,27S,28R)-6-[(1S)-2-Amino-1-hydroxy-2-oxoethyl]-18-(3-{[amino(imino)methyl]amino}propyl)-12-[(1S)-1-hydroxyethyl]-3-(hydroxy-methyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-L-leucinamid-bistrifluoracetat

### {Des-D-leucyllysobactin-bistrifluoracetat}

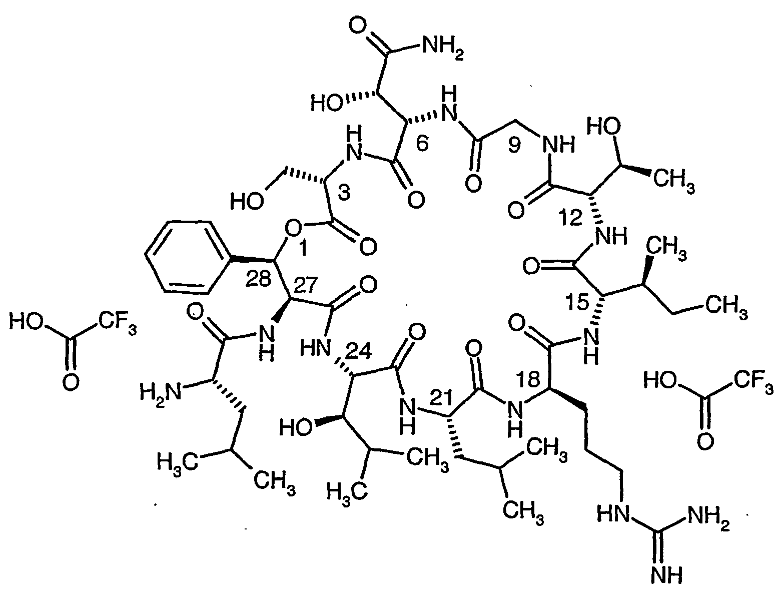

Thioharnstoff (Beispiel 10A) (300 mg, 0.2 mmol) wird gemäß der Allgemeinen Arbeitsvorschrift 2 umgesetzt. Das Rohprodukt wird gelchromatographiert (Methode 6; Methanol/0.25% Essigsäure) und anschließend mittels präparativer HPLC (Methode 8) feingereinigt. Man erhält 147 mg (65% d. Th.) Produkt.

HPLC/UV-Vis (Methode 13): Rₜ = 4.96 min,
λₘₐₓ (qualitativ) = 220 nm (s), 255-270 (w).
LC-MS (Methode 12): Rₜ = 3.84 min;
MS (ESIpos.): m/z (%) = 582.4 (100) [M + 2H]²⁺, 1164 (20) [M + H]⁺.
FT-ICR-HR-MS (Methode 23):
C₅₂H₈₈N₁₄O₁₆ [M + 2H]²⁺ ber. 582.32459, gef. 582.32460;
C₅₂H₈₇N₁₄NaO₁₆ [M + H + Na]²⁺ ber. 593.31556, gef. 593.31564.

Zur Aminosäure-Sequenzbestimmung wird eine analytische Probe des Produkts nach der Allgemeinen Arbeitsvorschrift 10 hydrolysiert.

MALDI-MS (Methode 20): m/z (%) = 1181.7 (100) [M + H]⁺.

### Alternatives Darstellungsverfahren in größerem Maßstab:

Beispiel 2A (6.47 g, 4.30 mmol) wird unter Argonatmosphäre in Pyridin (90 ml) gelöst. Dann wird Phenylisothiocyanat (1.16 g, 8.60 mmol, 2 Äquivalente) zugegeben und die Reaktionsmischung bei 37°C für 1 h gerührt. Anschließend wird das Lösungsmittel am Rotationsverdampfer abdestilliert und der Rückstand über Nacht am Ölpumpenvakuum getrocknet. Man erhält das Zwischenprodukt Beispiel 10A in einer Rohausbeute von 6.60 g. Das Zwischenprodukt wird ohne Reinigung weiter umgesetzt. Dazu wird Beispiel 10A (6.60 g) unter Argonatmosphäre in Trifluoressigsäure (107 ml) gelöst und 30 min bei Raumtemperatur gerührt. Dann wird die Lösung am Rotationsverdampfer unter Vakuum aufkonzentriert, kurz am Ölpumpenvakuum getrocknet, in Methyl-*tert*-butylether (250 ml) aufgenommen und so lange heftig gerührt, bis ein pulvriger amorpher Feststoff entstanden ist. Dieser wird mit Vakuum abfiltriert und mit Methyl-*tert*-butylether (200 ml) gewaschen, dann wird noch mit Dichlormethan (zweimal 100 ml) nachgewaschen. Der Feststoff wird in einen Kolben überführt und am Ölpumpenvakuum getrocknet. Man erhält Beispiel 11A in einer Rohausbeute von 6.0 g (quant.). Das Produkt kann ohne weitere Reinigung umgesetzt werden.

### Beispiel 12A

### N²-(Anilinocarbonothioyl)-N¹-{(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-L-leucinamid-monotrifluoracetat

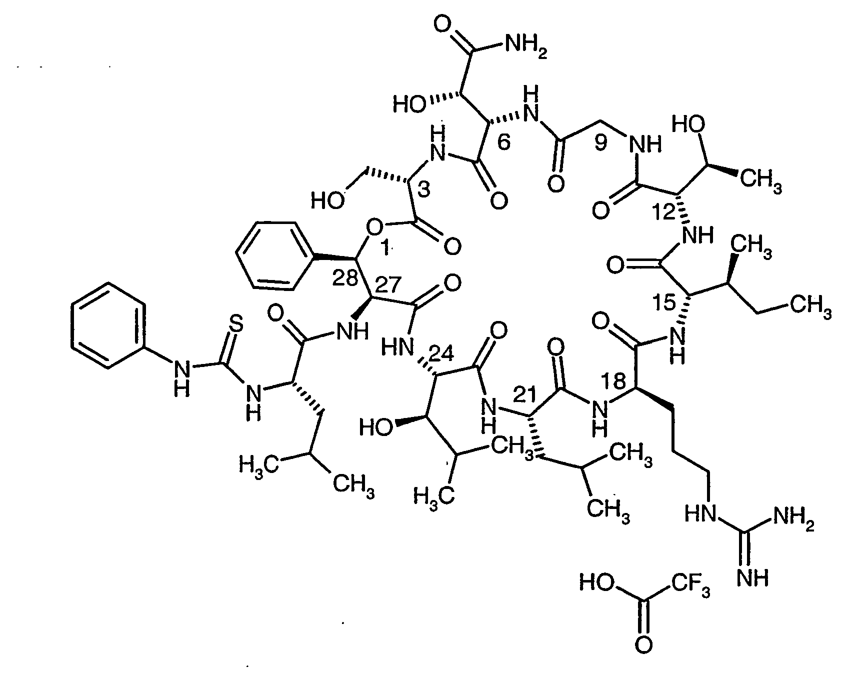

Gemäß der Allgemeinen Arbeitsvorschrift 1 wird Des-D-leucyllysobactin-bistrifluoracetat (Beispiel 11A, 255 mg, 0.18 mmol) umgesetzt. Man erhält 322mg (quant.) Produkt, das ungereinigt weiter umgesetzt werden kann.

Zur weiteren Aufreinigung kann das Rohprodukt gelchromatographiert (Methode 6; Methanol/0.1% Essigsäure) werden. Die Produkt-haltigen Fraktionen werden im Vakuum bei Raumtemperatur eingeengt und dann lyophilisiert.

HPLC/UV-Vis (Methode 13): Rₜ = 6.56 min,
λₘₐₓ (qualitativ) = 220 nm (s), 245 (m), 268 (m).
LC-MS (Methode 12): Rₜ = 4.85 min; MS (ESIpos.): m/z (%) = 1299 (100) [M + H]⁺.

### Beispiel 13A

### (2S)-2-{(3S,6S,12S,15S,18R,21S,24S,27S,28R)-27-Amino-18-(3-{[amino(imino)methyl]amino}-propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-6-yl}-2-hydroxyethanamid-bistrifluoracetat

### {Des(1-D-leucyl-2-L-leucyl)lysobactin-bistrifluoracetat}

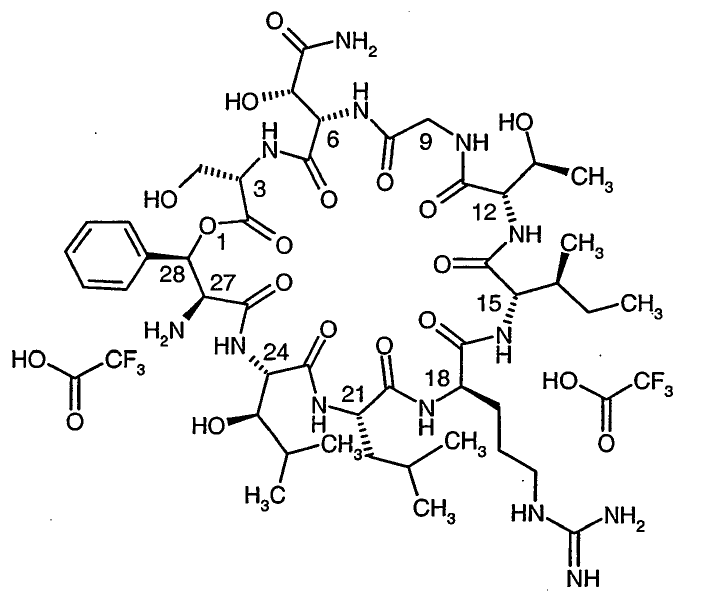

Der Thioharnstoff (Beispiel 12A, 66 mg, 34 µmol) wird gemäß der Allgemeinen Arbeitsvorschrift 2 umgesetzt. Das Rohprodukt kann durch zügige Gelchromatographie (Methode 6; Methanol/0.25% Essigsäure) vorgereinigt werden. Präparative HPLC (Methode 8 oder Methode 9 gefolgt von anschließendem Umsalzen des Chromatographieprodukts durch Zusatz von TFA (100 µmol)) liefert 45 mg (75% d. Th.) Produkt.

HPLC/UV-Vis (Methode 13): Rₜ = 4.71 min,
λₘₐₓ (qualitativ) = 220 nm (s), 255-270 (w). LC-MS (Methode 11): Rₜ = 1.65 min;
MS (ESIpos.): m/z (%) = 526 (100) [M + 2H]²⁺, 1051 (15) [M + H]⁺.

### Alternatives Darstellungsverfahren in größerem Maßstab:

Beispielverbindung 11A (6.47 g, 4.30 mmol) wird unter Argonatmosphäre in Pyridin (92 ml) gelöst. Dann wird Phenylisothiocyanat (8.75 g, 64.68 mmol, 15 Äquivalente) zugegeben und die Reaktionsmischung bei 37°C für 1 Stunde gerührt. Anschließend wird das Lösungsmittel am Rotationsverdampfer abdestilliert und der Rückstand über Nacht am Ölpumpenvakuum getrocknet. Man erhält Beispiel 12A in einer Rohausbeute von 6.0 g. Das Zwischenprodukt wird ohne Reinigung weiter umgesetzt. Dazu wird das rohe Beispiel 12A unter Argonatmosphäre in Trifluoressigsäure (82 ml) gelöst und 30 min bei Raumtemperatur gerührt. Dann wird die Lösung am Rotationsverdampfer unter Vakuum aufkonzentriert, kurz am Ölpumpenvakuum getrocknet, in Methyl-*tert*-butylether (250 ml) aufgenommen und so lange heftig gerührt, bis ein pulvriger amorpher Feststoff entstanden ist. Dieser wird mit Vakuum abfiltriert und mit weiterem Methyl-*tert*-butylether (200. ml) gewaschen, dann wird noch mit zwei Portionen je 100 ml Dichlormethan nachgewaschen. Der Feststoff wird in einen Kolben überführt und am Ölpumpenvakuum getrocknet. Man erhält die Titelverbindung in einer Rohausbeute von 5.4 g (quant.). Das Produkt wird durch präparative HPLC weiter aufgereinigt (Methode 31). Man erhält 1.79 g der Titelverbindung (32% d. Th.).

### Beispiel 14A

### N-(tert-Butoxycarbonyl)-D-leucyl-N¹-{(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyaooctacosan-27-yl}-3-cyclopropyl-L-alaninamid-monotrifluoracetat

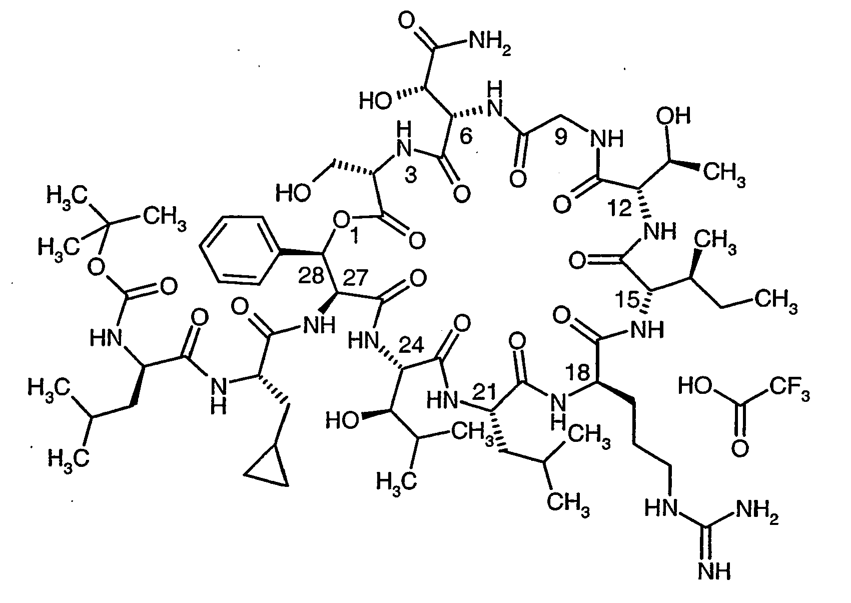

Des(1-D-leucyl-2-L-leucyl)lysobactin-bistrifluoracetat (Beispiel 13A, 112 mg, 88 µmol) und *N-*(*tert*-Butoxycarbonyl)-D-leucyl-3-cyclopropyl-L-alanin (Beispiel 5A, 120 mg, 350 µmol) werden gemäß der Allgemeinen Arbeitsvorschrift 4 umgesetzt. Das Rohprodukt wird durch präparative HPLC feingereinigt (Methode 8; bzw. Methode 7 gefolgt von anschließendem Umsalzen des Chromatographieprodukts durch Zusatz von TFA (200 µmol)). Man erhält 87 mg (63% d. Th.) Produkt.

HPLC/UV-Vis (Methode 13): Rₜ = 7.04 min,
λₘₐₓ (qualitativ) = 220 nm (s), 255-270 (w).
LC-MS (Methode 11): Rₜ = 2.61 min;
MS (ESIpos.): m/z (%) = 638 (100) [M - Boc + 2H]²⁺, 1375 (15) [M + H]⁺.
LC-MS (Methode 12): Rₜ = 5.27 min; MS (ESIpos.): m/z (%) = 638 (30) [M - Boc + 2H]²⁺, 1375 (100) [M +H]⁺.

### Beispiel 15A

### N-(tert-Butoxycarbonyl)-D-leucyl-N¹-{(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20, 23,26-nonaoxo-28-phenyl-1-oxa-4,7,10,13,16,19,22,25-octaazacyclooctacosan-27-yl}-L-norvalinamid-monotrifluoracetat

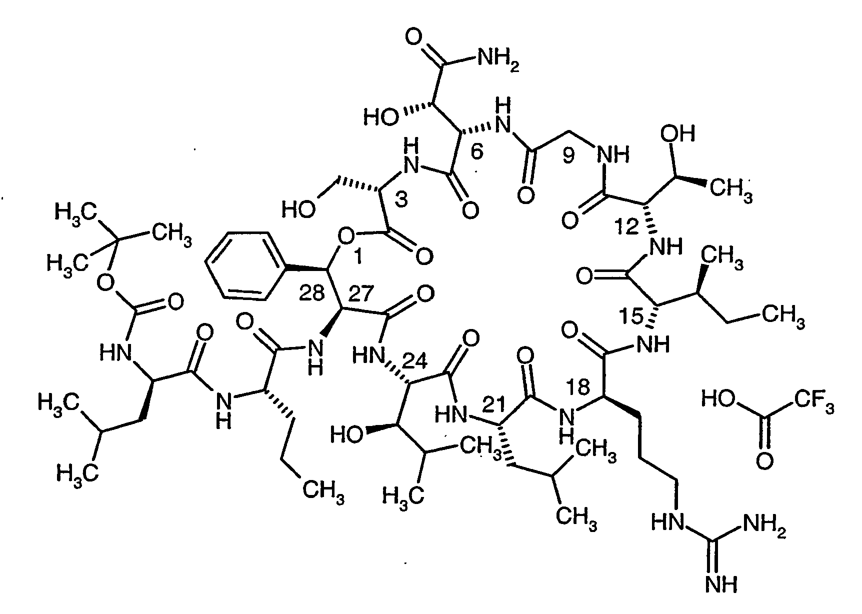

Des(1-D-leucyl-2-L-leucyl)lysobactin-bistrifluoracetat (Beispiel 13A, 5 mg, 4 µmol) und *N-*(*tert-*Butoxycarbonyl)-D-leucyl-L-norvalin (Beispiel 7A, 5 mg, 16 µmol) werden gemäß der Allgemeinen Arbeitsvorschrift 4 umgesetzt. Das Rohprodukt wird durch präparative HPLC feingereinigt (Methode 10; bzw. Methode 9 gefolgt von anschließendem Umsalzen des Chromategraphieprodukts durch Zusatz von TFA (10 µmol)). Man erhält 2.7 mg (51% d. Th.) Produkt.

HPLC/UV-Vis (Methode 13): Rₜ = 6.97 min,
λₘₐₓ (qualitativ) = 220 nm (s), 255-270 (w).
LC-MS (Methode 11): Rₜ = 2.49 min;
MS (ESIpos.): m/z (%) = 632 (100) [M - Boc + 2H]²⁺, 1363 (10) [M + H]⁺.

### Beispiel 16A

### N-(tert-Butoxycarbonyl)-D-leucyl-N¹-{(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-3-tert-butyl-L-alaninamid-monotrifluoracetat

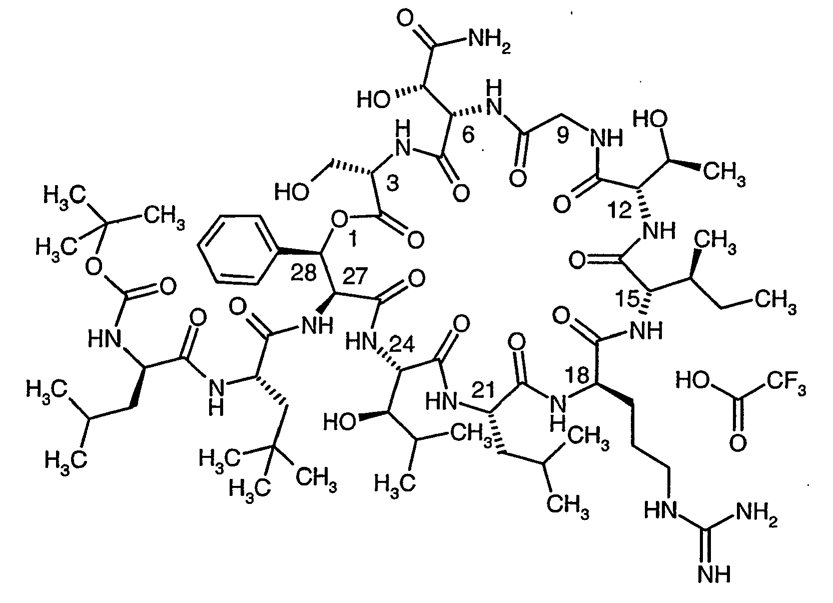

Des(1-D-leucyl-2-L-leucyl)lysobactin-bistrifluoracetat (Beispiel 13A, 21 mg, 14 µmol) und *N-*(*tert*-Butoxycarbonyl)-D-leucyl-3-*tert*-butyl-L-alanin (Beispiel 9A, 24 mg, 66 µmol) werden gemäß der Allgemeinen Arbeitsvorschrift 4 umgesetzt. Das Rohprodukt wird durch präparative HPLC feingereinigt (Methode 15; bzw. Methode 14 gefolgt von anschließendem Umsalzen des Chromatographieprodukts durch Zusatz von TFA (30 µmol)). Man erhält 19 mg (75% d. Th.) Produkt.

HPLGLTV-Vis (Methode 13): Rₜ = 7.31 min,
λₘₐₓ (qualitativ) = 220 nm (s), 255-270 (w).
LC-MS (Methode 11): Rₜ = 2.59 min;
MS (ESIpos.): m/z (%) = 646 (100) [M-Boc + 2H]²⁺, 1391 (20) [M + H]⁺.

### Beispiel 17A

### N-(tert-Butoxycarbonyl)-L-leucyl-N¹-{(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-L-leucinamid-monotrifluoracetat

### {N-(tert-Butoxycarbonyl)-epi-Lysobactin-monotrifluoracetat}

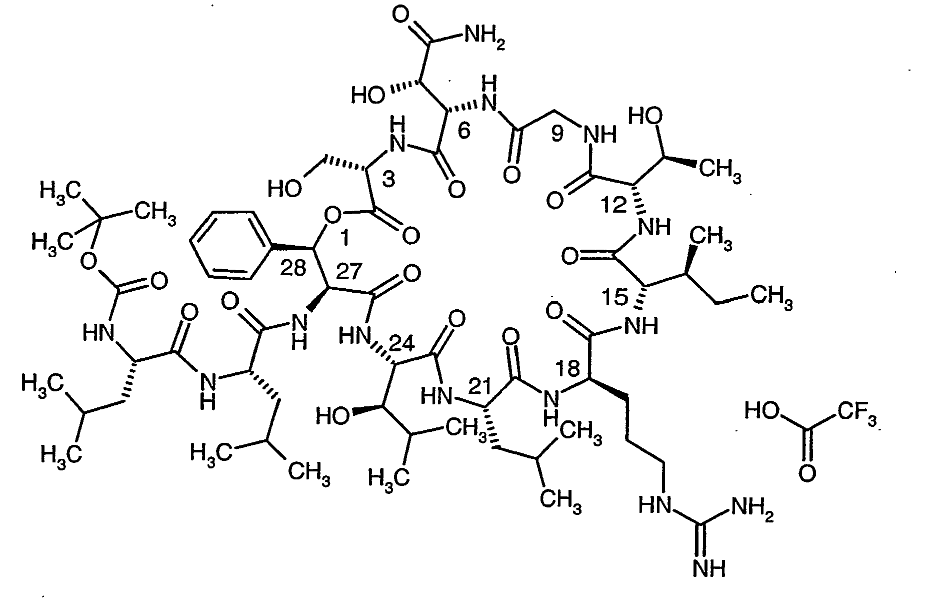

Analog der Allgemeinen Arbeitsvorschrift 3 wird Des-D-leucyllysobactin-bistrifluoracetat (Beispiel 11A, 2.2 mg, 2 µmol) mit *N*-(*tert*-Butoxycarbonyl)-leucin (7.9 mg, 32 µmol) umgesetzt. Das Rohprodukt wird gelchromatographiert (Methode 6; Methanol/0.25% Essigsäure) und anschließend mittels präparativer HPLC (Methode 14 gefolgt von anschließendem Umsalzen des Chromatographieprodukts durch Zusatz von TFA (10 µmol)) feingereinigt. Nach Gefriertrocknung der Produktfraktionen erhält man 1.5 mg (64% d. Th.) Produkt.

HPLC/UV-Vis (Methode 13): Rₜ = 6.97 min,
X. (qualitativ) = 220 nm (s), 255-270 (w).
LC-MS (Methode 12): Rₜ = 4.76 min;
MS (ESIpos.): m/z (%) = 1377 (100) [M + H]⁺;
MS (ESIneg.): m/z (%) = 1375 (100) [M - H]⁻.

### Beispiel 18A

### N²-{4-[(tert-Butoxycarbonyl)amino]butanoyl}-N¹-{(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-L-leucinamid-monotrifluoracetat

### [{4-[(tert-Butoxycarbonyl)amino]butanoyl}-des(leucyl)lysobactin-monotrifluoracetat]

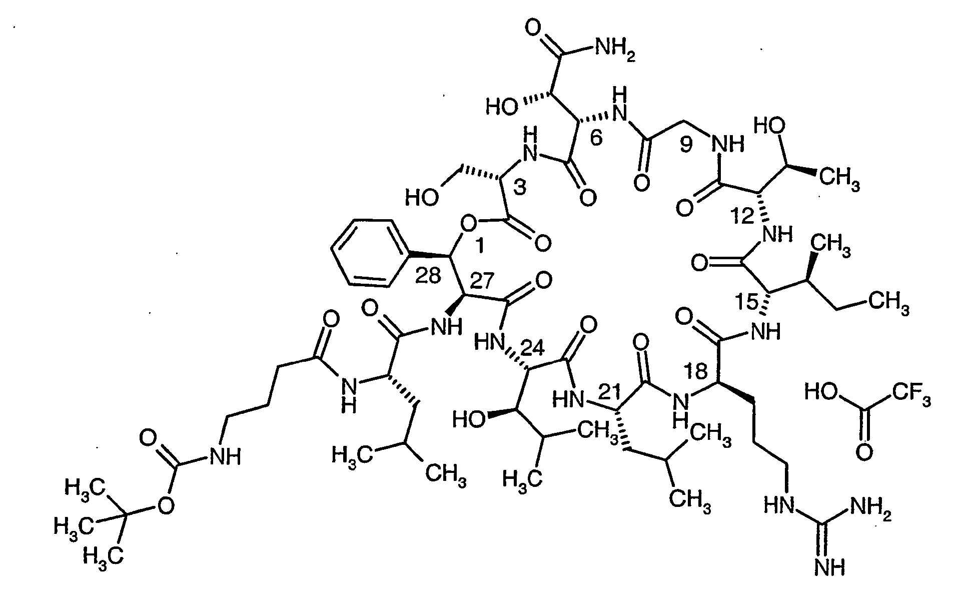

Analog der Allgemeinen Arbeitsvorschrift 3 wird Des-D-leucyllysobactin-bistrifluoracetat (Beispiel 11A, 4.0 mg, 3 µmol) mit 4-[(*tert*-Butoxycarbonyl)amino]-butansäure (11.7 mg, 57 µmol) umgesetzt. Das Rohprodukt wird gelchromatographiert (Methode 6; Methanol/0.25% Essigsäure) und anschließend mittels präparativer HPLC (Methode 14 gefolgt von anschließendem Umsalzen des Chromatographieprodukts durch Zusatz von TFA (10 µmol)) feingereinigt. Nach Gefriertrocknung der Produktfraktionen erhält man 2.3 mg (59% d. Th.) Produkt.

HPLC/UV-Vis (Methode 13): Rₜ = 6.67 min,
λₘₐₓ (qualitativ) = 220 nm (s), 255-270 (w).
LC-MS (Methode 12): Rₜ = 4.76 min;
MS (ESIpos.): m/z (%) = 1349 (100) [M + H]⁺;
MS (ESIneg.): m/z (%) = 1346 (100) [M - H]⁻.

### Beispiel 19A

### N-(tert-Butoxycarbonyl)-N-methyl-D-leucyl-N¹-{(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-L-leucinamid-monotrifluoracetat

### {N-(tert-Butoxycarbonyl)-N-methyl-lysobactin-monotrifluoracetat}

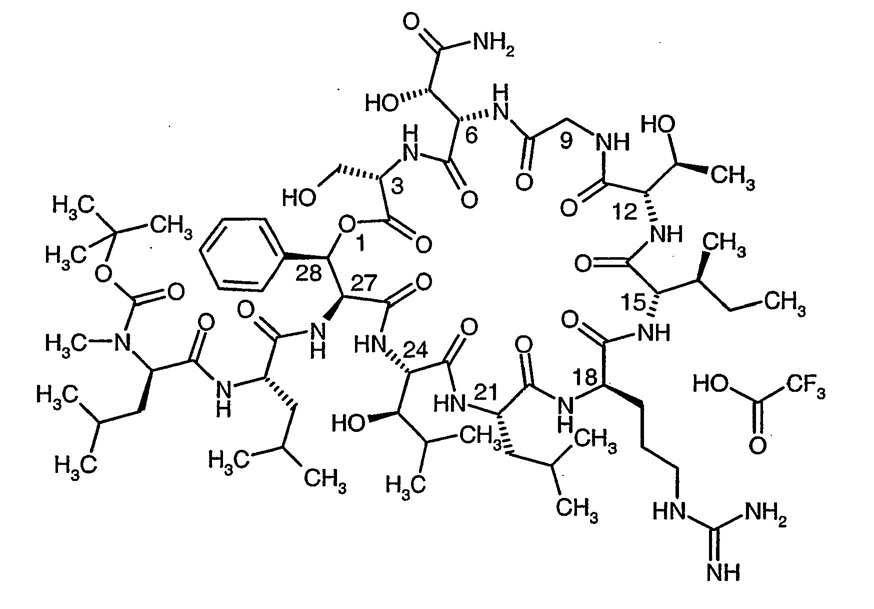

Analog der Allgemeinen Arbeitsvorschrift 3 wird Des-D-leucyllysobactin-bistrifluoracetat (Beispiel 11A, 5.0 mg, 4 µmol) mit *N*-(*tert*-Butoxycarbonyl)-*N*-methyl-D-Leucin-hydrat (17.6 mg, 72 µmol) umgesetzt. Das Rohprodukt wird gelchromatographiert (Methode 6; Methanol/0.25% Essigsäure) und anschließend mittels präparativer HPLC (Methode 14 gefolgt von anschließendem Umsalzen des Chromatographieprodukts durch Zusatz von TFA (10 µmol)) feingereinigt. Nach Gefriertrocknung der Produktfraktionen erhält man 2.0 mg (40% d. Th.) Produkt.

HPLC/UV-Vis (Methode 13): Rₜ = 7.41 min,
λₘₐₓ (qualitativ) = 220 nm (s), 255-270 (w).
LC-MS (Methode 12): Rₜ = 5.11 min;
MS (ESIpos.): m/z (%) =1391 (100) [M + H]⁺;
MS (ESIneg.): m/z (%) = 1389 (100) [M - H]⁻.

### Beispiel 20A

### N²-{6-[(tert-Butoxycarbonyl)amino]hexanoyl}-N¹-{(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-L-leucinamid-monotrifluoracetat

### [N-{6-[(tert-Butoxycarbonyl)amino]hexanoyl}-des(leucyl)lysobactin-monotrifluoracetat]

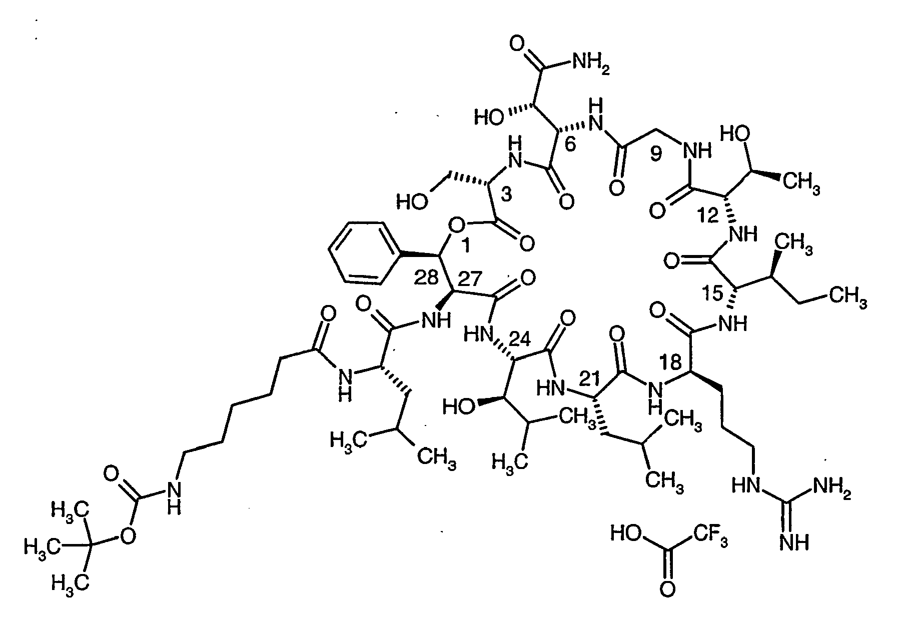

Analog der Allgemeinen Arbeitsvorschrift 3 wird Des-D-leucyllysobactin-bistrifluoracetat (Beispiel 11A, 5.0mag, 4 µmol) mit 6-[(*tert*-Butoxycarbonyl)amino]hexansäure (4.2 mg, 18 µmol) umgesetzt. Das Rohprodukt wird gelchromatographiert (Methode 6; Methanol/0.25% Essigsäure) und anschließend mittels präparativer HPLC (Methode 14 gefolgt von anschließendem Umsalzen des Chromatographieprodukts durch Zusatz von TFA (10 µmol)) feingereinigt. Nach Gefriertrocknung der Produktfraktionen erhält man 900 µg (18% d. Th.) Produkt.

LC-MS (Methode 11): Rₜ = 2.57 min;
MS (ESIpos.): m/z (%) = 639 (100) [M - Boc + 2H]²⁺, 1377 (10) [M + H]⁺;
MS (ESIneg.): m/z (%) = 1375 (100) [M - H]⁻.

### Beispiel 21A

### N²-{3-[(tert-Butoxycarbonyl)amino]propionyl}-N¹-{(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-L-leucinamid-monotrifluoracetat

### [N-{3-[(tert-Butoxycarbonyl)amino]propionyl)-des(leucyl)lysobactin-monotrifluoracetat]

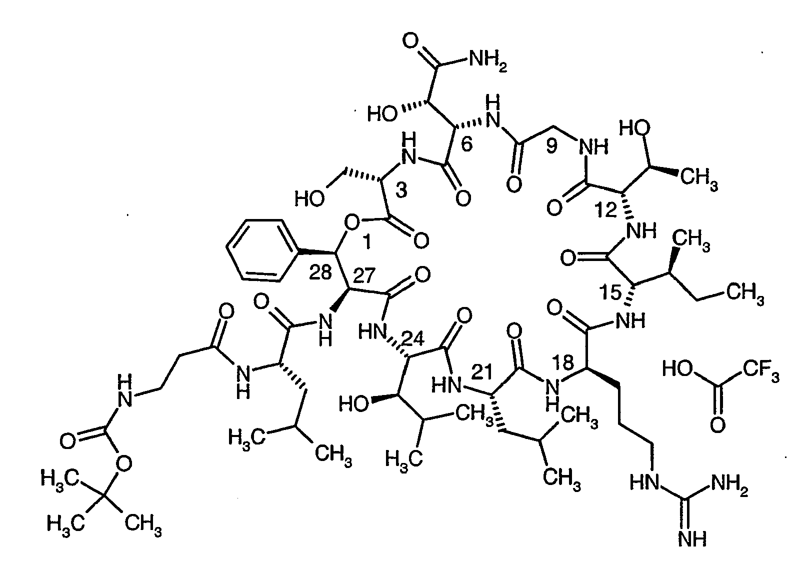

Analog der Allgemeinen Arbeitsvorschrift 3 wird Des-D-leucyllysobactin-bistrifluoracetat (Beispiel 11A, 4.4mg, 4 µmol) mit 3-[(*tert*-Butoxycarbonyl)amino]-propionsäure (700 µg, 20 µmol) umgesetzt. Das Rohprodukt wird gelchromatographiert (Methode 6; Methanol/0.25% Essigsäure) und anschließend mittels präparativer HPLC (Methode 14 gefolgt von anschließendem Umsalzen des Chromatographieprodukts durch Zusatz von TFA (10 µmol)) feingereinigt. Nach Gefriertrocknung der Produktfraktionen erhält man 2.0 mg (40% d. Th.) Produkt.

EPLC/UV-Vis (Methode 13): Rₜ = 6.45 min,
λₘₐₓ (qualitativ) = 220 nm (s), 255-270 (w).
LC-MS (Methode 12): Rₜ = 4.73 min;
MS (ESIpos.): m/z (%) = 1335.6 (100) [M + H]⁺;
MS (ESIneg.): m/z (%) = 1333 (100) [M - H]⁻.

### Beispiel 22A

### N²-({1-[(tert-Butoxycarbonyl)amino]cyclopropyl}carbonyl)-N¹-{(3S,6S,12S,15S,18R,21S,24S,-27S,28R)-6-[(1S)-2-amino-1-hydroxy-2-oxoethyl]-18-(3-{[amino-(imino)methyl]amino}propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclo-octacosan-27-yl -L-leucinamid-monotrifluoracetat

### [N-({1-[(tert-Butoxycarbonyl)amino]cyclopropyl}carbonyl)-des(leucyl)lysobactin-monotrifluoracetat]

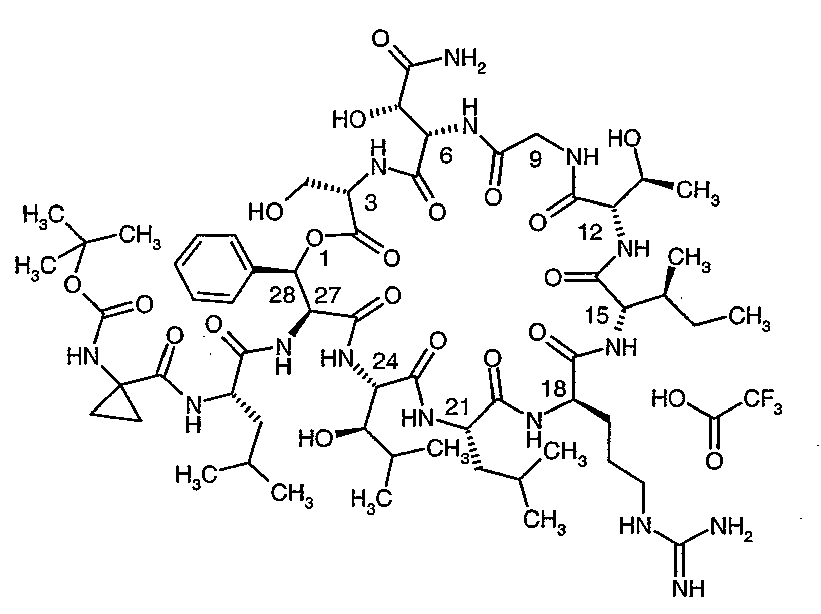

Analog der Allgemeinen Arbeitsvorschrift 3 wird Des-D-leucyllysobactin-bistrifluoracetat (Beispiel 11A, 5.0 mg, 4 µmol) mit 1-[(*tert-*Butoxycarbonyl)amino]cyclopropancarbonsäure (3.6 mg, 18 µmol) umgesetzt. Das Rohprodukt wird gelchromatographiert (Methode 6; Methanol/0.25% Essigsäure) und anschließend mittels präparativer HPLC (Methode 14 gefolgt von anschließendem Umsalzen des Chromatographieprodukts durch Zusatz von TFA (10 µmol)) feingereinigt. Nach Gefriertrocknung der Produktfraktionen erhält man 650 µg (13% d. Th.) Produkt.

LC-MS (Methode 11): Rₜ = 2.54 min;
MS (ESIpos.): m/z (%) = 624 (100) [M - Boc + 2H]²⁺, 1346 (20) [M + H]⁺;
MS (ESIneg.): m/z (%) = 1345 (100) [M - H]⁻.

### Beispiel 23A

### N-[(Benzyloxy)carbonyl]-3-[(tert-butoxycarbonyl)amino]-L-alanyl-N¹-{(3S,6S,12S,15S,18R,-21S,24S,27S,28R)-6-[(1S)-2-amino-1-hydroxy-2-oxoethyl]-18-(3-{[amino-(imino)methyl]amino}-propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13-,16,19,22,25-octaazacyclo-octacosan-27-yl}-L-leucinamid-monotrifluoracetat

### [N-[(Benzyloxy)carbonyl]-3-[(tert-butoxycarbonyl)amino]-L-alanyl}-des(leucyl)lysobactin-monotrifluoracetat]

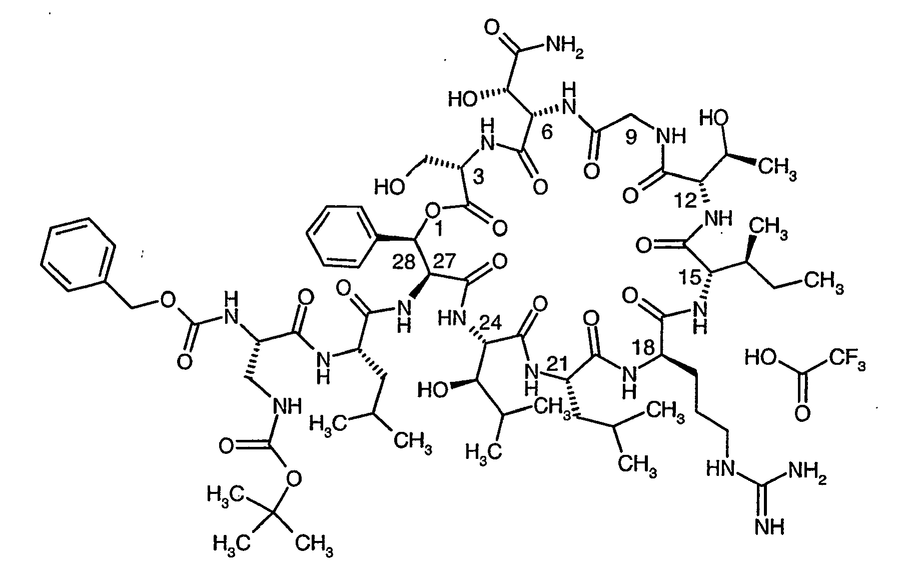

Analog der Allgemeinen Arbeitsvorschrift 3 wird Des-D-leucyllysobactin-bistrifluoracetat (Beispiel 11A, 1.9 mg, 1.3 µmol) mit *N*-[(Benzyloxy)carbonyl]-3-[(*tert*-butoxycarbonyl)amino]-L-alanin (2.3 mg, 6.7 µmol) umgesetzt. Das Rohprodukt wird gelchromatographiert (Methode 6; Methanol/0.25% Essigsäure) und anschließend mittels präparativer HPLC (Methode 14 gefolgt von anschließendem Umsalzen des Chromatographieprodukts durch Zusatz von TFA (10 µmol)) feingereinigt. Nach Gefriertrocknung der Produktfraktionen erhält man 900 µg (46% d. Th.) Produkts.

LC-MS (Methode 19): Rₜ = 2.79 min;
MS (ESIpos.): m/z (%) = 693 (100) [M-Boc + 2H]²⁺, 1484 (5) [M + H]⁺;
MS (ESIneg.): m/z (%) = 628 (100), 1482 (60) [M - H]⁻.

### Beispiel 24A

### N-(tert-Butoxycarbonyl)-D-leucyl-N¹-{(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-L-leucinamid-monotrifluoracetat

### {N-(tert-Butoxycarbonyl)-lysobactin-monotrifluoracetat}

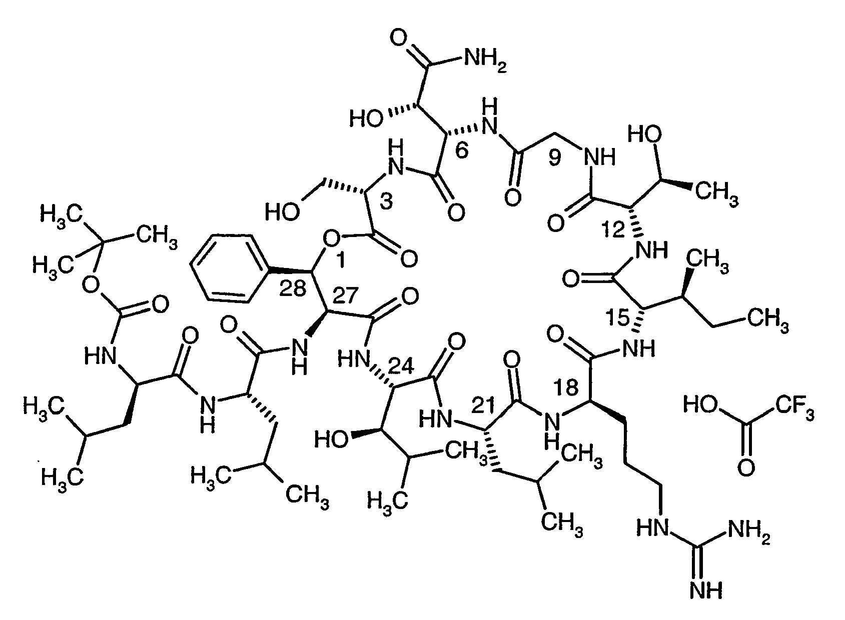

Analog der Allgemeinen Arbeitsvorschrift 3 wird Des-D-leucyllysobactin-bistrifluoracetat (Beispiel 11A, 4.4 mg, 4 µmol) mit *N-*(*tert*-Butoxycarbonyl)-D-Leucin-hydrat (16 mg, 64 µmol) umgesetzt. Das Rohprodukt wird gelchromatographiert (Methode 6; Methanol/0.25% Essigsäure) und anschließend mittels präparativer HPLC (Methode 14 gefolgt von anschließendem Umsalzen des Chromatographieprodukts durch Zusatz von TFA (20 µmol)) feingereinigt. Nach Gefriertrocknung der Produktfraktionen erhält man 1.3 mg (27% d. Th.) Produkt.

IHPLCIUV-Vis (Methode 13): Rₜ = 7.07 min,
λₘₐₓ (qualitativ) = 220 nm (s), 255-270 (w).
LC-MS (Methode 12): Rₜ = 5.01 min;
MS (ESIpos.): m/z (%) =1377 (100) [M + H]⁺;
MS (ESIneg.): m/z (%) = 1375 (100) [M - H]⁻.

### Aminosäuren und Derivate

### Beispiel 25A und Beispiel 26A

### (2R)-N-(tert-Butoxycarbonyl)-3-(trimethylsilyl)alanin und (2S)-N-(tert-Butoxycarbonyl)-3-(trimethylsilyl)alanin

Die Synthese erfolgt nach M. Merget, K. Günther, M. Bernd, E. Günther, R. Tacke, J. Organomet. Chem. 2001 628, 183-194. Die Trennung der Enantiomere erfolgt durch präparative HPLC an chiraler Phase:

Gilson Abimed HPLC, UV-Detektor 212 nm, Säule: Daicel Chiralpak AD-H 5 µm; 250 x 20 mm; Fluss: 15 ml/min; EluentA: *iso*-Hexan, EluentB: 0.2% Essigsäure/1% Wasser/2-Propanol; isokratisch.

### Beispiel 25A (2R Verbindung)

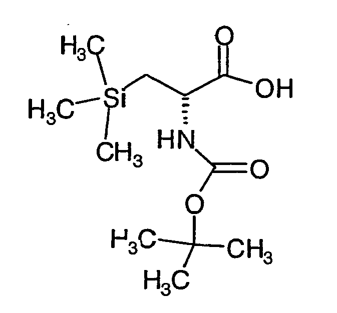

Präparative HPLC: Rₜ = 4.16 min
[α]_{D}²⁰= +1.1 (*c* = 0.83, Methanol)

### Beispiel 26A (2S Verbindung)

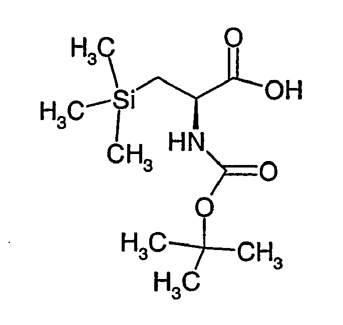

Präparative HPLC: Rₜ = 9.27 min
[α]_{D}²⁰ =-1.6 (*c* = 0.66, Methanol)

### Beispiel 27A

### (2Z)-2-{[(Benzyloxy)carbonyl]aminol-3-(1-methylcyclohexyl)acrylsäure-methylester

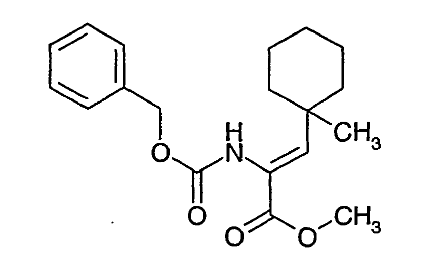

1-Methylcyclohexancarbaldehyd (2.66 g, 21.08 mmol) und Methyl{[(benzyloxy)carbonyl]-amino}(dimethoxyphosphoryl)acetat (6.63 g, 20.02mmol., 0.95 Äquivalente) werden in 75 ml Tetrahydrofuran gelöst und auf -78°C gekühlt. Bei -78°C wird *N,N,N,N*-Tetramethylguanidin (27.92 g, 0.24 mol, 11.5 Äquivalente) zugetropft und dann 15 min bei -78°C, anschließend 4 Tage bei Raumtemperatur gerührt. Dann wird mit Ethylacetat (zweimal 100 ml) gegen Wasser ausgeschüttelt, die vereinigten organischen Phasen mit gesättigter Natriumchloridlösung gewaschen und über Natriumsulfat getrocknet. Nach dem Einengen wird das Rohprodukt chromatographiert (Biotage 40M, Cyclohexan/Ethylacetat 6/1, 27.5 ml/min). Es werden 0.91 g (13% d. Th.) der Titelverbindung erhalten.

LC-MS (Methode 26): Rₜ = 2.74 min,
MS (ESIpos.): *m*/*z* (%) = 332.3 (70) [M + H]⁺.

### Beispiel 28A

### N-[(Benzyloxy)carbonyl]-3-(1-methylcyclohexyl)-D-alanin-methylester

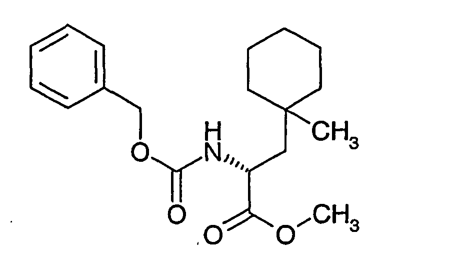

Beispielverbindung 27A (310 mg, 0.94 mmol) wird in Ethanol p.a. (60 ml) gelöst. Mit einer Kanüle wird etwa 5 min Argon hindurchgeleitet, dann wird (-)-1,2-Bis-[(2*R*,5*R*)diethylphospho-lano]benzen(cyclooctadien)rhodium(I)-Triflat (2.7 mg, 4 µmol, 0.004 Äquivalente) zugegeben und im Ultraschallbad gelöst. Es wird über 3 Tage bei 3 bar Wasserstoffdruck und Raumtemperatur hydriert. Der Ansatz wird mit einer weiteren Portion (2.7 mg, 4 µmol, 0.004 Äquivalente) des Katalysators versetzt und einen weiteren Tage bei 3 bar Wasserstoffdruck hydriert. Die Zugabe von Katalysator wird in Abständen von 24 h wiederholt, bis die Umsetzung vollständig ist. Die Reaktionskontrolle erfolgt über LC-MS (Methode 35). Dann wird über Kieselgel filtriert (Ethylacetat) und das Eluat eingeengt. Ausbeute: 181 mg (58% d. Th.) der Titelverbindung.

¹H NMR (300 MHz, CDCl₃): δ = 0.94 (s, 3H), 1.23-1.55 (m, 11H), 1.78 (dd, 1H), 3.71 (s, 3H), 4.42 (m, 1H), 5.02 (m, 1H), 5.11 (s, 1H), 7.36 (m, 5H).
LC-MS (Methode 35): Rₜ = 2.85 min,
MS (ESIpos.): *m*/*z* (%) = 334 (25) [M + H]⁺.
MS (DCI): *m*/*z* (%) = 351 (100) [M + NH₄]⁺.

### Beispiel 29A

### N-[(Benzyloxy)carbonyl]-3-(1-methylcyclohexyl)-D-alanin

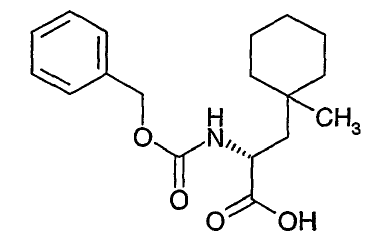

Die Beispielverbindung 28 A (180 mg, 0.54 mmol) wird in THF (3 ml) gelöst. Bei 0°C wird Lithiumhydroxid (2 M in Wasser, 0.59 ml, 1.19 mmol, 2.2 Äquivalente) zugetropft und dann über 4 h bei 0°C gerührt. Dann wird über Nacht bei 7°C stehengelassen. Der Ansatz wird unter Eiskühlung mit Trifluoressigsäure (0.12 ml, 1.62 mmol, 3 Äquivalente) versetzt, mit Ethylacetat extrahiert und der organische Extrakt über Natriumsulfat getrocknet. Das Rohprodukt wird mit präparativer HPLC (Methode 31) gereinigt. Ausbeute: 135 mg (78% d. Th.) der Titelverbindung.

¹H NMR (300 MHz, CDCl₃): δ = 0.95 (s, 3H), 1.20-1.54 (m, 11H), 1.90 (m, 1H), 4.43 (m, 1H), 5.01 (d, 1H), 5.13 (s, 2H), 7.28-7.40 (m, 5H).
HPLC (Methode 18): Rₜ = 4.8 min.
MS (Methode ESI: *m*/*z* (%) = 318.1 (4), [M - H]⁻.

### Beispiel 30A

### (2Z)-2-{[(Benzyloxy)carbonyl]amino}-3-(4-isopropylphenyl)acrylsäure-methylester

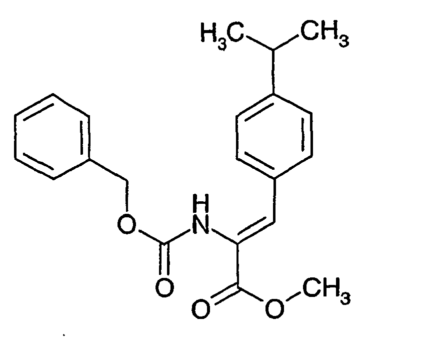

4-Isopropylbenzaldehyd (2.00 g, 13.50 mmol) und Methyl{[(benzyloxy)carbonyl]amino}-(dimethoxyphosphoryl)acetat (3.58 g, 10.80 mmol, 0.8 Äquivalente) werden in Tetrahydrofuran (20 ml) gelöst und auf -78°C gekühlt. Bei -78°C wird *N,N,N,N*-Tetramethylguanidin (27.92 g, 0.24 mol, 11.5 Äquivalente) zugetropft und dann 3 h bei -78°C, anschließend 3 Tage bei Raumtemperatur gerührt. Dann wird mit Ethylacetat (zweimal 100 ml) gegen Wasser ausgeschüttelt, die vereinigten organischen Phasen werden mit gesättigter Natriumchloridlösung gewaschen und es wird über Natriumsulfat getrocknet. Nach dem Einengen wird das Rohprodukt chromatographiert (Biotage 40M, Cyclohexan/Ethylacetat 5/1). Es werden 3.47 g (73% d. Th.) der Titelverbindung erhalten.

¹H NMR (300 MHz, CDCl₃): δ = (1.25 (d, 6H), 2.90 (sept, 1H), 3.80 (s, 3H), 5.13 (s, 2H), 6.22 (br s, 1H), 7.20 (d, 2H), 7.37 (m, 5H), 7.46 (d, 2H).
HPLC (Methode 28): Rₜ = 5.10 min.
MS (DCI): *m*/*z* (%) = 371.1 (100) [M + NH₄]⁺.

### Beispiel 31A

### (N-Benzyloxycarbonyl)-4-isopropyl-D-phenylalanin-methylester

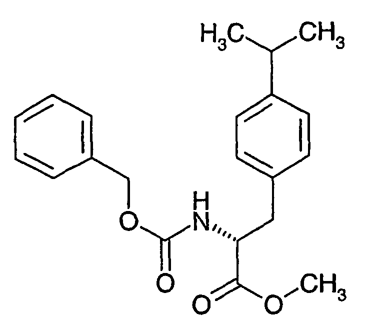

Die Beispielverbindung Beispiel 30A (3.47 g, 9.82 mmol) wird in Ethanol p.a. (60 ml) gelöst. Mit einer Kanüle wird etwa 10 min Argon hindurchgeleitet, dann wird (-)-1,2-Bis-((2*R*,5*R*)Diethyl-phospholano)benzen(cyclooctadien)rhodium(1)-Triflat (28 mg, 39 µmol, 0.004 Äquivalente) zugegeben und im Ultraschallbad gelöst. Es wird über 3 Tage bei 3 bar und Raumtemperatur hydriert. Der Ansatz wird mit einer weiteren Portion (28 mg, 39 µmol, 0.004 Äquivalente) des Katalysators versetzt und einen weiteren Tag bei 3 bar hydriert. Die Zugabe von Katalysator wird in Abständen von 24 h wiederholt, bis die Umsetzung vollständig ist. Die Reaktionskontrolle erfolgt über LC-MS (Methode 26). Dann wird über Kieselgel filtriert (Ethylacetat) und das Eluat eingeengt. Ausbeute: 3.27 g (89% d. Th.) der Titelverbindung.

¹H NMR (300 MHz, CDCl₃): δ = 1.21 (d, 6H), 2.87 (sept, 1H), 3.08 (d, 1H), 3.68 (s, 3H), 4.63 (m, 1H), 5.10 (s, 2H), 5.20 (m, 1H), 7.01 (d, 2H), 7.13 (d, 2H), 7.42 (m, 5H).
HPLC (Methode 36): Rₜ = 3.88 min.
LC-MS (Methode 26): Rₜ = 2.84 min,
MS (ESIpos.): *m*/*z* (%) = 356 (15) [M + H]⁺.
MS (DCI): *m*/*z* (%) = 373 (100) [M + NH₄]⁺.

### Beispiel 32A

### (N-Benzyloxycarbonyl)-4-isopropyl-D-phenylalanin

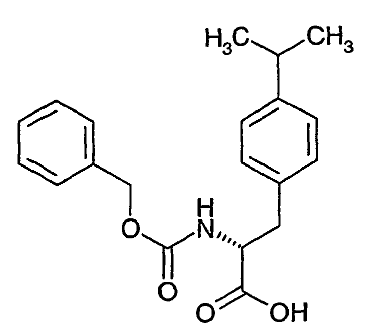

Die Beispielverbindung 31A (240 mg, 0.68 mmol) wird in Tetrahydrofuran (3 ml) gelöst. Bei 0°C wird Lithiumhydroxid (2 M in Wasser, 0.74 ml, 1.49 mmol, 2.2 Äquivalente) zugetropft und dann über 1 h bei 0°C gerührt. Der Ansatz wird unter Eiskühlung mit Trifluoressigsäure (0.16 ml, 2.03 mmol, 3 Äquivalente) versetzt, mit Ethylacetat extrahiert und der organische Extrakt über Natriumsulfat getrocknet. Das Rohprodukt wird mit präparativer HPLC (Methode 31) gereinigt. Ausbeute: 86% d. Th.

¹H NMR (200 MHz, CDCl₃): δ = 1.22 (d, 6H), 2.87 (sept, 1H), 3.13 (m, 2H), 4.69 (m, 1H), 5.10 (s, 2H), 5.18 (s,1H), 7.05 (d, 2H), 7.15 (d, 2H), 7.36 (m, 5H).
HPLC (Methode 36): Rₜ = 3.64 min.
MS (DCI): *m*/*z* (%) = 359.1 (100), [M + NH₄]⁺.

### Dipeptidester

### Beispiel 33A

### N-(tert-Butoxycarbonyl)-3-tert-butyl-D-alanyl-3-tert-butyl-L-alanin-benzylester

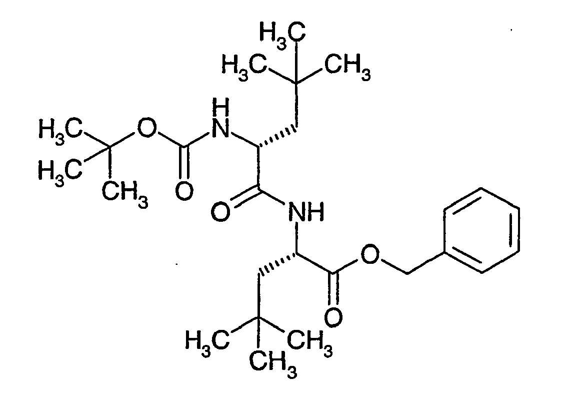

Nach der Allgemeinen Arbeitsvorschrift 6 (hier 5 Äquivalente *N-*Methylmorpholin) werden *N-*(*tert*-Butoxycarbonyl)-3-*tert*-butyl-D-alanin (3.28 g, 13.4 mmol) und Benzyl-3-*tert*-butyl-L-alaninat-hydrochlorid (4.0 g, 14.7 mmol; J. X. He, W. L. Cody, A. M. Doherty, J. Org. Chem. 1995, 60, 8262-8266) umgesetzt. Nach wässriger Aufarbeitung erhält man 6.0 g Produkt (97% d. Th.). Das Produkt kann mittels präparativer HPLC (Methode 16) feingereinigt werden.

¹H NMR (400 MHz, *d₆*-DMSO): δ = 0.85 (s, 9H, tBu), 0.86 (s, 9H, tBu), 1.31 (s, 9H, OtBu), 1.35 (m, 2H, β-CH₂), 1.55 (m, 2H, β-CH₂), 3.98 (m, 1H, α-CH), 4.22 (m, 1H, α-CH), 5.04 (d, *J* = 1.8 Hz, 2H, CH₂Ph), 6.72 (d, *J* = 9.2Hz, 1H, NH), 7.25-7.35 (m, 5H, Ph), 7.94 (d, *J* = 7.8 Hz, 1H, NH).
[α]²⁰_{Na} = + 17° (*c* = 0.1 in Methylenchlorid).
HPLC/UV-Vis (Methode 13): Rₜ = 9.6 min,
λₘₐₓ (qualitativ) = 220 nm (s), 250-275 (w).
LC-MS (Methode 26): Rₜ = 3.09 min;
MS (ESIpos.): *m*/*z* (%) = 363 (30) [M - Boc + H]⁺, 463 (100) [M + H]⁺, 926 (50) [2M + H]⁺.
HR-TOF-MS (Methode 21): C₂₆H₄₃N₂O₅ ber. 463.3172, gef. 463.3185 [M + H]⁺.

### Beispiel 34A

### N-(tert-Butoxycarbonyl)-3-(tert-butyl)-D-alanyl-3-(3-pyridyl)-L-alanin-methylester

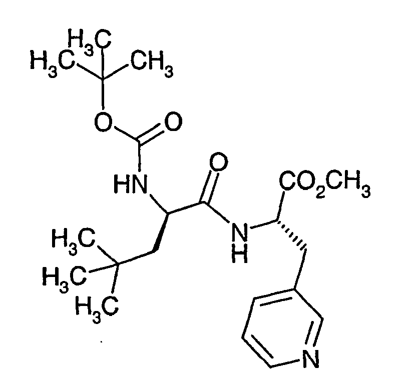

Zu einer Lösung *N-*(*tert*-Butoxycarbonyl)-3-*tert*-butyl-D-alanin (1.0 Äquivalente, 1.2 mmol) und 3-(3-Pyridyl)-L-alanin-methylester (1.0 Äquivalente, 1.2 mmol) in trockenem Dichlormethan (3 ml) werden bei -30°C langsam *N-*Methylmorpholin (5 Äquivalente, 6.0 mmol), EDC (2.5 Äquivalente, 3.0 mmol) und HOBt (2.5 Äquivalente, 3.0 mmol) gegeben. Das Reaktionsgemisch erwärmt sich langsam (ca. 12 h) auf Raumtemperatur, wobei vollständiger Umsatz mittels HPLC (Methode 36) beobachtet wird. Zur Aufarbeitung wird das Reaktionsgemisch mit Dichlormethan (20 ml) verdünnt und mit gesättigter Natriumhydrogencarbonat-Lösung (10 ml) gewaschen. Die organische Phase wird mittels Magnesiumsulfat getrocknet, filtriert und eingeengt. Das Rohprodukt wird mittels Flashchromatographie (Kieselgel, Gradient Dichlormethan 100% bis Dichlormethan/Methanol: 4/1) aufgereinigt, wobei man 466 mg (96% d. Th.) Produkt erhält.

¹H NMR (400 MHz, *d₆*-DMSO): δ = 0.78 (s, 9H, tBu), 1.17 (m, 2H, β-CH₂), 1.36 (s, 9H, OtBu), 3.00 (m, 2H, β-CH₂), 3.64 (s, 3H, OMe), 3.98 (m, 1H, α-CH), 4.49 (m, 1H, α-CH), 6.80 (d, *J* = 7.0 Hz, 1H, NH), 7.26 (dd, *J* = 4.0, 6.5 Hz, 1H, PyrH), 7.63 (d, *J* = 6.5 Hz, 1H, PyrH), 8.27 (d, *J*= 6.5 Hz, 1H, NH), 8.40 (d, *J* = 4.0 Hz, 1H, PyrH), 8.42 (s, 1H, PyrH).
[α]²⁰_{Na} = +5° (*c* = 0.19 in Methanol).
HPLC/UV-Vis (Methode 28): Rₜ = 4.0 min.
HPLC/UV-Vis (Methode 36): Rₜ = 3.80 min.
¹³C NMR (500 MHz, *d*₆-DMSO): δ = 172.9, 171.6, 154.7, 150.2, 147.7, 136.6, 132.6, 123.1, 77.8, 52.6, 51.9, 51.4, 44.9, 33.6, 30.0, 29.2, 28.1.
LC-MS (Methode 26): Rₜ = 1.75 min;
MS (ESIpos.): *m*/*z* (%) = 308 (60), 352 (100), 408 (100) [M + H]⁺;
MS (ESIneg.): *m*/*z* (%) = 332 (100), 406 (5) [M - H]⁻.
HR-TOF-MS (Methode 21): C₂₁H₃₄N₃O₅ [M + H]⁺ ber. 408.2498, gef. 408.2458.

**Tabelle 1: Dipeptidester**

| **Bsp.Nr.** | **Struktur Name** | **Analytische Daten** |
|---|---|---|
| | | **Herstellungsmethode** |
| 35A | 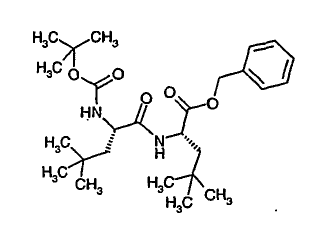 | [α]²⁰_{Na} =-44° (*c* = 0.1 in Methylenchlorid). HPLC/UV-Vis (Methode 13): Rₜ = 9.5 min. |
| | | ¹H NMR (400 MHz, *d₆*-DMSO): δ = 0.81 (s, 9H, tBu), 0.83 (s, 9H, tBu), 1.31 (s, 9H, OtBu), 1.36 (m, 2H, β-CH₂), 1.57 (m, 2H,β-CH₂), 3.99 (m, 1H, α-CH), 4.22 (m, 1H, α-CH), 5.04 (d, *J* = 1.8 Hz, 2H, CH₂Ph), 6.72 (d, *J* = 9.2 Hz, 1H, NH, 7.29 (m, 5H, Ph), 7.95 (d, *J* = 7.8 Hz, 1H, NH). |
| | | HR-TOF-MS (Methode 21): C₂₆H₄₃N₂O₅ [M + H]⁺ ber. 463.3172, gef. 463.3196. |
| | *N-tert*-Butoxycarbonyl-3-*tert*-butyl-L-alanyl-3-*tert*-butyl-L-alanin-benzylester | Allg. Arbeitsvorschrift 6 (hier 5 Äquivalente *N*-Methylmorpholin) aus *N*-*tert*-Butoxycarbonyl-3-*tert*-butyl-L-alanin (0.8 mmol) und 3-*tert*-Butyl-L-alanin-benzylester-hydrochlorid. Ausbeute: 95% d. Th. |
| 36A | 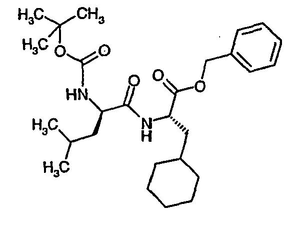 | [α]²⁰_{Na} = +16° (*c* = 0.1 in Methylenchlorid). |
| | | HPLC/UV-Vis (Methode 13): Rₜ = 9.9 min. |
| | | HR-TOF-MS (Methode 21): C₂₇H₄₃N₂O₅ [M + H]⁺ ber. 475.3172, gef. 475.3162. |
| | *N-tert*-Butoxycarbonyl-D-leucyl-3-cyclohexyl-L-alanin-benzylester | Allg. Arbeitsvorschrift 6 (hier 5 Äquivalente *N-*Methylmorpholin) aus *N-tert*-Butoxycarbonyl-D-leucin (0.97 mmol) und 3-Cyclohexyl-L-alanin-benzylester-hydrochlorid. |
| | | Ausbeute: 90% d. Th. |
| 37A | 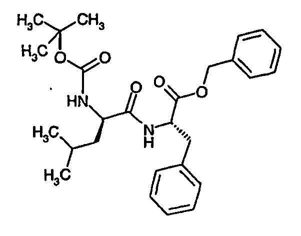 | HPLC/UV-Vis (Methode 13): Rₜ, = 9.07 min. |
| | | LC-MS (Methode 26): Rₜ = 2.94 min; |
| | | MS (ESIpos.): *m*/*z* (%) = 369 (50) [M- Boc + H]⁺, 469 (100) [M + H]⁺. |
| | *N-tert*-Butoxycarbonyl-D-leucyl-L-phenylalanin-benzylester | Allg. Arbeitsvorschrift 6 (hier 5 Äquivalente *N-*Methylmorpholin) aus *N-tert*-Butoxycarbonyl-D-leucin (3.12 mmol) und L-Phenylalanin-benzylester-hydrochlorid (3.43 mmol). |
| | | Ausbeute: 98% d. Th. |
| 38A | 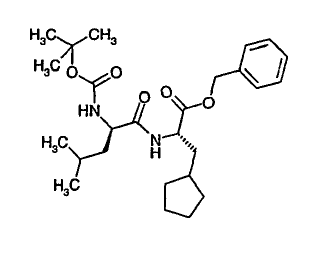 | HPLC/UV-Vis (Methode 13): Rₜ = 9.47 min. |
| | | HPLC/UV-Vis (Methode 28): Rₜ = 5.3 min. |
| | | LC-MS (Methode 29): Rₜ = 7.1 min; |
| | | MS (ESIpos.): *m*/*z* (%) = 361 (80) [M- Boc + H]⁺, 461 (100) [M + H]⁺. |
| | *N-tert*-Butoxycarbonyl-D-leucyl-3-cyclopentyl-L-alanin-benzylester | Allg. Arbeitsvorschrift 6 (hier 5 Äquivalente *N-*Methylmorpholin) aus *N-tert*-Butoxycarbonyl-D-leucin (0.34 mmol) und 3-Cyclopentyl-L-alanin-benzylester-hydrochlorid (0.37 mmol). |
| | | Ausbeute: 94% d. Th. |
| 39A | 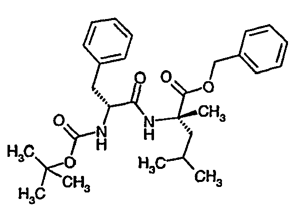 | HPLC/UV-Vis (Methode 13): Rₜ = 9.48 min. |
| | | LC-MS (Methode 26): Rₜ = 3.09 min; |
| | | MS (ESIpos.): *m*/*z* (%) = 383 (100) [M - Boc + H]⁺, 483 (90) [M + H]⁺. |
| | *N*-*tert*-Butoxycarbonyl-D-phenylalanyl-2-methyl-L-leucin-benzylester | Allg. Arbeitsvorschrift 6 (hier 5 Äquivalente *N-*Methylmorpholin) aus *N-tert*-Butoxycarbonyl-D-phenylalanin (0.17 mmol) und 2-Methyl-L-leucin-benzylester-hydrochlorid. |
| | | Ausbeute: 81% d. Th. |
| 40A | 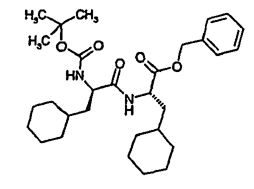 | [α]²⁰_{Na} =+13° (*c* = 0.5 in Methylenchlorid). |
| | | HR-TOF-MS (Methode 21): C₃₀R₄₇N₂O₅ [M + H]⁺ ber. 515.3485, gef. 515.3494. |
| | *N-tert*-Butoxycarbonyl-3-cyclohexyl-D-alanyl-3-cyclohexyl-L-alanin-benzylester | Allg. Arbeitsvorschrift 6 (hier 5 Äquivalente *N-*Methylmorpholin) aus *N-tert*-Butoxycarbonyl-3-cyclohexyl-D-alanin (1.22 mmol) und 3-Cyclohexyl-L-alanin-benzylester-hydrochlorid (1.34 mmol). |
| | | Ausbeute: 85% d. Th. (ee >99%) |
| 41A | 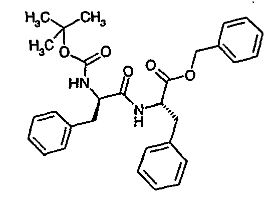 | HPLC/UV-Vis (Methode 13): Rₜ =9.16 min. |
| | | HPLC/UV-Vis (Methode 18): Rₜ = 5.1 min. |
| | | HR-TOF-MS (Methode 21): C₃₀H₃₅N₂O₅ [M + H]⁺ ber. 503.2546, gef. 503.2566. |
| | *N-tert*-Butoxycarbonyl-D-phenylalanyl-L-phenylalanin-benzylester | Allg. Arbeitsvorschrift 6 (hier 5 Äquivalente *N-*Methylmorpholin) aus *N-tert*-Butoxycarbonyl-D-phenylalanin (3.77 mmol) und L-Phenylalanin-benzylester-hydrochlorid (4.15 mmol). |
| | | Ausbeute: 96% d. Th. |
| 42A | 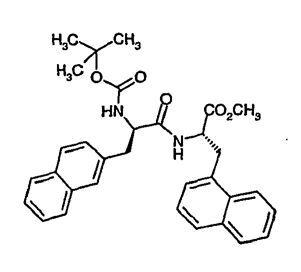 | HPLC/UV-Vis (Methode 13): Rₜ = 9.24 min. |
| | | HPLC/UV-Vis (Methode 28): Rₜ = 5.3 min; λₘₐₓ (qualitativ) = 222 nm (s), 282 (m). |
| | | LC-MS (Methode 22): Rₜ = 2.78 min; |
| | | MS (ESIpos.): *m*/*z* (%) = 427 (80) [M- Boc + H]⁺, 527 (100) [M + H]⁺. |
| | | HR-TOF-MS (Methode 21): C₃₂H₃₅N₂O₅ [M + H]⁺ ber. 527.2546, gef. 527.2552. |
| | *N-tert*-Butoxycarbonyl-3-(2-naphthyl)-D-alanyl-3-(1-naphthyl)-L-alanin-methylester | Allg. Arbeitsvorschrift 6 (hier 5 Äquivalente *N-*Methylmorpholin) aus *N-tert*-Butoxycarbonyl-3-(2-naphthyl)-D-alanin (0.75 mmol) und 3-(1-Naphthyl)-L-benzylester-hydrochlorid. |
| | | Ausbeute: 92% d. Th. |
| 43A | 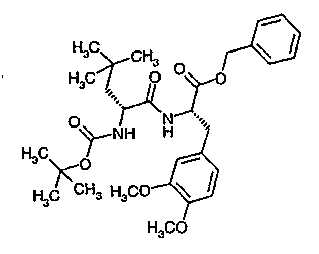 | HPLC/UV-Vis (Methode 13): Rₜ = 8.89 min. |
| | | LC-MS (Methode 26): Rₜ = 2.87 min; |
| | | MS (ESIpos.): *m*/*z* (%) = 443 (80) [M - Boc + H]⁺, 543 (100) [M+H]⁺. |
| | | HR-TOF-MS (Methode 21): C₃₀H₄₃N₂O₇ [M + H]⁺ ber. 543.3070, gef. 5473.3066. |
| | *N-tert*-Butoxycarbonyl-3-*tert*-butyl-D-alanyl-3-(3,4-dimethoxyphenyl)-L-alanin-benzylester | Allg. Arbeitsvorschrift 6 (hier 5 Äquivalente *N*-Methylmorpholin) aus *N-tert*-Butoxycarbonyl-3-*tert*-butyl-D-alanin (6.35 mmol) und 3-(3,4-Dimethoxyphenyl)-L-alanin-benzylester-hydrochlorid. |
| | | Ausbeute: 77% d. Th. |
| 44A | 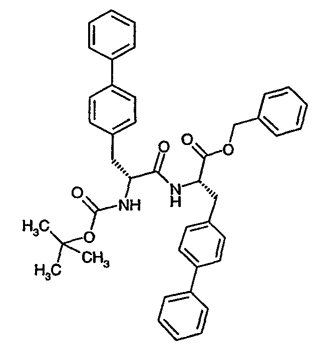 | [α]²⁰_{Na} = +16.7° (*c* = 0.6 in Methylenchlorid). |
| | | HPLC/UV-Vis (Methode 13): Rₜ = 10.31 min. |
| | | HPLC/UV-Vis (Methode 18): Rₜ = 5.7 min. |
| | | HR-TOF-MS (Methode 21): C₄₂H₄₂N₂O₅Na [M + Na]⁺ ber. 677.2991, gef. 677.3014. |
| | *N-tert*-Butoxycarbonyl-4-phenyl- D-phenylalanyl-4-phenyl- L-phenylalanin-benzylester | Allg. Arbeitsvorschrift 6 (hier 5 Äquivalente *N-*Methylmorpholin) aus *N-tert*-Butoxycarbonyl- 4-phenyl-D-phenylalanin (0.62 mmol) und 4-Phenyl-L-phenylalanin-benzylester-hydrochlorid (0.68 mmol). |
| | | Ausbeute: quant. |
| 45A | 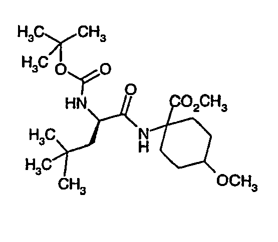 | Diastereomer 1: LC-MS (Methode 26): Rₜ = 2.39 min; |
| | | MS (ESIpos.): *m*/*z* (%) = 315 (100) [M- Boc + H]⁺, 415 (90) [M + H]⁺. |
| | | Diastereomer 2: LC-MS (Methode 26): Rₜ = 2.44 min; |
| | | MS (ESIpos.): *m*/*z* (%) = 315 (100) [M-Boc +H]⁺, 415 (70) [M + H]⁺. |
| | *N-tert*-Butoxycarbonyl-3-*tert*-butyl-D-alanyl-(1-amino-4-methoxy-cyclohexylcarbonsäure)-methylester | Allg. Arbeitsvorschrift 6 aus *N*-*tert*-Butoxycarbonyl-3-*tert*-butyl-D-alanin (2.04 mmol) und 1-Amino-4-methoxy-cyclohexylcarbonsäure-methylester (2.24 mmol). Ausbeute: 8% d. Th. (Diastereomerengemisch). |
| 46A | 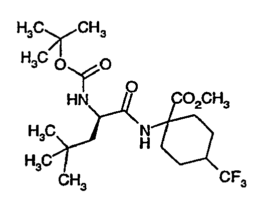 | HPLC/UV-Vis (Methode 13): Rₜ = 8.74 min. |
| | | HPLC/UV-Vis (Methode 18): Rₜ = 5.13 min. |
| | | LC-MS (Methode 26): Rₜ = 2.76 min; |
| | | MS (ESIpos.): *m*/*z* (%) = 353 (50) [M - Boc + H]⁺, 397 (60), 453 (100) [M + H]⁺. |
| | | HR-TOF-MS (Methode 21): C₂₁H₃₆N₂O₅F₃ [M + H]⁺ ber. 453.2576, gef. 453.2581. |
| | *N-tert*-Butoxycarbonyl-3-*tert*-butyl-D-alanyl-(1-amino-4-(trifluormethyl)cyclohexylcarbonsäure)-methylester | Allg. Arbeitsvorschrift 6 aus *N-tert*-Butoxycarbonyl-3-*tert*-butyl-D-alanin (2.04 mmol) und 1-Amino-4-(trifluormethyl)cyclohexylcarbonsäure-methylester (2.24 mmol). |
| | | Ausbeute: 63% d. Th. (Diastereomerengemisch). |
| 47A | 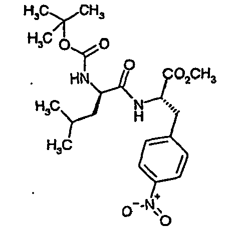 | HPLC/UV-Vis (Methode 13): Rₜ = 8.03 min. |
| | | LC-MS (Methode 37): Rₜ = 2.52 min; |
| | | MS (ESIpos.): *m*/*z* (%) = 338 (20) [M - Boc + H]⁺, 379 (30), 382 (100), 438 (30) [M + H]⁺. |
| | N-*tert*-Butoxycarbonyl-D-leucyl-4-nitro-L-phenylalanin-methylester | Allg. Arbeitsvorschrift 6 (hier 2.5 Äquivalente Triethylamin als Base) aus *N*-*tert*-Butoxycarbonyl-D-leucin (4.40 mmol) und 4-Nitro-L-phenylalanin-methylester-hydrochlorid (4.0 mmol). |
| | | Ausbeute: 95% d. Th. |
| 48A | 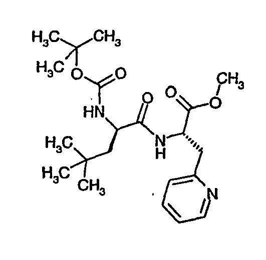 | HPLC/UV-Vis (Methode 28): Rₜ = 4.1 min. |
| | | HPLC/UV-Vis (Methode 36): Rₜ = 3.82 min. |
| | | LC-MS (Methode 26): Rₜ = 2.07 min; |
| | | MS (ESIpos.): *m*/*z* (%) = 352 (100), 408 (95) [M+H]⁺; |
| | | MS (ESIneg.): *m*/*z* (%) = 332 (100), 406 (5) [MH]⁻. |
| | | IR νₘₐₓ (NaCl, cm⁻¹): 3414, 1639, 1617, 1542, 1438,1385,1175,620. |
| | | ¹H NMR (400 MHz, *d₆*-DMSO): δ = 0.93 (s, 9H, tBu), 1.31-1.36 (m, 1H, β-CH), 1.41 (s, 9H, OtBu), 1.88 (m, 1H, β-CH), 3.31 (m, 2H, β-CH₂), 3.68 (s, 3H, OMe), 4.18 (m,1H, α-CH), 4.79 (d, *J* = 8.0 Hz, 1H), 4.92 (m, 1H, α-CH), 7.12-7.16 (m, 2H), 7.60 (dt, *J* = 2.0, 8.0 Hz, 1H), 7.76 (d, *J* = 6.5 Hz, 1H), 8.50 (d, *J* = 4.0 Hz, 1H, NH). |
| | | ¹³CNMR (500 MHz, CDCl₃): δ =172.7,171.8, 157.1, 155.2, 149.1, 136.7, 123.8, 121.9, 79.9, 52.4, 52.3, 51.6, 46.0, 38.3, 30.4, 29.6, 28.3. |
| | | HR-TOF-MS (Methode 21): C₂₁H₃₄N₃O₅ [M + H]⁺ ber. 408.2498, gef. 408.2494. |
| | *N-tert*-Butoxycarbonyl-3-*tert*-butyl-D-alanyl-3-(2-pyridyl)-L-alanin-methylester | Aus *N-tert*-Butoxycarbonyl-3-*tert*-butyl-D-alanin (2.0 mmol) und 2-pyridyl-L-alanin-methylester-dihydrochlorid (2.0 mmol). |
| | | Ausbeute: 41% d. Th. |
| 49A | 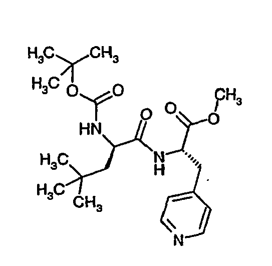 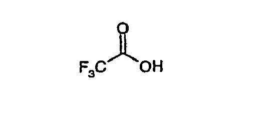 | [α]²⁰_{Na} = -18° (c = 0.28 in MeOH). |
| | | HPLC/UV-Vis (Methode 28): Rₜ = 4.0 min. |
| | | HPLC/UV-Vis (Methode 36): Rₜ = 3.80 min. |
| | | LC-MS (Methode 26): Rₜ = 1.61 min; |
| | | MS (ESIpos.): *m*/*z* (%) = 352 (100), 408 (20) [M+H]⁺; |
| | | MS (ESIneg.): *m*/*z* (%) = 332 (100), 406 (20) [MH]⁻. |
| | | IR νₘₐₓ (NaCl, cm⁻¹) 3414, 2959, 1680, 1509, 1203. |
| | | ¹H NMR (500 MHz, *d₆*-DMSO): δ = 0.77 (s, 9H, tBu), 1.19-1.36 (m, 1H, β-CH), 1.36 (s, 9H, OtBu), 3.18 (m, 1H, β-CH), 3.36 (m, 2H, β-CH), 3.66 (s, 3H, OMe), 3.88 (m, 1H, α-CH), 4.70 (m, 1H, α-CH), 6.85 (d, *J* = 8.5, 1H), 7.83 (d, *J* = 6.0 Hz, 2H), 8.36 (d, *J* = 8.0 Hz, 1H), 876 (m, 2H). |
| | | ¹³C NMR (500 MHz, *d₆*-DMSO): δ =173.1, 171.1, 158.4,158.1,154.9,142.7,127.4, 78.0, 52.2, 51.6, 44.8,36.1,30.7,30.1,29.4,29.0,28.2. |
| | | HR-TOF-MS (Methode 21): C₂₁H₃₄O₅ [M + H]⁺ ber. 408.2498, gef. 408.2525. |
| | *N-tert*-Butoxycarbonyl-3-*tert*-butyl-D-alanyl-3-(4-pyridyl)-L-alaninmethylester-trifluoracetate | Aus *N-tert*-Butoxycarbonyl-3-*tert*-butyl-D-alanin (1.26 mmol) und 4-pyridyl-L-alanin-methylester-dihydrochlorid (1.26 mmol). |
| | | Ausbeute: 75% d. Th. |
| 50A | 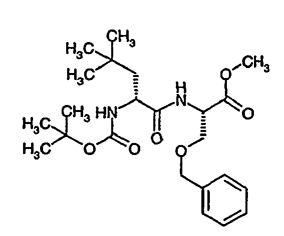 | HPLC/UV-Vis (Methode 36): Rₜ = 5.01 min. |
| | | LC-MS (Methode 26): Rₜ = 2.73 min, MS (ESIpos.): *m*/*z* (%) = 437.3 (50) [M + H]⁺. |
| | | ¹H NMR (300 MHz, *d₆*-DMSO): δ = 0.89 (s, 9H), 1.38 (s, 9H), 1.42-1.60 (m, 2 H), 3.60 (dd, 1H), 3.61 (s, 3H), 3.75 (dd, 1H), 4.07 (m, 1H), 4.46-4.51 (m, 3H), 6.96 (d, 1H), 7.25-7.40 (m, 5W, 7.95 (d, 1H). |
| | *N*-(*tert*-Butoxycarbonyl)-3-*tert*-butyl-D-alanyl-*O-*benzyl-L-serin-methylester | Allgemeine Arbeitsvorschrift 14 aus *O-*Benzyl-L-serin-methylester und *N-*(*tert*-Butoxycarbonyl)-3-*tert*-butyl-D-alanin. Ansatzgröße: 0.96 mmol. |
| | | Ausbeute: 64% d Th. |
| 51A | 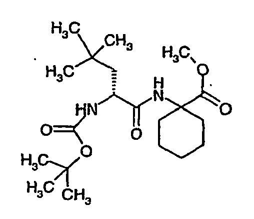 | HPLC/UV-Vis (Methode 36): Rₜ = 4.99 min. |
| | | ¹H NMR (300 MHz, *d₆*-DMSO): δ = 0.89 (s, 9H), 1.39 (s, 9H), 1.40-2.00 (m, 12 H), 3.52 (s, 3H), 4.00 (m, 1H), 6.88 (d, 1H), 7.57 (s, 1H). |
| | | MS (DCI): *m*/*z* (%) = 402.2 [M + NH₄]⁺. |
| | Methyl-1-{[*N-*(*tert-*Butoxycarbonyl)- 3-*tert*-butyl-D-alanyl]amino}cyclohexancarboxylat | Allgemeine Arbeitsvorschrift 15 aus Methylaminocyclohexancarboxylat und *N-(tert-*Butoxycarbonyl)- 3-*tert*-butyl-D-alanin. Ansatzgröße: 1.29 mmol. |
| | | Ausbeute: 61 % d. Th. |
| 52A | 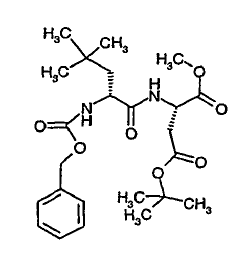 | HPLC/UV-Vis (Methode 36): Rₜ = 4.94 min. |
| | | LC-MS (Methode 26): Rₜ = 2.69 min, |
| | | MS (ESIpos.): *m*/*z* (%) = 465.3 (70) [M + H]⁺. |
| | | ¹H NMR (400 MHz, *d₆*-DMSO): δ = 0.89 (s, 9H), 1.38 (s, 9H), 1.50 (m, 2H), 2.58 (dd, 2H), 2.68 (dd, 2H), 3.61 (s, 3H), 4.10 (m, 1H), 4.58 (m, 1H), 5.00 (d, 1H), 5.06 (d, 1H), 7.25-7.38 (m, 5H), 7.42 (d, 1H), 8.27 (d, 1H). |
| | Methyl-(2S)-2-[(2-{ [(benzyloxy)-carbonyl]amino}-3-*tert*-butyl-D-alanyl)amino]-4-tert-butoxy-4-oxobutanoat | Allgemeine Arbeitsvorschrift 14 aus *O*⁴-*tert*-Butyl *O*¹-methyl L-aspartat und *N-*[(Benzyloxy)carbonyl]-3-*tert*-butyl-D-alanin. Ansatzgröße: 1.3 mmol. |
| | | Ausbeute: 69% d. Th. |
| 53A | 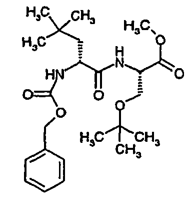 | HPLC/UV-Vis (Methode 36): Rₜ = 4.84 min. |
| | | LC-MS (Methode 26): Rₜ = 2.70 min, |
| | | MS (ESIpos.): *m*/*z* (%) = 437.3 (70) [M + H]⁺. |
| | *N*-[(Benzyloxy)carbonyl]-3*-tert*-butyl-D-alanyl*-O-*(*tert*-butyl)-L-serin-methylester | Allgemeine Arbeitsvorschrift 15, keine HPLC, Rohprodukt wird weiter umgesetzt. aus Methyl-*O-*(*tert* butyl)-L-serinat und *N-*[(Benzyloxy)carbonyl]-3-*tert*-butyl-D-alanin. Ansatzgröße: 0.43 mmol. |
| | | Ausbeute: 24% d. Th. |
| 54A | 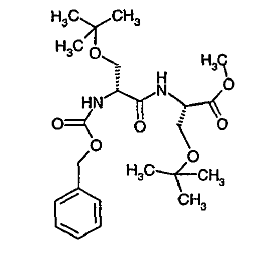 | HPLC/UV-Vis (Methode 36): Rₜ = 4.89 min. |
| | *N*-[(Benzyloxy)carbonyl]-*O*-(*tert*-butyl)-D-seryl-*O-*(*tert*-butyl)-L-serin-methylester | Allgemeine Arbeitsvorschrift 14 aus Methyl-*O-*(*tert*-butyl)-L-serinat und *N-*[(Benzyloxy)carbonyl]-*O-*(*tert*-butyl)-D-serin. Ansatzgröße: 2.85 mmol. |
| | | Ausbeute: 32% d. Th. |
| 55A | | HPLC (Methode 36): Rₜ = 4.92 min. |
| | 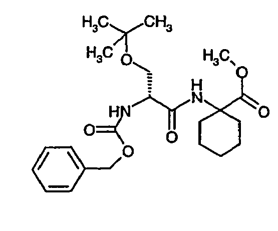 | LC-MS (Methode 26): Rₜ = 2.87 min, |
| | | MS (ESIpos.): *mlz* (%) = 435.4 (100) [M + H]⁺. |
| | | ¹H NMR (300 MHz, *d₆*-DMSO): δ = 1.10 (s, 9H), 1.39-1.68 (m, 8H), 1.89 (d, 1H), 2.00 (d, 1H), 3.39-3.49 (m, 2H), 3.55 (s, 3H), 4.19 (m, 1H), 5.01 (d, 1H), 5.05 (d, 1H), 7.08 (d, 1H), 7.30-7.38 (m, 5H), 7.71 (s, 1H). |
| | Methyl-1-{ [N [(benzyloxy)carbonyl]-*O*-(*tert*-butyl)-D-seryl]amino}cyclohexancarboxylat | Allgemeine Arbeitsvorschrift 14 aus Methyl-aminocyclohexancarboxylat und *N-*[(Benzyloxy)-carbonyl]-*O*-(*tert*-butyl)-D-serin. Ansatzgröße: 2.03 mmol. Ausbeute: 86% d. Th. |
| 56A | 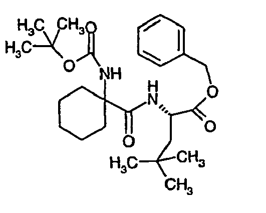 | HPLC/UV-Vis (Methode 36): Rₜ = 5.39 min. |
| | | LC-MS (Methode 26): Rₜ = 3.05 min, |
| | | MS (ESIpos.): *m*/*z* (%) = 461.4 (80) [M + H]⁺. |
| | Benzyl-*N*-({ 1-[(*tert*-butoxy-carbonyl)-amino]cyclohexyl carbonyl)-4-methyl-L-leucinat | Allgemeine Arbeitsvorschrift 16 aus 1-[(*tert-*Butoxycarbonyl)amino]cyclohexancarbonsäure und Benzyl-L-leucinat. An Stelle der RP-HPLC Flashchromatographie an C₁₈-Phase wird mit Ethylacetat gegen Natriumhydrogencarbonat und dann 5% Citronensäure gewaschen, getrocknet und eingeengt). Ansatzgröße: 0.82 mmol. |
| | | Ausbeute: 90% d. Th. |
| 57A | 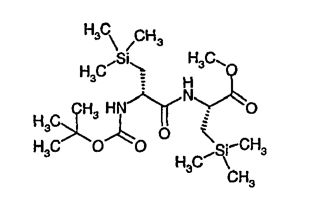 | HPLC/UV-Vis (Methode 36): Rₜ = 5.33 min. |
| | | LC-MS (Methode 26): Rₜ = 2.82 min, |
| | | MS (ESIpos.): *m*/*z* (%) = 419 (100) [M + H]⁺. |
| | Methyl-*N-*(*tert*-butoxycarbonyl)-3-(trimethylsilyl)-D-alanyl-3-(trimethylsilyl)-L-alaninat | Allgemeine Arbeitsvorschrift 14 aus 25A und 3-Tri-methylsilyl)-L-alanin-methylester. Ansatzgröße: 1.63 mmol. |
| | | Ausbeute: 35% d. Th. |
| 58A | 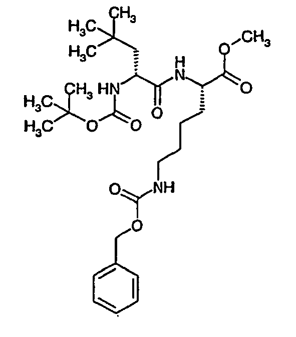 | HPLC/UV-Vis (Methode 36): Rₜ = 4.86 min. |
| | | LC-MS (Methode 26): Rₜ = 2.49 min, |
| | | MS (ESIpos.): *m*/*z* (%) = 522 (20) [M + H]⁺. |
| | | ¹H NMR (300 MHz, *d₆*-DMSO): δ = 0.89 (s, 9H), 1.20-1.35 (m, 2H), 1.40-2.77 (m, 4H), 2.95 (dd, 2H), 3.60 (s, 3H), 4.06 (m, 1H), 4.18 (m, 1H), 5.00 (s, 2H), 6.79 (d, 1H), 7.18 (m, 1H), 7.29-7.40 (m, 5H), 7.96 (d, 1H). |
| | *N*-(*tert*-Butoxycarbonyl)- 3-*tert*-butyl-D-alanyl-*N*⁶-[(benzyloxy)-carbonyl]-L-lysin-methylester | Allgemeine Arbeitsvorschrift 16 aus *N*⁶-[(Benzyloxy)carbonyl]-L-lysin-methylester und *N-*(*tert*-Butoxycarbonyl)- 3-*tert*-butyl-D-alanin. Ansatzgröße: 0.99 mmol. |
| | | Ausbeute: 61% d. Th. |

### Dipeptidsäuren

### Beispiel 59A

### N-(tert-Butoxycarbonyl)-3-tert-butyl-D-alanyl-3-tert-butyl-L-alanin

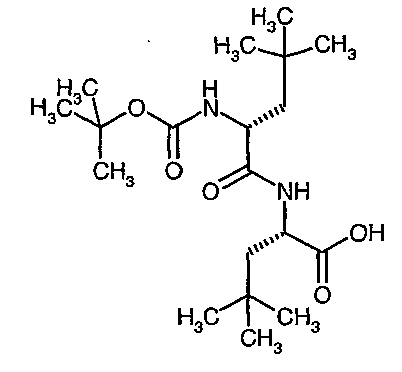

*N*-(*tert*-Butoxycarbonyl)-3-*tert*-butyl-D-alanyl-3-*tert*-butyl-L-alanin-benzylester (Beispiel 33A, 285 mg, 0.62 mmol) wird nach der Allgemeinen Arbeitsvorschrift 8 umgesetzt. Man erhält 225 mg (98% d. Th.) Produkt.

¹H NMR (400 MHz, *d₆*-DMSO): δ = 0.83 (s, br, 18H, tBu), 1.31 (s, 9H, OtBu), 1.40 (d, *J* = 6.1 Hz, 2H,β-CH₂), 1.48 (dd, *J* = 14.1, 9.4 Hz, 1H, β-CH), 1.59 (dd, *J* = 14.1,2.7 Hz, 1H, β-CH), 3.98 (m, 1H, α-CH), 4.12 (m, 1H, α-CH), 6.73 (d, *J* = 9.1 Hz, 1H, NH), 7.72 (d, *J* = 7.9 Hz, 1H, NH), 12.42 (s, br, 1H, CO₂H).
¹³C NMR (125 MHz, *d₆*-DMSO): δ = 28.49 (3C), 29.66 (3C), 29.78 (3C), 30.52, 30.58, 44.63 (β-CH₂), 45.24 (β-CH₂), 49.67 (α-CH), 52.40 (α-CH), 78.29, 155.05, 172.97, 174.61.
[α]²⁰_{Na} = + 25° (c = 0.1 in Chloroform).
HPLC/UV-Vis (Methode 13): Rₜ = 7.95 min.
LC-MS (Methode 22): Rₜ = 2.26 min;
MS (ESIpos.): *m*/*z* (%) = 373 (100) [M + H]⁺.
HR-TOF-MS (Methode 21): C₁₉H₃₇N₂O₅ ber. 373.2702, gef. 373.2717 [M + H]⁺.

### Beispiel 60A

### N-tert-Butoxycarbonyl-3-tert-butyl-D-alanyl-3-(3-pyridyl)-L-alanin

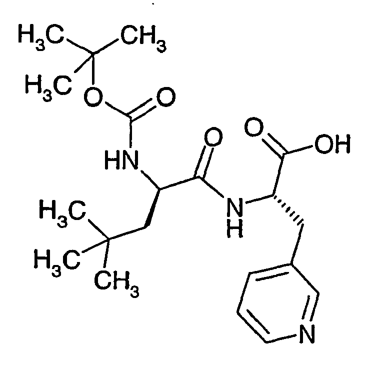

Zu einer Lösung 34A (1.0 Äquivalente, 12.8 mmol) in Tetrahydrofuran (104 ml) und Wasser (6.2 ml) wird bei -20°C eine Lösung Lithiumhydroxid-hydrat (2.5 Äquivalente, 31.9 mmol) in Wasser (1.2 ml) gegeben. Das Reaktionsgemisch erwärmt sich (ca. 1.5 h) auf +15°C, wobei vollständiger Umsatz mittels HPLC/UV-Vis (Methode 36) beobachtet wird. Zur Aufarbeitung wird Kaliumdihydrogenphosphat (10 Äquivalente, 127 mmol) zugegeben (pH 7). Das Reaktionsgemisch wird filtriert und im Vakuum aufkonzentriert. Das Rohprodukt (5.5 g) wird mittels Gelchromatographie (Methode 6, Laufmittel Methanol/Aceton 4/1) aufgereinigt, wobei man 3.7 g (70% d. Th.) Produkt erhält.
[α]²⁰Na = +39.3° (c = 0.33 in Methanol).
HPLC/UV-Vis (Methode 28): Rₜ = 3.8 min.
HPLC/UV-Vis (Methode 36): Rₜ = 3.64 min.
LC-MS (Methode 26): Rₜ = 1.59 min;
MS (ESIpos.): *m*/*z* (%) = 338 (100), 394 (40) [M + H]⁺;
MS (ESIneg.): *m*/*z* (%) = 318 (60), 392 (100) [M - H]⁻.
HR-TOF-MS (Methode 21): C₂₀H₃₂N₃O₅ [M + H]⁺ ber. 394.2342, gef. 394.2322.

**Tabelle 2: N-Geschützte Dipeptidsäuren**

| **Bsp. Nr.** | **Struktur** | **Analytische Daten** |
|---|---|---|
| | **Name** | **Herstellungsmethode** |
| 61A | 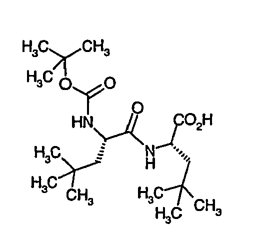 | HPLC/UV-Vis (Methode 13): Rₜ = 7.8 min. |
| | | ¹H NMR (400 MHz, *d₆*-DMSO, TMS): δ = 0.88 (s, br, 18H, tBu), 1.36 (s, 9H, OtBu), 1.41 (dd, *J* = 13.9, 9.2 Hz, 1H, β-CH), 1.49 (dd, *J* = 13.9, 3.2 Hz, 1H, β-CH'), 1.54 (dd, *J* = 13.9, 9.1 Hz, 1H, β-CH"), 1.65 (dd, *J* = 14.1, 2.5 Hz, 1H, β-CH"'), 4.02 (m, 1H, α-CH), 4.25 (m, 1H, α-CH), 6.87 (d, *J* = 8.9 Hz, 1H, NH), 7.85 (d, *J* = 8.2 Hz, 1H, NH), 12.48 (s, br, 1H, CO₂H). |
| | | HR-TOF-MS (Methode 21): C₁₉H₃₇N₂O₅ [M + H]⁺ ber. 373.2702, gef. 373.2680. |
| | *N-tert*-Butoxycarbonyl-3-*tert*-butyl-L-alanyl-3-*tert*-butyl-L-alanin | Allg. Arbeitsvorschrift 8 aus Beispiel 35A (0.6 mmol). |
| | | Ausbeute: 98% d. Th. |
| 62A | 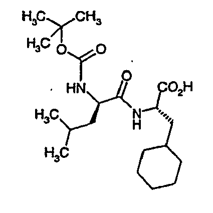 | BPLC/UV-Vis (Methode 13): Rₜ = 8.2 min. |
| | | HR-TOF-MS (Methode 21): C₂₀H₃₇N₂O₅ [M + H]⁺ ber. 385.2702, gef. 385.2693. |
| | *N-tert*-Butoxycarbonyl-D-leucyl-3-cyclohexyl-L-alanin | Allg. Arbeitsvorschrift 8 aus Beispiel 36A (0.65 mmol). |
| | | Ausbeute: 99% d. Th. |
| 63A | 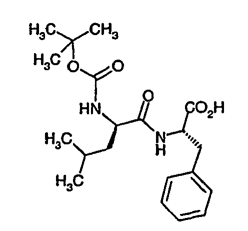 | [α]²⁰_{Na} = +43° (c = 0.1 in Methylenchlorid). |
| | | HPLC/UV-Vis (Methode 13): Rₜ = 7.52 min. |
| | | LC-MS (Methode 22): Rₜ = 2.12 min; |
| | | MS (ESIpos.): *m*/*z* (%) = 279 (100) [M - Boc + H]⁺, 379 (60) [M + H]⁺. |
| | | HR-TOF-MS (Methode 21): C₂₀H₃₁N₂O₅ [M + H]⁺ ber. 379.2233, gef. 379.2227. |
| | *N*-*tert-*Butoxycarbonyl-D-leucyl-L-phenylalanin | Allg. Arbeitsvorschrift 8 aus Beispiel 37A (1.49 mmol). |
| | | Ausbeute: 94% d. Th. |
| 64A | 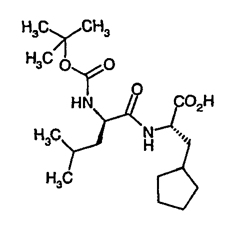 | [α]²⁰_{Na}=+3° (c = 0.1 in Methylenchlorid). |
| | | HPLC(UV-Vis (Methode 28): Rₜ = 4.7 min. |
| | | HR-TOF-MS (Methode 21): C₁₉H₃₅N₂O₅ [M + H]⁺ ber. 371.2546, gef. 371.2537. |
| | *N-tert*-Butoxycarbonyl-D-leucyl-3-cylopentyl-L-alanin | Allg. Arbeitsvorschrift 8 aus Beispiel 38A (0.24 mmol). |
| | | Ausbeute: 86% d. Th. |
| 65A | 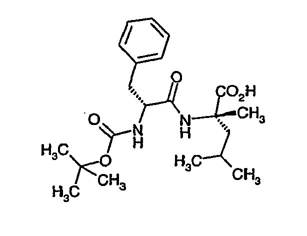 | HPLC/UV-Vis (Methode 18): Rₜ = 4.6 min. |
| | | HR-TOF-MS (Methode 21): C₂₁H₃₃N₂O₅ [M + H]⁺ber. 393.2389, gef. 393.2370. |
| | *N-tert*-Butoxycarbonyl-D-phenylalanyl-2-methyl-L-leucin | Allg. Arbeitsvorschrift 8 aus Beispiel 39A (0.11 mmol). |
| | | Ausbeute: 95% d. Th. |
| 66A | 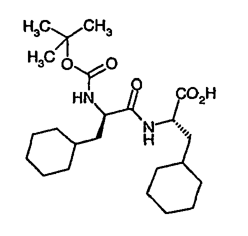 | IHPLC/UV-Vis (Methode 18): Rₜ = 5.1 min. |
| | | HR-TOF-MS (Methode 21): C₂₃H₄₁N₂₉O5 [M + H]⁺ber. 425.3015, gef. 425.3018. |
| | *N-tert*-Butoxycarbonyl-3-cyclohexyl-D-alanyl-3-cyclohexyl-L-alanin | Allg. Arbeitsvorschrift 8 aus Beispiel 40A (0.96 mmol). |
| | | Ausbeute: 88% d. Th. |
| 67A | 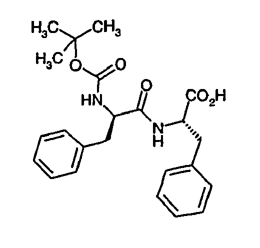 | [α]²⁰_{Na} = +33° (c = 0.6 in Methylenchlorid). |
| | | HPLC/UV-Vis (Methode 13): Rₜ = 6.64 min. |
| | | HPLC/UV-Vis (Methode 28): Rₜ = 4.4 min. |
| | | HR-TOF-MS (Methode 21): C₂₃H₂₉N₂O₅ [M + H]⁺ ber. 413.2076, gef. 413.2082. |
| | *N-tert*-Butoxycarbonyl-D-phenylalanyl-L-phenylalanin | Allg. Arbeitsvorschrift 8 aus Beispiel 41A (1.12 mmol). |
| | | Ausbeute: 98% d. Th. |
| 68A | | HPLC/UV-Vis (Methode 13): Rₜ = 8.66 min. |
| | 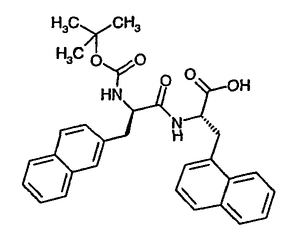 | HPLC/UV-Vis (Methode 28): Rₜ = 5.0 min. |
| | | LC-MS (Methode 29): Rₜ = 6.6 min; |
| | | MS (ESIpos.): *m*/*z* (%) = 413 (100) [M - Boc + H]⁺, 513 (80) [M + H]⁺. |
| | | HR-TOF-MS (Methode 21): C₃₁H₃₃N₂O₅ [M + H]⁺ ber. 513.2389, gef. 513.2388. |
| | *N-tert*-Butoxycarbonyl-3-(2-naphthyl)-D-alanyl-3-(1-naphthyl)-L-alanin | Allg. Arbeitsvorschrift 7 aus Beispiel 42A (0.49 mmol). |
| | | Ausbeute: 67% d. Th. |
| 69A | 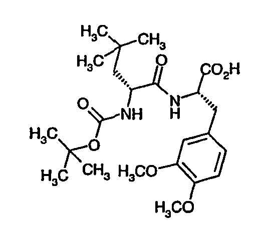 | [α]²⁰_{Na} = +31.6° (c = 0.6 in Methylenchlorid). |
| | | HPLC/UV-Vis (Methode 18): Rₜ = 4.3 min. |
| | | HPLC/UV-Vis (Methode 13): Rₜ = 7.7 min. |
| | | HR-TOF-MS (Methode 21): C₂₃H₃₇N₂O7 [M + H]⁺ ber. 453.2601, gef. 453.2576. |
| | *N-tert*-Butoxycarbonyl-3-*tert*-butyl-D-alanyl-3-(3,4-dimethoxyphenyl)-L-alanin | Allg. Arbeitsvorschrift 8 aus Beispiel 43A (0.46 mmol). |
| | | Ausbeute: quant. |
| 70A | 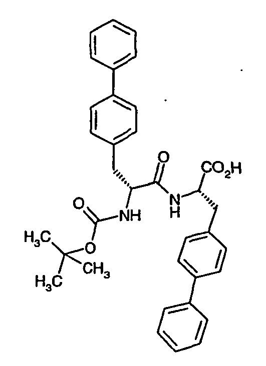 *N-tert*-Butoxycarbonyl-4-phenyl-D-phenylalanyl-4-phenyl-L-phenylalanin | [α]²⁰_{Na} = +31 ° (c = 0.1 in Methanol). |
| | | HPLC/UV-Vis (Methode 13): Rₜ = 9.15 min. |
| | | HPLC/UV-Vis (Methode 28): Rₜ = 5.30 min, λₘₐₓ (qualitativ) = 202 nm (s), 254 (m). |
| | | LC-MS (Methode 22): Rₜ = 2.80 min; |
| | | MS (ESIpos.): *m*/*z* (%) = 465 (100) [M - Boc + H]⁺ 509 (50), 565 (90) [M + H]⁺, 1129 (30). |
| | | HR-TOF-MS (Methode 21): C₃₅H₃₇N₂O₅ [M + H]⁺ ber. 565.2702, gef. 565.2686. |
| | | Allg. Arbeitsvorschrift 8 aus Beispiel 44A (0.53 mmol). |
| | | Ausbeute: 84% d. Th. |
| 71A | 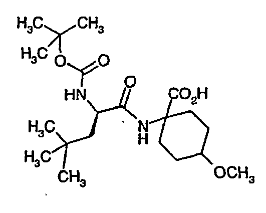 *N-tert*-Butoxycarbonyl-3-*tert*-butyl-D-alanyl-L-1-amino-4-methoxy-cyclohexylcarbonsäure | [α]²⁰_{Na} = +47.9° (*c* = 0.5 in Methylenchlorid). |
| | | HPLC/UV-Vis (Methode 13): Rₜ = 7.07 min. |
| | | HPLC/UV-Vis (Methode 18): Rₜ = 4.30 min. |
| | | HR-TOF-MS (Methode 21): C₂₀H₃₇N₂O₆ [M + H]⁺ ber. 401.2652, gef. 401.2667. |
| | | Allg. Arbeitsvorschrift 7 (hier 2 Äquivalente Lithiumhydroxid-hydrat) aus Beispiel 45A (0.66 mmol). |
| | | Ausbeute: 48% d. Th. |
| 72A | | [α]²⁰_{Na} = +37.0° (*c* = 1.05 in CH₂Cl₂). |
| | 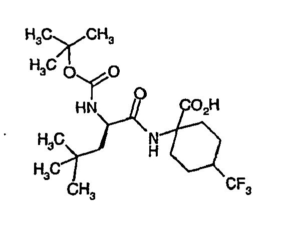 | HPLC/UV-Vis (Methode 13): Rₜ = 8.07 min. |
| | | HPLC/LUV-Vis (Methode 18): Rₜ = 4.79 min. |
| | | LC-MS (Methode 22): Rₜ = 2.32 min; |
| | | MS (ESIpos.): *m*/*z* (%) = 339 (60) [M - Boc + H]⁺, 383 (80), 439 (100) [M + H]⁺. |
| | | HR-TOF-MS (Methode 21): C₂₀H3₄₂O₅F₃ [M + H]⁺ ber. 439.2420, gef. 439.2398. |
| | *N-tert*-Butoxycarbonyl-3-*tert*-butyl-D-alanyl-L-1-amino-4-(trifluor- | Allg. Arbeitsvorschrift 7 (hier 2 Äquivalente Lithiumhydroxid-hydrat) aus Beispiel 46A (1.10 mmol). |
| | methyl)cyclohexylcarbonsäure | Ausbeute: 80% d. Th. |
| 73A | 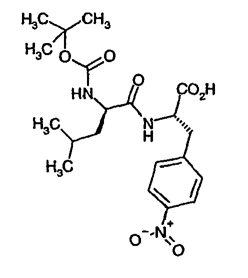 | Das Rohprodukt wird direkt zu Beispiel 178A weiter umgesetzt. |
| | *N*-*tert*-Butoycarbonyl-D-leucyl-4.-nitro-L-phenylalanin | Allg. Arbeitsvorschrift 7 (hier 2 Äquivalente Lithiumhydroxid-hydrat) aus Beispiel 47A (0.50 mol). |
| | | Ausbeute: 85% d. Th. |
| 74A | 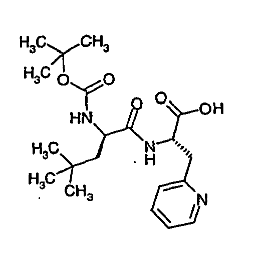 | [α]²⁰_{Na} = +53.8° (c = 1.05 in CHCl₃). |
| | | HPLC/UV-Vis (Methode 28): Rₜ = 3.9 min. |
| | | HPLC/UV-Vis (Methode 36): Rₜ = 3.60 min. |
| | | LC-MS (Methode 26): Rₜ = 1.70 min; |
| | | MS (ESIpos.): *m*/*z* (%) = 338 (100), 394 (70) [M+H]⁺; |
| | | MS (ESIneg.)- m/Z (%) = 318 (60), 392 (100) [M-H]⁻. |
| | | νₘₐₓ (NaCl, cm⁻¹) : 3407, 2957, 1709, 1650, 1522, 1367, 1170. |
| | | HR-TOF-MS (Methode 21): C₂₀H₃₂N₃O₅ [M + H]⁺ ber. 394.2342, gef. 394.2342. |
| | *N-tert*-Butoxycarbonyl-3-*tert*-butyl- | Aus Beispiel 48A (12.8 mmol). |
| | D-alanyl-3-(2-pyridyl)-L-alanin | Ausbeute: 70% d. Th. |
| 75A | 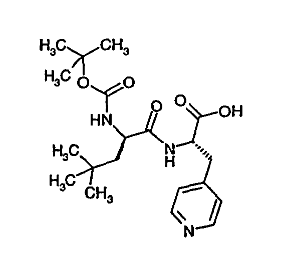 | HPLC/UV-Vis (Methode 28): Rₜ = 3.9 min. |
| | | HPLC/UV-Vis (Methode 36): Rₜ = 3.60 min. |
| | | LC-MS (Methode 26): Rₜ = 1.54 min; |
| | | MS (ESIpos.): *m*/*z*(%) = 338 (100), 394 (30) [M + H]⁺; |
| | | MS (ESIneg.): *m*/*z* (%) = 318 (60), 392 (100) [M-H]⁻. |
| | | HR-TOF-MS (Methode 21): C₂₀H₃₂N₃O₅ [M + H]⁺ ber. 394.2342, gef. 394.2320. |
| | *N-tert*-Butoxycarbonyl-3-*tert*-butyl-D-alanyl-3-(4-pyridyl)-L-alanin | Aus Beispiel 49A (0.25 mmol). |
| | | Ausbeute: 87% d. Th. |
| 76A | 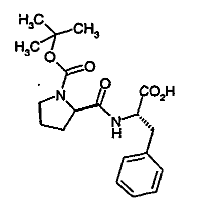 | HPLGMS (Methode 26): Rₜ = 2 min, |
| | | MS (ESIneg.): *m*/*z* (%) = 361 (57) [M - H]⁻ |
| | *N-tert*-Butoxycarbonyl-D-prolyl-L-phenylalanin | Allgemeine Arbeitsvorschrift 12 (1 mmol) aus *N-*(9-Fluorenyhnethoxycarbonyl)-L-phenylalanin und *N-*(*tert*-Butoxycarbonyl)-D-prolin. |
| | | Ausbeute: 40% d. Th. |
| 77A | 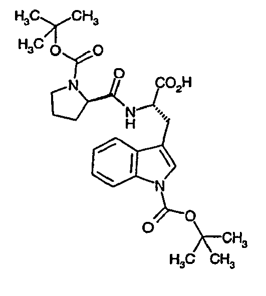 | HPLC/MS (Methode 26): Rₜ = 2.55 min, |
| | | MS (ESIneg.): *m*/*z* (%) = 500 (66) [M - H]- |
| | *N-tert*-Butoxycarbonyl-D-prolyl-*N-tert*-butoxycarbonyl-L-tryptophan | Allgemeine Arbeitsvorschrift 12 (1 mmol) aus *N*^{□}-(9-Fluorenylnlethoxycarbonyl)-*N*-*tert*-butoxycarbonyl-L-tryptophan und *N-(tert-* Butoxycarbonyl)-D-prolin. |
| | | Ausbeute: 39% d. Th. |
| 78A | 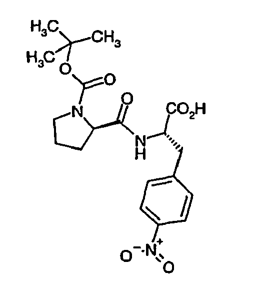 | HPLC/MS (Methode 26): Rₜ = 2.02 min, |
| | | MS (ESIneg.): *m*/*z* (%) = 406 (36) [M - H]- |
| | *N-tert*-Butoxycarbonyl-D-prolyl-4-nitro-L-phenylalanin | Allgemeine Arbeitsvorschrift 12 (1 mmol) aus *N-*(9-Fluorenylmethoxycarbonyl)-4-nitro-L-phenylalanin und *N-*(*tert*-Butoxycarbonyl)-D-prolin. |
| | | Ausbeute: 44% d. Th. |
| 79A | 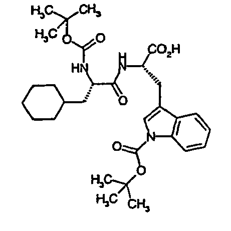 | HPLC/MS (Methode 26): Rₜ = 3.03 min, |
| | | MS (ESIneg.): *m*/*z* (%) = 557 (36) [M - -H]⁻ |
| | *N-tert*-Butoxycarbonyl-3- cyclohexyl-L-alanyl- *N^{indol}-tert-* butoxycarbonyl-L-tryptophan | Allgemeine Arbeitsvorschrift 12 (1 mmol) aus *N*^{□}-(9-Fluorenylmethoxycarbonyl)-*N*-*tert*-butoxycarbonyl-L-tryptophan und *N-(tert-*Butoxycarbonyl)-3-cyclohexyl-L-alanin. |
| | | Ausbeute: 29% d. Th. |
| 80A | 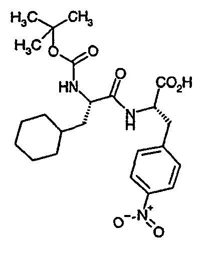 | HPLC/MS (Methode 26): Rₜ = 2.54 min, |
| | | MS (ESIneg.): *m*/*z* (%) = 462 (20) [M - H]⁻ |
| | *N-tert*-Butoxycarbonyl-3- cyclohexyl-L-alanyl-4-nitro-L-phenylalanin | Allgemeine Arbeitsvorschrift 12 (1 mmol) aus *N-*(9-Fluorenylmethoxycarbonyl)-4-nitro-L-phenylalanin und *N*-(*tert*-Butoxycarbonyl)- 3-cyclohexyl-L-alanin. |
| | | Ausbeute: 31 % d. Th. |
| 81A | 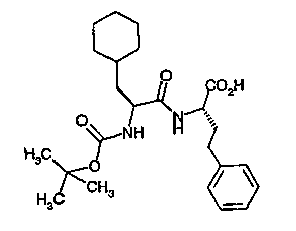 | HPLC/MS (Methode 26): Rₜ = 2.65 min, |
| | | MS (ESIneg.): *m*/*z* (%) = 431 (22) [M - H]⁻ |
| | *N-tert*-Butoxycarbonyl-3-cyclohexyl-L-alanyl-3-benzyl-L-alanin | Allgemeine Arbeitsvorschrift 12 (1 mmol) aus *N-*(9-Fluorenylmethoxycarbonyl)-3-benzyl-L-alanin und *N-*(*tert*-Butoxycarbonyl)-3-cyclohexyl-L-alanin. |
| | | Ausbeute: 44% d. Th. |
| 82A | 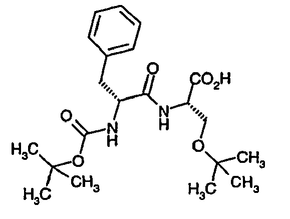 | HPLC/MS (Methode 26): Rₜ = 2.66 min, |
| | | MS (ESIneg.): *m*/*z* (%) = 521 (54) [M - H]⁻ |
| | *N-tert*-Butoxycarbonyl-D-phenylalanyl-*O*³-*tert*-butyl-L-serin | Allgemeine Arbeitsvorschrift 12 (1 mmol) aus *N*-(9-Fluorenyhmethoxycarbonyl)-*O*³-(*tert*-butyl)-L-serin und *N-*(*tert*-Butoxycarbonyl)-D-phenylalanin. |
| | | Ausbeute: 29% d. Th. |
| 83A | 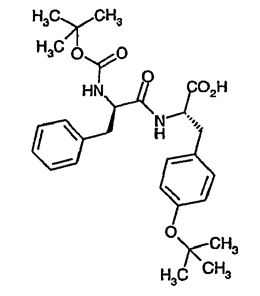 | HPLC/MS (Methode 26): Rₜ = 2.8 min, |
| | | MS (ESIneg.): m/,z (%) = 489 (27) [M - H]⁻ |
| | *N-tert*-Butoxycarbonyl-D-phenylalanyl-*O-tert*-butyl-L-tyrosin | Allgemeine Arbeitsvorschrift 12 (1 mmol)aus *N*-(9-Fluorenylmethoxycarbonyl)-*O*-*tert*-butyl-L-tyrosin und *N-*(*tert*-Butoxycarbonyl)-D-phenyl-alanin. Ausbeute: 58% d. Th. |
| 84A | 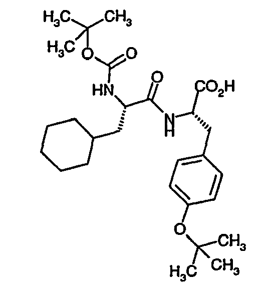 | HPLC/MS (Methode 26): Rₜ = 2.42 min, |
| | | MS (ESIneg.): *m*/*z*(%) = 449 (35) [M - H]⁻ |
| | *N-tert*-Butoxycarbonyl-3-cyclohexyl-1.-alanyl-*O-tert*-butyl-L-tyrosin | Allgemeine Arbeitsvorschrift 12 (1 mmol) aus *N*-(9-Fluorenylmethoxycarbonyl)-*O*-*tert*-butyl-L-tyrosin und *N-*(*tert*-Butoxycarbonyl)-3-cyclohexyl-L-alanin. |
| | | Ausbeute: 27% d. Th. |
| 85A | 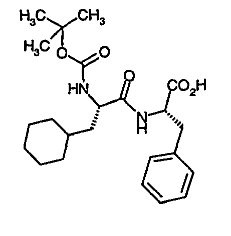 | HPLC/MS (Methode 26): Rₜ = 1.95. min, |
| | | MS (ESIneg.): *m*/*z*(%) = 359 (21) [M - H]⁻ |
| | *N-tert*-Butoxycarbonyl-3-cyclohexylalanyl-L-phenylalanin | Allgemeine Arbeitsvorschrift 12 (1 mmol) aus *N-*(9-Fluorenylmethoxycarbonyl)-L-phenylalanin und *N-*(*tert*-Butoxycarbonyl)-3-cyclohexyl-L-alanin. |
| | | Ausbeute: 21 % d. Th. |
| 86A | 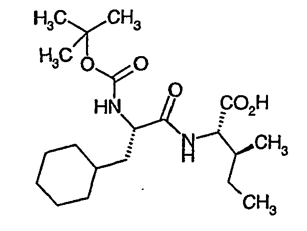 | HPLC/MS (Methode 26): Rₜ = 2.89 min, |
| | | MS (ESIneg.): *m*/*z* (%) = 588 (55) [M - H]⁻. |
| | *N-tert*-Butoxycarbonyl-3-cyclohexyl-L-alanyl-L-isoleucin | Allgemeine Arbeitsvorschrift 12 (1 mmol) aus *N-*(9-Fluorenylmethoxycarbonyl)- L-isoleucin und *N*-(*tert-*Butoxycarbonyl)-3-cyclohexyl-L-alanin. |
| | | Ausbeute: 41 % d. Th. |
| 87A | 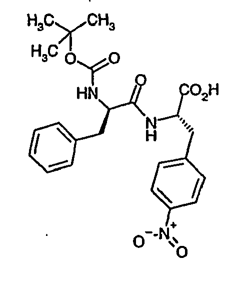 | HPLC/MS (Methode 26): Rₜ = 2.37 min, |
| | | MS (ESIneg.): *m*/*z* (%) = 516 (46) [M - H]⁻. |
| | *N-tert*-Butoxycarbonyl-D-phenylalanyl-4-nitro-L-phenylalanin | Allgemeine Arbeitsvorschrift 12 (1 mmol) aus *N-*(9-Fluorenylmethoxycarbonyl)-4-nitro-L-phenylalanin und *N-*(*tert*-Butoxycarbonyl)-D-phenylalanin. |
| | | Ausbeute: 45% d. Th. |
| 88A | 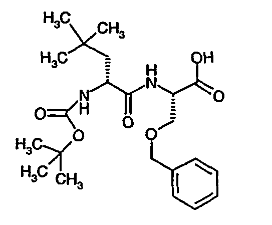 | HPLC/UV-Vis (Methode 36): Rₜ = 4.68 min. |
| | | LC-MS (Methode 26): Rₜ = 2.49 min, MS (ESIpos.): *m*/*z* (%) = 423 (100) [M + H]⁺. |
| | | ¹H NMR (200 MHz, *d*₆-DMSO): δ = 0.89 (s, 9H), 1.48 (s, 9H), 1.42-1.60 (m, 2H), 3.60 (dd, 1H), 3.75 (dd, 1H), 4.07 (m, 1H), 4.40-4.50 (m, 3H), 7.02 (d, 1H), 7.25-7.40 (m, 5H), 7.80 (d, 1H). |
| | *N-*(*tert*-Butoxycarbonyl)-3*-tert-*butyl-D-alanyl-*O*-benzyl-L-serin | Allgemeine Arbeitsvorschrift 17 aus Beispiel 50A. Ansatzgröße: 0.99 mmol. |
| | | Ausbeute: 76% d. Th. |
| 89A | 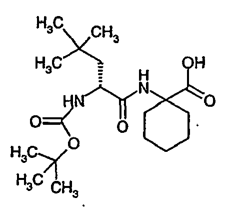 | HPLC/LTV-Vis (Methode 36): Rₜ = 4.99 min. |
| | | LC-MS (Methode 26): Rₜ = 2.17 min, MS (ESIpos.): *m*/*z* (%) = 371 (100) [M + H]⁺. |
| | 1-{ [*N-*(*tert*-Butoxycarbonyl)- *3-tert-*butyl-D-alanyl]amino}cyclohexan-carbonsäure | Allgemeine Arbeitsvorschrift 17, Reaktionszeit: 12 h bei Raumtemperatur aus Beispiel 51A. Ansatzgröße: 0.99 mmol. |
| | | Ausbeute: 46% d. Th. |
| 90A | 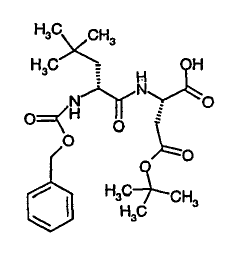 | HPLC/UV-Vis (Methode 36): Rₜ = 4.61 min. |
| | | LC-MS (Methode 26): Rₜ = 2.24 min, |
| | | MS (ESIpos.): *m*/*z* (%) = 451 (100) [M + H]⁺. |
| | | ¹H NMR (300 MHz, *d₆*-DMSO): δ = 0.89 (s, 9H), 1.48 (s, 9H), 1.45-1.60 (m, 2H), 2.56 (dd, 2H), 2.68 (dd, 2H), 4.10 (m, 1H), 4.50 (m, 1H), 4.97 (d, 1H), 5.06 (d, 1H), 7.25-7.38 (m, 5H), 7.42 (d, 1H), 8.00 (d, 1H). |
| | (2*S*)-2-[(2-{[(Benzyloxy)carbonyl]-aminol-3-*tert*-butyl-D-alanyl)amino]-4.-*tert*-butoxy-4-oxobuttersäure | Allgemeine Arbeitsvorschrift 17 aus Beispiel 52A. Ansatzgröße: 1.26 mmol. |
| | | Ausbeute: 78% d. Th. |
| 91A | | IHPL/U-Vis (Methode 36): Rₜ = 4.62 min. |
| | 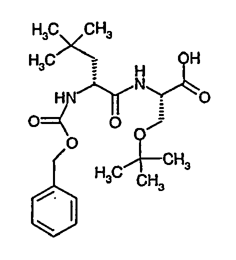 | LC-MS (Methode 26): Rₜ = 2.24 min, |
| | | MS (ESIpos.): *m*/*z* (%) = 423 (100), [M + H]⁺. |
| | | ¹H NMR (300 MHz, *d₆*-DMSO): δ = 0.89 (s, 9H), 1.09 (s, 9H), 1.48-1.60 (m, 2H), 3.48 (dd, 1H), 3.61 (dd, 1H), 4.12 (m, 1H), 4.35 (m, 1H), 5.01 (d, 1H), 5.06 (d, 1H), 7.30-7.40 (m, 5H), 7.53 (d, 1H), 7.65 (d, 1H), 12.65 (br s, 1H). |
| | *N-*[(Benzyloxy)carbonyl]-3*-tert-*butyl-D-alanyl-*O*-(*tert*-butyl)-L-serin | Allgemeine Arbeitsvorschrift 17 aus Beispiel 53A. Ansatzgröße: 0.38 mmol. |
| | | Ausbeute: 79% d. Th. |
| 92A | 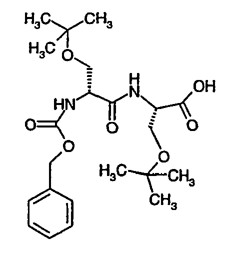 | HPLC/UV-Vis (Methode 36): Rₜ = 4.55 min. |
| | | LC-MS (Methode 26): Rₜ = 2.57 min, |
| | | MS (ESIpos.): *m*/*z* (%) = 439.4 (100) [M + H]⁺. |
| | *N*-[(Benzyloxy)carbonyl]-*O*-(*tert*-butyl)-D-seryl-*O-*(*tert*-butyl)-L-serin | Allgemeine Arbeitsvorschrift 17 aus Beispiel 54A. Ansatzgröße: 0.91 mmol. |
| | | Ausbeute: 54% d. Th. |
| 93A | 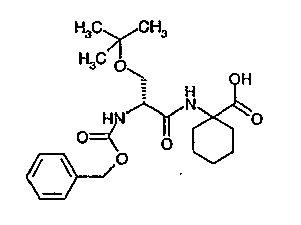 | HPLC/UV-Vis (Methode 36): Rₜ = 4.56 min. |
| | | LC-MS (Methode 26): Rₜ = 2.72 min, |
| | | MS (ESIpos.): *m*/*z* (%) = 435.4 (100) [M + H]⁺. |
| | | ¹H NMR (400 MHz, *d₆*-DMSO): δ = 1.10 (s, 9H), 1.39-1.68 (m, 8w, 1.91 (d, 1H), 2.03 (d, 1H), 3.39-3.49 (m, 2H), 4.20 (m, 1H), 5.04(s, 2H), 7.28-7.40 (m, 5H), 7.71 (s, 1H), 12.08 (br s, 1H). |
| | 1-{[*N-*[(Benzyloxy)carbonyl]-*O-*(*tert*-butyl)-D-seryl]amino-}cyclo-hexancarbonsäure | Allgemeine Arbeitsvorschrift 17 aus Beispiel 55A. Ansatzgröße: 1.69 mmol. |
| | | Ausbeute: 90% d. Th. |
| 94A | 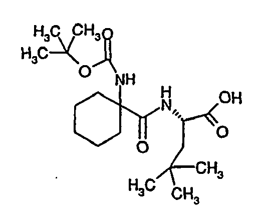 | HPLC/UV-Vis (Methode 36): Rₜ = 4.56 min. |
| | | LG-S (Methode 26): Rₜ = 2.36 min, |
| | | MS (ESIpos.): *m*/*z* (%) = 371.3 (100) [M + H]⁺. |
| | | ¹H NMR (300 MHz, *d₆*-DMSO): δ = 0.88 (s, 9H), 1.10-1.26 (m, 2H), 1.33 (s, 9H), 1.33-1.68 (m, 8H), 1.97 (m, 2H), 4.23 (m, 1H), 6.53 (br s, 1H), 7.29 (d, 1H), 12.38 (br s, 1H). |
| | *N-*({1-[(*tert* Butoxycarbonyl)-amino]cyclohexyl}carbonyl)-4-methyl-L-leucin | Allgemeine Arbeitsvorschrift 18 aus Beispiel 56A. Ansatzgröße: 1.05 mmol. |
| | | Ausbeute: 90% d. Th. |
| 95A | | HPLC/UV-Vis (Methode 36): Rₜ = 4.96 min. |
| | 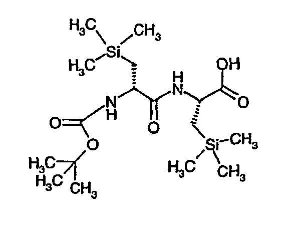 | LC-MS (Methode 26): Rₜ = 2.67 min, |
| | | MS (ESIpos.): *m*/*z* (%) = 405.3 (100) [M + H]⁺. |
| | | ¹H NMR (400 MHz, *d₆*-DMSO): δ = 0.00 (s, 18H), 0.85-1.08 (m, 4H), 1.38 (s, 9H), 4.04 (m, 1H), 4.16 (m, 1H), 6.77 (d, 1H), 7.72 (d, 1H), 12.45 (br s, 1H). |
| | *N-*(*tert*-Butoxycarbonyl)-3-trimethylsilyl)-D-alanyl-3-(trimethylsilyl)-L-alanin | Allgemeine Arbeitsvorschrift 17 aus Beispiel 57A. Ansatzgröße: 2.13 mmol. |
| | | Ausbeute: 53% d. Th. |
| 96A | 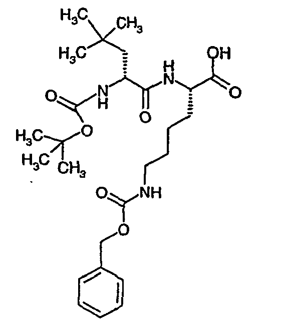 | HPLC/UV-Vis (Methode 36): Rₜ = 4.57 min. |
| | | LC-MS (Methode 26): Rₜ = 2.27 min, |
| | | MS (ESIpos.): *m*/*z* (%) = 508 (100) [M + H]⁺. |
| | | ¹H NMR (400 MHz, *d₆-* Aceton): δ = 0.95 (s, 9H), 1.40 (s, 9H), 1.40-1.98 (m, 6H), 3.16 (m, 2H), 4.19 (m, 1H), 4.42 (m, 1H), 5.08 (s, 2H), 6.16 (d, 1H), 6.42 (br s, 1H), 7.29-7.40 (m, 5H), 10.80 (br s, 1H). |
| | *N-*(*tert*-Butoxycarbonyl)- *3-tert-*butyl-D-alanyl-*N*⁶-[(benzyloxy)carbonyl]-L-lysin | Allgemeine Arbeitsvorschrift 17 aus Beispiel 58A. Ansatzgröße: 0.3 mmol. |
| | | Ausbeute: 91% d. Th. |

**Tabelle 3: N-Geschützte Nonadepsipeptide**

| **Bsp. Nr.** | **Struktur** | **Analytische Daten** |
|---|---|---|
| | **Name** | **Herstellungsmethode** |
| 97A | 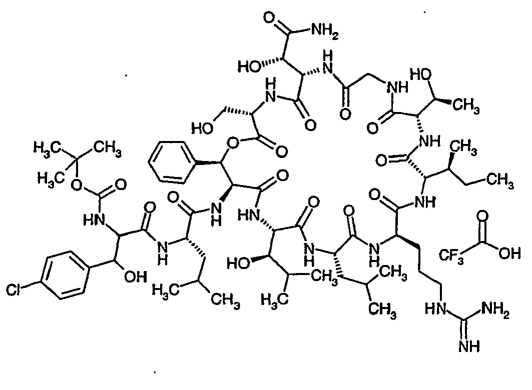 | HPLC/UV-Vis (Methode 13): Rₜ = 7.17 min. |
| | | LC-MS (Methode 26): Rₜ = 2.18 min; |
| | | MS (ESIpos.): *m*/*z* (%) = 681 (100) [M - Boc + 2H]²⁺, 1461 (20) [M + H]⁺. |
| | [*N*-*tert*-Butoxycarbonyl-3-(4-chlorphenyl)-*threo*-*rac*-seryl]-des(1-D-leucyl)lysobactin-trifluoracetat | Allgemeine Arbeitsvorschrift 11 aus Beispiel 11A (0.04 mmol) und *N-tert-*Butoxycarbonyl-3-(4-chlorphenyl)-threo-rac-serin (0.17 mmol). Reinigung nach Methode 7. |
| | | Ausbeute 34% d. Th. (trennbares Diastereomerengemisch) |
| 98A | 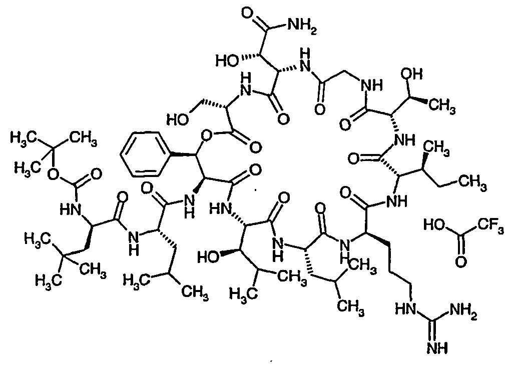 | HPLC/UV-Vis (Methode 13): Rₜ = 7.38 min. |
| | | LC-MS (Methode 26): Rₜ = 2.18 min; |
| | | MS (ESIpos.): *m*/*z* (%) = 646 (90) [M - Boc + 2H]²⁺, 1050 (20), 1391 (100) [M + H]⁺. |
| | *N-tert*-Butoxycarbonyl-3-*tert*-butyl-D-alanyldes(1-D-leucyl)lysobactin-trifluoracetat | Allg. Arbeitsvorschrift 4 aus Beispiel 11A (0.01 mmol) und *N-tert*-Butoxycarbonyl-3-*tert*-butyl-D-alanin (0.04 mmol). Reinigung nach Methode 7. |
| | | Ausbeute 65% d. Th. |
| 99A | 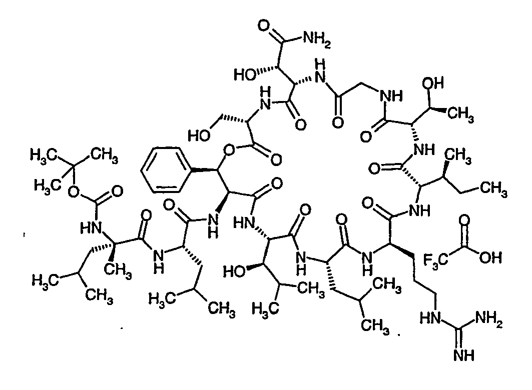 | HPLC/UV-Vis (Methode 13): Rₜ = 7.19 min. |
| | | LC-MS (Methode 26): Rₜ = 2.09 min; |
| | | MS (ESIpos.): *m*/*z* (%) = 646 (100) [M - Boc + 2H]²⁺, 1391 (30) [M + H]⁺. |
| | *N-tert*-Butoxycarbonyl-2-methyl-L-leucyl- des(1-D-leucyl)lysobactin-trifluoracetat | Allgemeine Arbeitsvorschrift 11 aus Beispiel 11A (0.11 mmol) und *N-(tert-*Butoxycarbonyl)-2-methyl-L-leucin (0.43 mmol). Reinigung nach Methode 7. |
| | | Ausbeute 13% d. Th. |
| 100A | 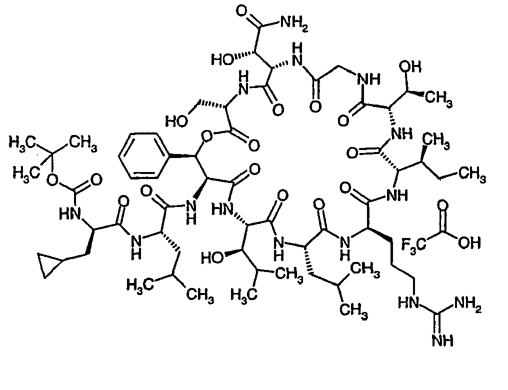 | HPLC/UV-Vis (Methode 13): Rₜ = 7.28 min. |
| | | LC-MS (Methode 26): Rₜ = 2.15 min; |
| | | MS (ESIpos.): *m*/*z* (%) = 638 (80) [M - Boc + 2H]²⁺, 688 (100), 1376 (15) [M+H]⁺. |
| | *N-tert*-Butoxycarbonyl-3-cyclopropyl-D-alanyl- des(1-D-leucyl)lysobactin-trifluoracetat | Allg. Arbeitsvorschrift 4 aus Beispiel 11A (0.01 mmol) und *N-*Cylohexylcyclohexan-aminium*-N-tert-*butoxycarbonyl-3-cyclopropyl-D-alaninat (0.04 mmol). Reinigung nach Methode 7. |
| | | Ausbeute 34% d. Th. |
| 101A | 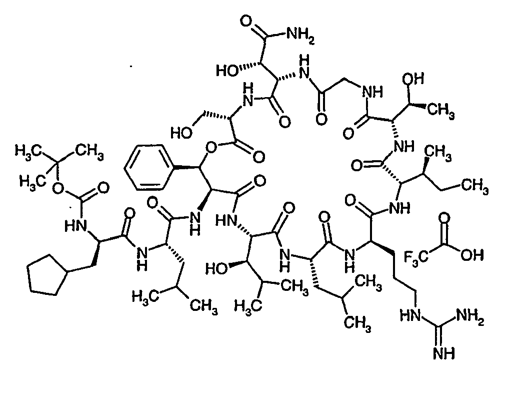 | HPLC/UV-Vis (Methode 13): Rₜ = 7.41 min. |
| | | LC-MS (Methode 26): Rₜ = 2.29 min; |
| | | MS (ESIpos.): *m*/*z* (%) = 652 (70) [M - Boc + 2H]²⁺, 702 (100), 1404 (30) [M + H]⁺. |
| | *N*-*tert*-Butoxycarbonyl-3-cylopentyl-D-alanyl-des(1-D-leucyl)lysobactin-trifluoroacetat | Allg. Arbeitsvorschrift 4 aus Beispiel 11A (0.01 mmol) und *N*-*tert*-Butoxycarbonyl-3-cyclopentyl-D-alanin (0.04 mmol). Reinigung nach Methode 7. |
| | | Ausbeute 57% d. Th. |
| 102A | 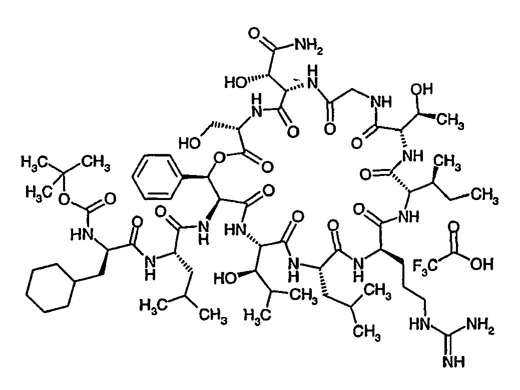 | HPLC/UV-Vis (Methode 13): Rₜ = 7.85 min. |
| | | LC-MS (Methode 26): Rₜ = 2.35 min; |
| | | MS (ESIpos.): *m*/*z* (%) = 709 (100) [M + 2H]²⁺, 718 (20) [M + H]⁺. |
| | *N*-*tert*-Butoxycarbonyl-3-cyclohexyl-D-alanyldes(1-D-leucyl)lysobactin-trifluoracetat | Allg. Arbeitsvorschrift 4 aus Beispiel 11A (0.01 mmol) und *N*-*tert*-Butoxycarbonyl-3-cyclohexyl-D-alanin (0.04 mmol). Reinigung nach Methode 7. |
| | | Ausbeute 50% d. Th. |
| 103A | 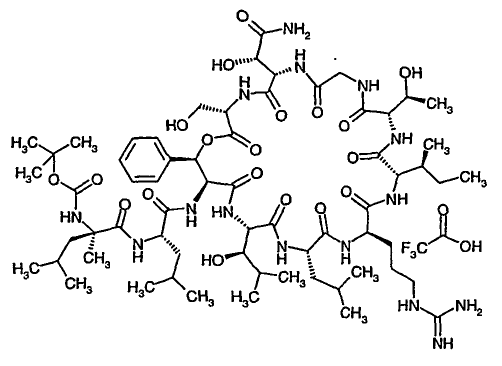 | HPLC/UV-Vis (Methode 13): Rₜ = 7.23 min. |
| | | LC-MS (Methode 26): Rₜ = 2.30 min; |
| | | MS (ESIpos.): *m*/*z* (%) = 646 (100) [M - Boc + 2H]²⁺, 1391 (30) [M + H]⁺. |
| | *N*-*tert*-Butoxycarbonyl-2-methyl-D-leucyldes(1-D-leucyl)lysobactin-trifluoracetat | Allg. Arbeitsvorschrift 4 aus Beispiel 11A (0.01 mmol) und *N*-*tert*-Butoxycarbonyl-2-methyl-D-leucin (0.04 mmol). Reinigung nach Methode 7. |
| | | Ausbeute 67% d. Th. |
| 104A | 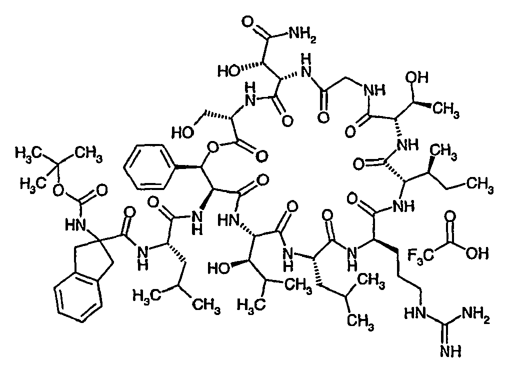 | HPLC/UV-Vis (Methode 13): Rₜ = 7.25 min. |
| | | LC-MS (Methode 26): Rₜ = 2.17 min; |
| | | MS (ESIpos.): *m*/*z* (%) = 662 (100) [M - Boc + 2H]²⁺, 1423 (30) [M + H]⁺. |
| | | HR-TOF-MS (Methode 21): C₆₇H₁₀₄N₁₅O₁₉ [M + H]⁺ ber. 1422.7633, gef. 1422.7612. |
| | (*N*-*tert*-Butoxycarbonyl-2-amino-2,3-dihydro-1*H*-indenylcarbonyl)-des(1-D-leucyl)lysobactin-trifluoracetat | Allgemeine Arbeitsvorschrift 11 aus Beispiel 11A (0.07 mmol) und *N-tert-*Butoxycarbonyl-2-amino-2,3-dihydro-1*H*-indenyl-carbonsäure (0.29 mmol). Reinigung nach Methode 7. |
| | | Ausbeute 76% d. Th. |
| 105A | 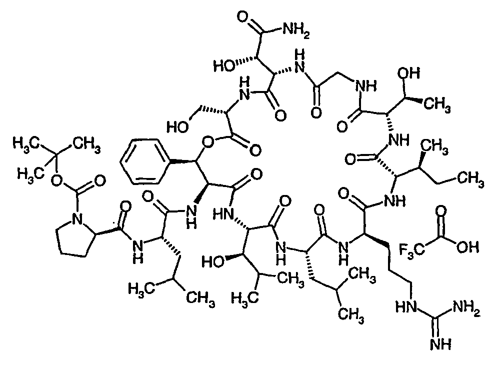 | HPLC/UV-Vis (Methode 13): Rₜ = 7.04 min. |
| | | LC-MS (Methode 26): Rₜ=2.10min; |
| | | MS (ESIpos.): *mlz* (%) = 631 (90) [M - Boc + 2H]²⁺, 681 (100), 1361 (30) [M + H]⁺. |
| | *N*-*tert*-Butoxycarbonyl-D-prolyl-des(1-D-leucyl)lysobactin-trifluoracetat | Allg. Arbeitsvorschrift 4 aus Beispiel 11A (0.01 mmol) und *N*-*tert*-Butoxycarbonyl-D-prolin (0.04 mmol). Reinigung nach Methode 7. |
| | | Ausbeute 55% d. Th. |
| 106A | 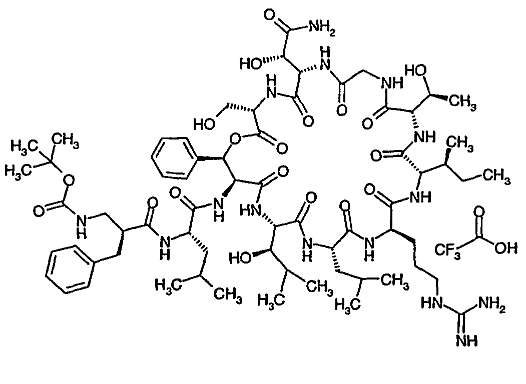 | LC-MS (Methode 26): Rₜ = 2.25 min; MS (ESIpos.): *m*/*z* (%) = 1424.9(15) [M + H]⁺; |
| | | MS (ESIneg.): *m*/*z* (%) = 711.0 (11) [M - 2H]²⁻, 1469.1 (18) [M +HCOOH- H]⁻. |
| | *(2S)*-2-Benzyl-3-(*tert*-butoxycarbonyl)amino- propanoyl-des(1-D-leucyl)lysobactin-trifluoracetat | Aus 50 mg Beispiel 11A und *(2S)*-2-Benzyl-3-(*tert*-butoxycarbonyl)amino-propionsäure (2 Äquivalente) nach allg. Arbeitsvorschrift 4 (2 Äquivalente HATU und |
| | | 4.5 Äquivalente *N*-Methylmorpholin) in Dimethylformamid bei Raumtemperatur und Chromatographie (Methode 8). |
| 107A | 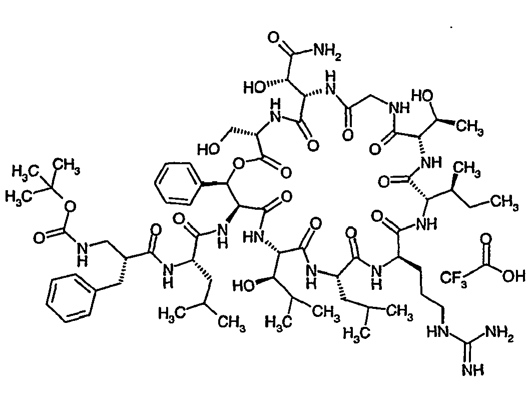 | LC-MS (Methode 26): Rₜ = 2.22 min (100%); |
| | | MS (ESIpos.): *m*/*z* (%) = 1424.7(15) [M + H]⁺; |
| | | MS (ESIneg.): *m*/*z* (%) = 711.1(100) [M - 2H]²⁻, 1468.9 (28) [M +HCOOH-H]⁻. |
| | *(2R)*-2-Benzyl-3-(*tert*-butoxycarbonyl)amino-propanoyl-des(1-D-leucyl)lysobactin-trifluoracetat | Analog Beispiel 106A aus Beispiel 11A und *(2R)*-2-Benzyl-3-(*tert*-butoxy-carbonyl)amino-propionsäure. |
| 108A | 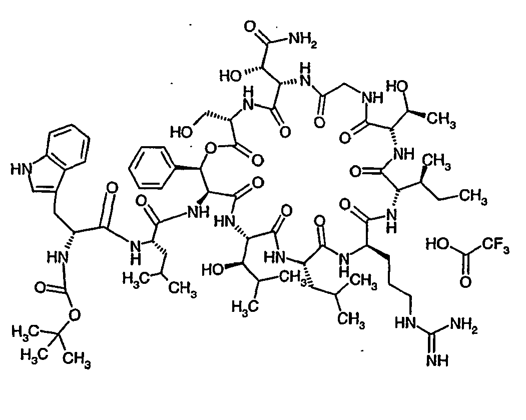 | HPLC/UV-Vis (Methode 36): Rₜ = 4.13 min. |
| | | LC-MS (Methode 26): Rₜ = 2.12 min, |
| | | MS (ESIpos.): *m*/*z* (%) = 725.9 (100) [M + 2H]²⁺. |
| | *N*-(*tert*-Butoxycarbonyl)-D-tryptophyl-des(1-D-leucyl)-lysobactin-trifluoracetat | Allgemeine Arbeitsvorschrift 19 aus Beispiel 11A (36 µmol) und *N-(tert-*Butoxycarbonyl)-D-Tryptophan (36 µmol). |
| | | Ausbeute: 84% d. Th. |
| 109A | 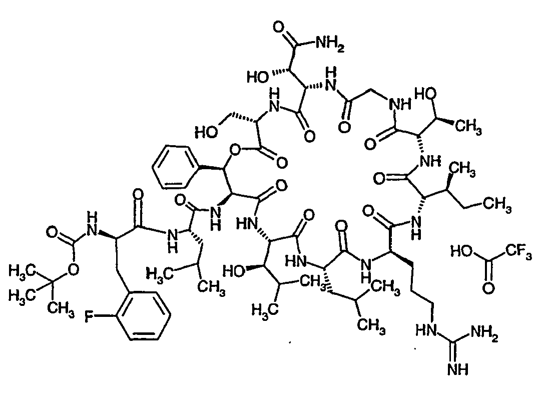 | HPLC/UV-Vis (Methode 36): Rₜ = 4.26 min. |
| | | LC-MS (Methode 26): Rₜ = 2.23 min, |
| | | MS (ESIpos.): *m*/*z* (%) = 1430.2 [M + H]⁺. |
| | *N*-(*tert*-Butoxycarbonyl)-2-fluor-D-phenylalanyl-des(1-D-leucyl)lysobactin-trifluoracetat | Allgemeine Arbeitsvorschrift 19 aus Beispiel 11A (40 µmol) und *N-tert-*Butoxycarbonyl-2-fluor-D-phenylalanin. |
| | | Ausbeute: 76% d. Th. |
| 110A | 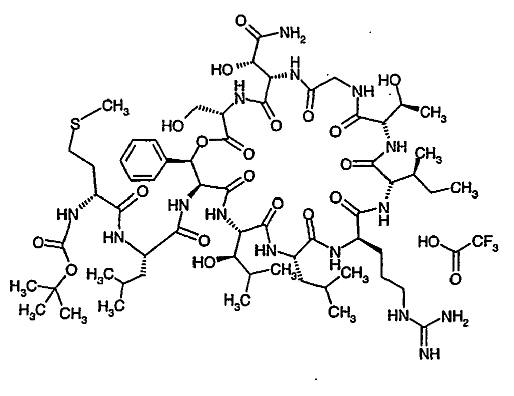 | HPLC/UV-Vis (Methode 36): Rₜ = 4.12 min. |
| | | LC-MS (Methode 26): Rₜ = 2.13 min, |
| | | MS (ESIpos.): *m*/*z* (%) = 698.3 [M + 2H]²⁺. |
| | *N*-(*tert*-Butoxycarbonyl)-D-methionyl-des(1-D-leucyl)-lysobactin-bistrifluoracetat | Allgemeine Arbeitsvorschrift 19 aus Beispiel 11A (140 µmol) und *N-tert-*Butoxycarbonyl-D-methionin. |
| | | Ausbeute: 64% d. Th. |
| 111A | 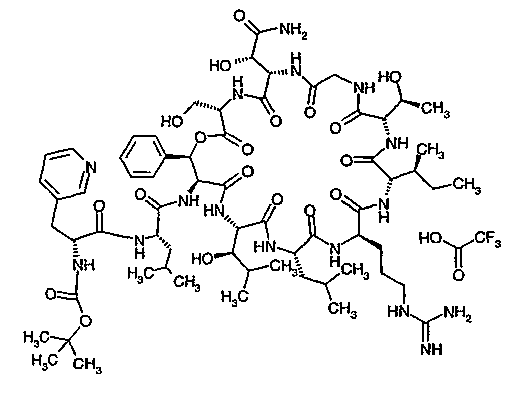 | HPLC/UV-Vis (Methode 36): Rₜ = 3.58 min. |
| | | LC-MS (Methode 26): Rₜ = 1.85 min, |
| | | MS (ESIpos.): *m*/*z* (%) = 706.9 (100) [M + 2H]²⁺. |
| | [*N*-(*tert*-Butoxycarbonyl)-3-pyrid-3-yl-D-alanyl]-des(1-D-leucyl)lysobactintrifluoracetat | Allgemeine Arbeitsvorschrift 19 aus Beispiel 11A (36 µmol) und (*N-tert-* Butoxycarbonyl-3-(3-pyridyl)-D-alanin. |
| | | Ausbeute: 68% d. Th. |
| 112A | 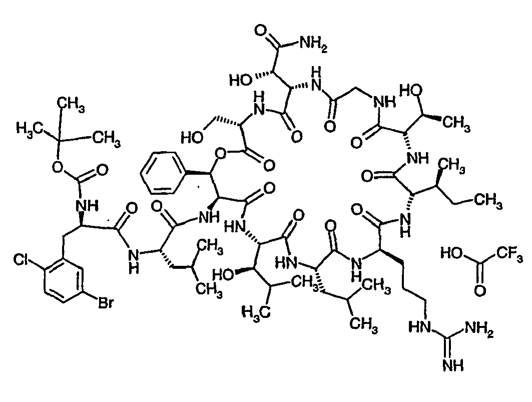 | HPLC/UV-Vis (Methode 36): Rₜ = 4.46 min. |
| | | LC-MS (Methode 26): Rₜ = 2.38 min, |
| | | MS (ESIpos.): *m*/*z* (%) = 1522.9 [M + H]⁺. |
| | | HR-TOF-MS (Methode 21): ber. 1522.6348, gef. 1522.6373 [M + H]⁺. |
| | [3-Brom-*N*-(*tert*-butoxycarbonyl)-6-chlor-D-phenylalanyl]-des(1-D-leucyl)lysobactin-trifluor-acetat | Allgemeine Arbeitsvorschrift 19 aus Beispiel 11A (36 µmol) und 3-Brom-*N*-(*tert*-butoxycarbonyl)-6-chlor-D-phenylalanin. |
| | | Ausbeute: 85% d. Th. |
| 113A | 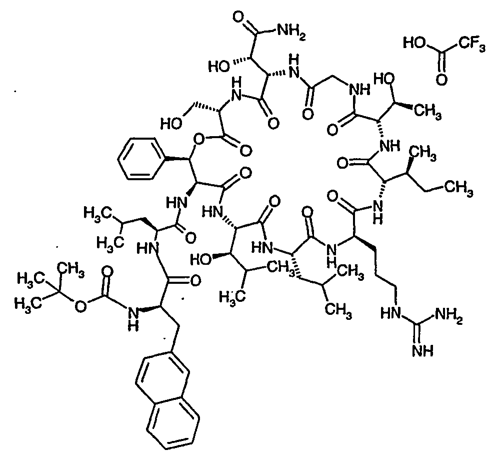 | HPLC/UV-Vis (Methode 36): Rₜ = 4.39 min. |
| | | LC-MS (Methode 26): Rₜ = 2.40 min, |
| | | MS (ESIpos.): *m*/*z* (%) = 1461.2 (10) [M + H]⁺. |
| | *N*-[(*tert*-Butoxycarbonyl)-3-(2-naphthyl)-D-alanyl]- des(1-D-leucyl)lysobactin-trifluoracetat | Allgemeine Arbeitsvorschrift 19 aus Beispiel 11A (36 µmol) und *N-*(*tert-*Butoxycarbonyl)-3-(2-naphthyl)-D-alanin. |
| | | Ausbeute: 72% d. Th. |
| 114A | 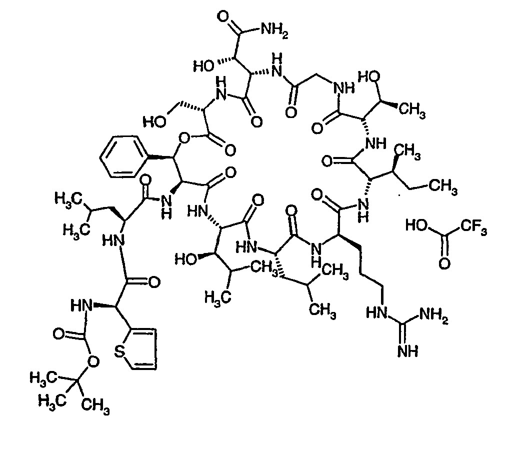 | HPLC/UV-Vis (Methode 36): Rₜ = 4.03 min. |
| | | LC-MS (Methode 26): Rₜ = 2.11 min, |
| | | MS (ESIpos.): *m*/*z* (%) = 1402.1 (1) [M + H]⁺. |
| | (2*R*)-[(*tert*-Butoxycarbonyl)amino]-(2-thienyl)acetyl-des(1-D-leucyl)lysobactin-trifluoracetat | Allgemeine Arbeitsvorschrift 19 aus Beispiel 11A (36 µmol) und (2*R*)-Butoxycarbonylamino-(2-thienyl)essigsäure. |
| | | Ausbeute: 20% d. Th. |
| 115A | 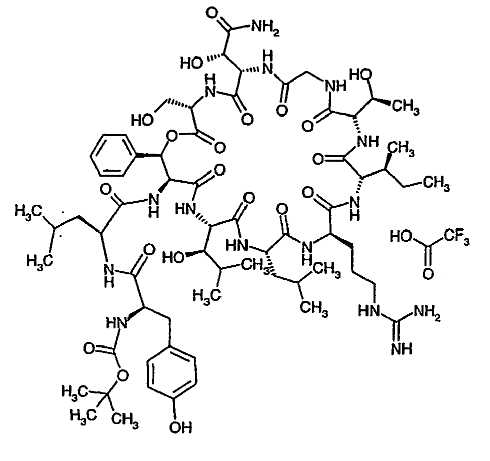 | HPLC/UV-Vis (Methode 36): Rₜ = 4.43 min. |
| | | LC-MS (Methode 26): Rₜ = 2.35 min, |
| | | MS (ESIpos.): *m*/*z* (%) = 1482.8 (10) [M + H]⁺. |
| | *N*-(*tert*-Butoxycarbonyl)-D-tyrosyl-des(1-D-leucyl)-lysobactin-trifluoracetat | Allgemeine Arbeitsvorschrift 19 aus Beispiel 11A (36 µmol) und *N-(tert-*Butoxycarbonyl)-D-Tyrosin. |
| | | Ausbeute: 70% d. Th. |
| 116A | 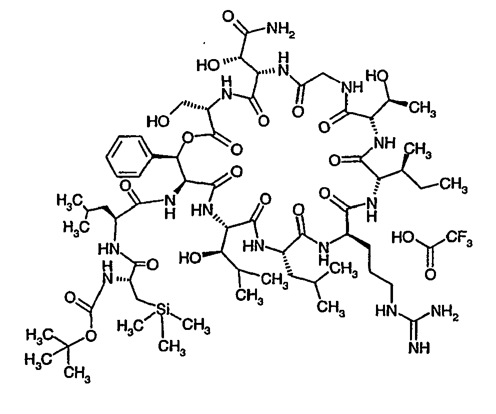 | HPLC/UV-Vis (Methode 36): Rₜ = 4.41 min. |
| | | LC-MS (Methode 26): Rₜ = 2.28 min, |
| | | MS (ESIpos.): *m*/*z* (%) = 1407 (15) [M + H]⁺. |
| | *N*-(*tert*-Butoxycarbonyl)-3-(Trimethylsilyl)-L-alanyl-des(1-D-leucyl)lysobactin-trifluoracetat | Allgemeine Arbeitsvorschrift 19 aus Beispiel 11A (287 µmol) und Beispiel 26A |
| | | Ausbeute: 75% d. Th. |
| 117A | 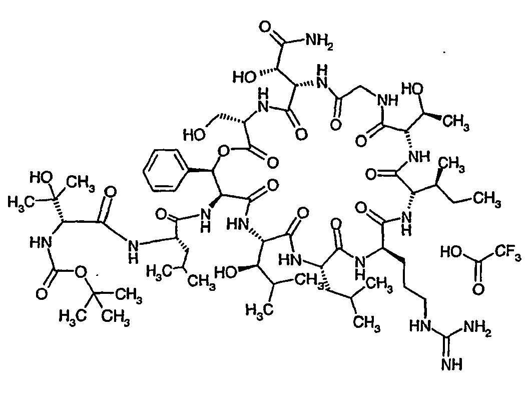 | HPLC/UV-Vis (Methode 36): Rₜ = 3.91 min. |
| | | LC-MS (Methode 26): Rₜ = 1.92 min, |
| | | MS (ESIpos.): *m*/*z* (%) = 1379.1 (20) [M + H]⁺. |
| | *N*-(*tert*-Butoxycarbonyl)-(3-hydroxy)-D-valyl-des(1-D-leucyl)lysobactin-trifluoracetat | Allgemeine Arbeitsvorschrift 19 aus Beispiel 11A (36 µmol) und *N-(tert-*Butoxycarbonyl)-(3-hydroxy)-D-valin. |
| | | Ausbeute: 54% d. Th. |
| 118A | 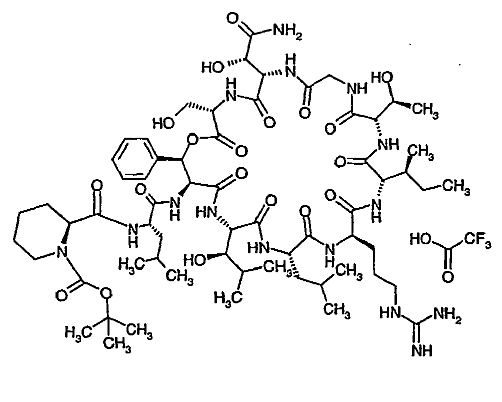 | HPLC/UV-Vis (Methode 36): Rₜ = 4.38 min. |
| | | LC-MS (Methode 26): Rₜ = 2.26 min, |
| | | MS (ESIpos.): *m*/*z* (%) = 1375.0 (10) [M + H]⁺. |
| | *N*-(*tert*-Butoxycarbonyl)-L-pipecolyl-des(1-D-leucyl)- lysobactin-trifluoracetat | Allgemeine Arbeitsvorschrift 19 aus Beispiel 11A (36 µmol) und *N-(tert-*Butoxycarbonyl)-L-pipecolinsäure. |
| | | Ausbeute: 62% d. Th. |
| 119A | 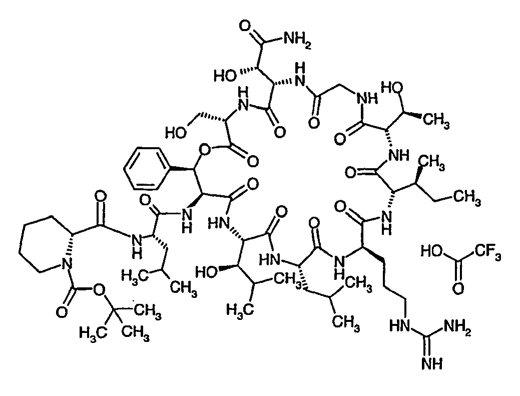 | IPLC/UV-Vis (Methode 36): Rₜ = 4.3 min. |
| | | LC-MS (Methode 26): Rₜ = 2.21 min, |
| | | MS (ESIpos.): *m*/*z* (%) = 1375.0 (10) [M + H]⁺. |
| | *N*-(*tert*-Butoxycarbonyl)-D-pipecolyl-des(1-D-leucyl)- lysobactin-trifluoracetat | Allgemeine Arbeitsvorschrift 19 aus Beispiel 11A (36 µmol) und *N-(tert-*Butoxycarbonyl)-D-pipecolinsäure. |
| | | Ausbeute: 64% d. Th. |
| 120A | 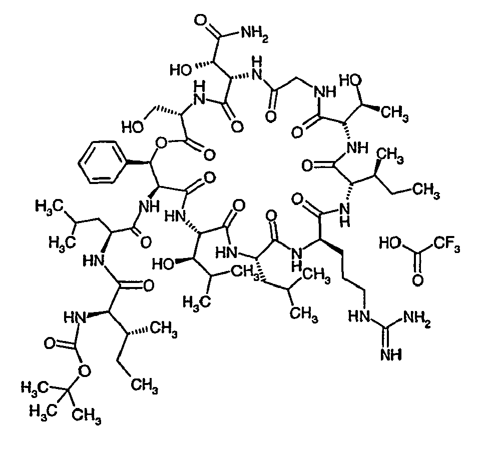 | LC-MS (Methode 26): Rₜ = 2.23 min, |
| | | MS (ESIneg.): *m*/*z* (%) = 1375.3 (20) [M - H]⁻. |
| | *N*-(*tert*-Butoxycarbonyl)-D-isoleucyl-des(1-D-leucyl)-lysobactin-trifluoracetat | Allgemeine Arbeitsvorschrift 19 aus Beispiel 11A (36 µmol) und *N-(tert-*Butoxycarbonyl)-D-isoleucin. |
| | | Ausbeute: 58% d. Th. |
| 121A | 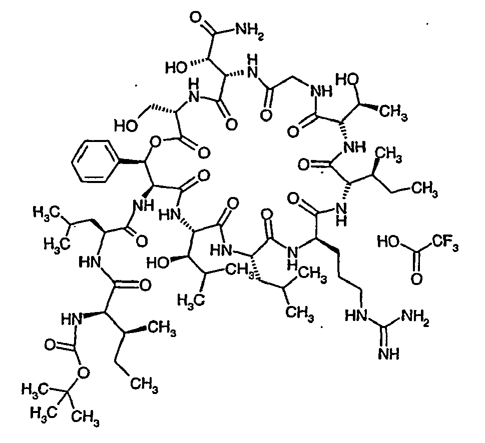 | LC-MS (Methode 26): Rₜ = 2.15 min, |
| | | MS (ESIpos.): *m*/*z* (%) = 1377.2 (20) [M + H]⁺. |
| | *N*-(*tert*-Butoxycarbonyl)-D-alloisoleucyl-des(1-D-leucyl)lysobactin-trifluoracetat | Allgemeine Arbeitsvorschrift 19 aus Beispiel 11A (36 µmol) und *N-*(*tert-*Butoxycarbonyl)-D-alloisoleucin. |
| | | Ausbeute: 49% d. Th. |
| 122A | 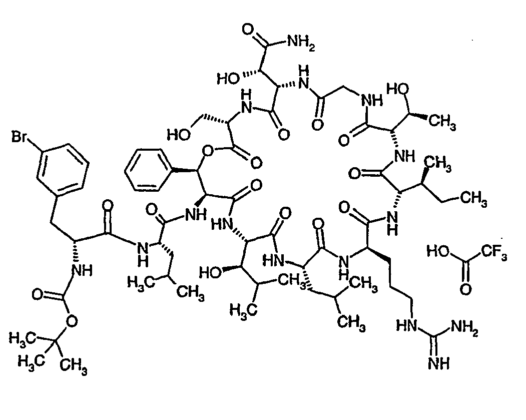 | HPLC/UV-Vis (Methode 36): Rₜ = 4.4 min. |
| | | LC-MS (Methode 26): Rₜ = 2.32 min, |
| | | MS (ESIpos.): *m*/*z* (%) = 1488.9 (1) [M + H]⁺. |
| | 3-Brom-*N*-(*tert*-butoxycarbonyl)-D-phenylalanyl- des(1-D-leucyl)lysobactin-trifluoracetat | Allgemeine Arbeitsvorschrift 19 aus Beispiel 11A (36 µmol) und 3-Brom-*N*-(*tert*-butoxycarbonyl)-D-phenylalanin. |
| | | Ausbeute: 44% d. Th. |
| 123A | 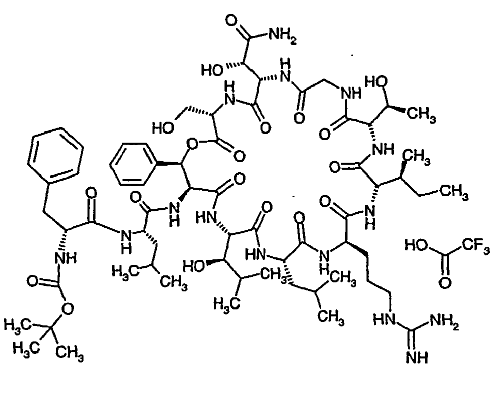 | HPLC/UV-Vis (Methode 36): Rₜ = min. |
| | | LC-MS (Methode 26): Rₜ = 2.18 min, |
| | | MS (ESIpos.): *m*/*z* (%) = 1411.2 [M + H]⁺. |
| | *N*-(*tert*-Butoxycarbonyl)-D-phenylalanyl-des(1-D-leucyl)lysobactin-trifluoracetat | Allgemeine Arbeitsvorschrift 19 aus Beispiel 11A (80 µmol) und *N-*(*tert-*Butoxycarbonyl)-D-phenylalanin. |
| | | Ausbeute: 55% d. Th. |
| 124A | 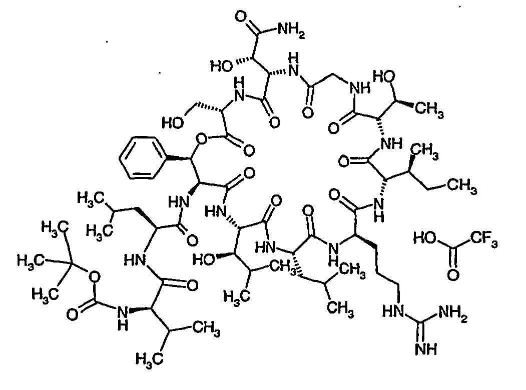 | HPLC/UV-Vis (Methode 36): Rₜ = 4.16 min. |
| | | LC-MS (Methode 26): Rₜ = 2.16 min, |
| | | MS (ESIpos.): *mlz* (%) = 1361.7 /20) [M + H]⁺. |
| | *N*-(*tert*-Butoxycarbonyl)-D-valyl-des(1-D-leucyl)-lysobactin-trifluoracetat | Allgemeine Arbeitsvorschrift 19 aus Beispiel 11A (50 µmol) und *N-*(*tert-*Butoxycarbonyl)-D-valin. |
| | | Ausbeute: 58% d. Th. |
| 125A | 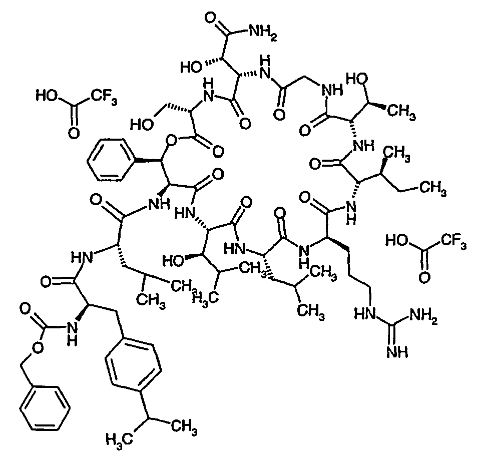 | HPLC/UV-Vis (Methode 36): Rₜ = 4.48 min. |
| | | LC-MS (Methode 26): Rₜ = 2.42 min, |
| | | MS (ESIpos.): *m*/*z* (%) = 1487.3 (20) [M + H]⁺. |
| | *N*-(Benzyloxycarbonyl)-(4-isopropyl-D-phenylalanyl) -des(1-D-leucyl)lysobactin-trifluoracetat | Allgemeine Arbeitsvorschrift 19 aus Beispiel 11A (36 µmol) und Beispiel 32A. |
| | | Ausbeute: 68% d. Th. |
| 126A | 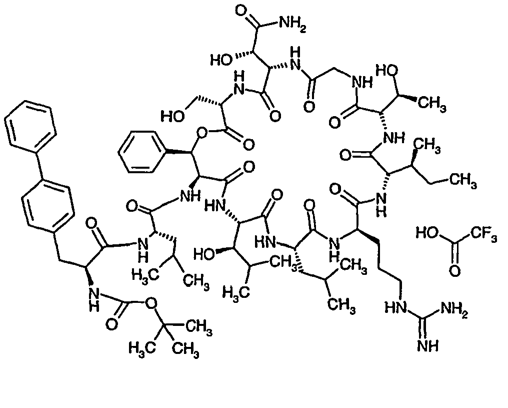 | HPLC/UV-Vis (Methode 36): Rₜ = 4.42 min. |
| | | LC-MS (Methode 26): Rₜ = 2.09 min, |
| | | MS (ESIpos.): *m*/*z* (%) = 1487.1 (10) [M + H]⁺. |
| | *N-*(*tert*-Butoxycarbonyl)-3-biphenyl-L-alanyl -des(1-D-leucyl)lysobactin-trifluoracetat | Allgemeine Arbeitsvorschrift 19 aus Beispiel 11A (110 µmol) und *N-*(*tert-*Butoxycarbonyl)-2-amino-3-biphenyl-L-alanin. |
| | | Ausbeute: 66% d. Th. |
| 127A | 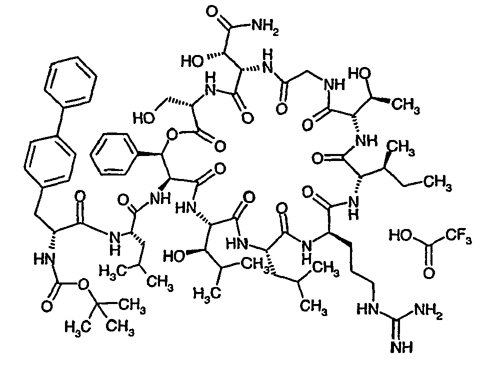 | HPLC/UV-Vis (Methode 36): Rₜ = 4.43 min. |
| | | LC-MS (Methode 26): Rₜ = 2.16 min, |
| | | MS (ESIpos.): *m*/*z* (%) = 1487.1 (10) [M + H]⁺. |
| | | HR-TOF-MS (Methode 21): ber. 1486.7946, gef. 1486.8008 [M + H]⁺. |
| | *N*-(*tert*-Butoxycarbonyl)-3-biphenyl-D-alanyldes(1-D-leucyl)lysobactin-trifluoracetat | Allgemeine Arbeitsvorschrift 19 aus Beispiel 11A (110 µmol) und *N-(tert-*Butoxycarbonyl)-2-Amino-3-biphenyl-D-alanin. |
| | | Ausbeute: 62% d. Th. |
| 128A | 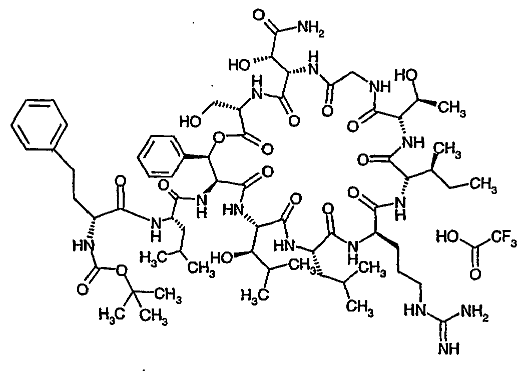 | HPLC/UV-Vis (Methode 36): Rₜ = 4.32 min. |
| | | LC-MS (Methode 26): Rₜ = 2.01 min, |
| | | MS (ESIpos.): *m*/*z* (%) = 1425.2 (20) [M + H]⁺. |
| | *N*-(*tert*-Butoxycarbonyl)-(2*R*)-2-Amino-4-phenylbutanoyldes(1-D-leucyl)lysobactin-trifluoracetat | Allgemeine Arbeitsvorschrift 19 aus Beispiel 11A (50 µmol) und *N-(tert-*Butoxycarbonyl)-(2*R*)-2-Amino-4-phenylbuttersäure. |
| | | Ausbeute: 79% d. Th. |
| 129A | 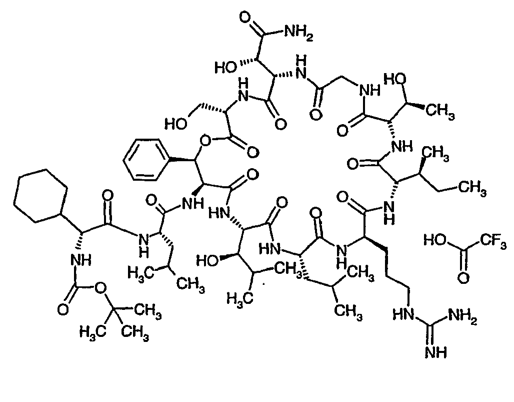 | HPLC/UV-Vis (Methode 36): Rₜ = 4.45 min. |
| | | LC-MS (Methode 26): Rₜ = 2.05 min, |
| | | MS (ESIpos.): *m*/*z* (%) = 1402.3 (10) [M + H]⁺. |
| | *N*-(*tert*-Butoxycarbonyl)-(2*R*)-amino(cyclohexyl)acetyl-des(1-D-leucyl)lysobactin-trifluoracetat | Allgemeine Arbeitsvorschrift 19 aus Beispiel 11A (100 µmol) und *N-*(*tert-*Butoxycarbonyl)-(2*R*)-amino(cyclohexyl)essigsäure. |
| | | Ausbeute: 28% d. Th. |
| 130A | 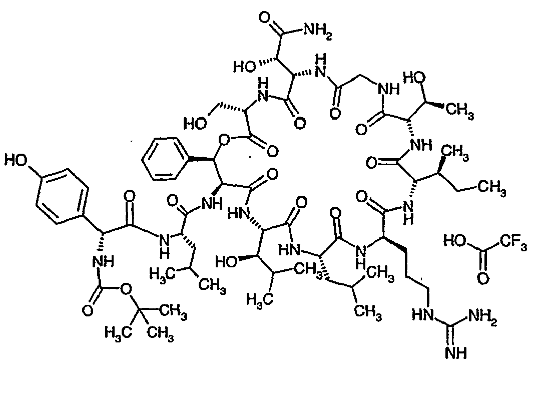 | HPLC/UV-Vis (Methode 36): Rₜ=3.81min. |
| | | LC-MS (Methode 26): Rₜ = 1.71 min, |
| | | MS (ESIpos.): *mlz* (%) = 1413.2 (10) [M + H]⁺. |
| | *N*-(*tert*-Butoxycarbonyl)-(2*R*)-amino(4-hydroxyphenyl)acetyl -des(1-D-leucyl)lysobactin-trifluoracetat | Allgemeine Arbeitsvorschrift 19 aus Beispiel 11A (100 µmol) und *N-(tert-*Butoxycarbonyl)-(2*R*)-amino(4-hydroxyphenyl)-essigsäure. |
| | | Ausbeute: 18% d. Th. |
| 131A | 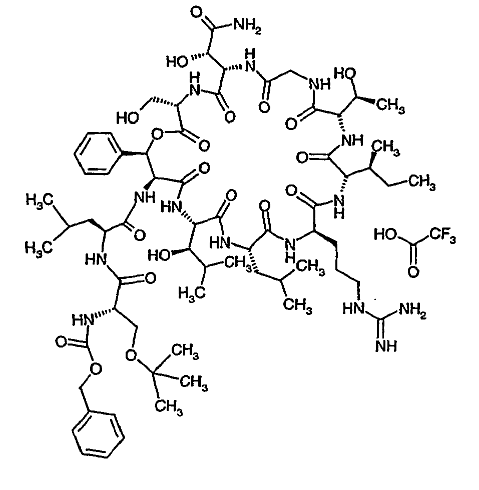 | HPLC/UV-Vis (Methode 36): Rₜ = 4.25 min. |
| | | LC-MS (Methode 26): Rₜ = 2.06 min, |
| | | MS (ESIpos.): *m*/*z* (%) = 1441.1 (30) [M + H]⁺. |
| | *N*-(Benzyloxycarbonyl)-*O*-(*tert*-butyl)-L-seryldes(1-D-leucyl)lysobactin-trifluoracetat | Allgemeine Arbeitsvorschrift 19 aus Beispiel 11A (110 µmol) und *N-(tert-*Butoxycarbonyl)-*O*-(*tert*-butyl)-L-serin. |
| | | Ausbeute: 70%. |
| 132A | 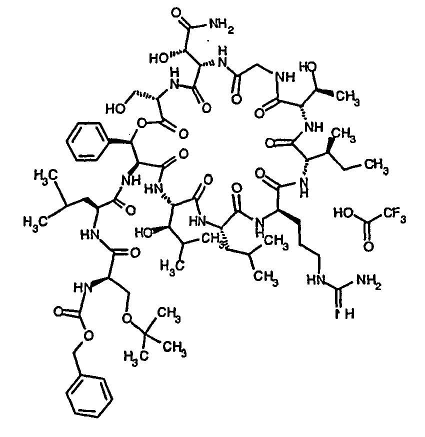 | HPLC/UV-Vis (Methode 36): Rₜ = 4.11 min. |
| | | LC-MS (Methode 26): Rₜ = 2.08 min, |
| | | MS (ESIpos.): *m*/*z* (%) = 1441.0 (80) [M + H]⁺. |
| | *N*-(Benzyloxycarbonyl)-*O*-(*tert*-butyl)-D-seryldes(1-D-leucyl)lysobactin-trifluoracetat | Allgemeine Arbeitsvorschrift 19 aus Beispiel 11A (310 µmol) und *N-*(*tert-*Butoxycarbonyl)-*O*-(*tert*-butyl)-D-serin. |
| | | Ausbeute: 47% d. Th. |
| 133A | 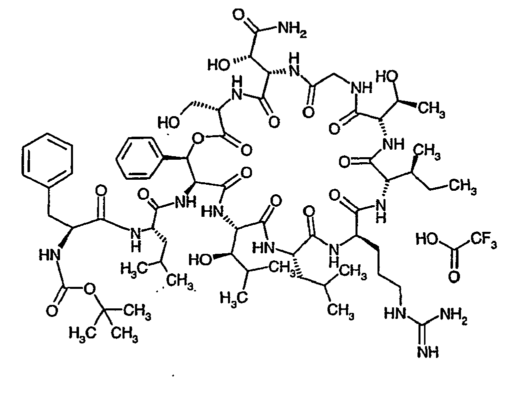 | HPLC/UV-Vis (Methode 36): Rₜ = 4.23 min. |
| | | LC-MS (Methode 26): Rₜ = 2.30 min, |
| | | MS (ESIpos.): *m*/*z* (%) = 1411.0 (30) [M + H]⁺. |
| | *N*-(*tert*-Butoxycarbonyl)-L-phenylalanyldes(1-D-leucyl)lysobactin-trifluoracetat | Allgemeine Arbeitsvorschrift 19 aus Beispiel 11A (110 µmol) und *N-*(*tert-*Butoxycarbonyl)-L-phenylalanin. |
| | | Ausbeute: 72% d. Th. |
| 134A | 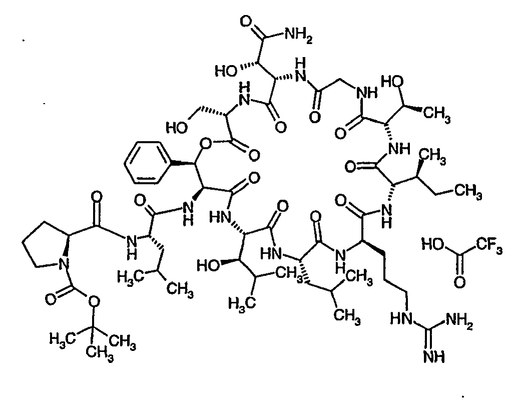 | HPLC/UV-Vis (Methode 36): Rₜ = 4.18 min. |
| | | LC-MS (Methode 26): Rₜ = 1.96 min, |
| | | MS (ESIpos.): *m*/*z* (%) = 1361.1 (10) [M + H]⁺. |
| | *N*-(*tert*-Butoxycarbonyl)-L-prolyl-des(1-D-leucyl)-lysobactin-trifluoracetat | Allgemeine Arbeitsvorschrift 19 aus Beispiel 11A (70 µmol) und *N-(tert-*Butoxycarbonyl)-L-prolin. |
| | | Ausbeute: 73% d. Th. |
| 135A | 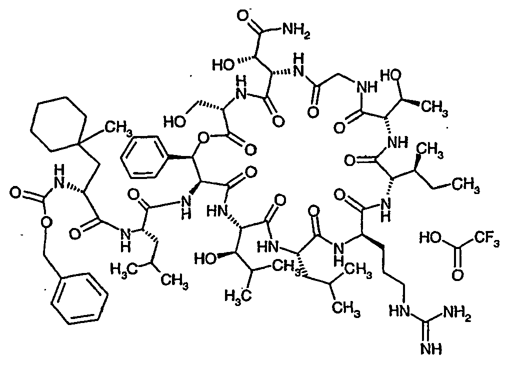 | HPLC/UV-Vis (Methode 36): Rₜ = 4.54 min. |
| | | LC-MS (Methode 26): Rₜ = 2.15 min, |
| | | MS (ESIpos.): *mlz* (%) = 1465.2 (40) [M + H]⁺. |
| | 3-(1-Methylcyclohexyl)-D-alanyl-des(1-D-leucyl)-lysobactin-trifluoracetat | Allgemeine Arbeitsvorschrift 19 aus Beispiel 11A (90 µmol) und Beispiel 29A. |
| | | Ausbeute: 66% d. Th. |
| 136A | 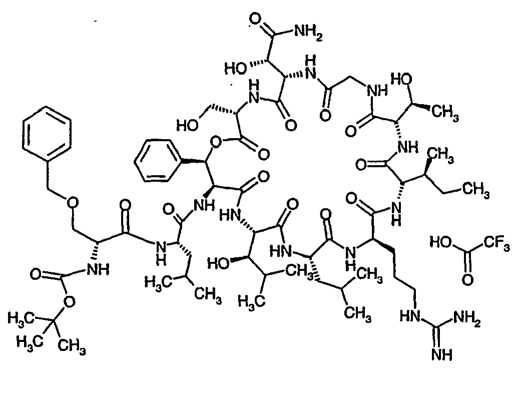 | HPLC/UV-Vis (Methode 36): Rₜ = 4.25 min. |
| | | LC-MS (Methode 26): Rₜ = 2.07 min, |
| | | MS (ESIpos.): *m*/*z* (%) = 1440.9 (30) [M + H]⁺. |
| | *N*-(*tert*-Butoxycarbonyl)-*O*-benzyl-D-seryldes(1-D-leucyl)lysobactin-trifluoracetat | Allgemeine Arbeitsvorschrift 19 aus Beispiel 11A (110 µmol) und *N-(tert-*Butoxycarbonyl)-*O*-benzyl-D-serin. |
| | | Ausbeute: 56% d. Th. |
| 137A | 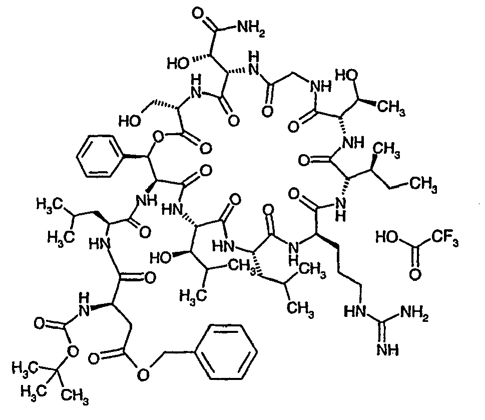 | HPLC/UV-Vis (Methode 36): Rₜ = 4.25 min. |
| | | LC-MS (Methode 26): Rₜ = 2.21 min, |
| | | MS (ESIpos.): *m*/*z* (%) = 1569.2 (10) [M + H]⁺. |
| | (2*R*)-4-(Benzyloxy)-2-[(*tert*-butoxycarbonyl)amino]-4-oxobutanoyl-des(1-D-leucyl)lysobactin-trifluoracetat | Allgemeine Arbeitsvorschrift 19 aus Beispiel 11A (180 µmol) und (2*R*)-4-(Benzyloxy)-2-[(*tert*-but-oxycarbonyl)amino]-4-oxo-buttersäure. |
| | | Ausbeute: 65% d. Th. |
| 138A | 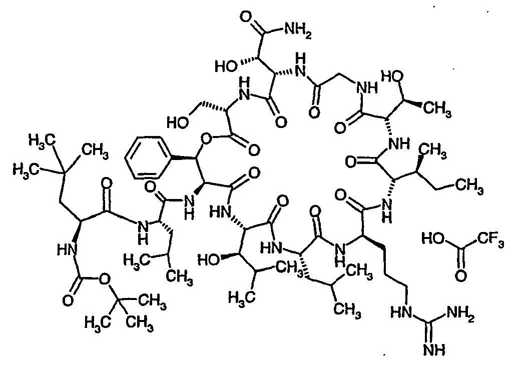 | HPLC/UV-Vis (Methode 36): Rₜ = 4.33 min. |
| | | LC-MS (Methode 26): Rₜ = 2.07 min, |
| | | MS (ESIpos.): *mlz* (%) = 1391.4 (5) [M + H]⁺. |
| | *N*-(*tert*-Butoxycarbonyl)-3-(*tert*-butyl)-L-alanyldes(1-D-leucyl)lysobactin-trifluoracetat | Allgemeine Arbeitsvorschrift 19 aus Beispiel 11A (110 µmol) und *N-*(*tert-*Butoxycarbonyl)-3-(*tert*-butyl)-L-alanin. |
| | | Ausbeute: 70% d. Th. |
| 139A | 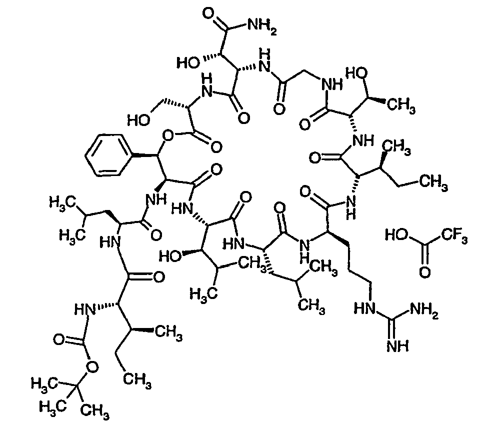 | HPLC/UV-Vis (Methode 36): Rₜ = 4.21 min. |
| | | LC-MS (Methode 26): Rₜ = 1.99 min, |
| | | MS (ESIpos.): *m*/*z* (%) = 1377.6 (1) [M + H]⁺. |
| | | HR-TOF-MS (Methode 21): ber. 1376.7789, gef. 1376.7765 [M + H]⁺. |
| | *N*-(*tert*-Butoxycarbonyl)-L-isoleucyl-des(1-D-leucyl)-lysobactin-trifluoracetat | Allgemeine Arbeitsvorschrift 19 aus Beispiel 11A (110 µmol) und *N-(tert-*Butoxycarbonyl)-L-isoleucin. |
| | | Ausbeute: 80% d. Th. |
| 140A | 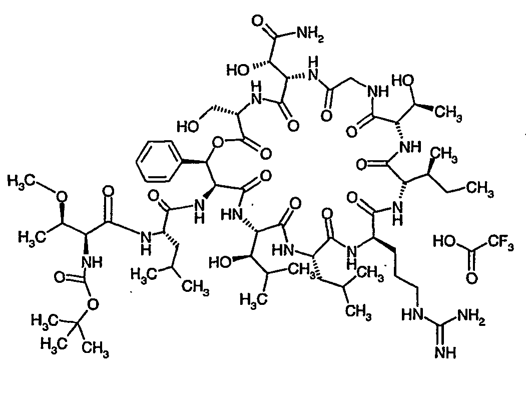 | HPLC/UV-Vis (Methode 36): Rₜ = 4.05 min. |
| | | LC-MS (Methode 26): Rₜ = 1.85 min, |
| | | MS (ESIpos.): *m*/*z* (%) = 1379.3 (80) [M + H]⁺. |
| | | HR-TOF-MS (Methode 21): ber. 1378.7582, gef. 1378.7576 [M + H]⁺ |
| | *N*-(*tert*-Butoxycarbonyl)-*O*-methyl-L-threonyldes(1-D-leucyl)lysobactin-trifluoracetat | Allgemeine Arbeitsvorschrift 19 aus Beispiel 11A (110 µmol) und *N-(tert-*Butoxycarbonyl)-*O*-methyl-L-threonin. |
| | | Ausbeute: 47% d. Th. |
| 141A | 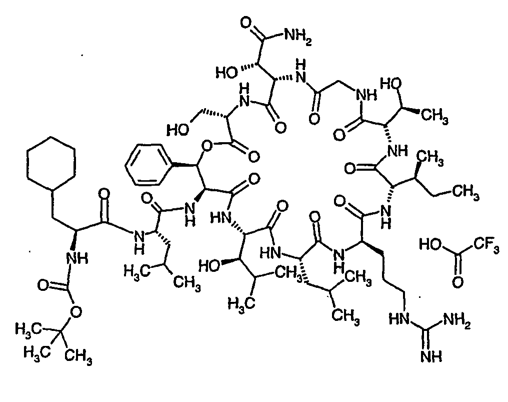 | HPLC/UV-Vis (Methode 36): Rₜ = 4.45 min. |
| | | LC-MS (Methode 26): Rₜ = 2.11 min, |
| | | MS (ESIpos.): *m*/*z* (%) = 1417.3 (10) [M + H]⁺. |
| | | HR-TOF-MS (Methode 21): ber. 1416.8102, gef. 1416.8116 [M + H]⁺. |
| | *N*-(*tert*-Butoxycarbonyl)-3-cyclohexyl-L-alanyldes(1-D-leucyl)lysobactin-trifluoracetat | Allgemeine Arbeitsvorschrift 19 aus Beispiel 11A (110 µmol) und *N-*(*tert-*Butoxycarbonyl)-3-cyclo-hexyl-L-alanin. |
| | | Ausbeute: 80% d. Th. |
| 142A | 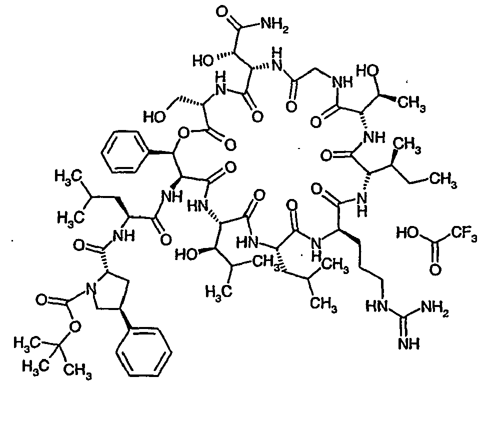 | HPLC/UV-Vis (Methode 36): Rₜ = 4.44 min. |
| | | LC-MS (Methode 26): Rₜ = 2.13 min, |
| | | MS (ESIpos.): *m*/*z* (%) = 1437 (20) [M + H]⁺. |
| | *N*-(*tert*-Butoxycarbonyl)-(4*S*)-4-phenyl-L-prolyldes(1-D-leucyl)lysobactin-trifluoracetat | Allgemeine Arbeitsvorschrift 19 aus Beispiel 11A (110 µmol) und *N-*(*tert-*Butoxycarbonyl)- (4*S*)-4-phenyl-L-prolin. |
| | | Ausbeute: 63% d. Th. |
| 143A | 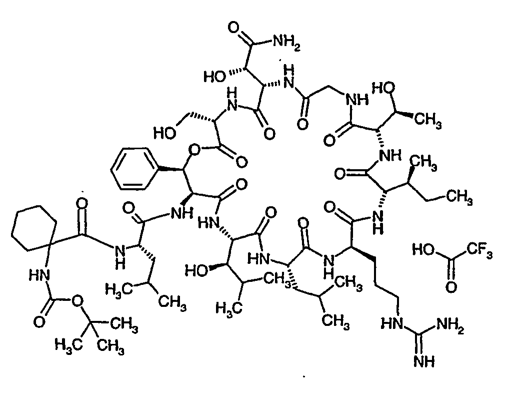 | HPLC/UV-Vis (Methode 36): Rₜ = 4.28 min. |
| | | LC-MS (Methode 26): Rₜ = 2.06 min, |
| | | MS (ESIpos.): *m*/*z* (%) = 1388.9 (10) [M + H]⁺. |
| | *N*-(*tert*-Butoxycarbonyl)-(1-aminocyclohexyl)carbonyldes(1-D-leucyl)lysobactin-trifluoracetat | Allgemeine Arbeitsvorschrift 19 aus Beispiel 11A (110 µmol) und *N-*(*tert-*Butoxycarbonyl)(1-aminocyclohexyl)carbonsäure. |
| | | Ausbeute: 27% d. Th. |
| 144A | 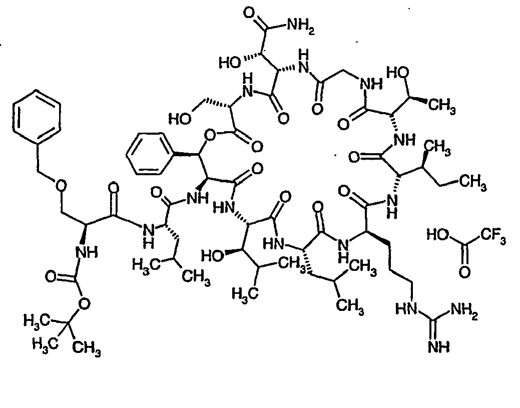 | HPLC/UV-Vis (Methode 36): Rₜ = 4.17 min. |
| | | LC-MS (Methode 26): Rₜ = 2.00 min, |
| | | MS (ESIpos.): *m*/*z* (%) = 1441.0 (20) [M + H]⁺. |
| | *N*-(*tert*-Butoxycarbonyl)-(*O*-benzyl)-L-seryldes(1-D-leucyl)lysobactin-trifluoracetat | Allgemeine Arbeitsvorschrift 19 aus Beispiel 11A (110 µmol) und *N-*(*tert-*Butoxycarbonyl)-(*O*-benzyl)-L-serin. |
| | | Ausbeute: 53% d. Th. |
| 145A | 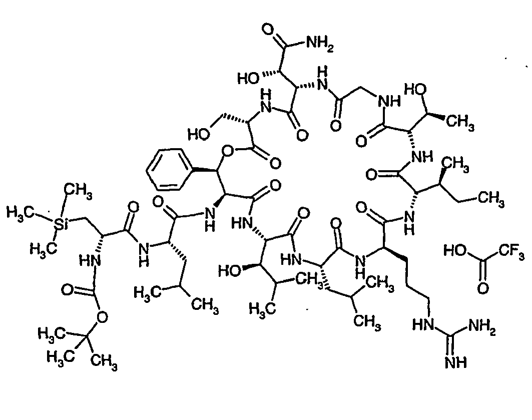 | HPLC/UV-Vis (Methode 36): Rₜ = 4.5 min. |
| | | LC-MS (Methode 26): Rₜ = 2.13 min, |
| | | *S* (ESIpos.): *mlz* (%) = 1407.0 (20) [M + H]⁺. |
| | *N*-(*tert*-Butoxycarbonyl)-3-(trimethylsilyl)-D-alanyldes(1-D-leucyl)lysobactin-trifluoracetat | Allgemeine Arbeitsvorschrift 19 aus Beispiel 11A (143 µmol) und Beispiel 25A. |
| | | Ausbeute: 40% d. Th. |
| 146A | 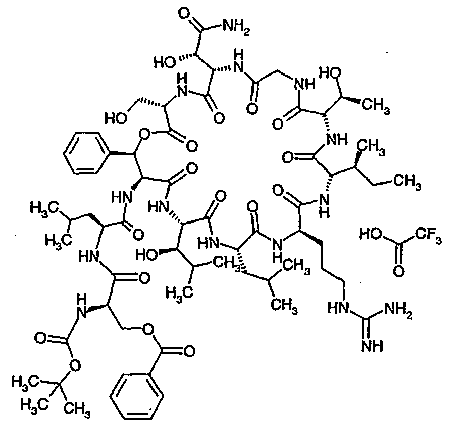 | HPLC/UV-Vis (Methode 36): Rₜ = 4.17 min. |
| | | LC-MS (Methode 26): Rₜ = 2.01 min, |
| | | MS (ESIpos.): *mlz* (%) = 1455.0 (20) [M + H]⁺. |
| | *N*-(*tert*-Butoxycarbonyl)-(*O*-benzoyl)-D-seryldes(1-D-leucyl)lysobactin-trifluoracetat | Allgemeine Arbeitsvorschrift 19 aus Beispiel 11A (110 µmol) und *N-*(*tert-*Butoxycarbonyl)-(*O*-benzoyl)-D-serin. |
| | | Ausbeute: 41 % d. Th. |
| 147A | 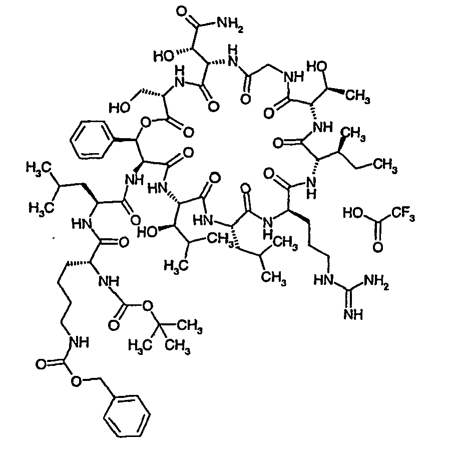 | HPLC/UV-Vis (Methode 36): Rₜ = 4.13 min. |
| | | LC-MS (Methode 26): Rₜ = 1.96 min, |
| | | MS (ESIpos.): *m*/*z* (%) = 1526.1 (50) [M + H]⁺. |
| | *N*⁶-[(Benzyloxy)carbonyl]-*N*²-(*tert*-butoxycarbonyl)-D-lysyl-des(1-D-leucyl)lysobactin-trifluoracetat | Allgemeine Arbeitsvorschrift 19 aus Beispiel 11A (110 µmol) und *N*⁶-[(Benzyloxy)carbonyl]-*N*²-(*tert*-butoxycarbonyl)-D-lysin. |
| | | Ausbeute: 50% d. Th. |
| 148A | 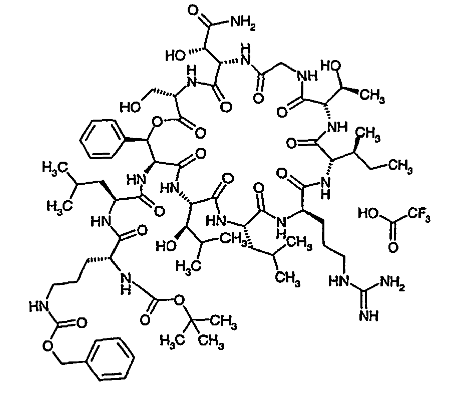 | LC-MS (Methode 26): Rₜ = 1.93 min, |
| | | MS (ESIpos.): *m*/*z* (%) = 1511.9 (30) [M + H]⁺. |
| | *N*⁵-[(Benzyloxy)carbonyl]-*N*²-[(butoxy)carbonyl]-D-ornithyl-des(1-D-leucyl)lysobactin-trifluoracetat | Allgemeine Arbeitsvorschrift 19 aus Beispiel 11A (110 µmol) und *N*⁵-[(Benzyloxy)carbonyl]-*N*²-[(butoxy)carbonyl]-D-ornithin. |
| | | Ausbeute: 48% d. Th. |
| 149A | 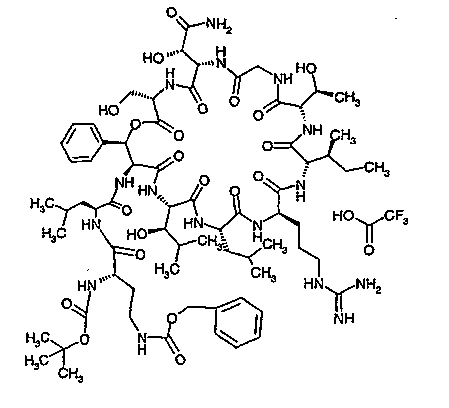 | HPLC/UV-Vis (Methode 36): Rₜ = 4.08 min. |
| | | LC-MS (Methode 26): Rₜ = 1.94 min, |
| | | MS (ESIpos.): *m*/*z* (%) = 1498.0 (20) [M + H]⁺. |
| | (2*S*)-4-{[(Benzyloxy)carbonyl]amino}-2-[(*tert*-butoxycarbonyl)amino]butanoyl-des(1-D-leucyl)-lysobactin-trifluoracetat | Allgemeine Arbeitsvorschrift 19 aus Beispiel 11A (110 µmol) und (2*S*)-4-{[(Benzyloxy)carbonyl]-amino}-2-[(*tert*-butoxycarbonyl) amino]buttersäure. |
| | | Ausbeute: 49% d. Th. |
| 150A | 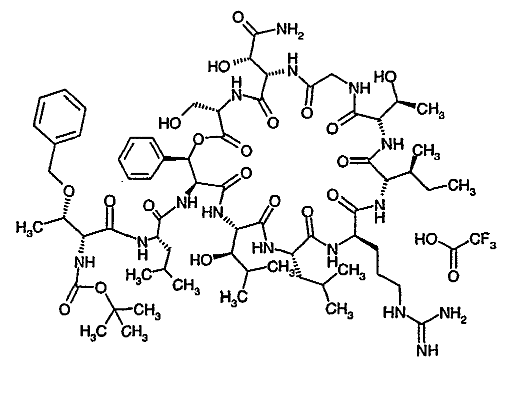 | HPLC/UV-Vis (Methode 36): Rₜ = 4.41 min. |
| | | LC-MS (Methode 26): Rₜ = 2.13 min, |
| | | MS (ESIpos.): *m*/*z* (%) = 1455.0 (30) [M + H]⁺. |
| | *N*-(*tert*-Butoxycarbonyl)-*O*-benzyl-D-threonyl- des(1-D-leucyl)lysobactin-trifluoracetat | Allgemeine Arbeitsvorschrift 19 aus Beispiel 11A (110 µmol) und *N-*(*tert-*Butoxycarbonyl)-*O*-benzyl-D-threonin. |
| | | Ausbeute: 46% d. Th. |
| 151A | 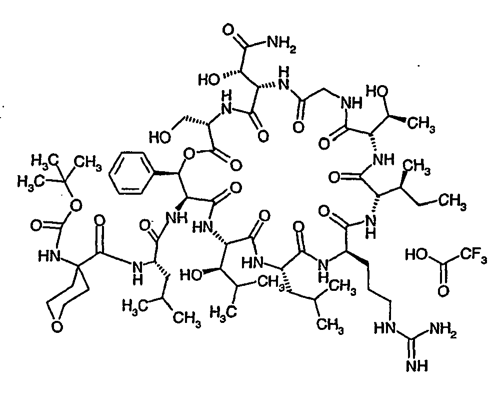 | HPLC/UV-Vis (Methode 36): Rₜ = 3.60 min. |
| | | LC-MS (Methode 26): Rₜ = 1.78 min, |
| | | MS (ESIneg.): *m*/*z* (%) = 1390.8 [M - H]⁻. |
| | 4-[(*tert*-Butoxycarbonyl)amino]tetrahydro-2*H*-pyran-4-carbonyl]-des(1-D-leucyl)lysobactin-trifluoracetat | Allgemeine Arbeitsvorschrift 19 aus Beispiel 11A (110 µmol) und 4-[(*tert*-Butoxycarbonyl)amino]-tetrahydro-2H-pyran-4-carbonsäure. |
| | | Ausbeute: 26% d. Th. |
| 152A | 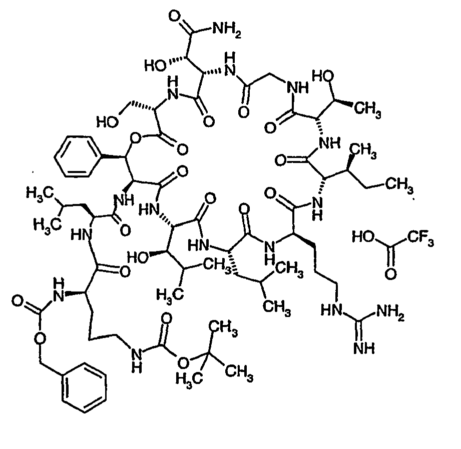 | HPLC/UV-Vis (Methode 36): Rₜ = 4.08 min. |
| | | LC-MS (Methode 26): Rₜ = 1.99 min, |
| | | MS (ESIpos.): *m*/*z* (%) = 1512.7 (10) [M + H]⁺. |
| | *N*²-(Benzyloxycarbonyl)-*N*⁵-(*tert*-butoxycarbonyl)-D-ornithyl-des(1-D-leucyl)lysobactin-trifluoracetat | Allgemeine Arbeitsvorschrift 19 aus Beispiel 11A (110 µmol) und *N²-*(Benzyloxycarbonyl)-*N*⁵-(*tert*-butoxycarbonyl)-D-ornithin. |
| | | Ausbeute: 60% d. Th. |
| 153A | 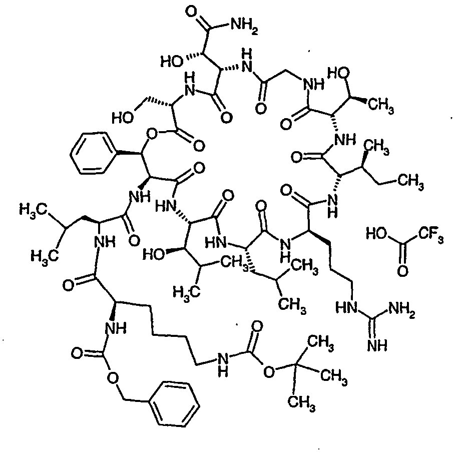 | HPLC/UV-Vis (Methode 36): Rₜ = 4.13 min. |
| | | LC-MS (Methode 26): Rₜ = 1.99 min, |
| | | MS (ESIpos.): *m*/*z* (%) = 1525.8 (20) [M + H]⁺. |
| | *N*²-(Benzyloxycarbonyl)-*N*⁶-(*tert*-Butoxycarbonyl)-D-lysyl-des(1-D-leucyl)lysobactin-trifluoracetat | Allgemeine Arbeitsvorschrift 19 aus Beispiel 11A (110 µmol) und *N*²-(Benzyloxycarbonyl)-*N*⁶-(*tert*-Butoxycarbonyl)-D-lysin. |
| | | Ausbeute: 82% d. Th. |
| 154A | 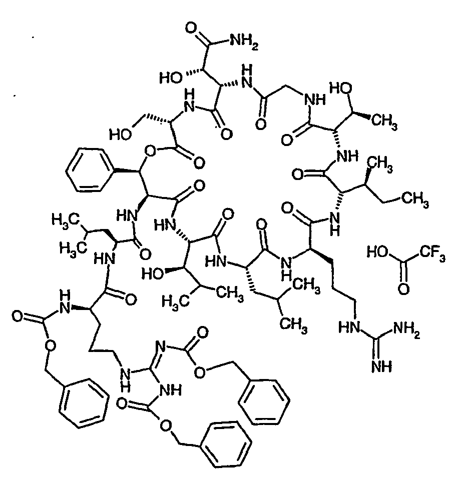 *N*²,*N*⁷,*N*⁸-tris-(Benzyloxycarbonyl)-D-arginyl-des(1-D-leucyl)lysobactin-trifluoracetat | HPLC/UV-Vis (Methode 36): Rₜ = 4.4 min. |
| | | LC-MS (Methode 26): Rₜ = 2.49 min, |
| | | MS (ESIpos.): *m*/*z* (%) = 1721.0 (10) [M + H]⁺. |
| | | Allgemeine Arbeitsvorschrift 19 aus Beispiel 11A (110 µmol) und *N*²,*N*⁷,*N*⁸-tris-(Benzyloxycarbonyl)-D-arginin. |
| | | Ausbeute: 60% d. Th. |

### (Beispiel 155A

### N-(tert-Butoxycarbonyl)-3-tert-butyl-D-alanyl-N¹-{(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-l'hydroxyethyl]-3-(hydroxymethyl)-24-[(1R)-1-hydroxy-2-methylpropyl]-21-isobutyl]-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-3-tert-butyl-L-alaninamid-trifluoracetat

### {N-tert-Butoxycarbonyl-3-tert-butyl-D-alanyl-3-tert-butyl-L-alanyl-des(1-D-leucyl-2-L-leucyl)-lysobactin-trifluoracetat}

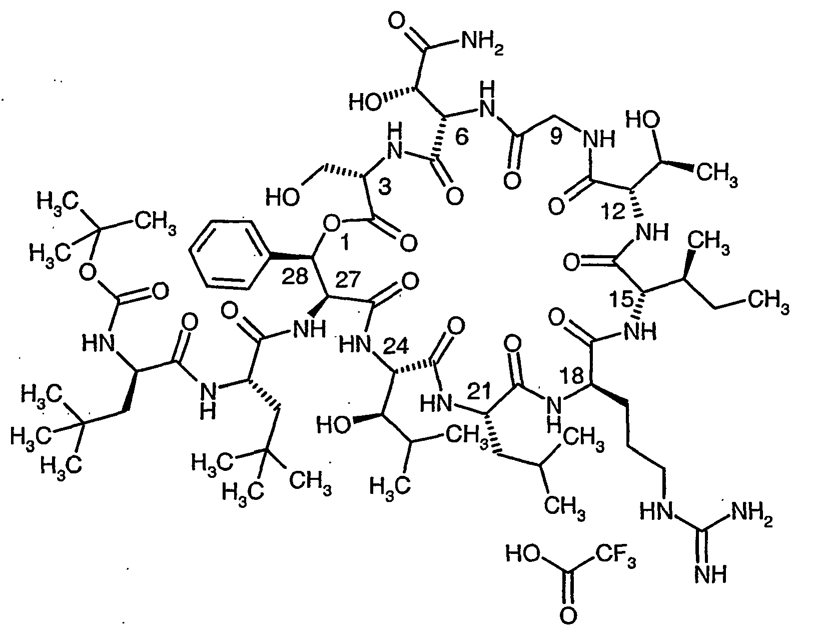

Des(1-D-leucyl-2-L-leucyl)lysobactin-bistrifluoracetat (Beispiel 13A, 500 mg, 0.39 mmol) und *N-*(*tert*-Butoxycarbonyl)-3-*tert*-butyl-D-alanyl-3-*tert*-butyl-L-alanin (Beispiel 59A, 583 mg, 1.56 mmol) werden gemäß der Allgemeinen Arbeitsvorschrift 4 umgesetzt. Das Rohprodukt wird durch präparative HPLC feingereinigt (Methode 24). Man erhält 445 mg (95% d. Th.) Produkt.

HPLC/UV-Vis (Methode 13): Rₜ = 7.9 min,
λₘₐₓ (qualitativ) = 220 nm (s), 255-270 (w).
LC-MS (Methode 26): Rₜ = 2.16 min;
MS (ESIpos.): *m*/*z* (%) = 653 (100) [M - Boc + 2H]²⁺, 1404 (30) [M + H]⁺.
MS (ESIneg.): *m*/*z* (%) = 701 (100), 1403 (20) [M - H]⁻, 1449 [M - H + HCO₂H]⁻.

LC-MS (Methode 29): Rₜ = 5.5 min;
MS (ESIpos.): *m*/*z* (%) = 653 (100) [M - Boc + 2H]²⁺, 1405 (90) [M + H]⁺.
HR-TOF-MS (Methode 21): C₆₅H₁₁₀N₁₅O₁₉ ber. 1404.8102, gef. 1404.8057 [M + H]⁺.

### Beispiel 156A

### N-tert-Butoxycarbonyl-3-tert-butyl-D-alanyl-3-(3-pyridyl)-L-alanyl-des(1-D-leucyl-2-L-leucyl)lysobactin-bistrifluoracetat

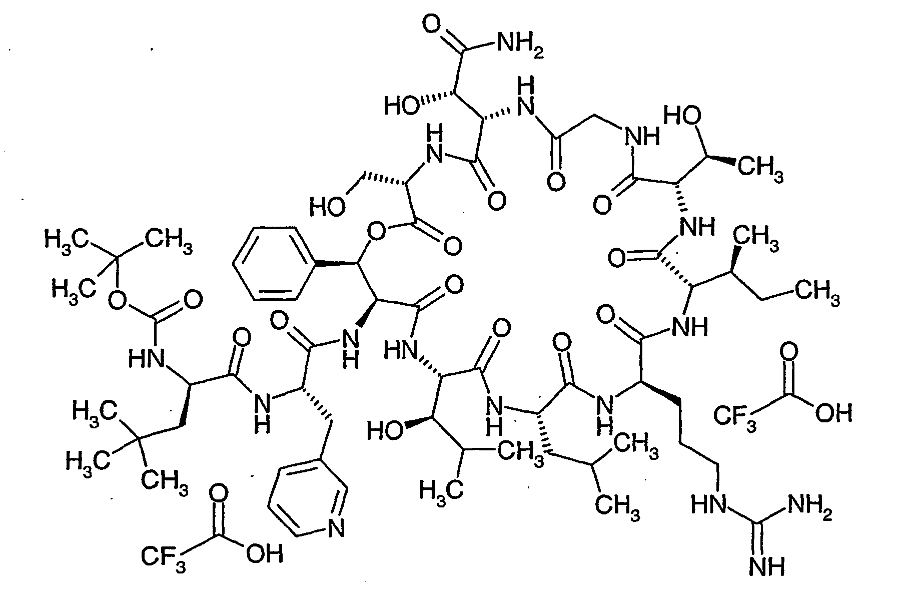

Zu einer Lösung von Beispiel 13A (1.0 Äquivalente, 0.03 mmol) und Beispiel 60A (2.5 Äquivalente, 0.08 mmol) in trockenem Dimethylformamid (2 ml) werden bei -78°C langsam *N-*Methylmorpholin (4 Äquivalente, 0.13 mmol) und HATU (2.6 Äquivalente, 0.08 mmol) gegeben. Das Reaktionsgemisch erwärmt sich langsam (ca. 2 h) auf 0°C, wobei vollständiger Umsatz mittels HPLC/UV-Vis (Methode 36) beobachtet wird. Die Reaktion wurde mit Kaliumdihydrogenphosphat (5.0 Äquivalente, 0.16 mmol) gestoppt. Das Reaktionsgemisch wird mittels Gelchromatographie (Methode 6, Laufmittel Methanol/Aceton 4/1) aufgereinigt, wobei man 53.6 mg (80% d. Th.) Produkt erhält.

HPLC/UV-Vis (Methode 36): Rₜ = 3.83 min.
LC-MS (Methode 26): Rₜ = 2.08 min;
MS (ESIpos.): *m*/*z* (%) = 713 (100) [M + 2H]²⁺, 1425 (15) [M + H]⁺;
MS (ESIneg.): *m*/*z* (%) = 711 (100) [M - 2H]²⁻, 1423 (30) [M - H]⁻.
HR-TOF-MS (Methode 21): C₆₆H₁₀₅N₁₆O₁₉ [M + H]⁺ ber. 1425.7742, gef. 1425.7722.

**Tabelle 4: N-Geschützte Nonadepsipeptide**

| **Bsp.- Nr.** | **Struktur Name** | **Analytische Daten** |
|---|---|---|
| | | **Herstellungsmethode** |
| 157A | 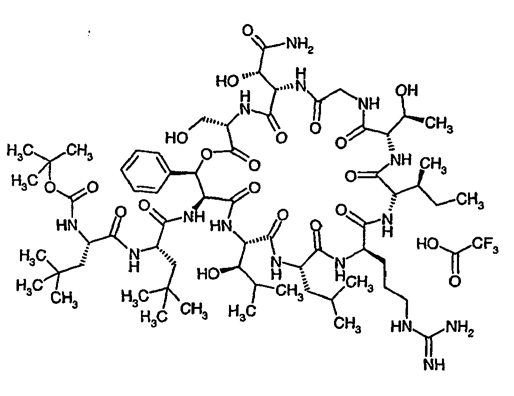 | HPLC/UV-Vis (Methode 13): Rₜ = 7.3 min. |
| | | LC-MS (Methode 27): Rₜ = 2.45 min; |
| | | MS (ESIpos.): *m*/*z* (%) = 653 (100) [M -Boc + 2H]²⁺, 1405 (30) [M + H]⁺. |
| | *N*-*tert*-Butoxycarbonyl-3-*tert*-butyl-L-alanyl-3-*tert*- butyl-L-alanyldes(1-D-leucyl-2-L-leucyl)lysobactin-trifluoracetat | Allg. Arbeitsvorschrift 4 aus Beispiel 13A (0.05 mmol) und Beispiel 61A (0.2 mmol). Reinigung nach Methode 7. |
| | | Ausbeute 51 % d. Th. |
| 158A | 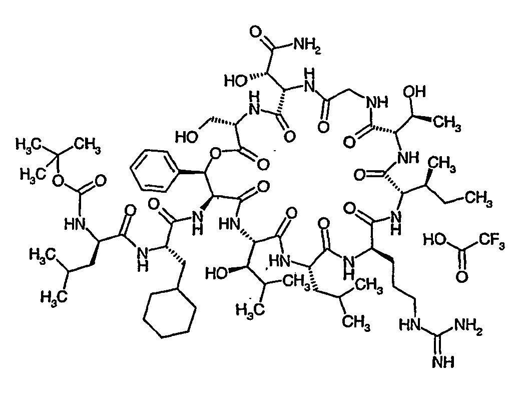 | HPLC/UV-Vis (Methode 13): Rₜ = 7.54 min, |
| | | λₘₐₓ (qualitativ) = 220 nm (s), 255-270 (m). |
| | | LC-MS (Methode 29): Rₜ = 5.41 min; |
| | | MS (ESIpos.): *m*/*z* (%) = 659 (100) [M-Boc + 2H]²⁺, 1417 (80) [M + H]⁺. |
| | *N*-*tert*-Butoxycarbonyl-D-leucyl-3-cyclohexyl-L-alanyldes(1-D-leucyl-2-L-leucyl)lysobactin-trifluoracetat | Allg. Arbeitsvorschrift 4 aus Beispiel 13A (0.02 mmol) und Beispiel 62A (0.06 mmol). Reinigung nach Methode 7. |
| | | Ausbeute 75% d. Th. |
| 159A | 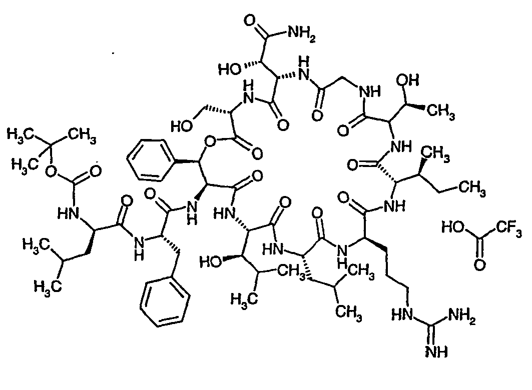 | HPLC/UV-Vis (Methode 13): Rₜ = 7.32 min, |
| | | λₘₐₓ (qualitativ) = 220 mn (s), 255-270 (m). |
| | | LC-MS (Methode 12): Rₜ = 5.42 min; |
| | | MS (ESIpos.): *m*/*z* (%) = 656 (80) [M - Boc + 2H]²⁺, 1411 (100) [M + H]⁺. |
| | *N*-*tert*-Butoxycarbonyl-D-leucyl-L-phenylalanyldes(1-D-leucyl-2-L-leucyl)lysobactin-trifluoracetat | Allg. Arbeitsvorschrift 4 aus Beispiel 13A (0.02 mmol) und Beispiel 63A (0.06 mmol). Reinigung nach Methode 7. |
| | | Ausbeute 57% d. Th. |
| 160A | 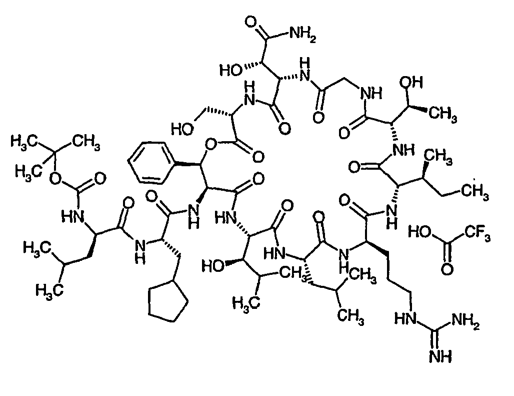 | HPLC/UV-Vis (Methode 13): Rₜ = 7.46 min, |
| | | λₘₐₓ (qualitativ) = 220 nm (s), 255-270 (m). |
| | | LC-MS (Methode 12): Rₜ = 5.46 min; |
| | | MS (ESIpos.): *m*/*z* (%) = 652 (100) [M - Boc + 2H]²⁺, 1403 - (90) [M + H]⁺. |
| | *N*-*tert*-Butoxycarbonyl-D-leucyl-3-cyclopentyl-L-alanyldes(1-D-leucyl-2-L-leucyl)lysobactin-trifluoracetat | Allg. Arbeitsvorschrift 4 aus Beispiel 13A (0.01 mmol) und Beispiel 64A (0.05 mmol). Reinigung nach Methode 7. |
| | | Ausbeute 55% d. Th. |
| 161A | 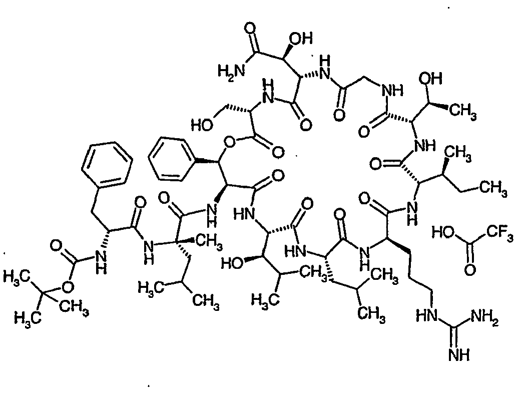 | HPLC/UV-Vis (Methode 13): Rₜ = 7.20 min, |
| | | λₘₐₓ (qualitativ) = 220 nm (s), 255-270 (m). |
| | | LC-MS (Methode 29): Rₜ = 5.33 min; |
| | | MS (ESIpos.): *m*/*z* (%) = 663 (100) [M - Boc + 2H]²⁺, 1425 (90) [M + H]⁺. |
| | *N*-*tert*-Butoxycarbonyl-D-phenylalanyl-2-methyl-L-leucyl-des(1-D-leucyl-2-L-leucyl)lysobactin-trifluoracetat | Allg. Arbeitsvorschrift 4 aus Beispiel 13A (0.01 mmol) und Beispiel 65A (0.05 mmol). Reinigung nach Methode 7. |
| | | Ausbeute 8% d. Th. |
| 162A | 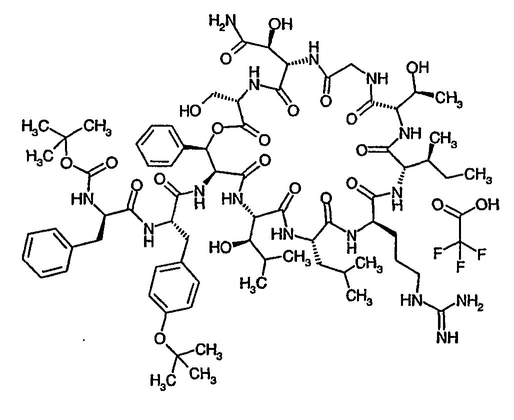 | EPLC/UV-Vis (Methode 13): Rₜ = 7.51 min. |
| | | LC-MS (Methode 29): Rₜ = 5.5 min; |
| | | MS (ESIneg.): *m*/*z* (%) = 1515 (100) [M - H]⁻. |
| | *N*-*tert*-Butoxycarbonyl-D-phenylalanyl-*O*-*tert*-butyl-L-tyrosyl-des(1-D-leucyl-2-L-leucyl)lysobactin-trifluoracetat | Allg. Arbeitsvorschrift 4 aus Beispiel 13A (0.02 mmol) und Beispiel 83A (0.06 mmol). Reinigung nach Methode 7. |
| | | Ausbeute 58% d. Th. |
| 163A | 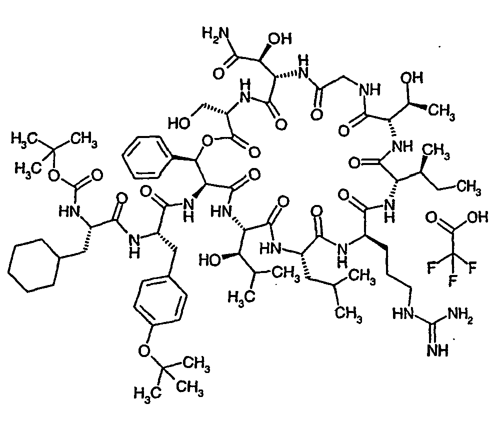 | HPLC/UV-Vis (Methode 13): Rₜ = 7.83 min. |
| | | LC-MS (Methode 29): Rₜ = 5.6 min; |
| | | MS (ESIpos.): *m*/*z* (%) = 734 (100) [M -Boc + 2H]²⁺, 1523 (100) [M + H]⁺. |
| | *N*-*tert*-Butoxycarbonyl-3-cyclohexyl-L-alanyl-*O*-*tert*butyl-L-tyrosyl-des(1-D-leucyl-2-L-leucyl)lysobactin-trifluoracetat | Allg. Arbeitsvorschrift 4 aus Beispiel 13A (0.02 mmol) und Beispiel 84A (0.06 mmol). Reinigung nach Methode 7. |
| | | Ausbeute 44% d. Th. |
| 164A | 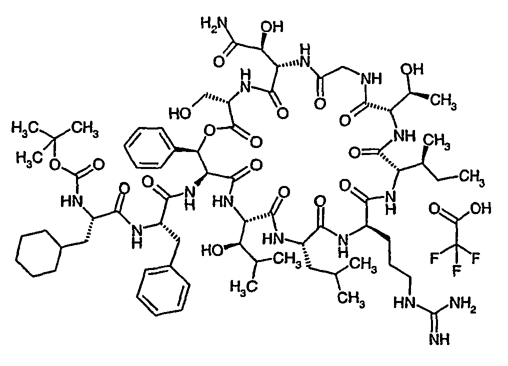 | HPLC/UV-Vis (Methode 13): Rₜ = 7.48 min. |
| | | LC-MS (Methode 29): Rₜ = 5.4 min; |
| | | MS (ESIpos.): *m*/*z* (%) = 676 (100) [M - Boc + 2H]²⁺, 698 (60), 1451 (60) [M + H]⁺. |
| | *N*-*tert*-Butoxycarbonyl-3-cyclohexyl-L-alanyl-L-phenylalanyl-des(1-D-leucyl-2-L-leucyl)lysobactin-trifluoracetat | All. Arbeitsvorschrift 4 aus Beispiel 13A (0.02 mmol) und Beispiel 85A (0.06 mmol). Reinigung nach Methode 7. |
| | | Ausbeute 55% d. Th. |
| 165A | 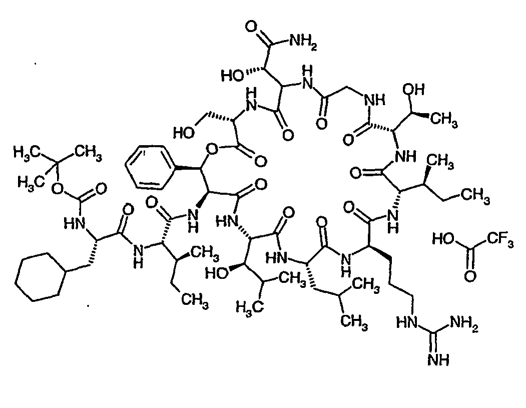 | HPLC/UV-Vis (Methode 13): Rₜ = 7.37 min. |
| | | LC-MS (Methode 26): Rₜ = 2.09 min; |
| | | MS (ESIpos.): *m*/*z* (%) = 659 (100) [M - Boc + 2H]⁺, 1417 (30) [M + H]⁺. |
| | *N*-*tert-*Butoxycarbonyl-3-cyclohexyl-L-alanyl-L-isoleucyl-des(1-D-leucyl-2-L-leucyl)lysobactin-trifluoracetat | Allg. Arbeitsvorschrift 4 aus Beispiel 13A (0.02 mmol) und Beispiel 86A (0.06 mmol). Reinigung nach Methode 7. |
| | | Ausbeute 41 % d. Th. |
| 166A | 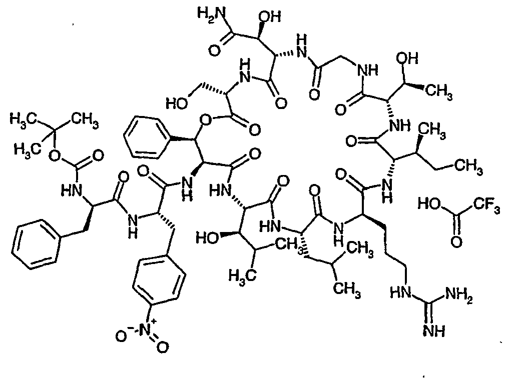 | HPLC/UV-Vis (Methode 13): Rₜ = 7.18 min. |
| | | LC-MS (Methode 29): Rₜ = 5.3 min; |
| | | MS (ESIpos.): *m*/*z* (%) = 696 (100) [M - Boc + 2H]²⁺, 1490 (80) [M + H]⁺. |
| | *N*-*tert*-Butoxycarbonyl-D-phenylalanyl-4 nitro-L-phenylalanyl-des(1-D-leucyl-2-L-leucyl)lysobactin-trifluoracetat | Allg. Arbeitsvorschrift 4 aus Beispiel 13A (0.02 mmol) und Beispiel 87A (0.06 mmol). Reinigung nach Methode 7. |
| | | Ausbeute 49% d. Th. |
| 167A | 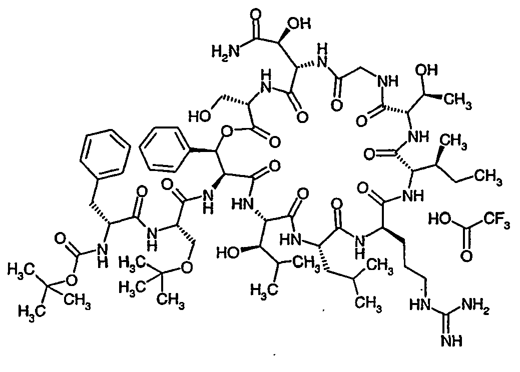 | HPLC/UV-Vis (Methode 13): Rₜ=7.19min. |
| | | LC-MS (Methode 29): Rₜ = 5.3 min; |
| | | MS (ESIpos.): *m*/*z* (%) = 671 (60) [M - Boc + 2H]²⁺, 1441 (100) [M + H]⁺. |
| | *N-tert*-Butoxycarbonyl-D-phenylalanyl-*O³*-*tert*-butyl-L-seryl-des(1-D-leucyl-2-L-leucyl)lysobactin-trifluoracetat | Allg. Arbeitsvorschrift 4 aus Beispiel 13A (0.03 mmol) und Beispiel 82A (0.13 mmol). Reinigung nach Methode 7. |
| | | Ausbeute 50% d. Th. |
| 168A | 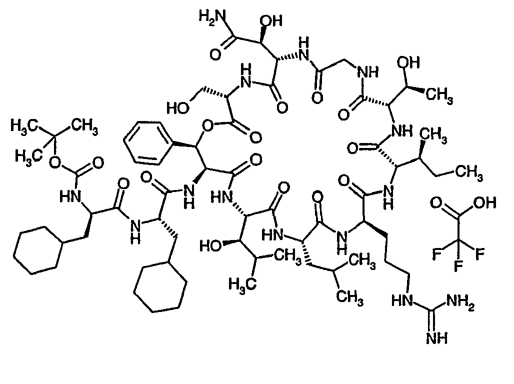 | HPLC/UV-Vis (Methode 13): Rₜ = 7.75 min. |
| | | LC-MS (Methode 29): Rₜ = 5.7 min; |
| | | MS (ESIpos.): *m*/*z* (%) = 679 (100) [M - Boc + 2H]²⁺, 1457 (100) [M + H]⁺. |
| | *N*-*tert*-Butoxycarbonyl-3-cyclohexyl-D-alanyl-3-cyclohexyl-L-alanyl-des(1-D-leucyl-2-L-leucyl)lysobactin-trifluoracetat | Allg. Arbeitsvorschrift 4 aus Beispiel 13A (0.02 mmol) und Beispiel 66A (0.06 mmol). Reinigung nach Methode 7. |
| | | Ausbeute 16% d. Th. |
| 169A | 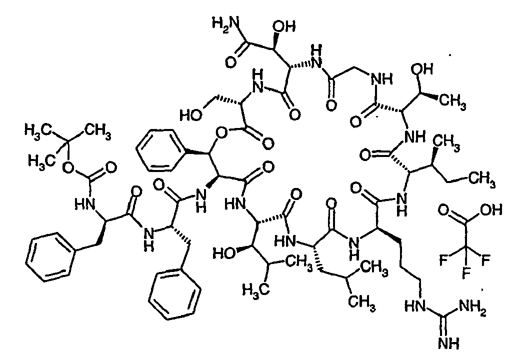 | HPLC/UV-Vis (Methode 13): Rₜ = 7.18 min. |
| | | LC-MS (Methode 29): Rₜ=5.31 min; |
| | | MS (ESIpos.): *m*/*z* (%) = 673 (50) [M - Boc + 2H]²⁺, 1445 (100) [M + H]⁺. |
| | *N*-*tert*-Butoxycarbonyl-D-phenylalanyl-L-phenylalanyl-des(1-D-leucyl-2-L-leucyl)lysobactin-trifluoracetat | Allg. Arbeitsvorschrift 4 aus Beispiel 13A (0.02 mmol) und Beispiel 67A (0.06 mmol). Reinigung nach Methode 7. |
| | | Ausbeute 74% d. Th. |
| 170A | 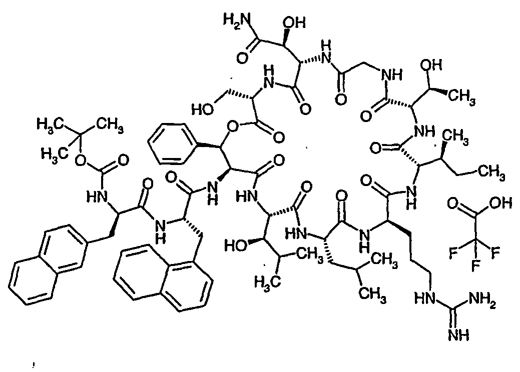 | HPLC/UV-Vis (Methode 13): Rₜ = 7.53 min, |
| | | λₘₐₓ (qualitativ) = 220 nm (s), 280 (m). |
| | | LC-MS (Methode 29): Rₜ = 5.52 min; |
| | | MS (ESIpos.): *m*/*z* (%) = 723 (100) [M - Boc + 2H]²⁺, 1545 (10) [M + H]⁺. |
| | *N*-*tert*-Butoxycarbonyl-3-(2-naphthyl)-D-alanyl-3-(1-naphthyl)-L-phenylalanyl-des(1-D-leucyl-2-L-leucyl)lysobactin-trifluoracetat | Allg. Arbeitsvorschrift 4 aus Beispiel 13A (0.02 mmol) und Beispiel 68A (0.06 mmol). Reinigung nach Methode 7. |
| | | Ausbeute 33% d. Th. |
| 171A | 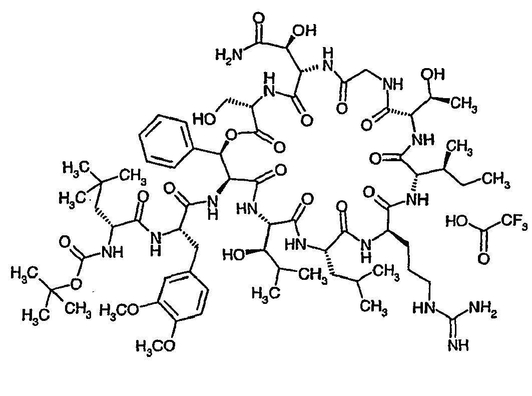 | HPLC/UV-Vis (Methode 13): R = 7.05 min. |
| | | LC-MS (Methode 29): Rₜ = 5.2 min; |
| | | MS (ESIpos.): *m*/*z* (%) = 693 (100) [M - Boc + 2H]²⁺, 1485 (60) [M + H]⁺. |
| | *N*-*tert*-Butoxycarbonyl-3-*ter*t-butyl-D-alanyl-3-(3,4-dimethoxyphenyl)-L-alanyl-des(1-D-leucyl-2-L-leucyl)lysobactin-trifluoracetat | Allgemeine Arbeitsvorschrift 11 aus Beispiel 13A (0.03 mmol) und Beispiel 69A (0.13 mmol). Reinigung nach Methode 7. |
| | | Ausbeute 23% d. Th. |
| 172A | 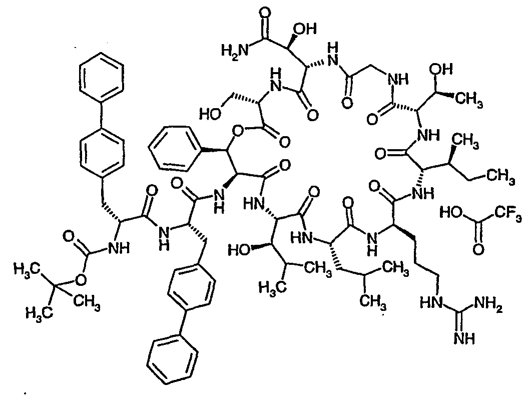 | HPLC/UV-Vis (Methode 13): Rₜ = 7.91 min. |
| | | LC-MS (Methode 29): Rₜ = 5.8 min; |
| | | MS (ESIpos.): *m*/*z* (%) = 749 (100) [M - Boc + 2H]²⁺, 1597 (30) [M + H]⁺. |
| | *N*-*tert*-Butoxycarbonyl-4-phenyl-D-phenylalanyl-4-phenyl-L-phenylalanyl-des(1-D-leucyl-2-L-leucyl)lysobactin-trifluoracetat | Allg. Arbeitsvorschrift 4 aus Beispiel 13A (0.03 mmol) und Beispiel 70A (0.13 mmol). Reinigung nach Methode 7. |
| | | Ausbeute 56% d. Th. |
| 173A | 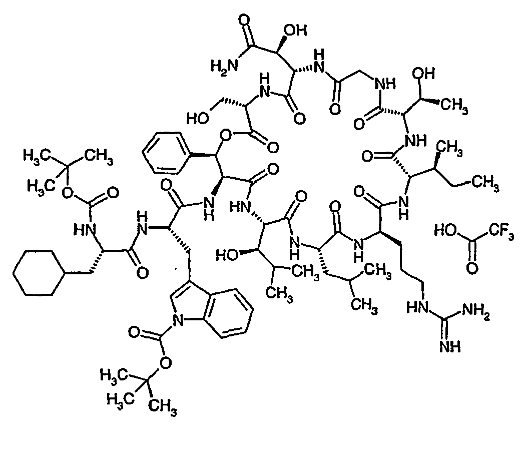 | HPLC/UV-Vis (Methode 13): Rₜ = 7.98 min. |
| | | LC-MS (Methode 29): Rₜ = 5.7 min; |
| | | MS (ESIpos.): *m*/*z* (%) = 746 (60) [M - Boc + 2H]²⁺, 1590 (100) [M + H]⁺. |
| | *N*-*tert*-Butoxycarbonyl-3-cyclohexyl-L-alanyl-*N^{indol}*-*tert*-butoxycarbonyl-L-tryptophyl-des(1-D-leucyl-2-L-leucyl)lysobactin-trifluoracetat | Allg. Arbeitsvorschrift 4 aus Beispiel 13A (0.02 mmol) und Beispiel 79A (0.06 mmol). Reinigung nach Methode 7. |
| | | Ausbeute 29% d. Th. |
| 174A | 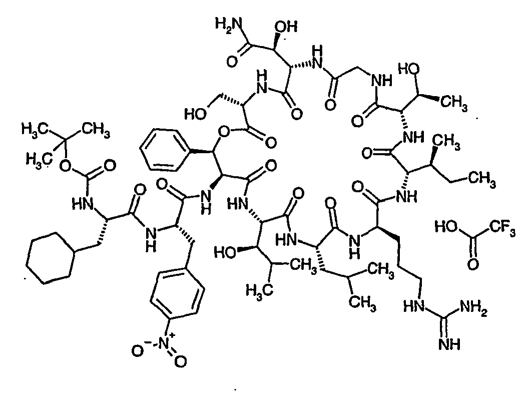 | HPLC/UV-Vis (Methode 13): Rₜ = 7.52 min, |
| | | λₘₐₓ (qualitativ) = 210 nm (s), 272 (m). |
| | | LC-MS (Methode 29): Rₜ = 5.5 min; |
| | | MS (ESIpos.): *m*/*z* (%) = 699 (100) [M - Boc + 2H]²⁺, 1496 (90) [M + H]⁺. |
| | *N*-*tert*-Butoxycarbonyl- 3-cyclohexyl-L-alanyl-4-nitro- L-phenylalanyl-des(1-D-leucyl-2-L-leucyl)lysobactin-trifluoracetat | Allg. Arbeitsvorschrift 4 aus Beispiel 13A (0.02 mmol) und Beispiel 80A (0.06 mmol). Reinigung nach Methode 7. |
| | | Ausbeute 17% d. Th. |
| 175A | 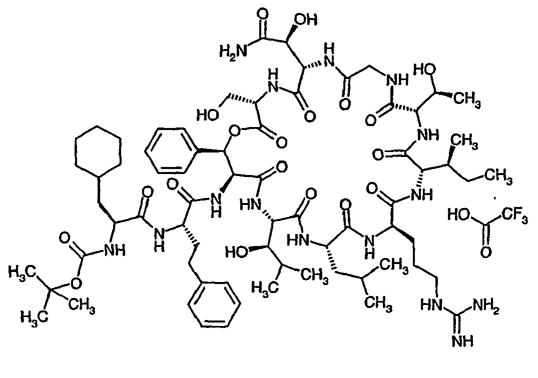 | HPLC/UV-Vis (Methode 13): Rₜ = 7.64 min. |
| | | LC-MS (Methode 29): Rₜ = 5.5 min; |
| | | MS (ESIpos.): *m*/*z* (%) = 683 (100) [M - Boc + 2H]²⁺, 1465 (50) [M + H]⁺. |
| | *N*-*tert*-Butoxycarbonyl-3-cyclohexyl-L-alanyl-3-benzyl-L-alanyl-des(1-D-leucyl-2-L-leucyl)lysobactin-trifluoracetat | Allg. Arbeitsvorschrift 4 aus Beispiel 13A (0.02 mmol) und Beispiel 81A (0.09 mmol). Reinigung nach Methode 7. |
| | | Ausbeute 52% d. Th. |
| 176A | 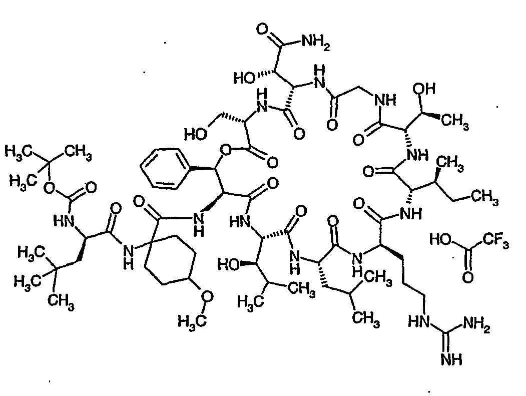 | HPLC/UV-Vis (Methode 13): Rₜ = 7.18 min. |
| | | LC-MS (Methode 26): Rₜ = 2.23 min; |
| | | MS (ESIpos.): *m*/*z* (%) = 667 (100) [M - Boc + 2H]²⁺, 1050 (10), 1433 (20) [M + H]⁺. |
| | *N*-*tert*-Butoxycarbonyl-3-*tert*-butyl-D-alanyl-(1-amino-4-methoxy-cyclohexylcarbonyl)-des(1-D-leucyl-2-L-leucyl)lysobactin-trifluoracetat | Allgemeine Arbeitsvorschrift 11 aus Beispiel 13A (0.05 mmol) und Beispiel 71A (0.19 mmol). Reinigung nach Methode 7. |
| | | Ausbeute 42% d. Th. |
| 177A | 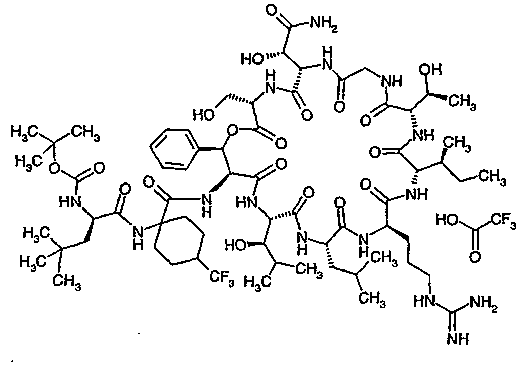 | HPLC/UV-Vis (Methode 13): Rₜ = 7.69 min. |
| | | LC-MS (Methode 26): Rₜ = 2.37 min; |
| | | MS (ESIpos.): *m*/*z* (%) = 686 (100) [M -Boc + 2H]²⁺, 1472 (30) [M + H]⁺. |
| | *N*-*tert*-Butoxycarbonyl-3-*tert*-butyl-D-alanyl-(1-amino-4-(trifluormethyl)cyclohexylcarbonyl)-des(1-D-leucyl-2-L-leucyl)lysobactin-trifluoracetat | Allgemeine Arbeitsvorschrift 11 aus Beispiel 13A (0.05 mmol) und Beispiel 72A (0.19 mmol). Reinigung nach Methode 7. |
| | | Ausbeute 33% d. Th. |
| 178A | 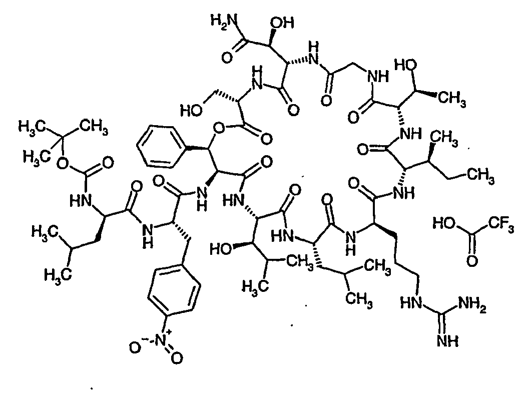 | HPLC/UV-Vis (Methode 13): Rₜ = 7.37 min. |
| | | LC-MS (Methode 26): Rₜ = 2.24 min; |
| | | MS (ESIpos.): *m*/*z* (%) = 679 (100) [M - Boc + 2H]²⁺, 1456 (30) [M + H]⁺. |
| | *N-tert*-Butoxycarbonyl-D-leucyl-4-nitro-L-phenylalanyldes(1-D-leucyl-2-L-leueyl)lysobactin-trifluoracetat | Allgemeine Arbeitsvorschrift 11 aus Beispiel 13A (0.08 mmol) und Beispiel 73A (0.31 mmol). Reinigung nach Methode 7. |
| | | Ausbeute 30% d. Th. |
| 179A | 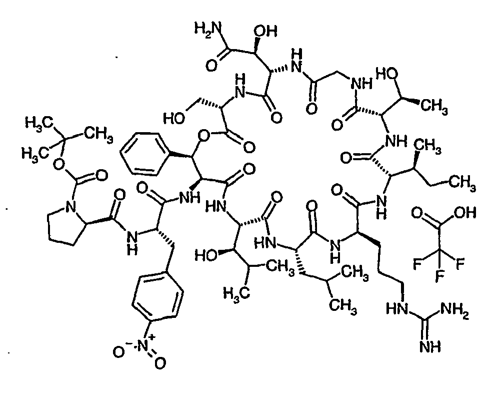 | HPLGL3V-Vis (Methode 13): Rₜ = 6.94 min, |
| | | λₘₐₓ (qualitativ) = 220 nm (s), 272 (m). |
| | | LC-MS (Methode 29): Rₜ = 5.2 min; |
| | | MS (ESIpos.): *m*/*z* (%) = 671 (100) [M - Boc + 2H]²⁺,1440 (70) [M + H]⁺. |
| | *N*-*tert*-Butoxycarbonyl-D-prolyl-4-nitro-L-phenylalanyldes(1-D-leucyl-2-L-leucyl)lysobactin-trifluoracetat | Allg. Arbeitsvorschrift 4 aus Beispiel 13A (0.02 mmol) und Beispiel 78A (0.06 mmol). Reinigung nach Methode 7. |
| | | Ausbeute 55% d. Th. |
| 180A | 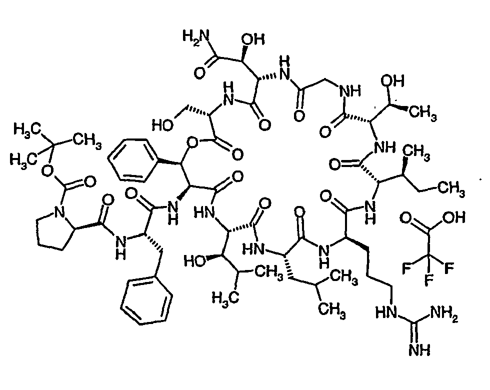 | HPLC/UV-Vis (Methode 13): Rₜ = 6.39 min. |
| | | LC-MS (Methode 29): Rₜ = 5.2 min; |
| | | MS (ESIpos.): *m*/*z* (%) = 648 (35) [M - Boc + 2H]²⁺, 1395 (100) [M + H]⁺. |
| | *N*-*tert*-Butoxycarbonyl-D-prolyl-L-phenylalanyldes(1-D-leucyl-2-L-leucyl)lysobacin-trifluoracetat | Allg. Arbeitsvorschrift 4 aus Beispiel 13A (0.016 mmol) und Beispiel 76A (0.063 mmol). Reinigung nach Methode 7. |
| | | Ausbeute 40% d. Th. |
| 181A | 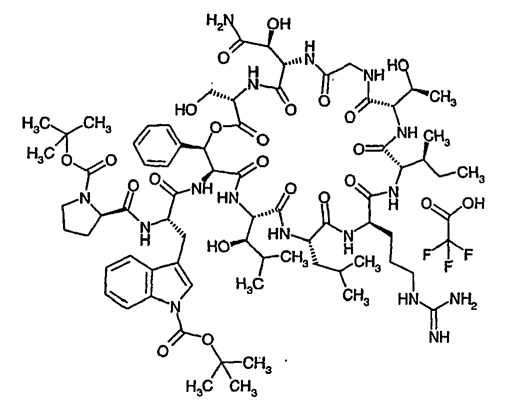 | HPLC/UV-Vis (Methode 13): Rₜ = 7.53 min, |
| | | λₘₐₓ (qualitativ) = 220 nm (s), 265 (m), 290 (sh). |
| | | LC-MS (Methode 26): Rₜ = 2.16 min; |
| | | MS (ESIpos.): *m*/*z* (%) = 718 (100) [M - Boc + 2H]²⁺, 1534 (30) [M + H]⁺. |
| | *N*-*tert-*Butoxycarbonyl-D-prolyl-*N*-*tert*-butoxycarbonyl-L-tryptophyldes(1-D-leucyl-2-L-leucyl)lysobactin-trifluoracetat | Allg. Arbeitsvorschrift 4 aus Beispiel 13A (0.02 mmol) und Beispiel 77A (0.06 mmol). Reinigung nach Methode 7. |
| | | Ausbeute 42% d. Th. |
| 182A | | HPLC/UV-Vis (Methode 36): Rₜ = 3.80 min. |
| | 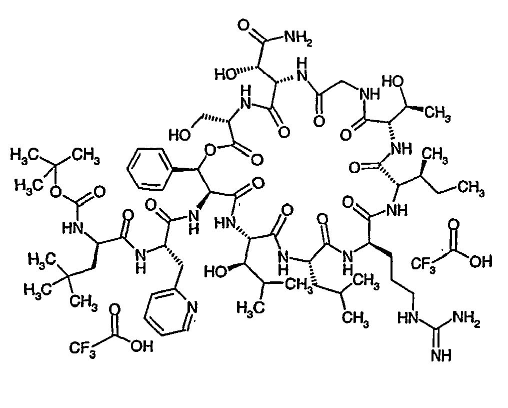 | LC-MS (Methode 22): Rₜ = 1.99 min; |
| | | MS (ESIpos.): *m*/*z* (%) = 713 (100) [M + 2H]²⁺, 1425 (80) [M + H]⁺; |
| | | MS (ESIneg.): *m*/*z* (%) = 711 (100) [M - 2H]²⁻ , 1423 (20) [M - H]⁻. |
| | | HR-TOF-MS (Methode 21): C₆₆H₁₀₅N₁₆₀O₁₉ [M + H]⁺ ber. 1425.7742, gef. 1425.7742. |
| | *N*-*tert*-Butoxycarbonyl-3-*tert*-butyl-D-alanyl-3- (2-pyridyl)-L-alanyl-des(1-D-leucyl-2-L-leucyl)lysobactin-bis-trifluoracetat | Allg. Arbeitsvorschrift 4 aus Beispiel 74A (0.23 mmol) und Beispiel 13A (0.09 mmol). |
| | | Ausbeute: 29% d. Th |
| 183A | | HPLC/UV-Vis (Methode 36): Rₜ = 3.78 min. |
| | 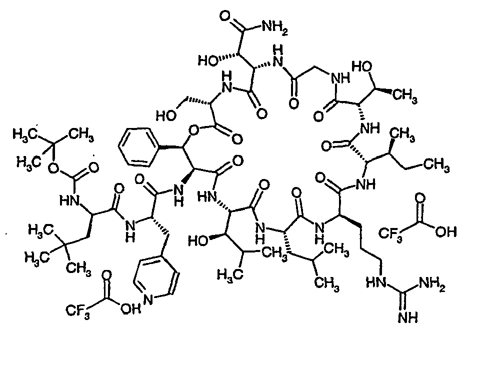 | LC-MS (Methode 22): Rₜ = 2.03 min; |
| | | MS (ESIpos.): *m*/*z* (%) = 713 (100) [M + 2H]²⁺, 1425 (10) [M+H]⁺; |
| | | MS (ESIneg.): *m*/*z* (%) = 711 (100) [M - 2H]²⁻ 1423 (20) [M - H]⁻. |
| | | HR-TOF-MS (Methode 21): C₆₆H₁₀₅N₁₆O₁₉ [M + H]⁺ ber. 1425.7742, gef. 1425.7773. |
| | *N*-*tert-*Butoxycarbonyl-3-*tert*-butyl-D-alanyl-3-(4-pyridyl)-L-alanyl-des(1-D-leucyl-2-L-leucyl)lysobactin-bis-trifluoracetat | Allg. Arbeitsvorschrift 4 aus *N*-*tert*-Butoxycarbonyl-3-*tert-*butyl-D-alanyl-3-(4-pyridyl)-L-alanin (0.15 mmol) und Beispiel 13A (0.06 mmol). |
| | | Ausbeute: 100% d. Th |
| 184A | 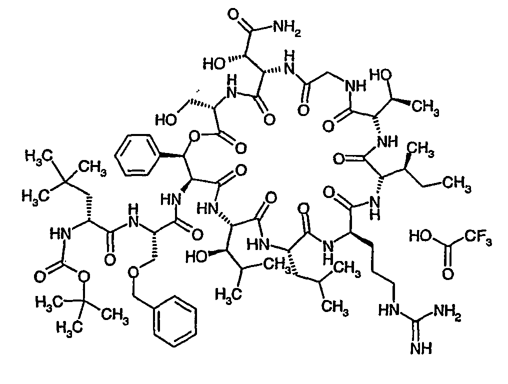 | HPLC/UV-Vis (Methode 36): Rₜ = 4.33 min. |
| | | LC-MS (Methode 26): Rₜ = 2.12 min, |
| | | MS (ESIpos.): *m*/*z* (%) = 1454.9 (10) [M + H]⁺. |
| | [*N*-(*tert*-Butoxycarbonyl)- 3-*tert*-butyl-D-alanyl)-(*O*-benzyl)-L-seryl-des(1-D-leucyl-2-L-leucyl)lysobactin-trifluoracetat | Allgemeine Arbeitsvorschrift 19 aus Beispiel 13A (100 *µ*mol) und Beispiel 88A |
| | | Ausbeute: 29% d. Th. |
| 185A | 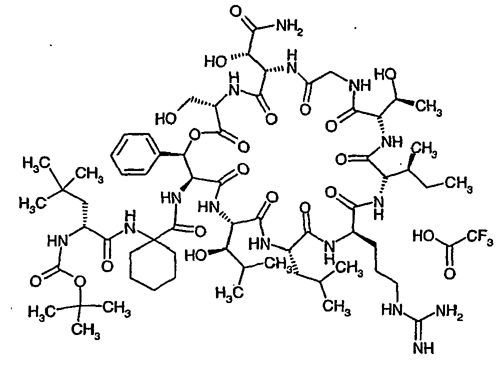 | HPLC/UV-Vis (Methode 36): Rₜ = 4.42 min. |
| | 1-{[*N*-(*tert*-Butoxycarbonyl)-3-*tert*-butyl-D-alanyl]an-amino)cyclohexancarbonyl-des(1-D-leucyl-2-L-leucyl)lysobactin-trifluoracetat | Allgemeine Arbeitsvorschrift 19 aus Beispiel 13A (80 µmol) und Beispiel 89A. |
| | | Ausbeute: 46% d. Th. |
| 186A | 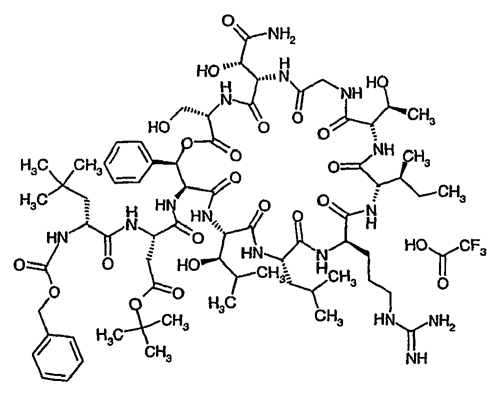 | HPLGLTV-Vis (Methode 36): Rₜ = 4.37 min. |
| | | LC-MS (Methode 26): Rₜ = 2.25 min, |
| | | MS (ESIpos.): *m*/*z* (%) = 1483.1 (20) [M + H]⁺. |
| | (2*S*)-2-[((2*R*)-2-{[(Benzyloxy)carbonyl]amino}-3-*tert-*butyl-D-alanyl)amino]-4-tert-butoxy-4-oxobutanoyl-des(1-D-leucyl-2-L-leucyl)lysobactin-trifluoracetat | Allgemeine Arbeitsvorschrift 19 aus Beispiel 13A (120 µmol) und Beispiel 90A. |
| | | Ausbeute: 72% d. Th. |
| 187A | 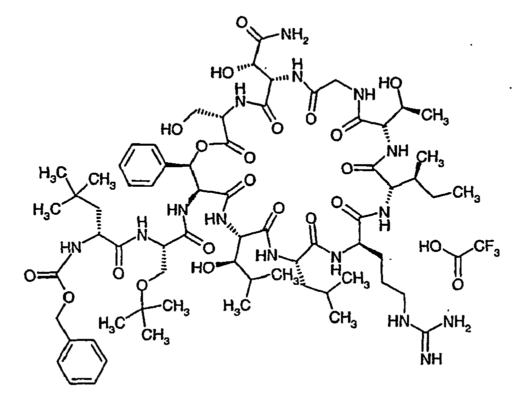 | HPLCIUV-Vis (Methode 36): Rₜ = 4.41 min. |
| | | LC-MS (Methode 26): Rₜ = 2.47 min, |
| | | MS (ESIpos.): *m*/*z* (%) = 1455.1 (30) [M + H]⁺. |
| | [*N*-(Benzyloxycarbonyl)-3-*tert*-butyl-D-alanyl]-O-(*tert*-butyl)-L-seryl-des(1-D-leucyl-2-L-leucyl)lysobactin-tri-fluoracetat | Allgemeine Arbeitsvorschrift 19 aus Beispiel 13A (90 µmol) und Beispiel 91A. |
| | | Ausbeute: 90% d. Th. |
| 188A | 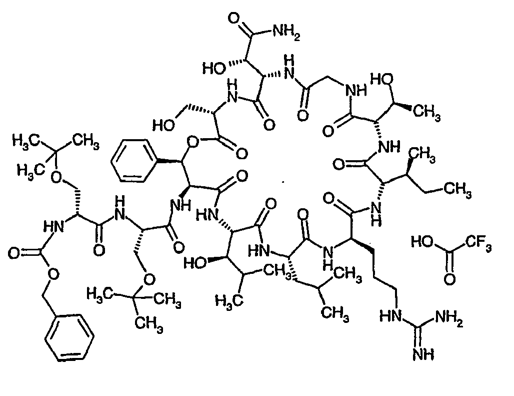 | HPLC/UV-Vis (Methode 36): Rₜ = 4.3 min. |
| | | LC-MS (Methode 26): Rₜ = 1471.1 (30) min, |
| | | MS (ESIpos.): *m*/*z* (%) = 1471.1 (30) [M+H]⁺. |
| | [*N*-(Benzyloxycarbonyl)-*O*-(*tert*-butyl)-D-seryl]-*O*-(*tert*-butyl)-L-seryl-des(1-D-leucyl-2-L-leucyl)-lysobactin-trifluoracetat | Allgemeine Arbeitsvorschrift 19 aus Beispiel 13A (120 µmol) und Beispiel 92A. |
| | | Ausbeute: 70% d. Th. |
| 189A | 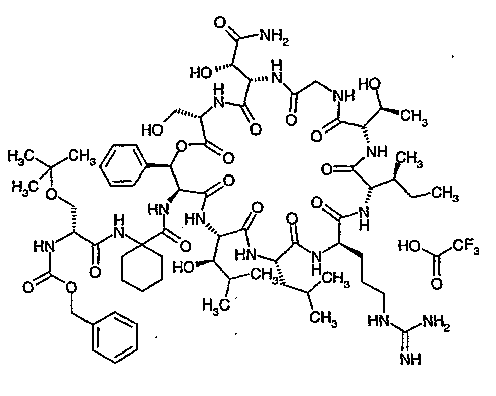 | HPLC/UV-Vis (Methode 36): Rₜ = 4.23 min. |
| | | LC-MS (Methode 26): Rₜ = 2.15 min, |
| | | MS (ESIpos.): *m*/*z* (%) = 1452.8 (40) [M+H]⁺. |
| | *N*-[(1-{[*N*[(Benzyloxy)carbonyl)-*O*-(*tert*-butyl)-D-seryl]amino)cylohexyl)carbonyl]-des(1-D-leucyl-2-L-leucyl)lysobactin-trifluoracetat | Allgemeine Arbeitsvorschrift 19 aus Beispiel 13A (120 *µ*mol) und Beispiel 93A. |
| | | Ausbeute: 18% d. Th. |
| 190A | 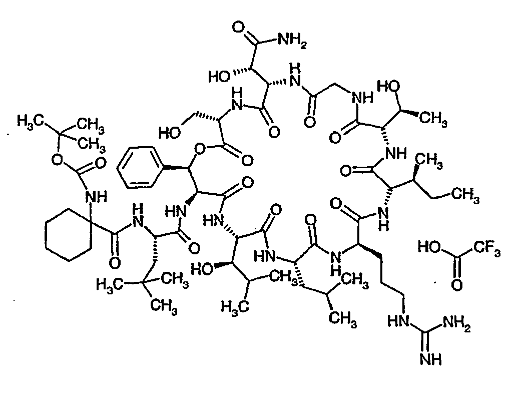 | HPLC/UV-Vis (Methode 36): Rₜ = 4.37 min. |
| | | LC-MS (Methode 26): Rₜ = 2.21 min, |
| | | MS (ESIpos.): *m*/*z* (%) = 1403.1 (60) [M + H]⁺. |
| | *N*-({1-[(*tert*-Butoxycarbonyl)amibo]-cyclohexyl}carbonyl)-3-*tert*-butyl-D-alanyl-des(1-D-leucyl-2-L-leucyl)lysobactin-trifluoracetat | Allgemeine Arbeitsvorschrift 19 aus Beispiel 13A (110 µmol) und Beispiel 94A. |
| | | Ausbeute: 76% d. Th. |
| 191A | 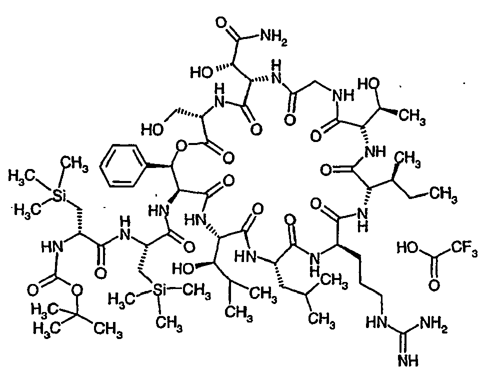 | HPLC/UV-Vis (Methode 36): Rₜ = 4.62 min. |
| | | LC-MS (Methode 26): Rₜ = 2.41 min, |
| | | MS (ESIpos.): *m*/*z* (%) = 1437.1 (30) [M + H]⁺. |
| | *N*-[(*tert*-Butoxycarbonyl)-3-(trimethylsilyl)-D-alanyl-3-(trimethylsilyl)-L-alanyl]-des(1-D-leucyl-2-L-leucyl)-lysobactin-trifluoracetat | Allgemeine Arbeitsvorschrift 19 aus Beispiel 13A (120 µmol) und Beispiel 95A. |
| | | Ausbeute: 86% d. Th. |
| 192A | 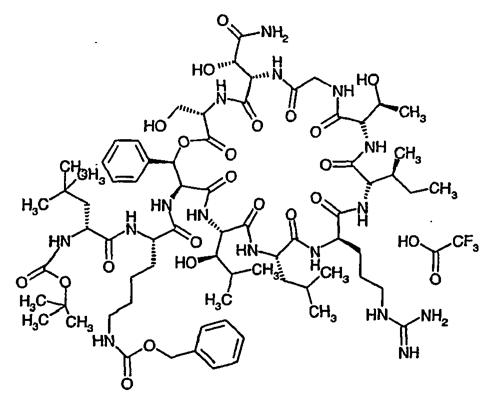 | HPLC/UV-Vis (Methode 36): Rₜ = 4.41 min. |
| | | LC-MS (Methode 26): Rₜ = 2.14 min, |
| | | MS (ESIpos.): *m*/*z* (%) = 1539.8 (20) [M + H]⁺. |
| | *N*-[(*tert*-Butoxycarbonyl)-4-methyl-D-leucyl]-*N*⁶-[(benzyloxy)carbonyl]-L-lysyl-des(1-D-leucyl- | Allgemeine Arbeitsvorschrift 19 aus Beispiel 13A (110 µmol) und Beispiel 96A. |
| | 2-L-leucyl)lysobactin-trifluoracetat | Ausbeute: 32% d. Th. |

### Ausführungsbeispiele

### Beispiel 1

### D-Leucyl-N¹-{(3S,6S,12S,15S,18R,21S,24S,27S,28R)-6-[(1S-2-amino-1-hydroxy-2-oxoethyl]-18-(3-{[amino(imino)methyl]amino)propyl)-12-[(1S)-1-hydroxyethyl]-3-(hydroxynxethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-3-cyclopropyl-L-alaninamid-bistrifluoracetat

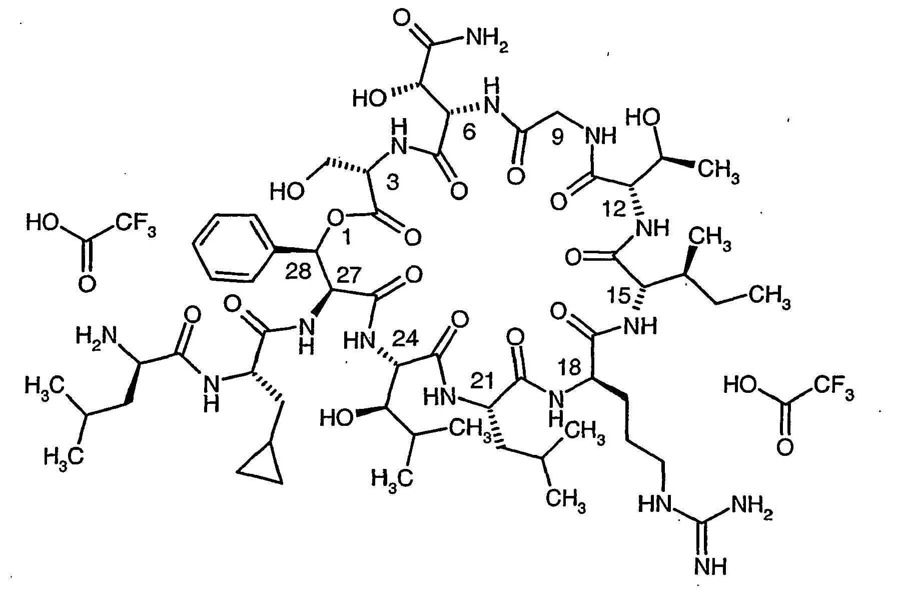

Gemäß der Allgemeinen Arbeitsvorschrift 5 wird das *N*-(*tert*-Butoxycarbonyl)-Depsipeptid (Beispiel 14A, 40 mg, 30 *µ*mol) umgesetzt. Nach chromatographischer Reinigung mittels präparativer HPLC (Methode 8; bzw. Methode 7 gefolgt von anschließendem Umsalzen des Chromatographieprodukts durch Zusatz von TFA (300 *µ*mol) erhält man durch Gefriertrocknung 36 mg (89% d. Th.) Produkt.

HPLC/UV-Vis (Methode 13): Rₜ = 5.64 min,
λₘₐₓ (qualitativ) = 220 nm (s), 255-270 (w).
LC-MS (Methode 11): Rₜ = 2.0 min;
MS (ESIpos.): *m*/*z* (%) = 638 (100) [M + 2H]²⁺, 1275 (8) [M + H]⁺.
FT-ICR-HR-MS (Methode 23):
C₅₈H₉₇N₁₅O₁₇ [M + 2H]²⁺ ber. 637.85880, gef. 637.85878;
C₅₈H₉₆N₁₅NaO₁₇ [M + H + Na]²⁺ ber. 648.84977, gef. 648.84990.

Zur Aminosäure-Sequenzbestimmung wird eine analytische Probe des Produkts nach der Allgemeinen Arbeitsvorschrift 10 hydrolysiert.

MALDI-MS (Methode 20): *m*/*z* (%) = 1292.5 (100) [M + H]⁺.

### Beispiel 2

### D-Leucyl-N¹-{(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-3-cyclopropyl-L-norvalin- amid-bistrifluoracetat

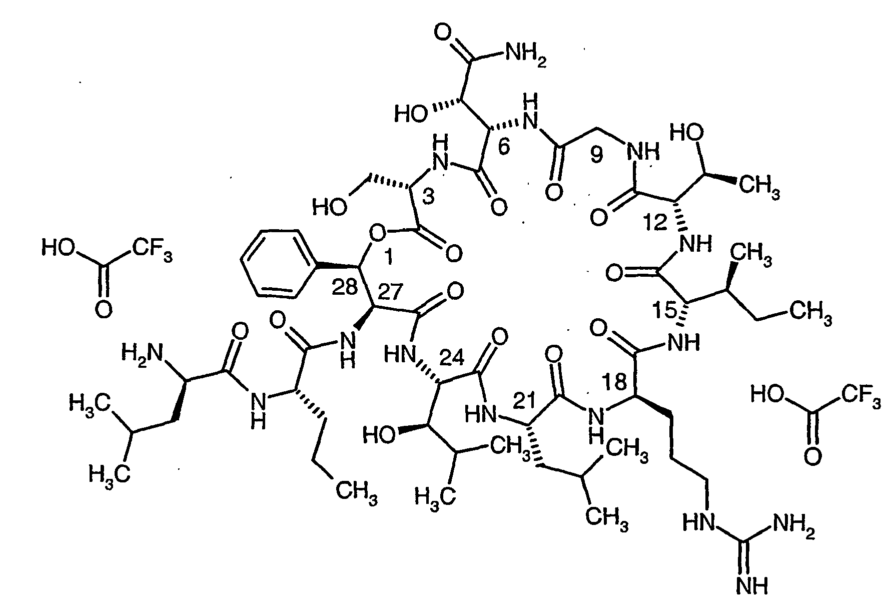

Gemäß der Allgemeinen Arbeitsvorschrift 5 wird das *N-*(*tert*-Butoxycarbonyl)-Depsipeptid (Beispiel 15A, 3.1 mg, 2 *µ*mol) umgesetzt. Nach chromatographischer Reinigung mittels präparativer HPLC (Methode 10; bzw. Methode 9 gefolgt von anschließendem Umsalzen des Chromatographieprodukts durch Zusatz von TFA (30 µmol) erhält man durch Gefriertrocknung 3 mg (quant.) Produkt.
HPLC/UV-Vis (Methode 13): Rₜ = 5.59 min,
λₘₐₓ (qualitativ) = 220 nm (s), 255-270 (w).

LC-MS (Methode 11): Rₜ = 2.01 min;
MS (ESIpos.): m/z (%) = 632 (100) [M + 2H]²⁺, 1263 (10) [M + H]⁺;
LC-MS (Methode 12): Rₜ = 4.05 min;
MS (ESIpos.): m/z (%) = 632 (100) [M + 2H]²⁺, 1263 (10) [M + H]⁺.
FT-ICR-HR-MS (Methode 23):
C₅₇H₉₇N₁₅O₁₇ [M + 2H]²⁺ ber. 631.85879, gef. 631.85913.

Zur Aminosäure-Sequenzbestimmung wird eine analytische Probe des Produkts nach der Allgemeinen Arbeitsvorschrift 10 hydrolysiert.

MALDI-MS (Methode 20): m/z (%) = 1280.5 (100) [M + H]⁺.

### Beispiel 3

### D-Leucyl-N¹-{(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-2?-yl}-3-tert-butyl-L-alaninamid-bistrifluoracetat

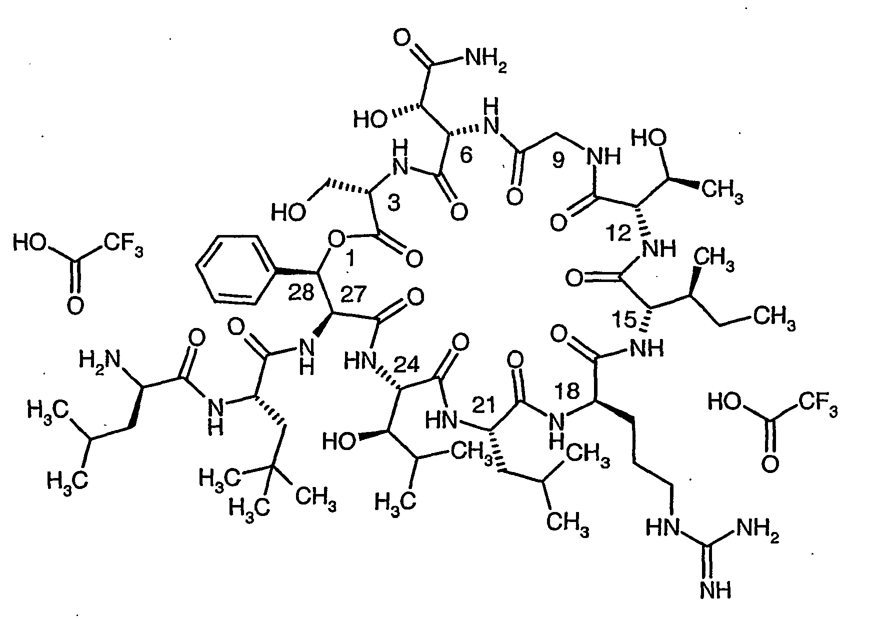

Gemäß der Allgemeinen Arbeitsvorschrift 5 wird das *N*-(*tert-*Butoxycarbonyl)-Depsipeptid (Beispiel 16A, 17 mg, 113 *µ*mol) umgesetzt. Nach chromatographischer Reinigung mittels präparativer HPLC (Methode 15) erhält man durch Gefriertrocknung 15 mg (quant.) Produkt.

HPLC/UV-Vis (Methode 13): Rₜ = 5.87 min,
λₘₐₓ (qualitativ) = 220 nm (s), 255-270 (w).
LC-MS (Methode 11): Rₜ = 1.95 min ;
MS (ESIpos.): m/z (%) = 646 (100) [M + 2H]²⁺, 1291 (10) [M + H]⁺;
MS (ESIneg.): m/z (%) = 644 (100) [M- 2H]²⁻, 1289 (10) [M - H]⁻.

### Beispiel 4

### L-Leucyl-N¹-{(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-1-hydroxyethyl]-3-(hydroxymethyl)-24-[(1R)-1-hydroxy-2-rnethylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacylooctacosan-27-yl)-L-leucinamid-bistrifluoracetat

### {epi-Lysobactin-bistrifluoracetat}

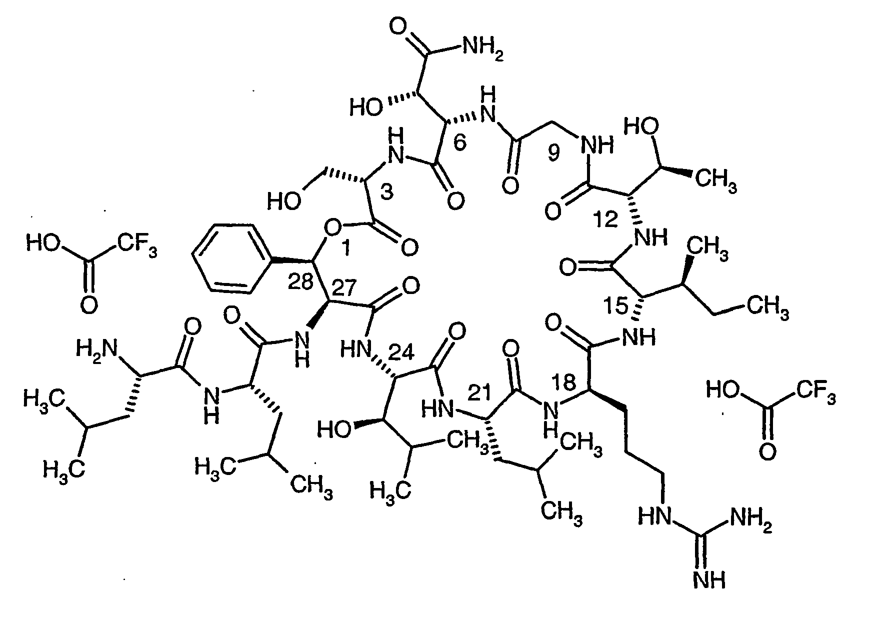

Analog der Allgemeinen Arbeitsvorschrift 5 wird *N*-(*tert*-Butoxycarbonyl)-*epi*-lysobactin-monotrifluoracetat (Beispiel 17A, 1.3 mg, 1 µmol) umgesetzt. Das Rohprodukt wird mittels präparativer HPLC (Methode 15) gereinigt. Nach Gefriertrocknung der Produktfraktionen erhält 1.0 mg (42% d. Th.) Produkt.

HPLC/W-Vis (Methode 13): Rₜ = 5.68 min,
λₘₐₓ (qualitativ) = 220 nm (s), 255-270 (w).

LC-MS (Methode 12): Rₜ = 4.35 min;
MS (ESIpos.): m/z (%) = 639 (100) [M+2H]²⁺, 1277 (5) [M + H]⁺;
MS (ESIneg.): m/z (%) = 1275 (100) [M-H]⁻.
HR-TOF-MS (Methode 21): ber. 1276.7265, gef. 1276.7261 [M + H]+.

### Beispiel 5

### N²-(4-Aminobutanoyl)-N¹-{(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-1-hydroxyethyl]-3-(hydroxymethyl)-24-[(1R)-1-hydroxy-2-methylpropyl]-21-isobutyl-15-[(1S)-1-methylpropyl1-2,5,8,11,14,-17,20,23,26-nonaoxo-28-phenyl-1-oxa-4,7,10,13,16,19,22,25-octaazacyclooctacosan-27-yl}-L-leucinamid-bistrifluoracetat

### {4-Aminobutanoyl-des(leucyl)lysobactin-bistrifluoracetat}

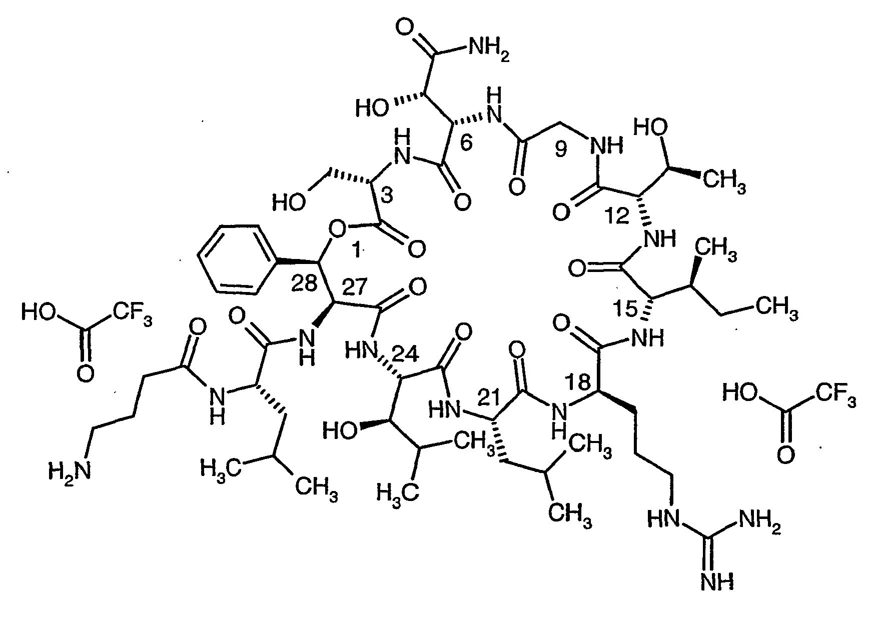

Analog der Allgemeinen Arbeitsvorschrift 5 wird {4-[(*tert*-Butoxycarbonyl)amino]butanoyl}-des(leucyl)lysobactin-trifluoracetat (Beispiel 18A, 2.3 mg, 2 µmol) umgesetzt. Das Rohprodukt wird mittels präparativer HPLC (Methode 15) gereinigt. Nach Gefriertrocknung der Produktfraktionen erhält man 1.5 mg (35% d. Th.) Produkt.

LC-MS (Methode 12): Rₜ = 3.93 min;
MS (ESIpos.): m/z (%) = 625 (100) [M + 2H]²⁺, 1248 (15) [M + H]⁺;
MS (ESIneg.): m/z (%) = 1247 (100) [M - H]⁻.

### Beispiel 6

### N-Methyl-D-leucyl-N¹-{(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,-17,20,23,26-nonaoxo-28-phenyl-1-oxa-4,7,10,13,16,19,22,25-octaazacylooctacosan-27-yl}-L-leucinamid-bistrifluoracetat

### {N-Methyl-lysobactin-bistrifluoracetat}

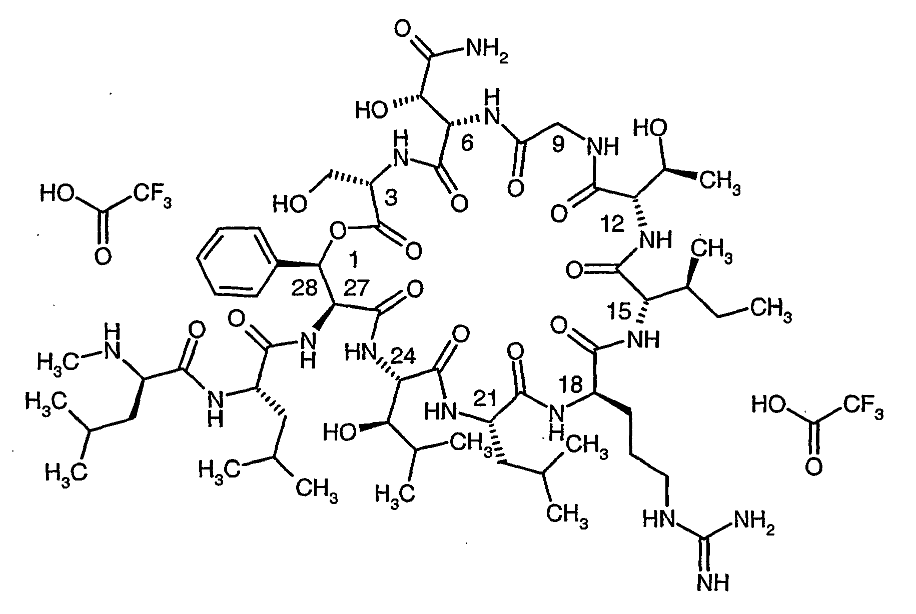

Analog der Allgemeinen Arbeitsvorschrift 5 wird *N*-(*tert*-Butoxycarbonyl)-*N*-methyl-lysobactin-monotrifluoracetat (Beispiel 19A, 1.9 mg, 1 *µ*mol) umgesetzt. Das Rohprodukt wird mittels präparativer HPLC (Methode 15) gereinigt. Nach Gefriertrocknung der Produktfraktionen erhält man 1.8 mg (quant.) Produkt.

LC-MS (Methode 12): Rₜ = 4.27 min;
MS (ESIpos.): m/z (%) = 646 (100) [M + 2H]²⁺, 1291 (15) [M + H]⁺;
MS (ESIneg.): m/z (%) = 1289 (100) [M - H]⁻.
HR-TOF-MS (Methode 21): C₅₉H₁₀₀N₁₅O₁₇ [M + H]⁺ ber. 1290.7422, gef. 1290.7415.

### Beispiel 7

### N²-(6-Aminohexanoyl)-N¹-{(3S,6S,12S,15S,18R,21S,24S,27S,28R)-6-[(1S)-2-amino-1-hydroxy-2-oxoethyl]-18-(3-{ [amino(imino)methyl]amino}propyl)-12-[(1S)-1-hydroxyethyl]-3-(hydroxymethyl)-24-[(1R)-1-hydroxy-2-methylpropyl)-21-isobutyl-15-[(1S)-1-methylpropyl]-2,5,8,11,14,-17,20,23,26-nonaoxo-28-phenyl-1-oxa-4,7,10,13,16,19,22,25-octaazacyclooctacosan-27-yl}-L-leucinamid-bistrifluoracetat

### {N-(6-Aminohexanoyl)-des(leucyl)lysobactin-bistrifluoracetat}

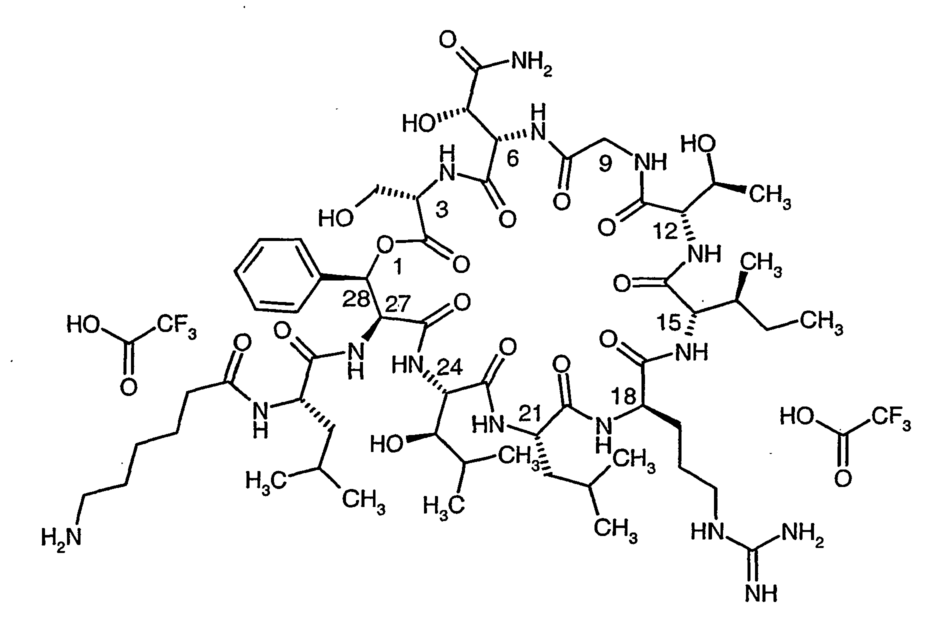

Analog der Allgemeinen Arbeitsvorschrift 5 wird {6-[(*tert*-Butoxycarbonyl)amino]hexanoyl}-des(leucyl)lysobactin-monotrifluoracetat (Beispiel 20A, 350 µg, 1 µmol) umgesetzt. Das Rohprodukt wird mittels präparativer HPLC (Methode 15) gereinigt. Nach Gefriertrocknung der Produktfraktionen erhält man 300 µg (quant.) Produkt.

HPLC/UV-Vis (Methode 13): Rₜ = 5.28 min,
λₘₐₓ (qualitativ) = 220 nm (s), 255-270 (w).
LC-MS (Methode 12): Rₜ = 4.00 min;
MS (ESIpos.): m/z (%) = 639 (100) [M + 2H]²⁺, 1277 (10) [M + H]⁺;
MS (ESIneg.): m/z (%) = 1275 (100) [M - H]⁻.

### Beispiel 8

### N²-(3-Aminopropionyl)-N¹-{(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,-17,20,23,26-nonaoxo-28-phenyl-1-oxa-4,7,10,13,16,19,22,25-octaazacyclooctacosan-27-yl}-L-leucinamid-bistrifluoracetat

### {N-(3-Aminopropionyl)-des(leucyl)lysobactin-bistrifluoracetat}

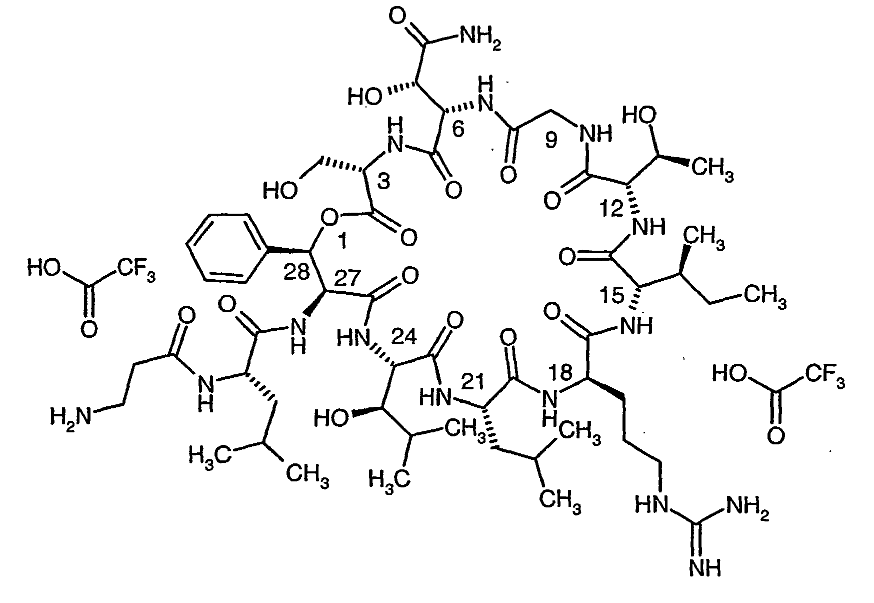

Analog der Allgemeinen Arbeitsvorschrift 5 wird {6-[(*tert*-Butoxycarbonyl)amino)hexanoyl}-des(leucyl)lysobactin-monotrifluoracetat (Beispiel 21A, 2.2 mg, 1.5 µmol) umgesetzt. Das Rohprodukt wird mittels präparativer HPLC (Methode 15) gereinigt. Nach Gefriertrocknung der Produktfraktionen erhält man 200 µg (11% d. Th.) Produkt.

HPLC/UV-Vis (Methode 13): Rₜ = 4.96 min,
λₘₐₓ (qualitativ) = 220 nm (s), 255-270 (w).
LC-MS (Methode 12): Rₜ = 3.86 min;
MS (ESIpos.): m/z (%) = 618 (100) [M + 2H]⁺, 1235 (15) [M + H]⁺;
MS (ESIneg.): m/z (%) = 1233 (100) [M - H]⁻.

Zur Aminosäure-Sequenzbestimmung wird eine analytische Probe des Produkts nach der Allgemeinen Arbeitsvorschrift 10 hydrolysiert.

MALDI-MS (Methode 20): m/z (%) = 1252.8 (100) [M + H]⁺.

### Beispiel 9

### N²-({1-Aminocyclopropyl}carbonyl)-N¹-{(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-L-leucinamid-bistrifluoracetat

### [N-({1-Aminocyclopropyl}carbonyl)-des(leucyl)lysobactin-bistrifluoracetat]

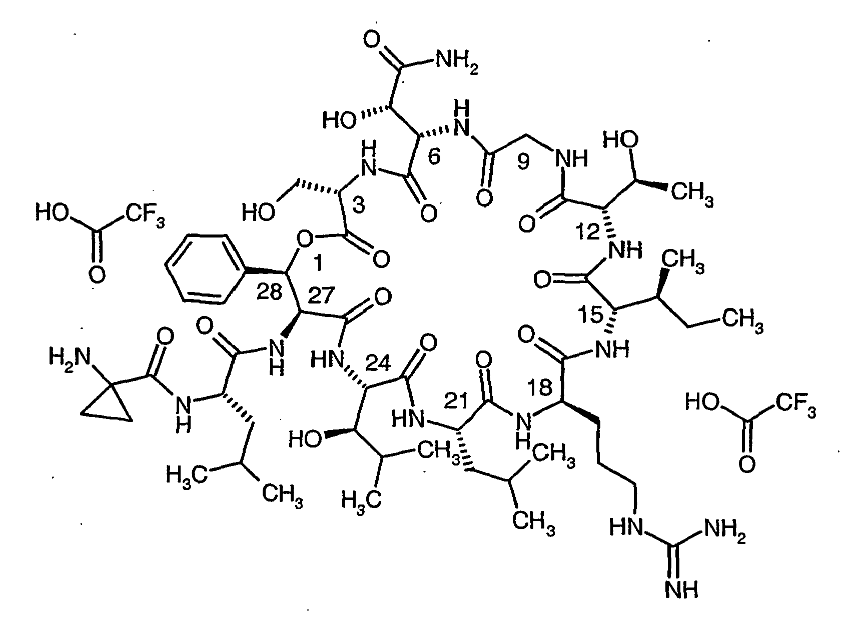

Analog der Allgemeinen Arbeitsvorschrift 5 wird *N*-({1-[(*tert*-Butoxycarbonyl)amino]cyclopropyl}carbonyl)-des(leucyl)lysobactin-monotrifluoracetat (Beispiel 22A, 400 µg, 0.3 µmol) umgesetzt. Das Rohprodukt wird mittels präparativer HPLC (Methode 15) gereinigt. Nach Gefriertrocknung der Produktfraktionen erhält man 200 µg (50% d. Th.) Produkt.

HPLC/UV-Vis (Methode 13): Rₜ = 5.29 min,
λₘₐₓ (qualitativ) = 220 nm (s), 255-270 (w).
LC-MS (Methode 12): Rₜ = 4.08 min;
MS (ESIpos.): m/z (%) = 1247 (100) [M + H]⁺;
MS (ESIneg.): m/z (%) = 1245 (100) [M - H]⁻.

### Beispiel 10

### 3-Amino-N-[(benzyloxy)carbonyl]-L-alanyl-N¹-{(3S,6S,12S,15S,18R,21S,24S,27S,28R)-6-[(1S)-2-amino-1-hydroxy-2-oxoethyl]-18-(3-{ [amino(imino)methyl]amino}propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-L-leucinamid-bistrifluoracetat

### [3-Amino-N-[(benzyloxy)carbonyl]-L-alanyl}-des(leucyl)lysobactin-bistrifluoracetat]

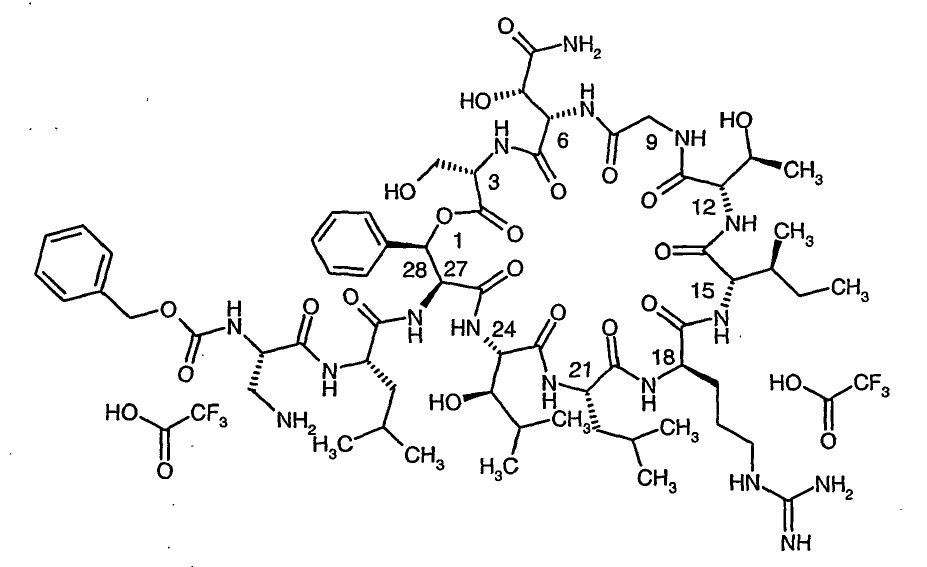

Analog der Allgemeinen Arbeitsvorschrift 5 wird {*N*-[(Benzyloxy)carbonyl]-3-[(*tert*-butoxycarbonyl)amino]-L-alanyl}-des(leucyl)lysobactin-monotrifluoracetat (Beispiel 23A, 300 µg, 0.2 µmol) umgesetzt. Das Rohprodukt wird mittels präparativer HPLC (Methode 15) gereinigt. Nach Gefriertrocknung der Produktfraktionen erhält man 200 µg (65% d. Th.) Produkt.

LC-MS (Methode 12): Rₜ = 4.20 min;
MS (ESIpos.): m/z (%) = 692.5 (100) [M + 2H]²⁺, 1384 (5) [M + H]⁺;
MS (ESIneg.): m/z (%) = 1382 (100) [M - H]⁻.

**Tabelle 5: Edman^{1.0}-Route**

| **Bsp.- Nr.** | **Struktur** | **Analytische Daten** |
|---|---|---|
| | **Name** | **Herstellungsmethode** |
| 11 | 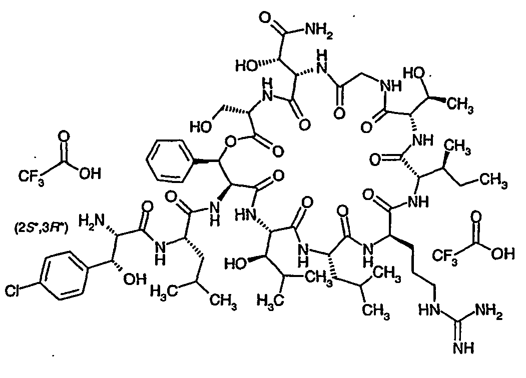 | HR-TOF-MS (Methode 21): C₆₁H₉₅N₁₅O₁₈Cl [M+H]⁺ ber. 1360.6668, gef. 1360.6697. |
| | [3-(4-Chlorphenyl)-*threo*-*rac*-seryl]-des(1-D-leucyl)lysobactin-bistrifluoracetat | Allg. Arbeitsvorschrift 5 aus Beispiel 97A (0.01 mmol). Reinigung nach Methode 8 bzw. Methode 25. |
| | | Ausbeute: 35% d. Th. |
| 12 | 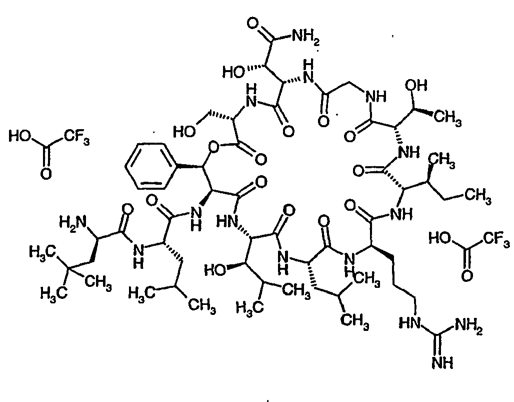 | HPLC/UV-Vis (Methode 13): Rₜ = 6.01 min. |
| | | LC-MS (Methode 26): Rₜ = 1.61 min; |
| | | MS (ESIpos.): *m*/*z* (%) = 646 (100) [M + 2H]²⁺, 1291 (30) [M+H]⁺. |
| | 3-*tert*-Butyl-D-alanyl-des(1-D-leucyl)lysobactin-bis-trifluoracetat | Allg. Arbeitsvorschrift 5 aus Beispiel 98A (0.01 mmol). Reinigung nach Methode 8 bzw. Methode 25. |
| | | Ausbeute: 34% d. Th. |
| 13 | 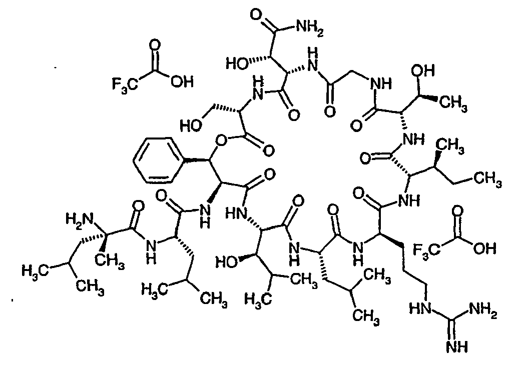 | HPLC/UV-Vis (Methode 13): Rₜ = 5.82 min. |
| | | LC-MS (Methode 26): Rₜ = 1.55 min; |
| | | MS (ESIpos.): *m*/*z* (%) = 646 (100) [M + 2H]²⁺, 1291 (30) [M + H]⁺. |
| | 2-Methyl-L-leucyl-des(1-D-leucyl)lysobactin-bistri-fluoracetat | Allg. Arbeitsvorschrift 5 aus Beispiel 99A (0.01 mmol). Reinigung nach Methode 8 bzw. Methode 25. |
| | | Ausbeute: 47% d. Th. |
| 14 | 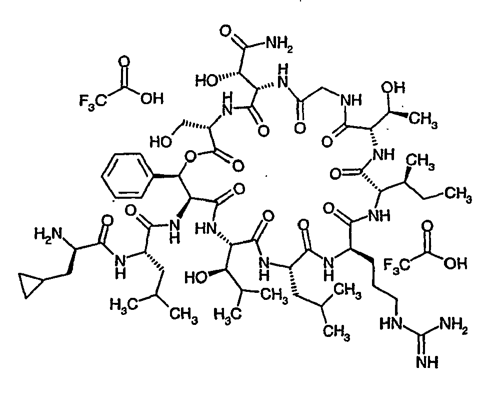 | HPLC/UV-Vis (Methode 36): Rₜ = 3.5 min. |
| | | LC-MS (Methode 29): Rₜ = 4.29 min; |
| | | MS (ESIpos.): *m*/*z* (%) = 638 (100) [M + 2H]²⁺, 1275 (30) [M + H]⁺. |
| | 3-Cyclopropyl-D-alanyl-des(1-D-leucyl)lysobactin-bis-trifluoracetat | Allg. Arbeitsvorschrift 5 aus Beispiel 100A (0.004 mmol). Reinigung nach Methode 8 bzw. Methode 25. |
| | | Ausbeute: 70% d. Th. |
| 15 | 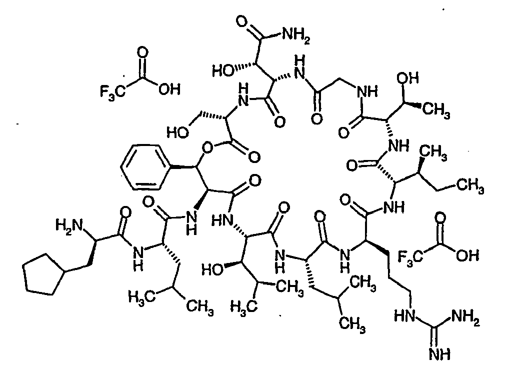 | HPLC/UV-Vis (Methode 13): Rₜ=6.19min. |
| | | LC-MS (Methode 26): Rₜ= 1.66 min; |
| | | MS (ESIpos.): *m*/*z* (%) = 652 (100) [M + 2H]²⁺, 1303 (30) [M + H]⁺. |
| | 3-Cyclopentyl-D-alanyl-des(1-D-leucyl)lysobactin-bis-trifluoracetat | Allg. Arbeitsvorschrift 5 aus Beispiel 101A (0.002 mmol). Reinigung nach Methode 8 bzw. Methode 25. |
| | | Ausbeute: quant. |
| 16 | 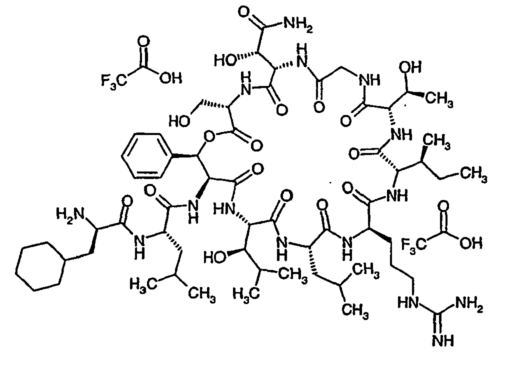 | HPLC/UV-Vis (Methode 13): Rₜ = 6.17 min. |
| | | LC-MS (Methode 26): Rₜ = 1.73 min; |
| | | MS (ESIpos.): *m*/*z* (%) = 659 (100) [M + 2H]²⁺_{.} |
| | 3-Cyclohexyl-D-alanyl-des(1-D-leucyl)lysobactin-bistri-fluoracetat | Allg. Arbeitsvorschrift 5 aus Beispiel 102A (0.01 mmol). Reinigung nach Methode 8 bzw. Methode 25. |
| | | Ausbeute: 51% d. Th. |
| 17 | 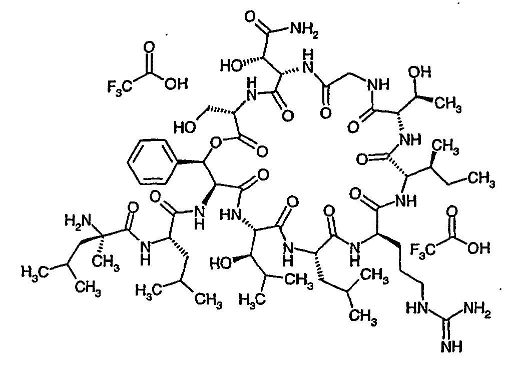 | HPLC/UV-Vis (Methode 13): Rₜ = 5.76 min. |
| | | LC-MS (Methode 26): Rₜ=1.60 min; |
| | | MS (ESIpos.): *m*/*z* (%) = 646 (100) [M + 2H]²⁺, 1291 (30) [M + H]⁺. |
| | 2-Methyl-D-leucyl-des(1-D-leucyl)lysobactin-bistri-fluoracetat | Allg. Arbeitsvorschrift 5 aus Beispiel 103A (0.01 mmol). Reinigung nach Methode 8 bzw. Methode 25. |
| | | Ausbeute: 68% d. Th. |
| 18 | 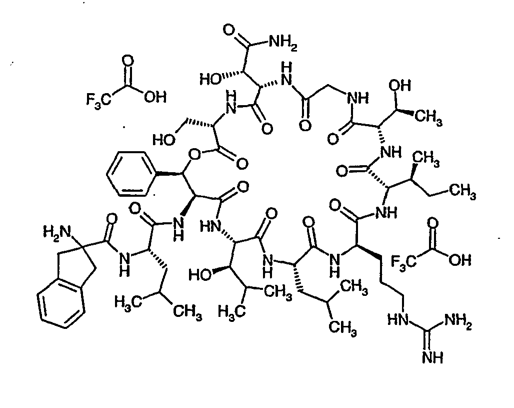 | EPLCILTV-Vis (Methode 13): Rₜ = 5.96 min. |
| | | HR-TOF-MS (Methode 21): C₆₂H₉₆N₁₅O₁₇ [M + H]⁺ ber. 1322.7109, gef. 1322.7076. |
| | (2-Amino-2,3-dihydro-1*H-*indenylcarbonyl)-des(1-D-leucyl)lysobactin-bistrifluoracetat | Allg. Arbeitsvorschrift 5 aus Beispiel 104A (0.06 mmol). Reinigung nach Methode 8 bzw. Methode 25. |
| | | Ausbeute: 54% d. Th. |
| 19 | 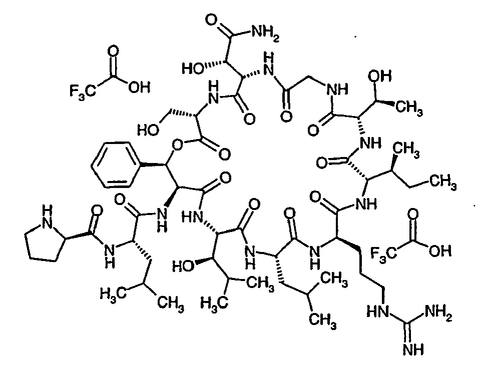 | HPLC/UV-Vis (Methode 13): Rₜ = 5.79 min. |
| | | LC-MS (Methode 26): Rₜ = 1.45 min; |
| | | MS (ESIpos.): *m*/*z* (%) = 1261 (100) [M + 2H]²⁺, 631 (30) [M + H]⁺. |
| | D-Prolyl-des(1-D-leucyl)lysobactin-bistrifluoracetat | Allg. Arbeitsvorschrift 5 aus Beispiel 105A (0.01 mmol). Reinigung nach Methode 8 bzw. Methode 25. |
| | | Ausbeute: 95% d. Th. |
| 20 | 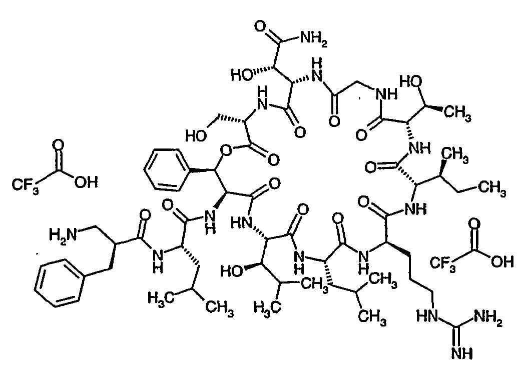 | LC-MS (Methode 26): Rₜ = 1.62 min (100%); |
| | | MS (ESIpos.): *m*/*z* (%) = 663.2 (100) [M + 2H]²⁺, 1324.8(8) [M + H]⁺; |
| | | MS (ESIneg.): *m*/*z* (%) = 661.0 (100) [M - 2H]²⁻, 1368.9 (30) [M + HCOOH - H]⁻. |
| | | HR-TOF-MS (Methode 21): C₆₂H₉₈N₁₅O₁₇ [M + H]⁺ ber. 1324.7265, gef. 1324.7230. |
| | (*(2S)*-3-Amino-2-benzyl-propanoyl)-des(1-D-leucyl)lysobactin-bistrifluoracetat | Aus Beispiel 106A nach allg. Arbeitsvorschrift 5 und Chromatographie (Methode 8). |
| | | Ausbeute: 8.3 mg (14%) über 2 Stufen. |
| 21 | 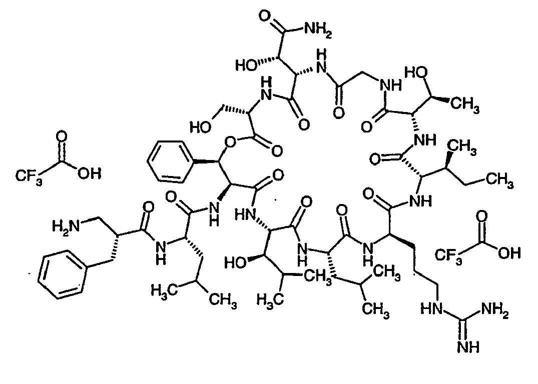 | LC-MS (Methode 26): Rₜ = 1.55 min (100%); |
| | | MS (ESIpos.): *m*/*z* (%) = 663.2 (100) [M + 2H]²⁺, 1324.8(8) [M + H]⁺; |
| | | MS (ESIneg.): *m*/*z* (%) = 661.1(100) [M-2H]²⁻, 1322.8 (25) [M - H]⁻, 1368.9 (36) [M +HCOOH- H]⁻. |
| | | HR-TOF-MS (Methode 21): C₆₂H₉₈N₁₇ [M + H]⁺ ber. 1324.7265, gef. 1324.7230. |
| | [(2*R*)-3-Amino-2-benzyl-propanoyl]-des(1-D-leucyl)lysobactin-bistrifluoracetat | Aus Beispiel 107A nach allg. Arbeitsvorschrift 5 und Chromatographie (Methode 8). |
| | | Ausbeute: 3.1 mg (5%) über 2 Stufen |
| 22 | 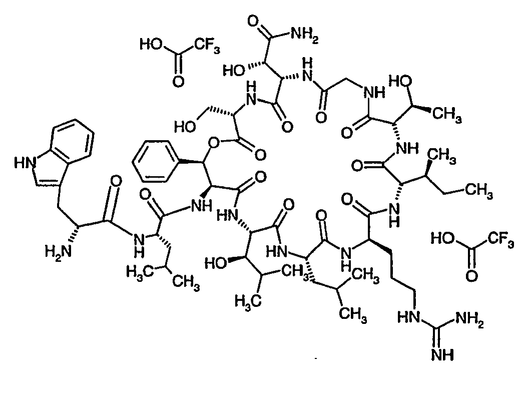 | HPLC/UV-Vis (Methode 36): Rₜ = 3.55 min. |
| | | LC-MS (Methode 26): Rₜ = 1.56 min, |
| | | MS (ESIpos.): *m*/*z* (%) = 675 (100) [M + 2H]²⁺. |
| | | HR-TOF-MS (Methode 21): ber. 1349.7218, gef. 1349.7202 [M + H]⁺. |
| | D-Tryptophyl-des(1-D-leucyl)lysobactin-bistrifluor-acetat | Allgemeine Arbeitsvorschrift 20 aus Beispiel 108A (26 µmol). |
| | | Ausbeute: 56% |
| 23 | 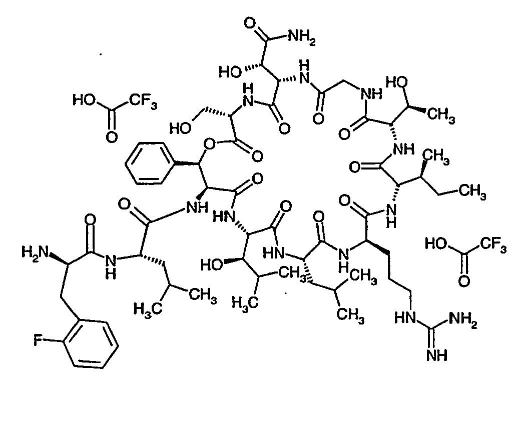 | HPLC/UV-Vis (Methode 36): Rₜ = 3.55 min. |
| | | LC-MS (Methode 26): Rₜ = 1.60 min, |
| | | MS (ESIpos.): *m*/*z* (%) = 1327 (35) [M + H]⁺. |
| | | HR-TOF-MS (Methode 21): ber. 1328.7014, gef. 1328.6971 [M + H]⁺. |
| | (2-Fluor-D-phenylalanyl)-des(1-D-leucyl)lysobactin-bis-trifluoracetat | Allgemeine Arbeitsvorschrift 20 aus Beispiel 109A (26 µmol). |
| | | Ausbeute: 44% d. Th. |
| 24 | 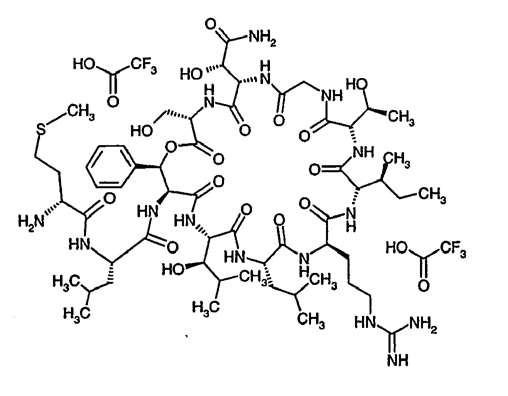 | HPLC/UV-Vis (Methode 36): Rₜ = 3.47 min. |
| | | LC-MS (Methode 26): Rₜ = 1.52 min, |
| | | MS (ESIpos.): *m*/*z* (%) = 1293 [M]⁺. |
| | | HR-TOF-MS (Methode 21): ber. 1294.6829, gef. 1294.6788 [M + H]⁺. |
| | D-Methionyl-des(1-D-leucyl)lysobactin-bistrifluoracetat | Allgemeine Arbeitsvorschrift 20 aus Beispiel 110A (26 µmol). |
| | | Ausbeute: 36% d. Th. |
| 25 | 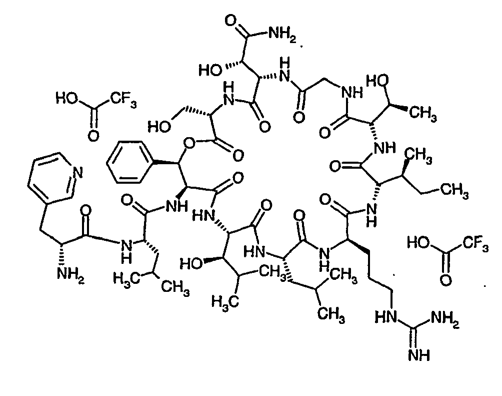 | HPLC/UV-Vis (Methode 36): Rₜ = 3.28 min. |
| | | LC-MS (Methode 26): Rₜ = 1.45 min, |
| | | MS (ESIpos.): *m*/*z* (%) = 656.8 [M + 2H]²⁺ (35). |
| | (3-Pyrid-3-yl-D-alanyl)-des(1-D-leucyl)lysobactin-bis-trifluoracetat | Allgemeine Arbeitsvorschrift 20 aus Beispiel 111A (24 µmol). |
| | | Ausbeute: 15% d. Th. |
| 26 | 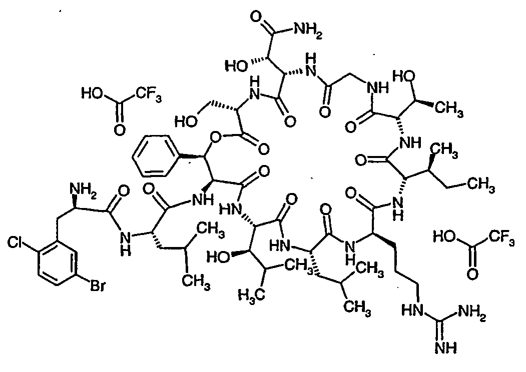 | HPLC/UV-Vis (Methode 36): Rₜ = 3.65 min. |
| | | LC-MS (Methode 26): Rₜ = 1.75 min, |
| | | MS (ESIpos.): *m*/*z* (%) = 711.5 (100) [M + 2H]²⁺. |
| | | HR-TOF-MS (Methode 21): ber. 1422.5824, gef.1422.5838 [M + H]⁺. |
| | (3-Brom-6-chlor-D-phenylalanyl) -des(1-D-leucyl)lysobactin-bistrifluoracetat | Allgemeine Arbeitsvorschrift 20 aus Beispiel 112A (79 µmol). |
| | | Ausbeute: 38% d. Th. |
| 27 | 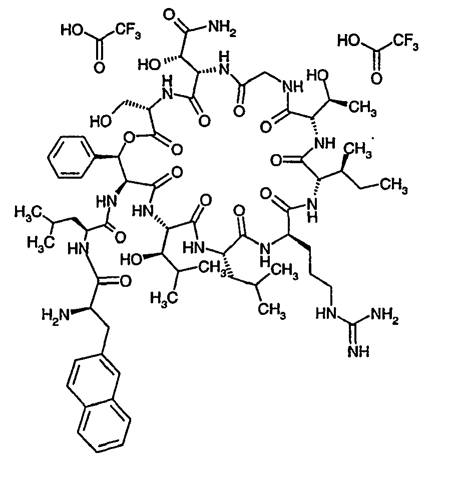 | HPLC/UV-Vis (Methode 36): Rₜ = 3.68 min. |
| | | LC-MS (Methode 26): Rₜ = 1.82 min, |
| | | MS (ESIpos.): *m*/*z* (%) = 681.3 (100) [M]²⁺. |
| | | HR-TOF-MS (Methode 21): ber. 1360.7265, gef. 1360.7285 [M+H]⁺. |
| | [3-(2-Naphthyl)-D-alanyl]-des(1-D-leucyl)lysobactin-bistrifluoracetat | Allgemeine Arbeitsvorschrift 20 aus Beispiel 113A (25 µmol). |
| | | Ausbeute: 37% |
| 28 | 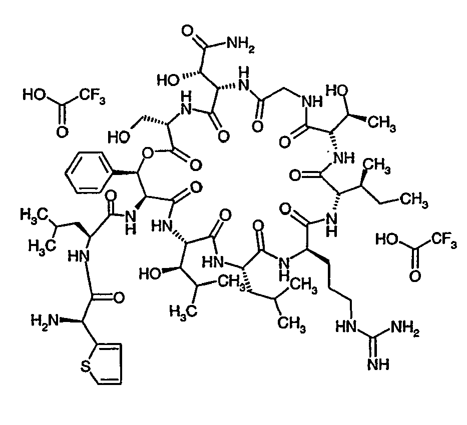 | HPLC/UV-Vis (Methode 36): Rₜ = 3.46 min. |
| | | LC-MS (Methode 26): Rₜ = 1.60 min, |
| | | MS (ESIpos.): *m*/*z* (%) = 1303 (5) [M+H]⁺. |
| | | HR-TOF-MS (Methode 21): ber. 1302.6694, gef. 1302.6698 [M+H]⁺. |
| | [(2*R*)-Amino(2-thienyl)acetyl]-des(1-D-leucyl)-lysobactin-bistrifluoracetat | Allgemeine Arbeitsvorschrift 20 aus Beispiel 114A (8 µmol). |
| | | Ausbeute: 45% |
| 29 | 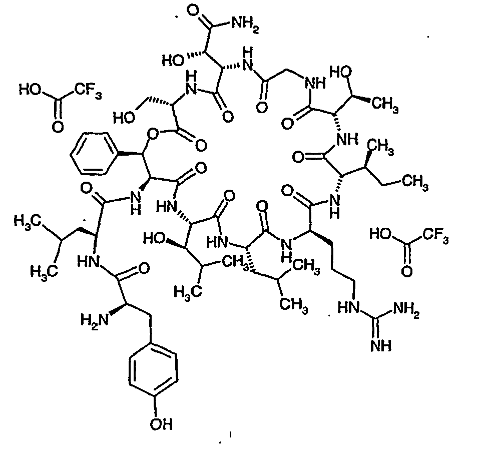 | HPLC/UV-Vis (Methode 36): Rₜ = 3.39 min. |
| | | LC-MS (Methode 26): Rₜ = 1.43 min, |
| | | MS (ESIpos.): *m*/*z* (%) = 664.3 (100) [M + 2H]²⁺. |
| | | HR-TOF-MS (Methode 21): ber. 1326.7058, gef. 1326.7032 [M + H]⁺. |
| | D-Tyrosyl-des(1-D-leucyl)lysobactin-bistrifluoracetat | Allgemeine Arbeitsvorschrift 20 aus Beispiel 115A (25 µmol). |
| | | Ausbeute: 50% |
| 30 | 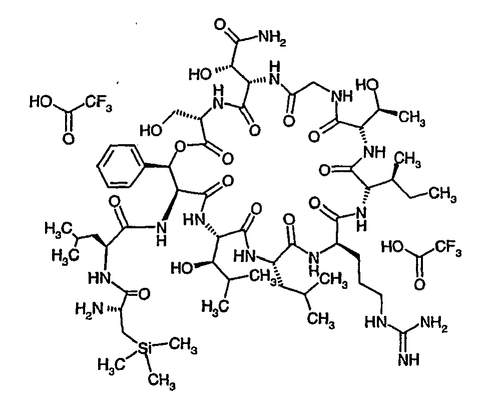 | HPLC/UV-Vis (Methode 36): Rₜ = 3.64 min. |
| | | LC-MS (Methode 26): Rₜ = 1.56 min, |
| | | MS (ESIpos.): *m*/*z* (%) = 654.3 (100) [M + 2H]²⁺; 1307 (2) [M+H]⁺. |
| | | HR-TOF-MS (Methode 21): ber. 1306.7191, gef. 1306.721 [M+H]⁺. |
| | [3-(Trimethylsilyl)-L-alanyl]-des(1-D-leucyl)lysobactin-bistrifluoracetat | Allgemeine Arbeitsvorschrift 20 aus Beispiel 116A (216 µmol). |
| | | Ausbeute: 36% |
| 31 | 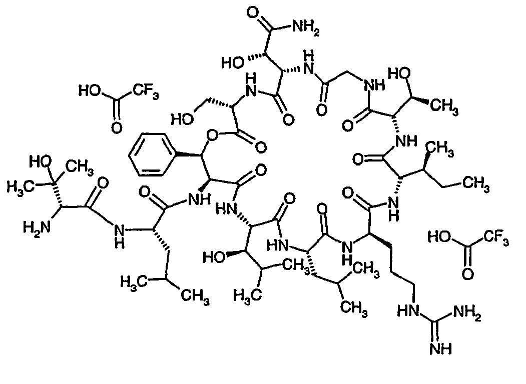 | HPLC/UV-Vis (Methode 36): Rₜ = 3.39 min. |
| | | LC-MS (Methode 26): Rₜ = 1.59 min, |
| | | MS (ESIpos.): *m*/*z* (%) = 640.1 (100) [M + 2H]²⁺. |
| | | HR-TOF-MS (Methode 21): ber. 1278.7058, gef. 1278.7039 [M+H]⁺. |
| | (3-Hydroxy-D-valyl)-des(1-D-leucyl)lysobactin-bistri-fluoracetat | Allgemeine Arbeitsvorschrift 20 aus Beispiel 117A (19 µmol). |
| | | Ausbeute: 22% d. Th. |
| 32 | 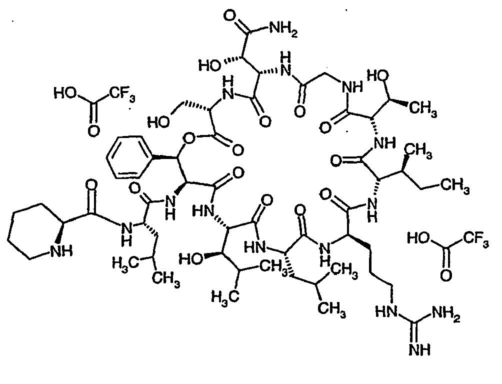 | HPLC/UV-Vis (Methode 36): Rₜ = 3.39 min. |
| | | LC-MS (Methode 26): Rₜ = 1.35 min, |
| | | MS (ESIpos.): *m*/*z* (%) = 638.3 (100) [M + 2H]²⁺. |
| | | HR-TOF-MS (Methode 21): ber. 1274.7109, gef. 1274.7087 [M+H]⁺. |
| | L-Pipecolyl-des(1-D-leucyl)lysobactin-bistrifluoracetat | Allgemeine Arbeitsvorschrift 20 aus Beispiel 118A (21 µmol). |
| | | Ausbeute: 40% d. Th. |
| 33 | 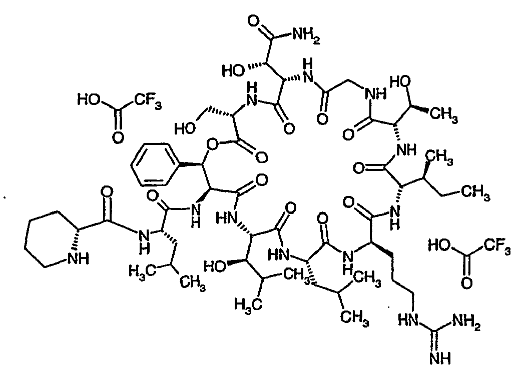 | HPLC/UV-Vis (Methode 36): Rₜ = 3.42 min. |
| | | LC-MS (Methode 26): Rₜ = 1.38 min, |
| | | MS (ESIpos.): *m*/*z* (%) = 1275 (10) [M + H]⁺. |
| | | HR-TOF-MS (Methode 21): ber. 1274.7109, gef. 1274.083 [M+H]⁺. |
| | D-Pipecolyl-des(1-D-leucyl)lysobactin-bistrifluoracetat | Allgemeine Arbeitsvorschrift 20 aus Beispiel 119A (22 µmol). |
| | | Ausbeute: 36% d. Th. |
| 34 | 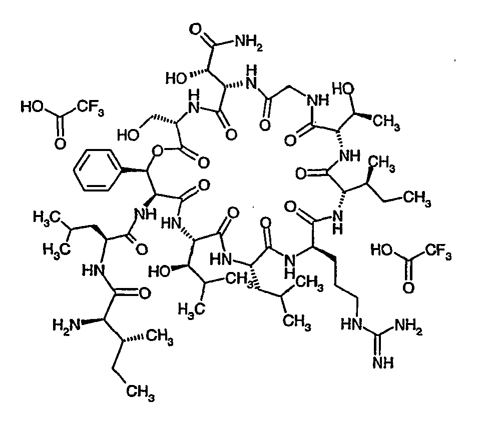 | HPLC/UV-Vis (Methode 36): Rₜ = 3.55 min. |
| | | LC-MS (Methode 26): Rₜ = 1.54 min, |
| | | MS (ESIpos.): *m*/*z* (%) = 639.3 (100) [M+2H]²⁺. |
| | D-Isoleucyl-des(1-D-leucyl)lysobactin-bistrifluoracetat | Allgemeine Arbeitsvorschrift 20 aus Beispiel 120A (20 µmol). |
| | | Ausbeute: 36% d. Th. |
| 35 | 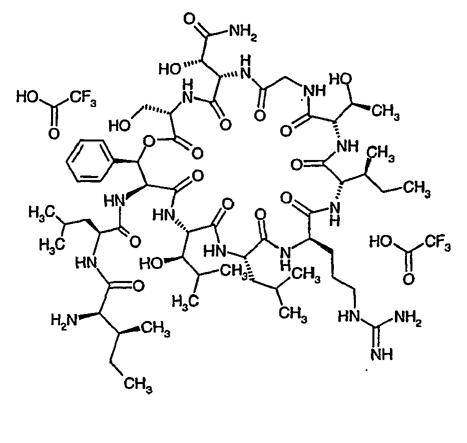 | HPLC/UV-Vis (Methode 36): Rₜ = 3.56 min. |
| | | LC-MS (Methode 26): Rₜ = 1.57 min, |
| | | MS (ESIpos.): *m*/*z* (%) = 639.3 (100) [M+2H]²⁺_{.} |
| | D-Alloisoleucyl-des(1-D-leucyl)lysobactin-bistrifluor-acetat | Allgemeine Arbeitsvorschrift 20 aus Beispiel 121A (17 µmol). |
| | | Ausbeute: 6% d. Th. |
| 36 | 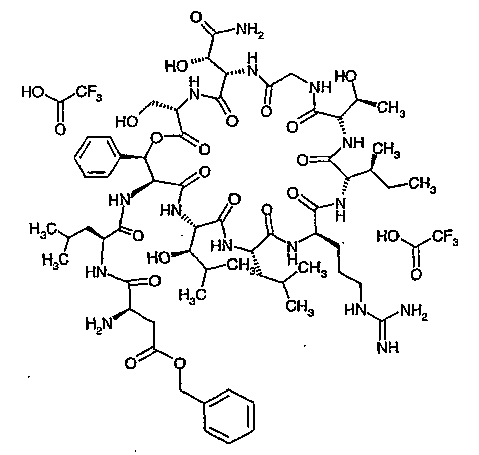 | HPLC/UV-Vis (Methode 36): Rₜ = 3.62 min. |
| | | LC-MS (Methode 26): Rₜ = 1.60 min, |
| | | MS (ESIpos.): *m*/*z* (%) = 685 (100) [M + 2H]²⁺. |
| | | HR-TOF-MS (Methode 21): ber. 1368.7133, gef. 1368.7163 [M+H]⁺. |
| | [(2*R*)-2-Amino-4-(benzyloxy)-4-oxobutanoyl]-des(1-D-leucyl)lysobactin-bistrifluoracetat | Allgemeine Arbeitsvorschrift 20 aus Beispiel 137A (117 µmol). |
| | | Ausbeute: 28% d. Th. |
| 37 | 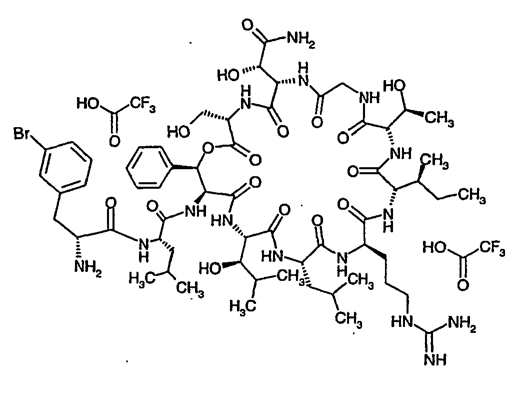 | HPLC/UV-Vis (Methode 36): Rₜ = 3.64 min. |
| | | LC-MS (Methode 26): Rₜ = 1.70 min, |
| | | MS (ESIpos.): *m*/*z* (%) = 695.1 (100) [M + 2H]²⁺. |
| | | HR-TOF-MS (Methode 21): ber. 1388.6214, gef. 1388.6243 [M+H]⁺. |
| | (3-Brom-D-phenylalanyl)-des(1-D-leucyl)lysobactin-bistrifluoracetat | Allgemeine Arbeitsvorschrift 20 aus Beispiel 122A (22 µmol). |
| | | Ausbeute: 22% d. Th. |
| 38 | 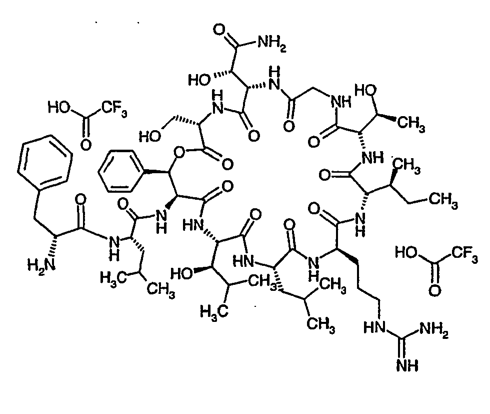 | HPLC/UV-Vis (Methode 36): Rₜ = 3.56 min. |
| | | LC-MS (Methode 26): Rₜ = 1.59 min, |
| | | MS (ESIpos.): *m*/*z* (%) = 656 (100) [M + 2H]²⁺. |
| | | HR-TOF-MS (Methode 21): ber. 1310.7157, gef. 1310.7109 [M+H]⁺. |
| | D-Phenylalanyl-des(1-D-leucyl)lysobactin-bistrifluor-acetat | Allgemeine Arbeitsvorschrift 20 aus Beispiel 123A (43 µmol). |
| | | Ausbeute: 33% d. Th. |
| 39 | 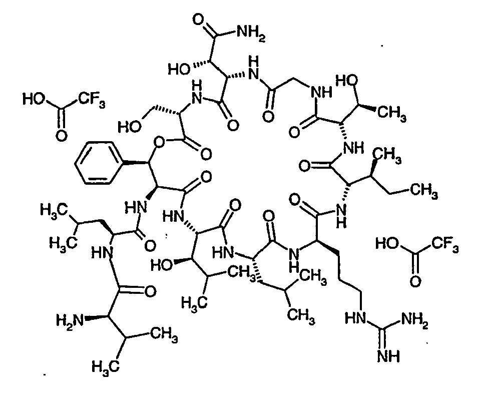 | HPLC/UV-Vis (Methode 36): Rₜ = 4.49 min. |
| | | LC-MS (Methode 26): Rₜ= 1.47 min, |
| | | MS (ESIpos.): *m*/*z* (%) = 632 (100) [M + 2H]²⁺. |
| | | HR-TOF-MS (Methode 21): ber. 1262.7168, gef. 1262.7109 [M+H]⁺. |
| | D-Valyl-des(1-D-leucyl)lysobactin-bistrifluoracetat | Allgemeine Arbeitsvorschrift 20 aus Beispiel 124A (29 µmol). |
| | | Ausbeute: 39% d. Th. |
| 40 | 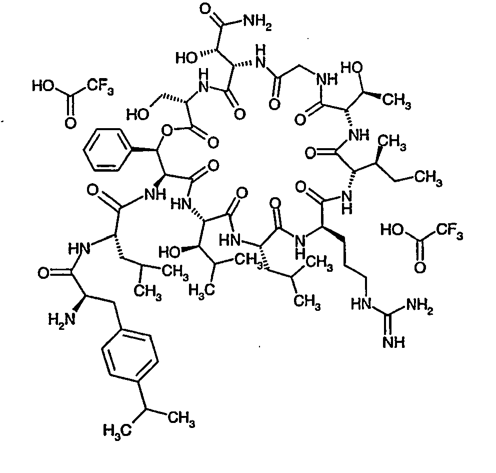 | HPLC/UV-Vis (Methode 36): Rₜ = 3.76 min. |
| | | LC-MS (Methode 26): Rₜ = 1.75 min, |
| | | MS (ESIpos.): *m*/*z* (%) = 677.4 (100) [M + 2H]²⁺. |
| | | HR-TOF-MS (Methode 21): ber. 1352.7578, gef. 1352.7510 [M+H]⁺. |
| | (4-Isopropyl-D-phenylalanyl)-des(1-D-leucyl)-lysobactin-bistrifluoracetat | Allgemeine Arbeitsvorschrift 22 aus Beispiel 125A (24 µmol). |
| | | Ausbeute: 52% d. Th. |
| 41 | 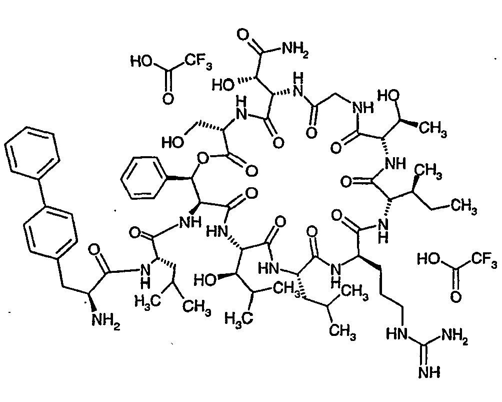 | HPLC/UV-Vis (Methode 36): Rₜ = 3.73 min. |
| | | LC-MS (Methode 26): Rₜ= 1.80 min, |
| | | MS (ESIpos.): *m*/*z* (%) = 694.3 (100) [M + 2H]²⁺. |
| | | HR-TOF-MS (Methode 21): ber. 1386.7422, gef. 1386.7412 [M+H]⁺. |
| | (3-Biphenyl-L-alanyl)-des(1-D-leucyl)lysobactin-bistri-fluoracetat | Allgemeine Arbeitsvorschrift 20 aus Beispiel 126A (37 µmol). |
| | | Ausbeute: 28% d. Th: |
| 42 | 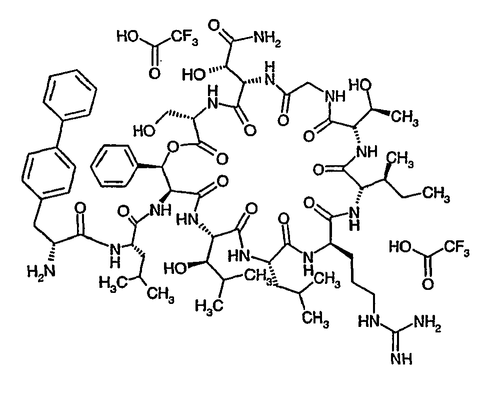 | HPLC/UV-Vis (Methode 36): Rₜ = 3.74 min. |
| | | LC-MS (Methode 26): Rₜ = 1.58 min, |
| | | MS (ESIpos.): *m*/*z* (%) = 694.3 (100) [M + 2H]²⁺. |
| | | HR-TOF-MS (Methode 21): ber. 1386.7422, gef. 1386.7407 [M+H]⁺. |
| | (3-Biphenyl-D-alanyl)-des(1-D-leucyl)lysobactin-bistri-fluoracetat | Allgemeine Arbeitsvorschrift 20 aus Beispiel 127A (74 µmol). |
| | | Ausbeute: 45% d. Th. |
| 43 | 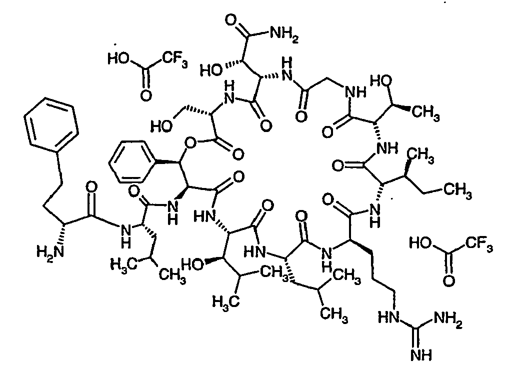 | HPLC/UV-Vis (Methode 36): Rₜ = 3.64 min. |
| | | LC-MS (Methode 26): Rₜ = 1.47 min, |
| | | MS (ESIpos.): *m*/*z* (%) = 663.3 (100) [M + 2H]²⁺. |
| | | HR-TOF-MS (Methode 21): ber. 1324.7265, gef. 13234.7226 [M+H]⁺. |
| | ((2*R*)-2-Amino-4-phenylbutanoyl)-des(1-D-leucyl)-lysobactin-bistrifluoracetat | Allgemeine Arbeitsvorschrift 21 aus Beispiel 128A (37 µmol). |
| | | Ausbeute: 27% d. Th. |
| 44 | 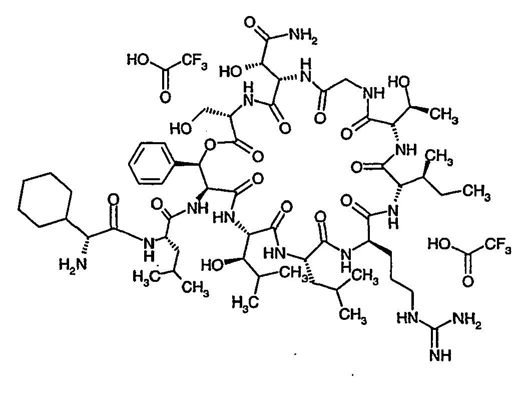 | HPLC/UV-Vis (Methode 36): Rₜ = 3.6 min. |
| | | LC-MS (Methode 26): Rₜ = 1.44 min, |
| | | MS (ESIpos.): *m*/*z* (%) = 652.3 (100) [M + 2H]²⁺_{.} |
| | | HR-TOF-MS (Methode 21): ber. 1302.7422, gef. 1302.7346 [M+ H]⁺. |
| | [(2*R*)-Amino(cyclohexyl)acetyl]-des(1-D-leucyl)-lysobactin-bistrifluoracetat | Allgemeine Arbeitsvorschrift 21 aus Beispiel 129A (36 µmol). |
| | | Ausbeute: 38% d. Th. |
| 45 | 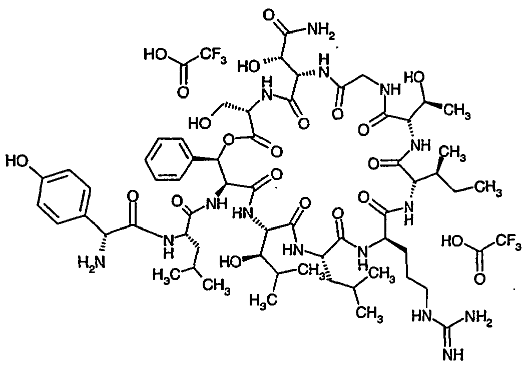 | HPLC/UV-Vis (Methode 36): Rₜ = 3.35 min. |
| | | LC-MS (Methode 26): Rₜ = 1.24 min, |
| | | MS (ESIpos.): *m*/*z* (%) = 657.3 (100) [M + 2 H]²⁺, 1313.1 (3) [M +H]+. |
| | | HR-TOF-MS (Methode 21): ber. 1312.6901, gef. 1312.6916 [M+H]⁺. |
| | [(2*R*)-Amino(4-hydroxyphenyl)acetyl]-des(1-D-leucyl)-lysobactin-bistrifluoracetat | Allgemeine Arbeitsvorschrift 21 aus Beispiel 130A (37 µmol). |
| | | Ausbeute: 24% d. Th. |
| 46 | 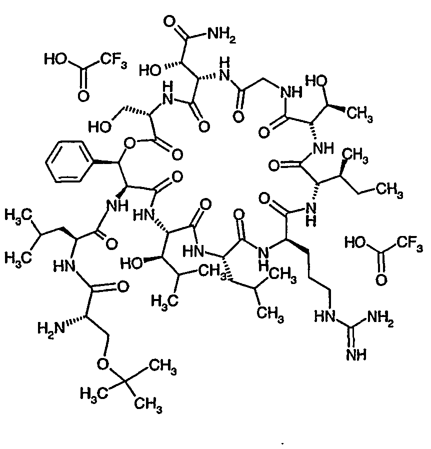 | HPLC/UV-Vis (Methode 36): Rₜ = 3.52 min. |
| | | LC-MS (Methode 26): Rₜ = 1.54 min, |
| | | MS (ESIpos.): *m*/*z* (%) = 1307 (3) [M+H]⁺. |
| | | HR-TOF-MS (Methode 21): ber. 1306.7371, gef. 1306.7307 [M+H]⁺. |
| | [*O*-(*tert*-Butyl)-L-seryl]-des(1-D-leucyl)lysobactin-bis-trifluoracetat | Allgemeine Arbeitsvorschrift 22 aus Beispiel 131A (76 µmol). |
| | | Ausbeute: 48% d. Th. |
| 47 | 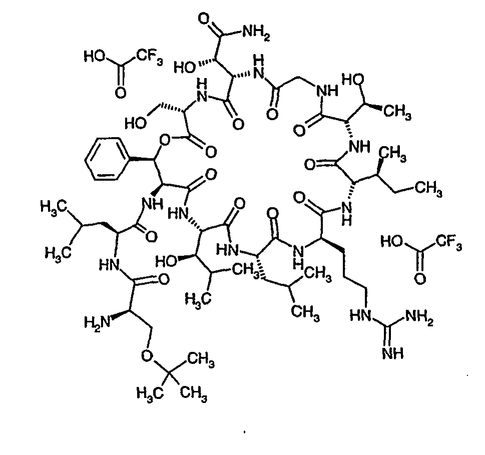 | HPLC/UV-Vis (Methode 36): Rₜ = 3.56 min. |
| | | LC-MS (Methode 26): Rₜ = 1.47 min, |
| | | MS (ESIpos.): *m*/*z* (%) = 1306.9 (3)[M+H]⁺. |
| | | HR-TOF-MS (Methode 21): ber. 1306.7371, gef. 1306.7393 [M+ H]⁺. |
| | [*O*-(*tert*-Butyl)-D-seryl]-des(1-D-leucyl)lysobactin-bis-trifluoracetat | Allgemeine Arbeitsvorschrift 22 aus Beispiel 132A (37 µmol). |
| | | Ausbeute: 79% d. Th. |
| 48 | 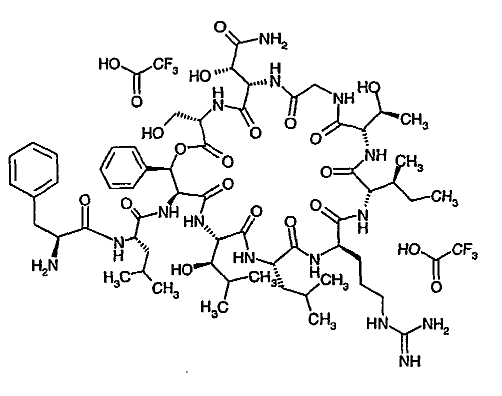 | HPLC/UV-Vis (Methode 36): Rₜ=3.52 min. |
| | | LC-MS (Methode 26): Rₜ 1.44 min, |
| | | MS (ESIpos.): *m*/*z* (%) = 1311.1 (5) [M + H]⁺. |
| | | HR-TOF-MS (Methode 21): ber. 1310.7109, gef. 1310.7153 [M+H]⁺. |
| | L-Phenylalynyl-des(1-D-leucyl)lysobactin-bistrifluor-acetat | Allgemeine Arbeitsvorschrift 21 aus Beispiel 133A (77 µmol). |
| | | Ausbeute: 63% d. Th. |
| 49 | 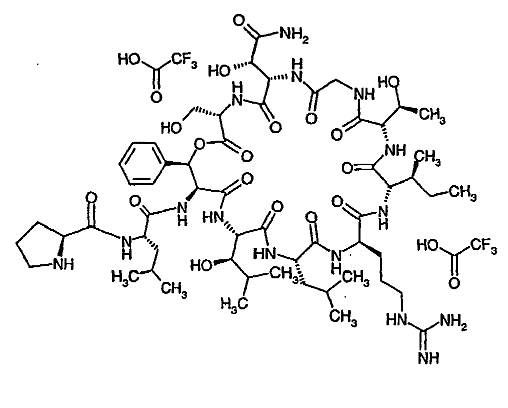 | HPLC/UV-Vis (Methode 36): Rₜ = 3.32 min. |
| | | LC-MS (Methode 26): Rₜ 1.20 min, |
| | | MS (ESIpos.): *m*/*z* (%) = 631.2 (100) [M+2H]²⁺. |
| | | HR-TOF-MS (Methode 21): ber. 1260.6952, gef. 1260.6962 [M+H]⁺ |
| | L-Prolyl-des(1-D-leucyl)lysobactin-bistrifluoracetat | Allgemeine Arbeitsvorschrift 21 aus Beispiel 134A (52 µmol). |
| | | Ausbeute: 64% d. Th. |
| 50 | 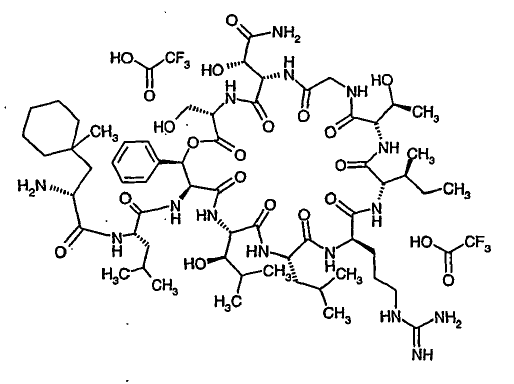 | HPLC/UV-Vis (Methode 36): Rₜ = 3.71 min. |
| | | LC-MS (Methode 26): Rₜ = 1.61 min, |
| | | MS (ESIpos.): *m*/*z* (%) = 666.3 (100) [M + 2H]²⁺. |
| | | HR-TOF-MS (Methode 21): ber. 1330.7735, gef. 1330.7759 [M+H]⁺. |
| | [3-(1-Methylcyclohexyl)-D-alanyl]-des(1-D-leucyl)-lysobactin-bistrifluoracetat | Allgemeine Arbeitsvorschrift 22 (als Lösungsmittel wird reine Essigsäure verwendet) aus Beispiel 135A (57 µmol). |
| | | Ausbeute: 30% d. Th. |
| 51 | 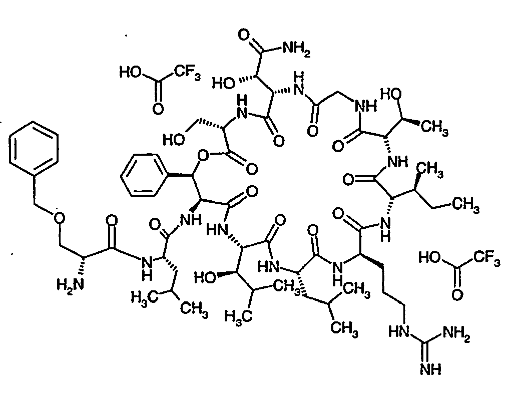 | HPLC/UV-Vis (Methode 36): Rₜ = 3.57 min. |
| | | LC-MS (Methode 26): Rot 1.51 min, |
| | | MS (ESIpos.): *m*/*z* (%) = 671.3 (100) [M+2H]²⁺, 1340.9 (1) [M+H]⁺. |
| | | HR-TOF-MS (Methode 21): ber. 1340.7214, gef. 1340.7155 [M+H]⁺. |
| | (O-Benzyl-D-seryl)-des(1-D-leucyl)lysobactb-bistri-fluoracetat | Allgemeine Arbeitsvorschrift 21 aus Beispiel 136A (67 µmol). |
| | | Ausbeute: 28% d. Th. |
| 52 | 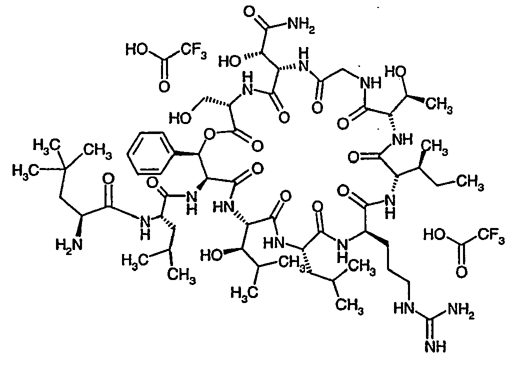 | HPLC/UV-Vis (Methode 36): Rₜ = 3.57 min. |
| | | LC-MS (Methode 26): Rₜ = 1.59 min, |
| | | MS (ESIpos.): *m*/*z* (%) = 646.4 (100) [M + 2H]²⁺_{.} |
| | | HR-TOF-MS (Methode 21): ber. 1290.7422, gef. 1290.7373 [M+H]⁺. |
| | [3-(*tert*-Butyl)-L-alanyl]-des(1-D-leucyl)lysobactin-bis-trifluoracetat | Allgemeine Arbeitsvorschrift 21 aus Beispiel 138A (74 µmol). |
| | | Ausbeute: 41% d. Th. |
| 53 | 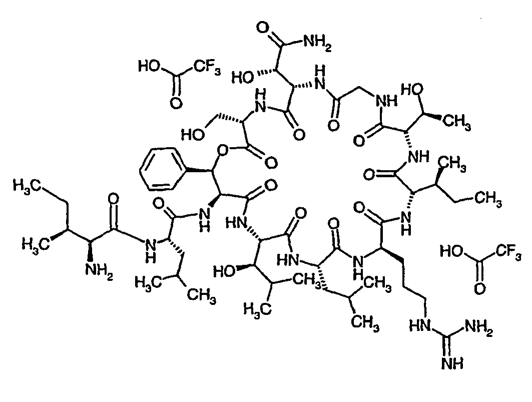 | HPLC/UV-Vis (Methode 36): Rₜ = 3.46 min. |
| | | LC-MS (Methode 26): Rₜ= 1.50 min, |
| | | MS (ESIpos.): *m*/*z* (%) = 639.4 (100) [M + 2H]²⁺. |
| | | HR-TOF-MS (Methode 21): ber. 1276.7265, gef. 1276.7290 [M+H]⁺. |
| | L-Isoleucyl-des(1-D-leucyl)lysobactin-bistrifluoracetat | Allgemeine Arbeitsvorschrift 21 aus Beispiel 139A (95 µmol). |
| | | Ausbeute: 33% d. Th. |
| 54 | 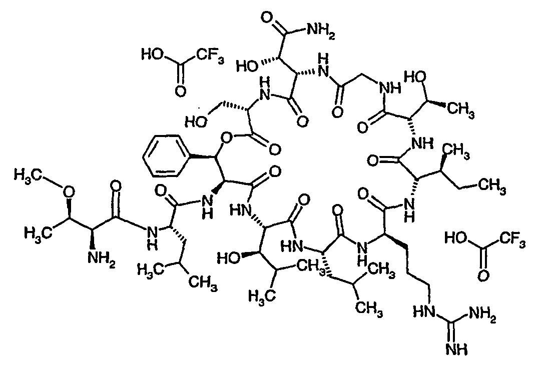 | HPLC/UV-Vis (Methode 36): Rₜ = 3.31 min. |
| | | LC-MS (Methode 26): Rₜ = 1.32 min, |
| | | MS (ESIpos.): *m*/*z* (%) = 640.2 (100) [M + 2H]²⁺. |
| | | HR-TOF-MS (Methode 21): ber. 1278.7058, gef. 1278.7037. |
| | (*O*-Methyl-L-threonyl)-des(1-D-leucyl)lysobactin-bistri-fluoracetat | Allgemeine Arbeitsvorschrift 21 aus Beispiel 140A (50 µmol). |
| | | Ausbeute: 77% d. Th. |
| 55 | 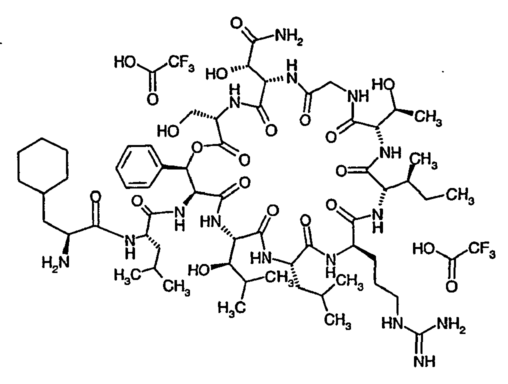 | HPLC/UV-Vis (Methode 36): Rₜ = 3.63 min. |
| | | LC-MS (Methode 26): Rₜ= 1.61 min, |
| | | MS (ESIpos.): *m*/*z* (%) = 659.3 (100) UM + 2H]²⁺. |
| | | HR-TOF-MS (Methode 21): ber. 1316.7578, gef. 1316.7532 [M + H]⁺. |
| | 3-Cyclohexyl-L-alanyl-des(1-D-leucyl)lysobactin-bistri-fluoracetat | Allgemeine Arbeitsvorschrift 21 aus Beispiel 141A (95 µmol). |
| | | Ausbeute: 40% d. Th. |
| 56 | 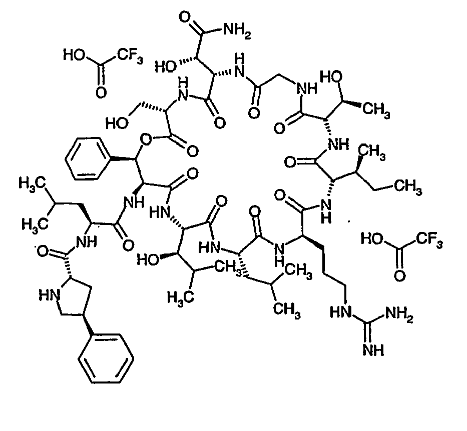 | HPLC/UV-Vis (Methode 36): Rₜ = 3.65 min. |
| | | LC-MS (Methode 26): Rₜ = 1.54 min, |
| | | MS (ESIpos.): *m*/*z* (%) = 669.1 (100) [M + 2H]²⁺. |
| | | HR-TOF-MS (Methode 21): ber. 1316.7265, gef. 1316.7242 [M+H]⁺. |
| | (4*S*)-4-Phenyl-L-prolyl-des(1-D-leucyl)lysobactin-bistri-fluoracetat | Allgemeine Arbeitsvorschrift 21 aus Beispiel 142A (68 µmol). |
| | | Ausbeute: 58% d. Th. |
| 57 | 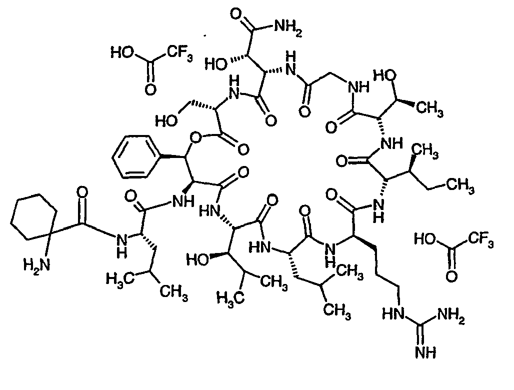 | HPLC/UV-Vis (Methode 36): Rₜ = 3.48 min. |
| | | LC-MS (Methode 26): Rₜ = 1.50 min, |
| | | MS (ESIpos.): *m*/*z* (%) = 645.2 (100) [M + 2H]²⁺. |
| | | HR-TOF-MS (Methode 21): ber. 1288.7264, gef. 1288.7291 [M+H]⁺. |
| | N-(1-Aminocyclohexyl)carbonyl-des(1-D-leucyl)-lysobactin-bistrifluoracetat | Allgemeine Arbeitsvorschrift 21 aus Beispiel 143A (28 µmol). |
| | | Ausbeute: 92% d. Th. |
| 58 | 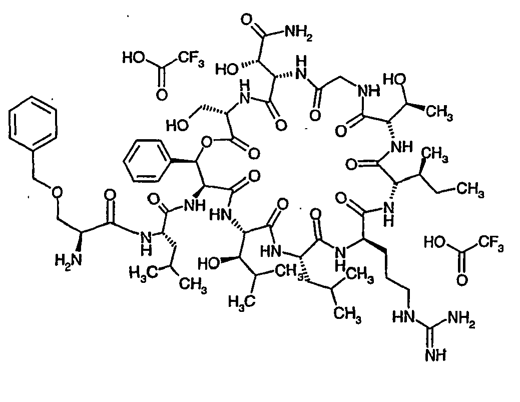 | HPLC/UV-Vis (Methode 36): Rₜ = 3.45 min. |
| | | LC-MS (Methode 26): Rₜ = 1.55 min, |
| | | MS (ESIpos.): *m*/*z* (%) =1340.8 (5) [M+H]⁺. |
| | | HR-TOF-MS (Methode 21): ber. 1340.7214, gef. 1340.7214 [M+H]⁺. |
| | (*O*-Benzyl)-L-seryl-des(1-D-leucyl)lysobactin-bistri-fluoracetat | Allgemeine Arbeitsvorschrift 21 aus Beispiel 144A (55 µmol). |
| | | Ausbeute: 71 % d. Th. |
| 59 | 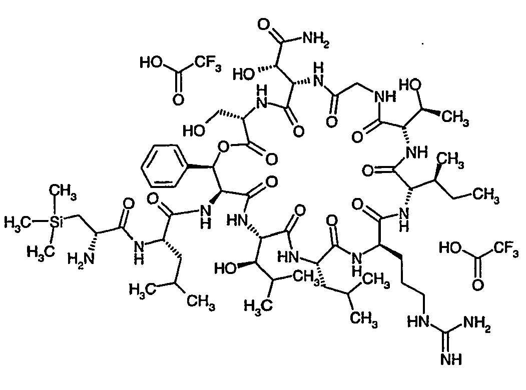 | HPLC/UV-Vis (Methode 36): Rₜ = 3.67 min. |
| | | LC-MS (Methode 26): Rₜ= 1.56 min, |
| | | MS (ESIpos.): *mlz* (%) = 654.2 (100) [M + 2H]²⁺. |
| | | HR-TOF-MS (Methode 21): ber. 1306.7191, gef. 1306.7228 [M+H]⁺. |
| | 3-(Trimethylsilyl)-D-alanyl-des(1-D-leucyl)lysobactin-bistrifluoracetat | Allgemeine Arbeitsvorschrift 21 aus Beispiel 145A (58 µmol). |
| | | Ausbeute: 40% d. Th. |
| 60 | 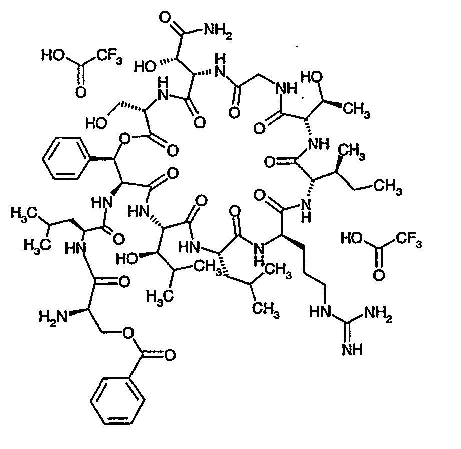 | HPLC/UV-Vis (Methode 36): Rₜ = 3.56 min. |
| | | LC-MS (Methode 26): Rₜ = 1.52 min, |
| | | MS (ESIpos.): *m*/*z* (%) = 678.2 (100) [M + 2H]²⁺. |
| | | HR-TOF-MS (Methode 21): ber. 1354.7007, gef. 1354.6946 [M+H]⁺. |
| | (*O*³-Benzoyl)-D-seryl-des(1-D-leucyl)lysobactin-bistri- fluoracetat | Allgemeine Arbeitsvorschrift 21 aus Beispiel 146A (31 µmol). |
| | | Ausbeute: 58% d. Th. |
| 61 | 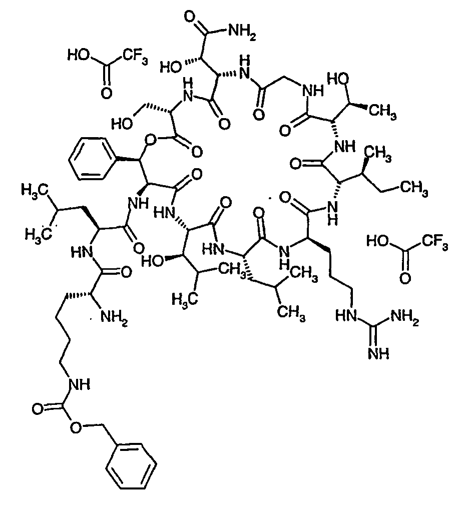 | HPLC/UV-Vis (Methode 36): Rₜ= 3.64 min. |
| | | LC-MS (Methode 26): Rₜ= 1.48 min, |
| | | MS (ESIpos.): *m*/*z* (%) = 713.7 (100) [M + 2H]²⁺. |
| | | HR-TOF-MS (Methode 21): ber. 1425.7742, gef. 1425.7769 [M+H]⁺. |
| | *N*⁶-[(Benzyloxy)carbonyl]-D-lysyl-des(1-D-leucyl)-lysobactin-bistrifluoracetat | Allgemeine Arbeitsvorschrift 21 aus Beispiel 147A (54 µmol). |
| | | Ausbeute: 51% d. Th. |
| 62 | 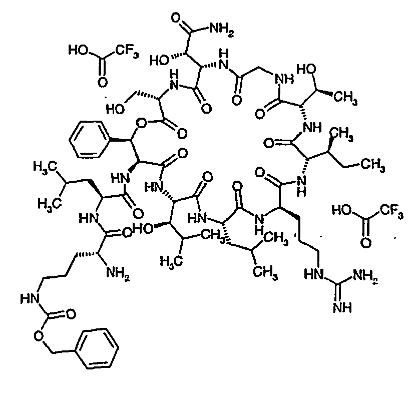 | HPLC/UV-Vis (Methode 36): Rₜ = 3.60 min. |
| | | LC-MS (Methode 26): Rₜ = 1.43 min, |
| | | MS (ESIpos.): *m*/*z* (%) = 706.8 (100) [M + 2H]²⁺. |
| | | HR-TOF-MS (Methode 21): ber. 1411.7585, gef. 1411.7596 [M+H]⁺. |
| | *N*⁵-[(Benzyloxy)carbonyl]-D-omithyl-des(1-D-leucyl)-lysobactin-bistrifluoracetat | Allgemeine Arbeitsvorschrift 21 aus Beispiel 148A (51 µmol). |
| | | Ausbeute: 63% d. Th. |
| 63 | 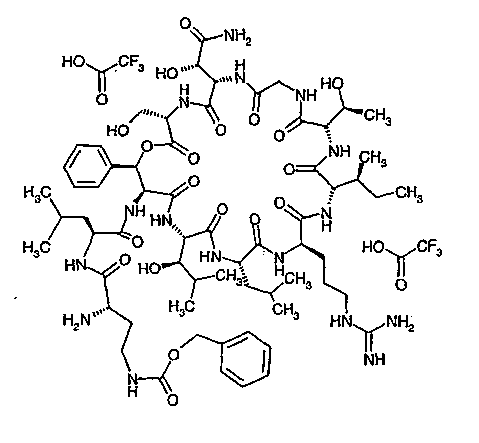 | HPLC/UV-Vis (Methode 36): Rₜ = 3.55 min. |
| | | LC-MS (Methode 26): Rₜ= 1.48 min, |
| | | MS (ESIpos.): *m*/*z* (%) = 699.7 (100) [M 2H]²⁺. |
| | | HR-TOF-MS (Methode 21): ber. 1397.7429, gef. 1397.7367 [M+H]⁺. |
| | (2*S*)-2-Amino-4-{[(benzyloxy)carbonyl]amino}-butanoyl-des(1-D-leucyl)lysobactin-bistrifluoracetat | Allgemeine Arbeitsvorschrift 21 aus Beispiel 149A (52 µmol). |
| | | Ausbeute: 48% d. Th. |
| 64 | 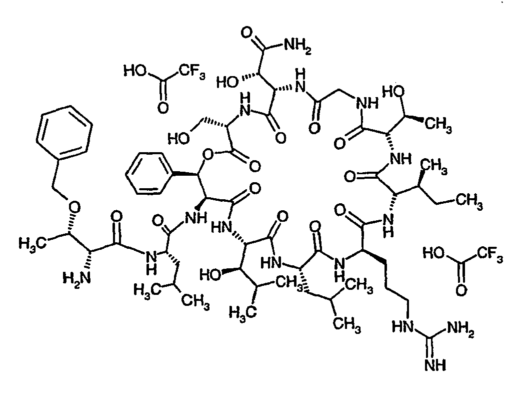 | HPLC/UV-Vis (Methode 36): Rₜ = 3.67 min. |
| | | LC-MS (Methode 26): Rₜ = 1.54 min, |
| | | MS (ESIpos.): *m*/*z* (%) = 678.1 (100) [M+2H]²⁺. |
| | | HR-TOF-MS (Methode 21): ber. 1354.7371, gef. 1354.7358 [M + H]⁺. |
| | *O*⁶-Benzyl-D-threonyl-des(1-D-leucyl)lysobactin-bistri-fluoracetat | Allgemeine Arbeitsvorschrift 21 aus Beispiel 150A (50 µmol). |
| | | Ausbeute: 46% d. Th. |
| 65 | 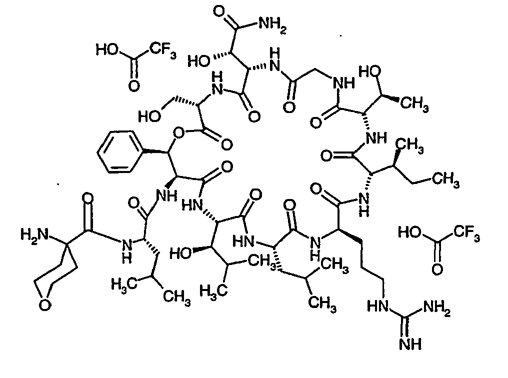 | HPLC/UV-Vis (Methode 36): Rₜ = 3.36 min. |
| | | LC-MS (Methode 26): Rₜ= 1.43 min, |
| | | MS (ESIpos.): *m*/*z* (%) = 646.1 (100) [M + 2H]²⁺_{.} |
| | | HR-TOF-MS (Methode 21): ber. 1290.7058, gef. 1290.7075 [M+H]⁺. |
| | *N*-[(4-Ambotetrahydro-2*H*-pyran-4-yl)carbonyl]-des(1-D-leucyl)lysobactin-bistrifluoracetat | Allgemeine Arbeitsvorschrift 21 aus Beispiel 151A (28 µmol). |
| | | Ausbeute: 7% d. Th. |
| 66 | 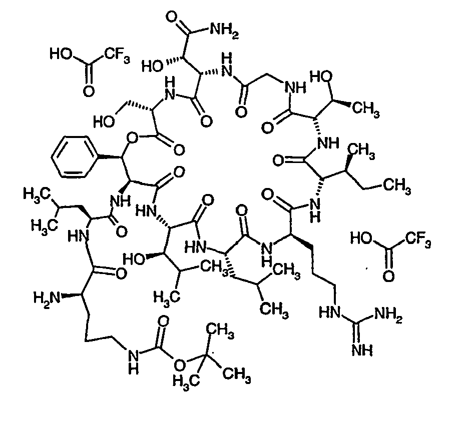 | HPLC/UV-Vis (Methode 36): Rₜ = 3.56 min. |
| | | LC-MS (Methode 26): Rₜ = 1.49 min, |
| | | MS (ESIpos.): *m*/*z* (%) = 689.7 (100) [M + 2H]²⁺. |
| | | HR-TOF-MS (Methode 21): ber. 1377.7742, gef. 1377.7769 [M+H]⁺. |
| | *N*⁵-(*tert*-Butoxycarbonyl)-D-ornithyl-des(1-D-leucyl)-lysobactin-bistrifluoracetat | Allgemeine Arbeitsvorschrift 23 aus Beispiel 152A (60 µmol). |
| | | Ausbeute: 36% d. Th. |
| 67 | 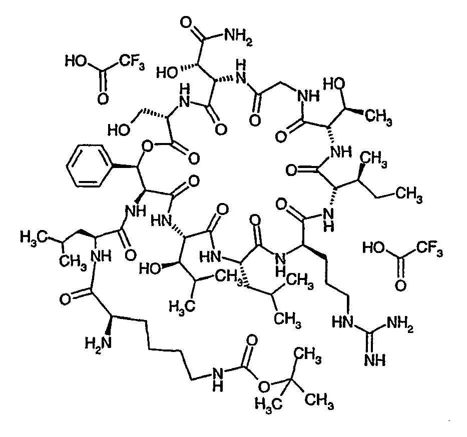 | HPLC/UV-Vis (Methode 36): Rₜ = 3.60 min. |
| | | LC-MS (Methode 26): Rₜ = 1.48 min, |
| | | MS (ESIpos.): *m*/*z* (%) = 696.8 (100) [M + 2H]²⁺. |
| | | HR-TOF-MS (Methode 21): ber. 1391.7898, gef. 1391.7869 [M + H]⁺. |
| | *N*⁶-(*tert*-Butoxycarbonyl)-D-lysyl-des(1-D-leucyl)-lysobactin-bistrifluoracetat | Allgemeine Arbeitsvorschrift 23 aus Beispiel 153A (69 µmol). |
| | | Ausbeute: 46% d. Th. |

### Beispiel 68

### 3-tert-Butyl-D-alanyl-N¹-1(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-1-hydroxyethyl]-3-(hydroxymethyl)-24-[(1R)-1-hydroxy-2-methylpropyl]-21-isobuty1-15-[(1S)-1-methylpropyl]-2,5,8,11,14,-17,20,23,26-nonaoxo-28-phenyl-1-oxa-4,7,10,13,16,19,22,25-octaazacyclooctacosan-27-yl}-3-tert-butyl-L-alaninamid-bistrifluoracetat

### {3-tert-Butyl-D-alanyl-3-tert-butyl-L-alanyl-des(1-D-leucyl-2-L-leucyl)lysobactin-bistrifluoracetat}

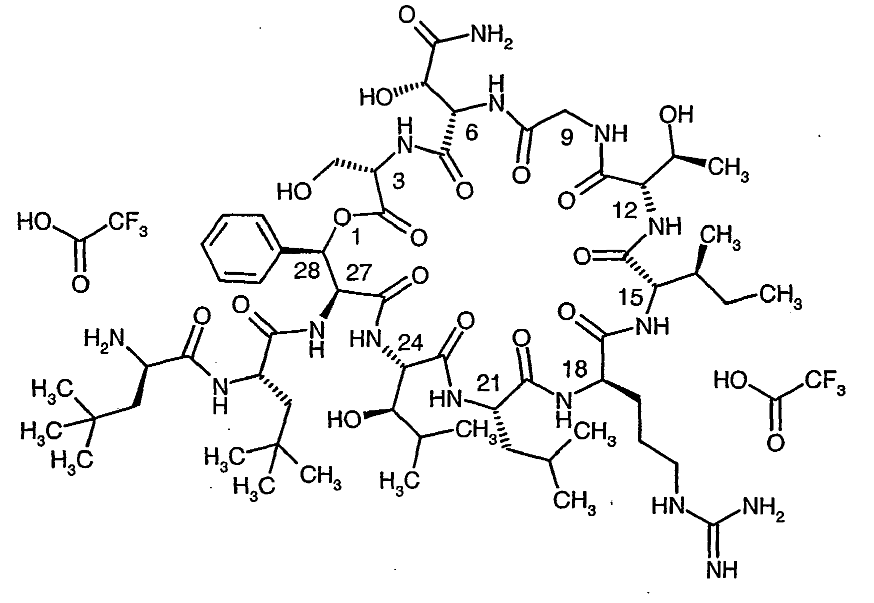

Gemäß der Allgemeinen Arbeitsvorschrift 5 wird das *N*-(*tert*-Butoxycarbonyl)-Depsipeptid (Beispiel 155A, 103 mg, 0.07 mmol) umgesetzt. Nach chromatographischer Reinigung mittels präparativer HPLC (Methode 25) erhält man durch Gefriertrocknung 75.5 mg (73% d. Th.) Produkt.

HPLC/UV-Vis (Methode 13): Rₜ = 6.09 min,
λₘₐₓ (qualitativ) = 220 nm (s), 255-270 (w).
LC-MS (Methode 12): Rₜ = 4.62 min;
MS (ESIpos.): *m*/*z* (%) = 653 (100) [M + 2H]²⁺, 1305 (10) [M + H]⁺;
MS (ESIneg.): *m*/*z* (%) = 651 (40) [M-2H]²-, 1303 (100) [M-H]⁻.
LC-MS (Methode 26): Rₜ =1.64 min;
MS (ESIpos.): m/z (%) = 653 (100) [M + 2H]²⁺, 1305 (5) [M + H]⁺;
MS (ESIneg.): *m*/*z* (%) = 651 (100) [M-2H]²-, 1303 (10) [M-H]⁻.
HR-TOF-MS (Methode 21): C₆₀H₁₀₂N₁₅O₁₇ ber. 1304.7578, gef. 1304.7606 [M + H]⁺.

### Beispiel 69

### 3-tert-Butyl-D-alanyl-3-(3-pyridyl)-L-alanyl-des(1-D-leucyl-2-L-leucyl)lysobactin-tristrifluoracetat

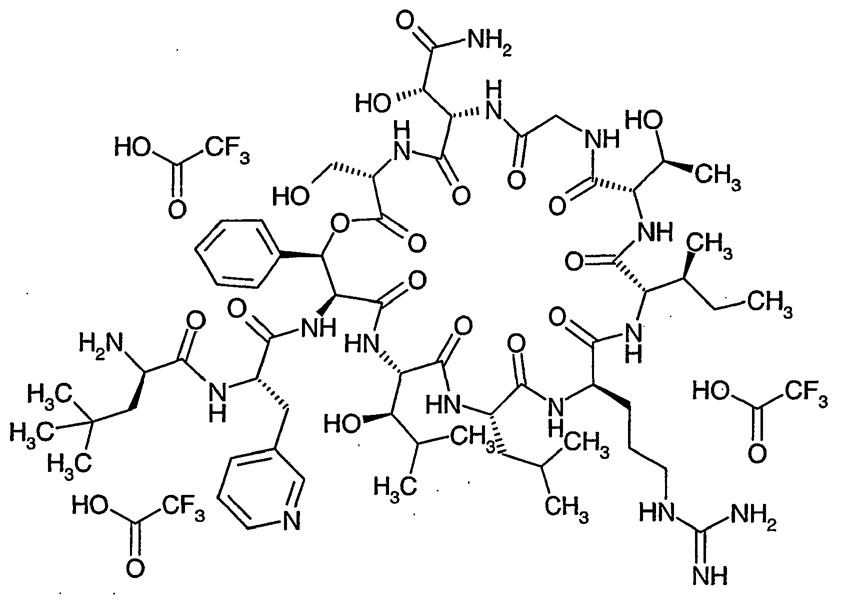

Zu einer Lösung von Beispiel 156A (1.0 Äquivalente, 0.03 mmol) in Dichlormethan (3 ml) wird bei 0°C langsam Trifluoressigsäure (13.0 mmol, 1 ml) zugetropft. Das Reaktionsgemisch wird bei 0°C gerührt (50 min), wobei vollständiger Umsatz mittels EPLC/UV-Vis (Methode 36) beobachtet wird. Das Reaktionsgemisch wird einrotiert und mittels Gelchromatographie (Methode 6, Laufmittel Methanol/Aceton 4/1) aufgereinigt. Das Rohprodukt wird mittels präparativer HPLC (Methode 8) aufgereinigt, wobei man 34 mg (59% d. Th.) Produkt erhält.

[α]²⁰_{Na} = -36° (c = 0.24 in Wasser)
BPLC/UV-Vis (Methode 28): Rₜ = 3.6 min.
HPLC/UV-Vis (Methode 36): Rₜ = 3.28 min.
LC-MS (Methode 26): Rₜ = 1.49 min;
MS (ESIpos.): *m*/*z* (%) = 663 (100) [M + 2H]²⁺, 1325 (10) [M +H]⁺;
MS (ESIneg.): *m*/*z* (%) = 661 (100) [M-2H]², 1323 (40) [M-H]⁻.
HR-MS (Methode 21): C₆₁H₉₇N₁₆O₁₇ [M + H]⁺ ber. 1325.7218, gef. 1325.7261.

**Tabelle 6: Edman^{2.0}-Route**

| **Bsp.-Nr.** | **Struktur** | **Analytische Daten** |
|---|---|---|
| | **Name** | **Herstellungsmethode** |
| 70 | 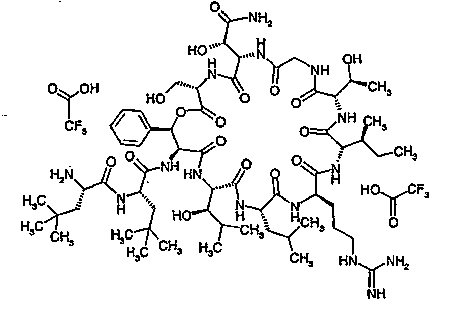 | HPLC/UV-Vis (Methode 13): Rₜ = 5.9 min. |
| | | LC-MS (Methode 26): Rₜ = 1.68 min; |
| | | MS (ESIpos.): *m*/*z* (%) = 653 (100) [M + 2H]²⁺, 1305 (5) [M+H]⁺. |
| | 3-*tert*-Butyl-L-alanyl-3-*tert*-butyl-L-alanyl-des(1-D-leucyl-2-L-leucyl)lysobactin-bistrifluoracetat | Allg. Arbeitsvorschrift 5 (kurze Reaktionszeit) aus Beispiel 157A (0.023 mmol). Reinigung nach Methode 8 bzw. Methode 25. |
| | | Ausbeute: 57% d. Th. |
| 71 | 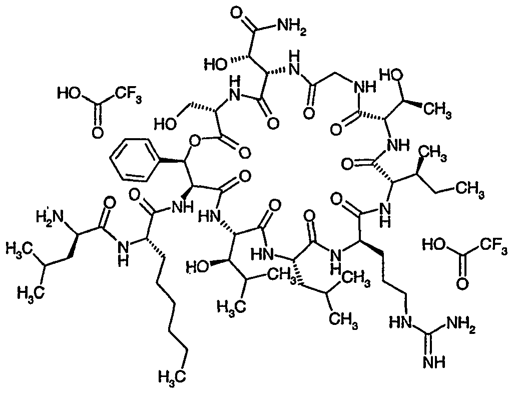 | HPLC/UV-Vis (Methode 13): Rₜ = 6.12 min, |
| | | λₘₐₓ (qualitativ) = 220 nm (s), 255-270 (m). |
| | | LC-MS (Methode 29): Rₜ = 4.61 min; |
| | | MS (ESIpos.): *m*/*z* (%) = 653 (100) [M+2H]²⁺, 1305 (5) [M+H]⁺. |
| | | HR-TOF-MS (Methode 21): C₆₀H₁₀₂N₁₅O₁₇ [M + H]⁺ ber. 1304.7578, gef. 1304.7610. |
| | D-Leucyl-3-pentyl-L-alanyl-des(1-D-leucyl-2-L-leucyl)lysobactin-bistrifluoracetat | Analog Beispiel 68A aus Beispiel 13A (0.016 mmol) und (2*S*)-2-{[*N*(tert-Butoxy-carbonyl)-D-leucyl]amino}-octansäure (0.063 mmol). Reinigung nach Methode 8 bzw. Methode 25. |
| | | Ausbeute: 16% d. Th. (über 2 Stufen) |
| 72 | 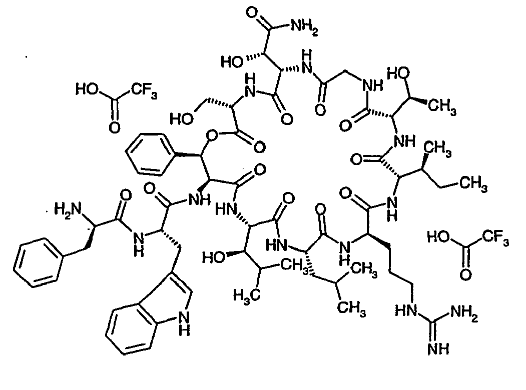 | HPLC/UV-Vis (Methode 13): Rₜ = 5.92 min. |
| | | LC-MS (Methode 29): Rₜ = 4.37 min; |
| | | MS (ESIpos.): *m*/*z* (%) = 693 (100) [M + 2H]²⁺, 1384 (5) [M+H]⁺. |
| | | HR-TOF-MS (Methode 21): C₆₆H₉₅N₁₆O₁₇ + H]⁺ ber. 1383.7061, gef. 1383.7098. |
| | D-Phenylalanyl-L-tryptophyl-des(1-D-leucyl-2-L-leucyl)lysobactin-bistrifluoracetat | Analog Beispiel 68A aus Beispiel 13A (0.016 mmol) und N-tert-Butoxycarbonyl-D-phenylalanyl-L-tryptophan (0.063 mmol). Reinigung nach Methode 8 bzw. Methode 25. |
| | | Ausbeute: 47% d. Th. (über 2 Stufen) |
| 73 | 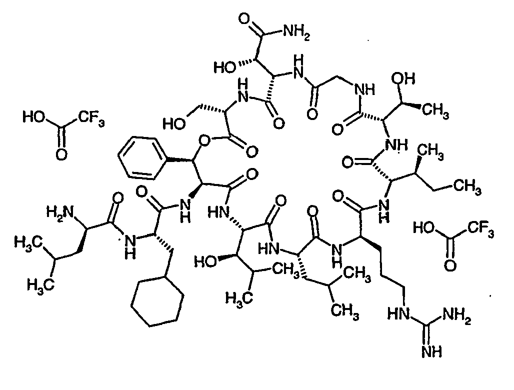 | HPLC/UV-Vis (Methode 13): Rₜ = 6.14 min. |
| | | LC-MS (Methode 29): Rₜ = 4.54 min; |
| | | LC-MS (ESIpos.): *m*/*z* (%) = 659 (100) [M + 2H]²⁺, 1317 (15) [M+H]⁺. |
| | | HR-TOF-MS (Methode 21): C₆₁H₁₀₂N₁₅O₁₇ [M + H]⁺ ber. 1316.7578, gef. 1316.7616. |
| | D-Leucyl-3-cyclohexyl-L-alanyl-des(1-D-leucyl-2-L-leucyl)lysobactin-bistrifluoracetat | Allg. Arbeitsvorschrift 5 aus Beispiel 158A (0.01 mmol). Reinigung nach Methode 8 bzw. Methode 25. |
| | | Ausbeute: 49% d. Th. |
| 74 | 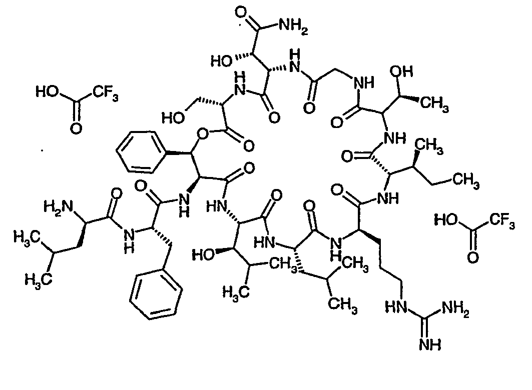 | HPLC/UV-Vis (Methode 13): Rₜ = 6.03 min, |
| | | λₘₐₓ (qualitativ) = 220 nm (s), 255-270 (m). |
| | | LC-MS (Methode 12): Rₜ=4.51 min; |
| | | MS (ESIpos.): *m*/*z* (%) = 656 (100) [M+2H]²⁺,1311 (5) [M+H]⁺. |
| | | HR-TOF-MS (Methode 21): C₆₁H₉₆N₁₅O₁₇ [M+H]⁺ ber. 1310.7109, gef. 1310.7094. |
| | D-Leucyl-L-phenylalanyl-des(1-D-leucyl-2-L-leucyl)lysobactin-bistrifluoracetat | Allg. Arbeitsvorschrift 5 aus Beispiel 159A (0.01 mmol). Reinigung nach Methode 8 bzw. Methode 25. |
| | | Ausbeute: 74% d. Th. |
| 75 | 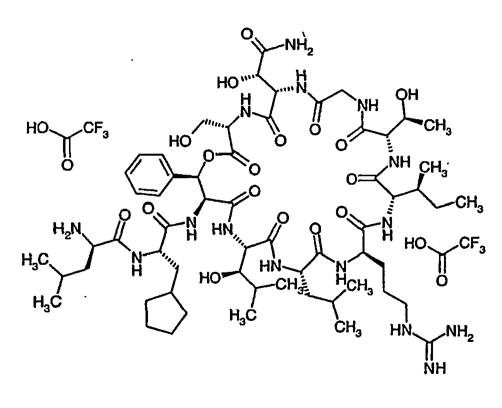 | HPLC/UV-Vis (Methode 13): Rₜ = 6.16 min, |
| | | λₘₐₓ (qualitativ) = 220 nm (s), 255-270 (m). |
| | | LC-MS (Methode 12): - Rₜ = 4.55 min; |
| | | MS (ESIpos.): *m*/*z* (%) = 652 (100) [M + 2H]²⁺, 1303 (20) [M + H]⁺. |
| | | HR-TOF-MS (Methode 21): C₆₀H₁₀₀N₁₅O₁₇ [M+ H]⁺ ber. 1302.7422, gef. 1302.7404. |
| | D-Leucyl-3-cyclopentyl-L-alanyl-des(1-D-leucyl-2-L-leucyl)lysobactin-bistrifluoracetat | Allg. Arbeitsvorschrift 5 aus Beispiel 160A (0.01 mmol). Reinigung nach Methode 8 bzw. Methode 25. |
| | | Ausbeute: 92% d. Th. |
| 76 | 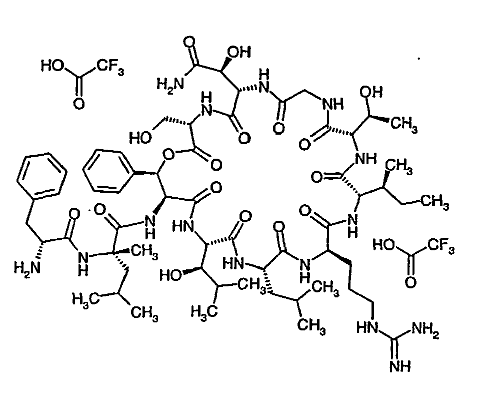 | HPLC/UV-Vis (Methode 13): Rₜ = 6.02 min, |
| | | λₘₐₓ (qualitativ) = 220 nm (s), 255-270 (m). |
| | | LC-MS (Methode 26): Rt = 2.25 min; |
| | | MS (ESIpos.): *m*/*z* (%) = 663 (100) [M + 2H]²⁺. |
| | D-Phenylalanyl-2-methyl-L-leucyl-des(1-D-leucyl-2-L-leucyl)lysobactin-bistrifluoracetat | Allg. Arbeitsvorschrift 5 aus Beispiel 161A (0.012 mmol). Reinigung nach Methode 8 bzw. Methode 25. |
| | | Ausbeute: 8.5% d. Th. |
| 77 | 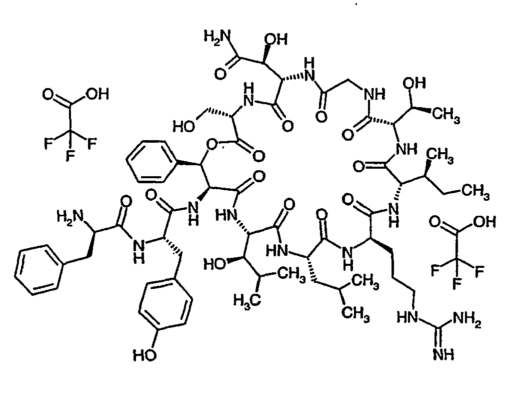 | HPLC/UV-Vis (Methode 13): Rₜ= 5.39 min. |
| | | LC-MS (Methode 29): Rt = 4.2 min; |
| | | MS (ESIpos.): *m*/*z* (%) = 681 (100) [M + 2H]²⁺, 1361 (10) [M+H]⁺. |
| | | HR-TOF-MS (Methode 21): C₆₄H₉₄N₁₅O₁₈ [M + H]⁺ ber. 1360.6901, gef. 1360.6901. |
| | D-Phenylalanyl-L-tyrosyl-des(1-D-leucyl-2-L-leucyl)lysobactin-bistrifluoracetat | Allg. Arbeitsvorschrift 5 aus Beispiel 162A (0.01 mmol). |
| | | Ausbeute: 93% d. Th. |
| 78 | 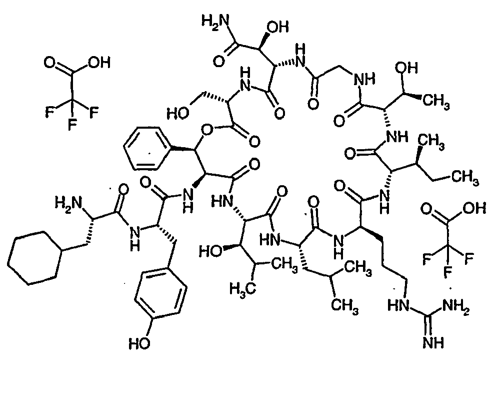 | HPLC/UV-Vis (Methode 13): Rₜ = 5.66 min. |
| | | LC-MS (Methode 29): Rₜ = 4.4 min; |
| | | MS (ESIpos.): *m*/*z* (%) = 684 (100) [M + 2H]²⁺,1367 (10) [M+H]⁺. |
| | | HR-TOF-MS (Methode 21): C₆₄H₁₀₀N₁₅O₁₈ [M+H]⁺ ber. 1366.7371, gef. 1366.7335. |
| | 3-Cyclohexyl-L-alanyl-L-tyrosyl-des(1-D-leucyl-2-L-leucyl)lysobactin-bistrifluoracetat | Allg. Arbeitsvorschrift 5 aus Beispiel 163A (0.01 mmol). |
| | | Ausbeute: 97% d. Th. |
| 79 | 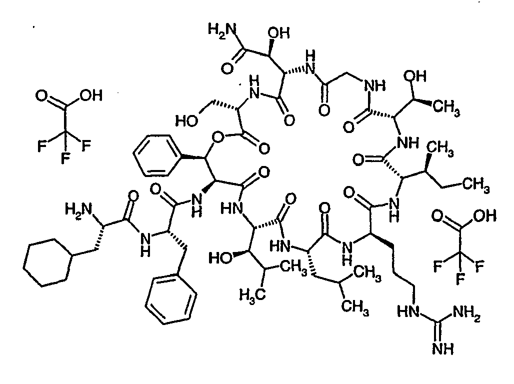 | HPLC/UV-Vis (Methode 13): Rₜ = 6.01 min. |
| | | LC-MS (Methode 29): Rₜ = 4.6 min; |
| | | MS (ESIpos.): *m*/*z* (%) = 677 (100) [M + 2H]²⁺, 1352 (10) [M + H]⁺. |
| | | HR-TOF-MS (Methode 21): C₆₄H₁₀₀N₁₅O₁₇ [M + H]⁺ ber. 1350.7422, gef. 1350.7393. |
| | 3-Cyclohexyl-L-alanyl-L-phenylalanyl-des(1-D-leucyl-2-L-leucyl)lysobactin-bistrifluoracetat | Allg. Arbeitsvorschrift 5 aus Beispiel 164A (0.01 mmol). |
| | | Ausbeute: 95% d. Th. |
| 80 | 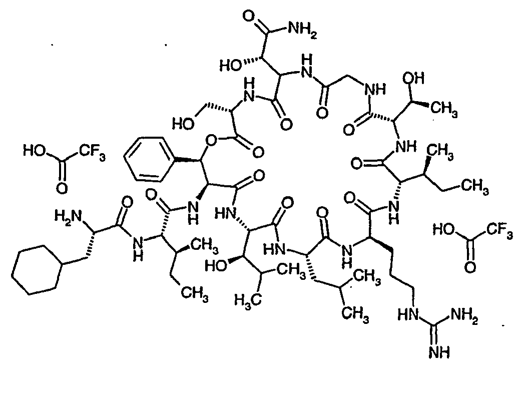 | LC-MS (Methode 26): Rₜ = 1.53 min; |
| | | MS (ESIpos.): *m*/*z* (%) = 659 (100) [M+2H]²⁺, 1317(5) [M+H]⁺. |
| | | HR-TOF-MS (Methode 21): C₆₁H₁₀₂N₁₅O₁₇ [M+H]⁺ ber. 1316.7578, gef. 1316.7574. |
| | 3-Cyclohexyl-L-alanyl-L-isoleucyl-des(1-D-leucyl-2-L-leucyl)lysobactin-bistrifluoracetat | Allg. Arbeitsvorschrift 5 aus Beispiel 165A (0.006 mmol). |
| | | Ausbeute: 99% d. Th. |
| 81 | 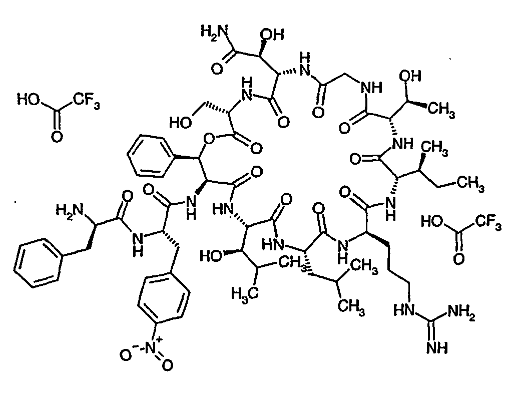 | HPLC/UV-Vis (Methode 13): Rₜ = 5.83 min. |
| | | LC-MS (Methode 29): Rₜ = 4.5 min; |
| | | MS (ESIpos.): *m*/*z* (%) = 696 (100) [M + 2H]²⁺, 1390 (10) [M + H]⁺. |
| | | HR-TOF-MS (Methode 21): C₆₄H₉₃N₁₆O₁₉ [M + H]⁺ ber. 1389.6803, gef. 1389.6823. |
| | D-Phenylalanyl-4-nitro-L-phenylalanyl-des(1-D-leucyl-2-L-leucyl)lysobactin-bistrifluoracetat | Allg. Arbeitsvorschrift 5 aus Beispiel 166A (0.007 mmol). Reinigung nach Methode 8 bzw. Methode 25. |
| | | Ausbeute: 46% d. Th. |
| 82 | 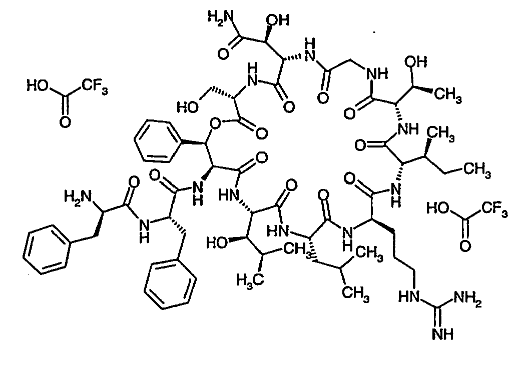 | HPLC/UV-Vis (Methode 13): Rₜ = 5.84 min, |
| | | λₘₐₓ (qualitativ) = 220 nm (s), 255-270 (m). |
| | | LC-MS (Methode 12): Rₜ = 4.5 min; |
| | | MS (ESIpos.): *m*/*z* (%) = 674 (100) [M + 2H]²⁺, 1345 (50) [M + H]⁺. |
| | | HR-TOF-MS (Methode 21): C₆₄H₉₄N₁₅O₁₇ [M + H]⁺ ber. 1344.6952, gef. 1344.6927. |
| | D-Phenylalanyl-L-phenylalanyl-des(1-D-leucyl-2-L-leucyl)lysobactin-bistrifluoracetat | Allg. Arbeitsvorschrift 5 aus Beispiel 169A (0.06 mmol). Reinigung nach Methode 8 bzw. Methode 25. |
| | | Ausbeute: 82% d. Th. |
| 83 | 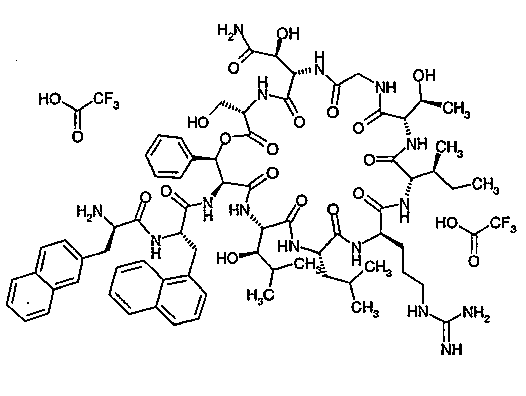 | LC-MS/UV-Vis (Methode 12): Rₜ = 4.8 min; |
| | | λₘₐₓ (qualitativ) = 224 nm (s), 280 (m); |
| | | MS (ESIpos.): *m*/*z* (%) = 723 (100) [M + 2H]²⁺, 1445 (10) [M + H]⁺. |
| | | HR-TOF-MS (Methode 21): C₇₂H₉₈N₁₅O₁₇ [M+H]⁺ ber. 1444.7265, gef. 1444.7290. |
| | 3-(2-Naphtyl)-D-alanyl-3-(1-naphtyl)-L-alanyl-des(1-D-leucyl-2-L-leucyl)lysobactin-bistrifluoracetat | Allg. Arbeitsvorschrift 5 aus Beispiel 170A (0.005 mmol). Reinigung nach Methode 8 bzw. Methode 25. |
| | | Ausbeute: 86% d. Th. |
| 84 | 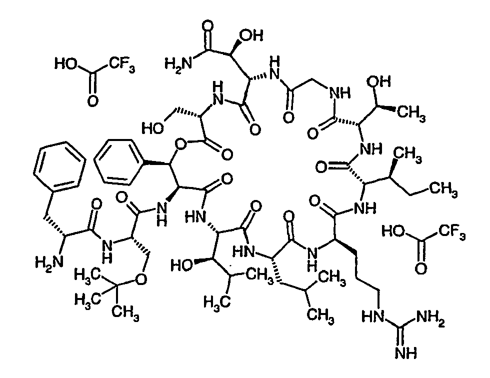 | HR-TOF-MS (Methode 21): C₆₂H₉₈N₁₅O₁₈ ber. 1340.7214, gef. 1340.7245 [M + H]⁺. |
| | D-Phenylalanyl-*O*³-*tert*-butyl-L-seryl-des(1-D-leucyl-2-L-leucyl)lysobactin-bistrifluoracetat | Allg. Arbeitsvorschrift 5 aus Beispiel 167A (0.015 mmol). |
| | | Ausbeute: 94% d. Th. |
| 85 | 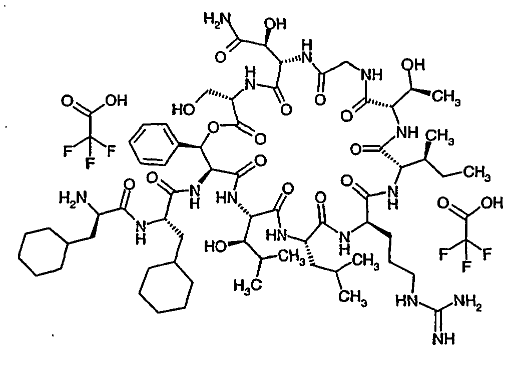 | HPLC/UV-Vis (Methode 13): Rₜ = 6.57 min. |
| | | LC-MS/UV-Vis (Methode 29): Rₜ = 4.7 min; |
| | | MS (ESIneg.): *m*/*z* (%) = 1355 (100), [M - H]⁻. |
| | | HR-TOF-MS (Methode 21): C₆₄H₁₀₆N₁₅O₁₇ ber. 1356.7891, gef. 1356.7921 [M+H]⁺. |
| | 3-Cyclohexyl-D-alanyl-3-cyclohexyl-L-alanyl-des(1-D-leucyl-2-L-leucyl)lysobactin-bistrifluoracetat | Allg. Arbeitsvorschrift 5 aus Beispiel 168A (0.003 mmol). |
| | | Ausbeute: 94% d. Th. |
| 86 | 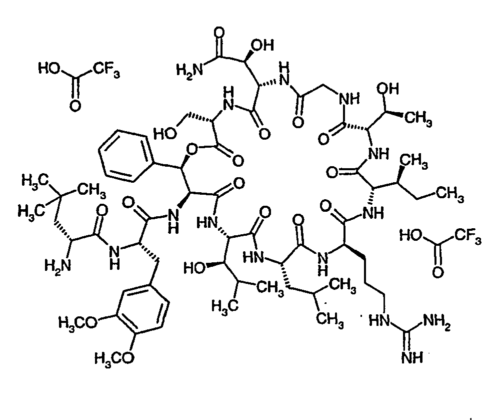 | HPLC/UV-Vis (Methode 13): Rₜ = 5.86 min. |
| | | LC-MS (Methode 29): Rₜ = 4.3 min; |
| | | MS (ESIpos.): *m*/*z* (%) = 693 (100) [M+2H]²⁺, 1384 (30) [M + H]⁺. |
| | | HR-TOF-MS (Methode 21): C₆₄H₁₀₂N₁₅O₁₉ ber. 1384.7476, gef. 1384.7466 [M + H]⁺. |
| | 3-*tert*-Butyl-D-alanyl-3-(3,4-dimethoxyphenyl)-L-alanyl-des(1-D-leucyl-2-L-leucyl)lysobactin-bistri-fluoracetat | Allg. Arbeitsvorschrift 5 aus Beispiel 171A (0.006 mmol). Reinigung nach Methode 8 bzw. Methode 25. |
| | | Ausbeute: 69% d. Th. |
| 87 | 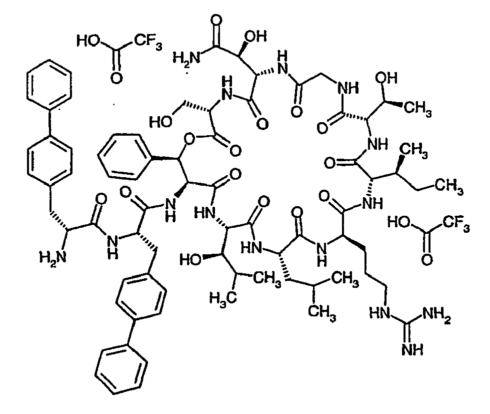 | HPLC/UV-Vis (Methode 13): Rₜ = 6.69 min. |
| | | LC-MS (Methode 29): Rₜ = 4.9 min; |
| | | MS (ESIpos.): *m*/*z* (%) = 749 (100) [M + 2H]²⁺, 1497 (10) [M + H]⁺. |
| | | HR-TOF-MS (Methode 21): C₇₆H₁₀₂N₁₅O₁₇ [M + H]⁺ ber. 1496.7578, gef. 1496.7604. |
| | 4-Phenyl-D-phenylalanyl-4-phenyl-L-phenylalanyl-des(1-D-leucyl-2-L-leucyl)lysobactin-bistrifluoracetat | Allg. Arbeitsvorschrift 5 aus Beispiel 172A (0.011 mmol). |
| | | Ausbeute: quant. |
| 88 | 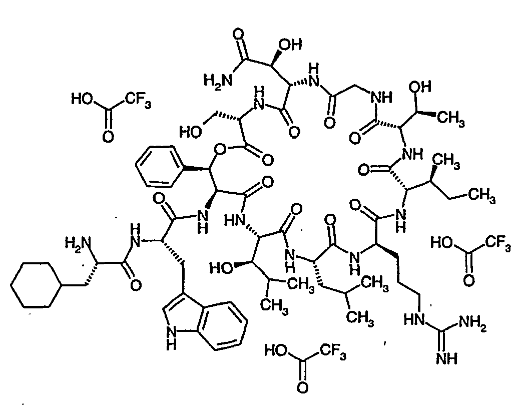 | HPLC/UV-Vis (Methode 13): Rₜ = 6.8 min. |
| | | LC-MS (Methode 29): Rₜ = 4.6 min; |
| | | MS (ESIneg.): *m*/*z* (%) = 1388 (100) [M - H]⁺. |
| | | HR-TOF-MS (Methode 21): C₆₆H₁₀₁N₁₆O₁₇ ber. 1389.7531, gef. 1389.7559 [M+H]⁺. |
| | 3-Cyclohexyl-L-alanyl-L-tryptophyl-des(1-D-leucyl-2-L-leucyl)lysobactin-tristrifluoracetat | Allg. Arbeitsvorschrift 5 aus Beispiel 173A (0.004 mmol). Reinigung nach Methode 8 bzw. Methode 25. |
| | | Ausbeute: 72% d. Th. |
| 89 | 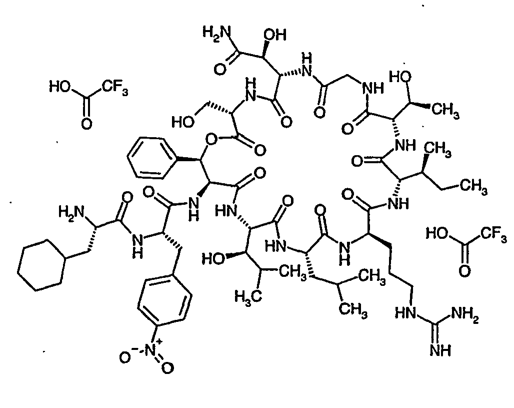 | HPLC/UV-Vis (Methode 13): Rₜ = 6.06 min. |
| | | LC-MS (Methode 29): Rₜ = 4.5 min; |
| | | MS (ESIpos.): *m*/*z* (%) = 699 (100) [M+2H]²⁺, 1395 (20) [M+H]⁺. |
| | | HR-TOF-MS (Methode 21): C₆₄H₉₉N₁₆O₁₉ [M + H]⁺ ber. 1395.7272, gef. 1395.7247. |
| | 3-Cyclohexyl-L-alanyl-4-nitro-L-phenylalanyl-des(1-D-leucyl-2-L-leucyl)lysobactin-bistrifluoracetat | Allg. Arbeitsvorschrift 5 aus Beispiel 174A (0.004 mmol). Reinigung nach Methode 8 bzw. Methode 25. |
| | | Ausbeute: 87% d. Th. |
| 90 | 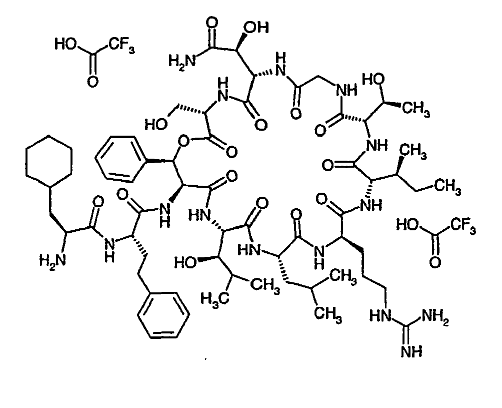 | LC-MS (Methode 29): Rₜ = 4.7 min; |
| | | MS (ESIpos.): *m*/*z* (%) = 683 (100) [M+2H]²⁺, 1364 (20) [M+H]⁺. |
| | | HR-TOF-MS (Methode 21): C₆₅H₁₀₂N₁₅O₁₇ ber. 1364.7578, gef. 1364.7626 [M + H]⁺. |
| | 3-Cyclohexyl-L-alanyl-3-benzyl-L-alanyl-des(1-D-leucyl-2-L-leucyl)lysobactin-bistrifluoracetat | Allg. Arbeitsvorschrift 5 aus Beispiel 175A (0.012 mmol). |
| | | Ausbeute: quant. |
| 91 | 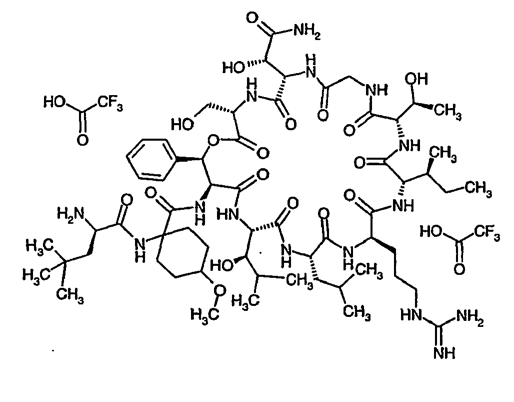 | HPLC/UV-Vis (Methode 13): Rₜ = 5.88 min. |
| | | LC-MS (Methode 26): Rₜ = 1.52 min; |
| | | MS (ESIpos.): *m*/*z* (%) = 667 (100) [M+2H]²⁺, 1051(10), 1333 (10) [M+H]⁺. |
| | | HR-TOF-MS (Methode 21): C₆₁H₁₀₂N₁₅O₁₈ [M + H]⁺ ber. 1332.7527, gef. 1332.7561. |
| | 3-*tert*-Butyl-D-alanyl-(1-amino-4-methoxy-cyclohexylcarbonyl)-des(1-D-leucyl-2-L-leucyl)lysobactin-bistrifluoracetat | Allg. Arbeitsvorschrift 5 aus Beispiel 176A (0.019 mmol). Reinigung nach Methode 8 bzw. Methode 25. |
| | | Ausbeute: 91 % d. Th. |
| 92 | 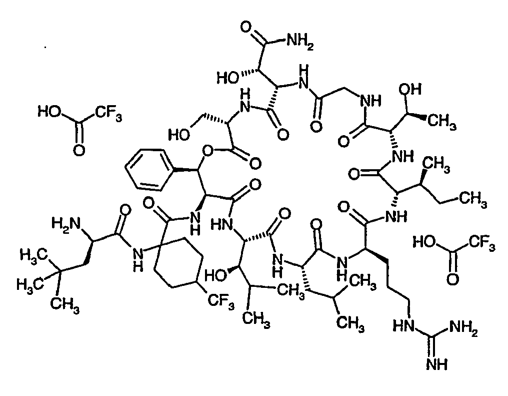 | HPLC/UV-Vis (Methode 13): Rₜ = 6.68 min, |
| | | λₘₐₓ (qualitativ) = 220 nm (s), 255-270 (m). |
| | | HR-TOF-MS (Methode 21): C₆₁H₉₉N₁₅O₁₇F₃ [M+H]⁺ ber. 1370.7295, gef. 1370.7263. |
| | 3-tert-Butyl-D-alanyl-(1-amino-4-(trifluormethyl)cyclo-hexylcarbonyl)-des(1-D-leucyl-2-L-leucyl)lysobactin-bistrifluoracetat | Allg. Arbeitsvorschrift 5 aus Beispiel 177A (0.02 mmol). Reinigung nach Methode 8 bzw. Methode 25. |
| | | Ausbeute: 81% d. Th. |
| 93 | 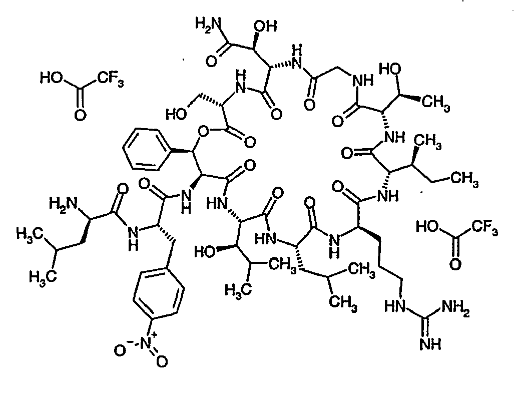 | HPLC/UV-Vis (Methode 13): Rₜ = 5.96 min, |
| | | λₘₐₓ (qualitativ) = 220 nm (s), 255-270 (m). |
| | | LC-MS (Methode 26): Rₜ = 1.60 min; |
| | | MS (ESIpos.): *m*/*z* (%) = 679 (100) [M + 2H]²⁺, 1356 (10) [M+H]⁺. |
| | | HR-TOF-MS (Methode 21): C₆₁H₉₅N₁₆O₁₉ [M+H]⁺ ber. 1355.6959, gef. 1355.6943. |
| | D-Leucyl-4-nitro-L-phenylalanyl-des(1-D-leucyl-2-L-leucyl)lysobactin-bistrifluoracetat | Allg. Arbeitsvorschrift 5 aus Beispiel 178A (0.02 mmol). Reinigung nach Methode 8 bzw. Methode 25. |
| | | Ausbeute: 40% d. Th. |
| 94 | 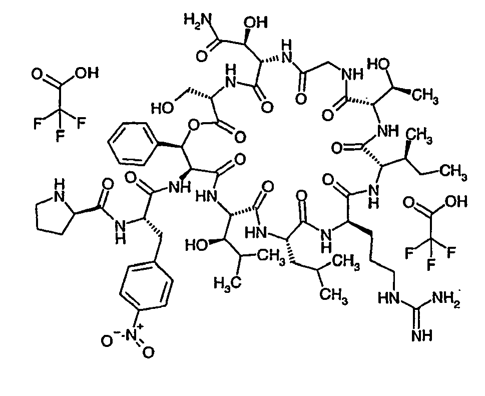 | HR-TOF-MS (Methode 21): C₆₀H₉₁N₁₆O₁₉ ber. 1339.6646, gef. 1339.6602 [M+H]⁺. |
| | D-Prolyl-4-nitro-L-phenylalanyl-des(1-D-leucyl-2-L-leucyl)lysobactin-bistrifluoracetat | Allg. Arbeitsvorschrift 5 aus Beispiel 179A (0.003 mmol). Reinigung nach Methode 8 bzw. Methode 25. |
| | | Ausbeute: quant. |
| 95 | 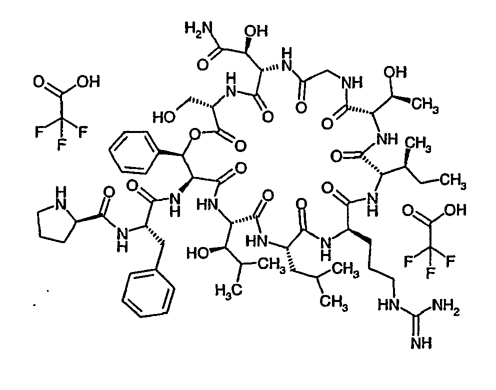 | HPLC/UV-Vis (Methode 13); Rₜ = 5.40 min. |
| | | LC-MS (Methode 29): Rₜ = 4.2 min; |
| | | MS (ESIpos.): *m*/*z* (%) = 648 (100) [M + 2H]²⁺, 1295 (50) [M+H]⁺. |
| | D-Prolyl-L-phenylalanyl-des(1-D-leucyl-2-L-leucyl)lysobactin-bistrifluoracetat | Allg. Arbeitsvorschrift 5 aus Beispiel 180A (0.01 mmol). Reinigung nach Methode 8 bzw. Methode 25. |
| | | Ausbeute: 90% d. Th. |
| 96 | 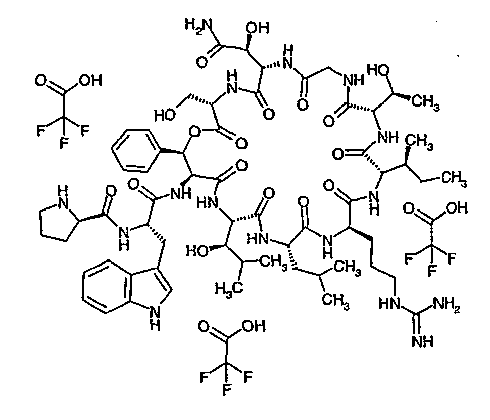 | HPLC/UV-Vis (Methode 13): Rₜ = 5.40 min. |
| | | LC-MS (Methode 29): Rₜ=4.1 min; |
| | | MS (ESIpos.): *m*/*z* (%) = 668 (100) [M+2H]²⁺; |
| | | ms (ESIneg.): *m*/*z* (%) = 1332 (100) [M-H]⁺. |
| | | HR-TOF-MS (Methode 21): C₆₂H₉₃N₁₆O₁₇[M+H]⁺ ber. 1333.6905, gef. 1333.6885. |
| | D-Prolyl-L-tryptophyl-des(1-D-leucyl-2-L-leucyl)lysobactin-tristrifluoracetat | Allg. Arbeitsvorschrift 5 aus Beispiel 181A (0.003 mmol). Reinigung nach Methode 8 bzw. Methode 25. |
| | | Ausbeute: 55% d. Th. |
| 97 | 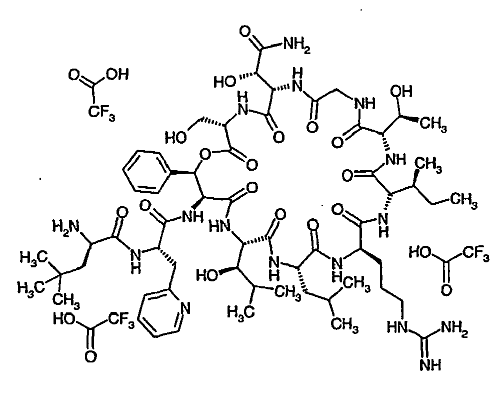 | HPLC/UV-Vis (Methode 36): Rₜ = 3.53 min. |
| | | LC-MS (Methode 26): Rₜ = 1.58 min; |
| | | MS (ESIpos.): *m*/*z* (%) = 663 (100) [M+2H]²⁺, 1325(10) [M+H]⁺; |
| | | MS (ESIneg.): *m*/*z* (%) = 661 (100) [M-2H]²-, 1323 (20) [M-H]⁻. |
| | | HR-MS (Methode 21): C₆₁H₉₇N₁₆O₁₇ [M+H]⁺ ber. 1325.7218, gef. 1325.7178. |
| | 3-*tert*-Butyl-D-alanyl-3-(2-pyridyl)-L-alanyl-des(1-D-leucyl-2-L-leucyl)lysobactin-tristrifluoracetat | Allg. Arbeitsvorschrift 5 aus Beispiel 182A (0.03 mmol). |
| | | Ausbeute: 38% d. Th |
| 98 | 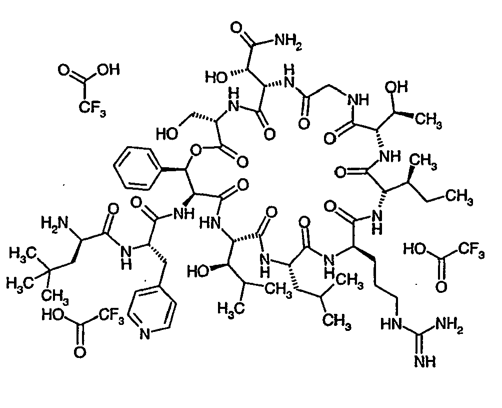 | HPLC/UV-Vis (Methode 36): Rₜ = 3.24 min. |
| | | LC-MS (Methode 26): Rₜ = 1.31 min; |
| | | MS (ESIpos.): *m*/*z* (%) = 663 (100) [M + 2H]²⁺, 1325 (5) [M+H]⁺; |
| | | MS (ESIneg.): *m*/*z* (%) = 661 (100) [M-2H]²⁻, 1323 (20) [M-H]⁻. |
| | | HR-MS (Methode 21): C₆₁H₉₇N₁₆O₁₇ [M + H]⁺ ber. 1325.7218, gef. 1325.7186. |
| | 3-*tert*-Butyl-D-alanyl-3-(4-pyridyl)-L-alanyl-des(1-D-leucyl-2-L-leucyl)lysobactin-tristrifluoracetat | Allg. Arbeitsvorschrift 5 aus Beispiel 98A (0.05 mmol). |
| | | Ausbeute: 8% d. Th |
| 99 | 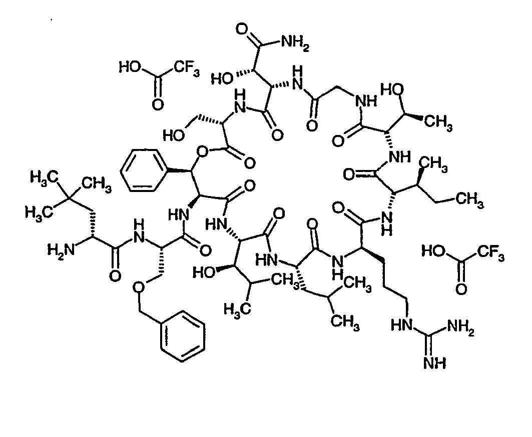 | HPLC/UV-Vis (Methode 36): Rₜ = 3.69 min. |
| | | LC-MS (Methode 26): Rₜ = 1.60 min, |
| | | MS (ESIpos.): *m*/*z* (%) = 678.3 (100) [M + 2H]²⁺. |
| | | HR-TOF-MS (Methode 21): ber. 1354.7371 gef.1354.7323 C₆₃H₉₉N₁₅O₁₈ [M+H]⁺. |
| | (3-*tert*-Butyl-D-alanyl-O-benzyl-L-seryl)-des(1-D-leucyl-2-L-leucyl)lysobactin-bistrifluoracetat | Allgemeine Arbeitsvorschrift 21 aus Beispiel 184A (68 µmol). |
| | | Ausbeute: 47% d. Th. |
| 100 | 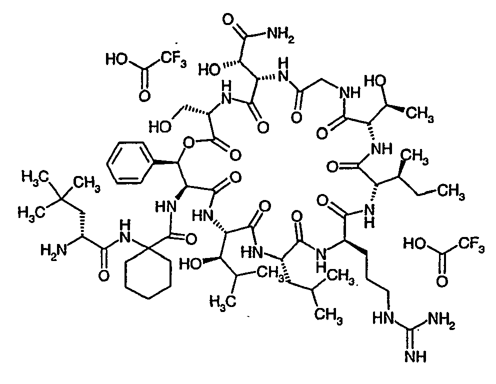 | HPLC/UV-Vis (Methode 36): Rₜ = 3.64 min. |
| | | LC-MS (Methode 26): Rₜ= 1.58 min, |
| | | MS (ESIpos.): *m*/*z* (%) = 652.24 (100) [M + 2H]²⁺. |
| | | HR-TOF-MS (Methode 21): ber. 1302.7422 gef: 1302.7471 [M + H]⁺. |
| | *N*-({1-[(3-*tert*-Butyl-D-alanyl)amino]cyclohexyl}carbonyl) -des(1-D-leucyl-2-L-leucyl)lysobactin-bistrifluoracetat | Allgemeine Arbeitsvorschrift 21 aus Beispiel 185A (36 µmol). |
| | | Ausbeute: 48% d. Th. |
| 101 | 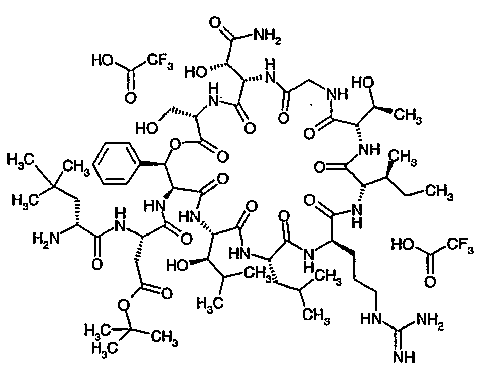 | HPLC/UV-Vis (Methode 36): Rₜ = 3.67 min. |
| | | LC-MS (Methode 26): Rₜ = 1.69 min, |
| | | MS (ESIpos.): *m*/*z* (%) = 675.3 (100) [M + 2H]²⁺. |
| | | HR-TOF-MS (Methode 21): ber. 1348.7476 gef. 1348.7478 [M + H]⁺. |
| | (2*S*)-2-[(3-*tert*-Butyl-D-alanyl)amino]-4-*tert*-butoxy-4-oxobutanoyl-des(1-D-leucyl-2-L-leucyl)lysobactin-bis-trifluoracetat | Allgemeine Arbeitsvorschrift 22 aus Beispiel 186A (85 µmol). |
| | | Ausbeute: 33% d. Th. |
| 102 | 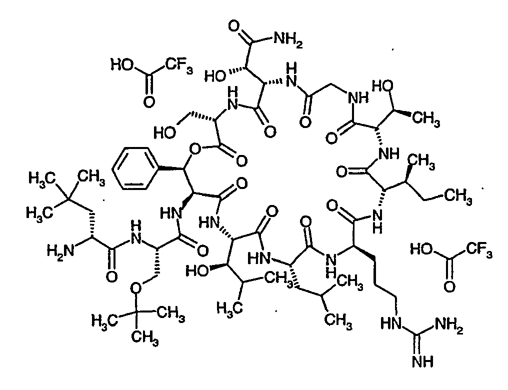 | HPLC/UV-Vis (Methode 36): Rₜ = 3.69 min. |
| | | LC-MS (Methode 26): Rₜ = 1.75 min, |
| | | MS (ESIpos.): *m*/*z* (%) = 661.3 (100) [M + 2H]²⁺. |
| | | HR-TOF-MS (Methode 21): ber. 1320.7572 gef. 1320.7477 [M + H]⁺. |
| | [3-*tert*-Butyl-D-alanyl -*O*-(*tert*-butyl)-L-seryl]-des(1-D-leucyl-2-L-leucyl)lysobactin-bistrifluoracetat | Allgemeine Arbeitsvorschrift 22 aus Beispiel 187A (77 µmol). |
| | | Ausbeute: 49% d. Th. |
| 103 | 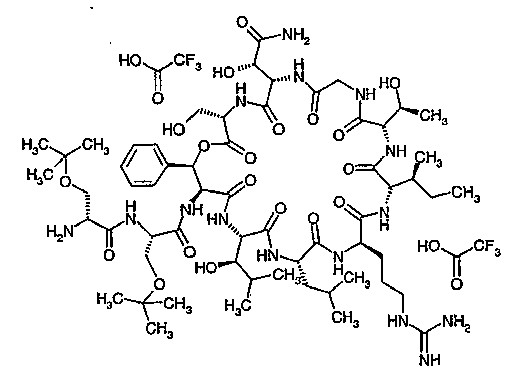 | HPLC/UV-Vis (Methode 36): Rₜ = 3.61 min. |
| | | LC-MS (Methode 26): Rₜ = 1.64 min, |
| | | MS (ESIpos.): *m*/*z* (%) = 669.2 (100) [M + 2H]²⁺. |
| | | HR-TOF-MS (Methode 21): ber. 1336.7476 gef. 1336.7483 [M+H]⁺. |
| | [*O*-(*tert*-Butyl)-D-seryl-*O*-(tert-butyl)-L-seryl] -des(1-D-leucyl-2-L-leucyl)lysobactin-bistrifluoracetat | Allgemeine Arbeitsvorschrift 22 aus Beispiel 188A (84 µmol). |
| | | Ausbeute: 16% d. Th. |
| 104 | 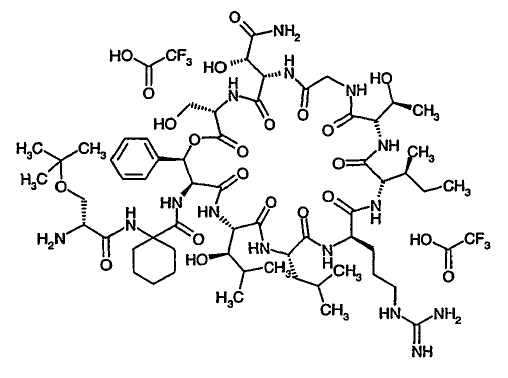 | HPLC/UV-Vis (Methode 36): Rₜ = 3.61 min. |
| | | LC-MS (Methode 26): Rₜ = 1.62 min, |
| | | MS (ESIpos.): *m*/*z* (%) = 1318.7 (5) [M + H]⁺. |
| | | HR-TOF-MS (Methode 21): ber. 1318.7371 gef. 1318.7351 [M+H]⁺. |
| | *N*-[(1-([*O*-(*tert*-Butyl)-D-seryl]amino)-cyclohexyl)carbonyl]-des(1-D-leucyl-2-L-leucyl)-lysobactin-bistrifluoracetat | Allgemeine Arbeitsvorschrift 22 aus Beispiel 189A (24 µmol). |
| | | Ausbeute: 75% d. Th. |
| 105 | 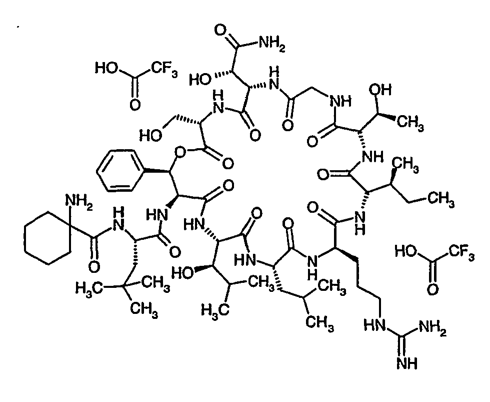 | HPLC/UV-Vis (Methode 36): Rₜ = 3.54 min. |
| | | LC-MS (Methode 26): Rₜ = 1.61 min, |
| | | MS (ESIpos.): *m*/*z* (%) =1302.9 [M+H]⁺. |
| | | HR-TOF-MS (Methode 21): ber. 1302.7422 gef. 1302.7373 [M + H]⁺. |
| | *N*-[(1-Aminocyclohexyl)carbonyl]-(3-*tert*-butyl-D-alanyl)-des(1-D-leucyl-2-L-leucyl)lysobactin-bistri-fluoracetat | Allgemeine Arbeitsvorschrift 21 aus Beispiel 190A (35 µmol). |
| | | Ausbeute: 81 % d. Th. |
| 106 | 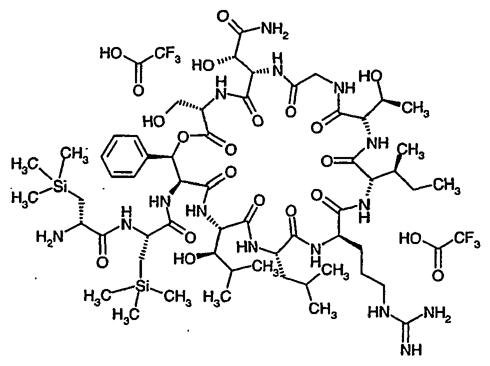 | HPLC/UV-Vis (Methode 36): Rₜ = 3.74 min. |
| | | LC-MS (Methode 26): Rₜ = 1.76 min, |
| | | MS (ESIpos.): *m*/*z* (%) = 1336.9 (3) [M + H]⁺. |
| | | HR-TOF-MS (Methode 21): ber. 1336.7117 gef. 1336.7100 [M+H]⁺. |
| | 3-(Trimethylsilyl)-D-alanyl-3-(trimethylsilyl)-L-alanyl -des(1-D-leucyl-2-L-leucyl)lysobactin-bistrifluoracetat | Allgemeine Arbeitsvorschrift 21 aus Beispiel 191A (84 µmol). |
| | | Ausbeute: 63% d. Th. |
| 107 | 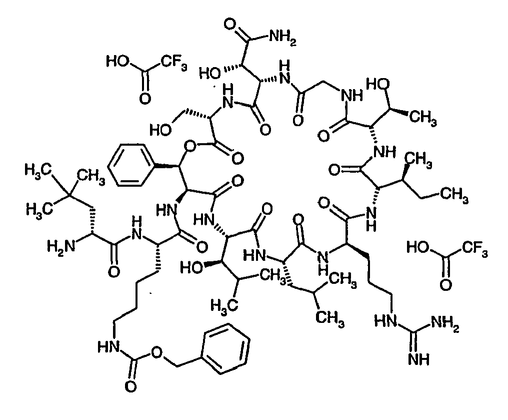 | HPLC/UV-Vis (Methode 36): Rₜ = 3.67 min. |
| | | LC-MS (Methode 26): Rₜ = 1.62 min, |
| | | MS (ESIpos.): *m*/*z* (%) = 720.7 (100) [M+ 2H]²⁺. |
| | | HR-TOF-MS (Methode 21): ber. 1439.7898, gef. 1439.7909 [M + H]⁺. |
| | {(3-*tert*-Butylalanyl)-[*N*⁶-(benzyloxycarbonyl)lysyl])-des(1-D-leucyl-2-L-leucyl)lysobactin-bistrifluoracetat | Allgemeine Arbeitsvorschrift 21 aus Beispiel 192A (41 µmol). |
| | | Ausbeute: 69% d. Th. |
| 108 | 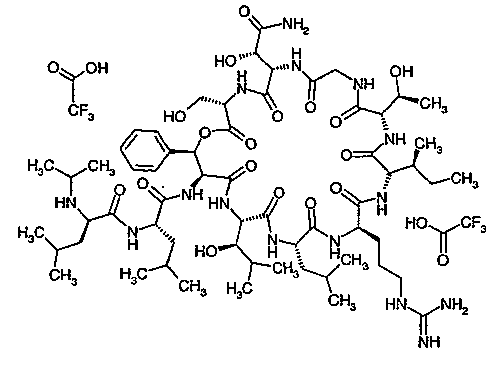 | LC-MS (Methode 19): Rₜ = 2.73 min; |
| | | MS (ESIpos.): *m*/*z* (%) = 660.4 (100) [M+2H]²⁺. |
| | *N*-(iso-Propyl)lysobactin-bistrifluoracetat | Allgemeine Arbeitsvorschrift 13 aus Beispiel 1A (0.010 mmol) und Aceton. |
| | | Ausbeute: 21% d. Th. |
| 109 | 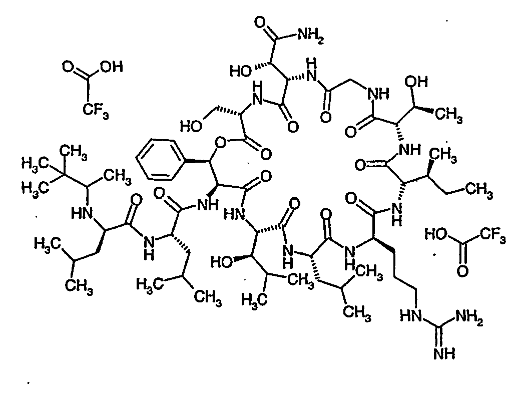 | LC-MS (Methode 19): Rₜ = 2.88 min; |
| | | MS (ESIpos.): *m*/*z* (%) = 681.4 (100) [M+2H]²⁺. |
| | *N*-(2,3,3- Trimethylpropyl)lysobactin-bistrifluoracetat | Allgemeine Arbeitsvorschrift 13 aus Beispiel 1A (0.010 mmol) und Methyl-*tert*-butylketon. |
| | | Ausbeute: 27% d. Th. |

### B. Bewertung der physiologischen Wirksamkeit

Die *in vitro-*Wirkung der erfmdungsgemäßen Verbindungen kann in folgenden Assays gezeigt werden:

### Bestimmung der minimalen Hemmkonzentration (MHK):

Die MHK wird im Flüssigdilutionstest gemäß der NCCLS-Richtlinien bestimmt. Übernachtkulturen von *Staplzylococcus aureus* 133, *Entercococcus faecalis* 27159, *E. faecium* 4147 und *Streptococcus pneumoniae* G9a werden mit den beschriebenen Testsubstanzen in einer 1:2 Verdünnungsreihe inkubiert. Die MHK-Besrimmung wird mit einer Zellzahl von 10⁵ Keimen pro ml in Isosensitest-Medium (Fa. Difco, Irvine/USA) durchgeführt, mit Ausnahme von S. *pneumoniae,* der in BHI-Bouillon (Fa. Difco, Irvine/USA) mit 10% Rinderserum bei einer Zellzahl von 10⁶ Keimen pro ml getestet wird. Die Kulturen werden bei 37°C für 18-24 Stunden inkubiert, *S. pneumoniae* in Gegenwart von 10% CO₂.

Die jeweils niedrigste Substanzkonzentration, bei der kein sichtbares Bakterienwachstum mehr auftritt, wird als MIK definiert. Die MHK-Werte werden in µg/ml angegeben.

Repräsentative in-vitro-Wirkdaten für die erfmdungsgemäßen Verbindungen sind in Tabelle A wiedergegeben:

**Tabelle A**

| **Beispiel-Nr.** | **MHK** ***S. aureus* 133** | **MHK** ***S. pneumoniae*** | **MHK** ***E.faecium* L4001** | **MHK** ***E. faecalis* ICB 27159** |
|---|---|---|---|---|
| **1** | 0.125 | 0.125 | 0.5 | 0.5 |
| **3** | 0.125 | 0.031 | 0.25 | 0.25 |
| **18** | 1 | 2 | 1 | 1 |
| **23** | 0.125 | 0.125 | 0.5 | 1 |
| **24** | 0.25 | 0.5 | 1 | 2 |
| **30** | 0.25 | 0.25 | 0.5 | 1 |
| **38** | 0.125 | 0.063 | 0.5 | 1 |
| **47** | 0.25 | 0.25 | 1 | 2 |
| **48** | 0.125 | 0.125 | 0.25 | 0.5 |
| **57** | 0.5 | 0.25 | 1 | 1 |
| **59** | 0.125 | <0.063 | 0.25 | 0.25 |
| **62** | 0.25 | 0.5 | 0.5 | 1 |
| **68** | 0.125 | 0.031 | 0.5 | 0.5 |
| **69** | 0.5 | 0.5 | 1 | 1 |
| **74** | 0.125 | 0.063 | 0.5 | 1 |
| **81** | 0.5 | 0.25 | 0.25 | 0.25 |
| **82** | 0.125 | 0.125 | 0.5 | 1 |
| **86** | 0.25 | 0.125 | 1 | 0.5 |
| **97** | 1 | 0.125 | 1 | 0.5 |
| **99** | 0.25 | ≤0.063 | 0.063 | 0.063 |
| **101** | 1 | 0.5 | 1 | 1 |
| **105** | 0.5 | 0.063 | 0.5 | 1 |
| **107** | 0.25 | 0.25 | 0.5 | 1 |
| **1A** | 0.25 | 0.063 | 0.5 | 1 |

Die Eignung der erfmdungsgemäßen Verbindungen zur Behandlung von bakteriellen Infektionen kann im folgenden Tiermodell gezeigt werden:

### Systemische Infektion mit Staphylococcus aureus 133:

Zellen von *S. aureus* 133 werden über Nacht in BHI-Bouillon (Fa. Oxoid, New York/ USA) angezüchtet. Die Übernachtkultur wird 1:100 in frische BHI-Bouillon verdünnt und für 3 Stunden inkubiert. Die dann in der logarithmischen Wachstumsphase befindlichen Zellen werden abzentrifugiert und zweimal mit gepufferter, physiologischer Kochsalzlösung gewaschen. Danach wird photometrisch eine Zellsuspension in Kochsalzlösung mit einer Extinktion von 50 Einheiten eingestellt. Nach einem Verdünnungsschritt (1:15) wird diese Suspension 1:1 mit einer 10%igen Mucinlösung gemischt. Von dieser Infektionslösung werden 0.25 ml/20 g Maus intraperitoneal (entsprechend 1x10⁶ Keimen/Maus) appliziert. Die Therapie erfolgt intraperitoneal oder intravenös 30 Minuten nach der Infektion. Für den Infektionsversuch werden weibliche CFWI-Mäuse verwendet. Das Überleben der Tiere wird über 6 Tage protokolliert.

Die Eigenschaften der erfindungsgemäßen Verbindungen im Hinblick auf die Nierenverträglichkeit können im folgenden Tiermodell gezeigt werden:

### Maus-Modell zur Bestimmung nephrotoxischer Effekte:

Nephrotoxische Nebenwirkungen der Nonadepsipeptide werden durch histopathologische Untersuchungen der Nieren in Mäusen nach mehrfacher Gabe einer bestimmten Dosierung analysiert. Dafür werden 5 - 6 Tiere täglich entweder intravenös (i.v.) oder intraperitoneal (i.p.) mit Substanzen behandelt, die in wässriger Lösung oder unter Zusatz von Solutol gelöst werden. Nierentoxische Effekte werden durch lichtmikroskopische Auswertung Hämatoxilin und Eosin (H&E) gefärbter Paraffinschnitte der Nieren bestimmt. Eine 'Periodic-Acid Schiff (PAS)-Reaktion wird wahlweise zur besseren Darstellung von Glykoproteinen durchgeführt. Nephrotoxische Effekte werden semiquantitativ für jedes Tier als Schweregrade der auftretenden tubulären Basophilie und Degeneration/Regeneration festgelegt (Schweregrade: 0 = kein Effekt; 1 = minimaler Effekt; 2 = leichter Effekt; 3 = moderater Effekt; 4 = schwere Läsionen). Der durchschnittliche Schweregrad der tubulären Degeneration/Regeneration sowie die Inzidenz (Anzahl der betroffenen Tiere) wird pro Tiergruppe oder Derivat berechnet. Darüber hinausgehende Nierenveränderungen wie tubuläre Dilatation sowie Nekrosen und Ansammlung nekrotischen Materials werden ebenfalls aufgeführt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus Wirkstoff, Lactose und Stärke wird mit einer 5%-igen Lösung (m/m) des PVPs in Wasser granuliert. Das Granulat wird nach dem Trocknen mit dem Magnesiumstearat für 5 min gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der Verbindung von Beispiel 1, 1000 mg Ethanol (96%), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, der Wirkstoff wird der Suspension zugefügt. Unter Rühren erfolgt die Zugabe des Wassers. Bis zum Abschluss der Quellung des Rhodigels wird ca. 6h gerührt.

### Intravenös applizierbare Lösung:

### Zusammensetzung:

100-200 mg der Verbindung von Beispiel 1, 15 g Polyethylenglykol 400 und 250 g Wasser für Injektionszwecke.

### Herstellung:

Die Verbindung von Beispiel 1 wird zusammen mit Polyethylenglykol 400 in dem Wasser unter Rühren gelöst. Die Lösung wird sterilfiltriert (Porendurchmesser 0,22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 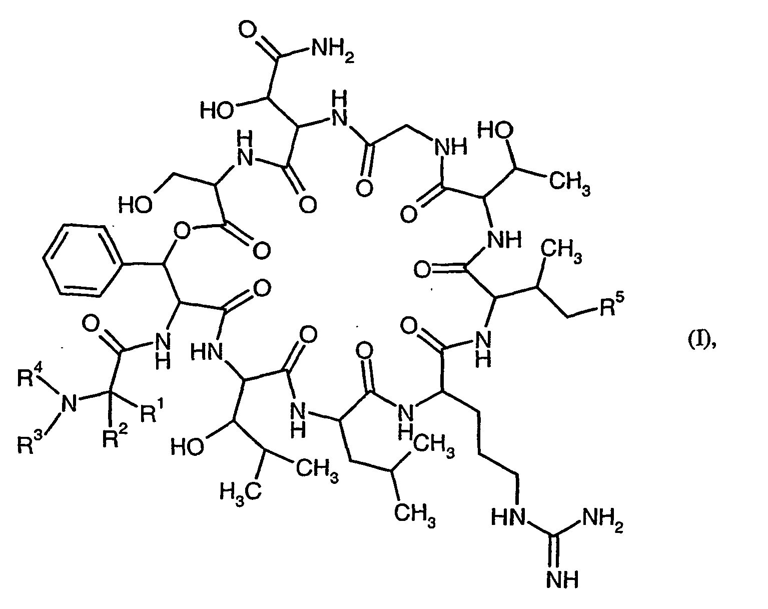 in welcher
R¹ Wasserstoff, C₃-C₆-Cycloalkyl, C₅-C₆-Cycloalkenyl, C₃-C₆-Cycloalkylmethyl, 5-bis 7-gliedriges Heterocyclylmethyl, Methyl, Ethyl, n-Propyl, iso-Propyl, 1-Methylprop-1-yl, 2-Methylprop-1-yl, 2,2-Dimethylprop-1-yl, 1,1-Dimethylprop-1-yl, 1-Ethyl-prop-1-yl, 1-Ethyl-l-methylprop-1-yl, n-Butyl, 2-Methylbut-1-yl, 3-Methylbut-1-yl, 1-Ethylbut-1-yl, *tert*-Butyl, 4-Methylpent-1-yl, n-Hexyl, Alkenyl oder Aryl bedeutet,
wobei R¹ substituiert sein kann mit 0, 1, 2 oder 3 Substituenten unabhängig voneinander ausgewählt aus der Gruppe bestehend aus Halogen, Hydroxy, Amino, Cyano, Trimethylsilyl, Alkyl, Alkoxy, Benzyloxy, C₃-C₆-Cycloalkyl, Aryl, 5- bis 10-gliedriges Heteroaryl, Alkylamino, Arylamino, Alkylcarbonylamino, Arylcarbonylamino, Alkylcarbonyl, Alkoxycarbonyl, Arylcarbonyl und Benzyloxycarbonylamino,
worin Aryl und Heteroaryl ihrerseits substituiert sein können mit 0, 1, 2 oder 3 Substituenten unabhängig voneinander ausgewählt aus der Gruppe bestehend aus Halogen, Hydroxy, Amino, Cyano, Nitro, Alkyl, Alkoxy und Phenyl,
R² Wasserstoff oder C₁-C₄-Alkyl bedeutet,
oder
R¹ und R² zusammen mit dem Kohlenstoffatom, an das sie gebunden sind, einen C₃-C₆-Cycloalkyl-Ring oder einen 5- bis 7-gliedrigen Heterocyclyl-Ring bilden, wobei der Cycloalkyl-Ring und der Heterocyclyl-Ring substituiert sein können mit 0, 1, 2 oder 3 Substituenten unabhängig voneinander ausgewählt aus der Gruppe bestehend aus Trifluormethyl, Alkyl, Alkoxy und Alkylcarbonyl,
R³ Alkyl, C₃-C₆-Cycloalkyl, 5- bis 7-gliedriges Heterocyclyl, Aryl, 5- oder 6-gliedriges Heteroaryl, Alkylcarbonyl, Alkoxycarbonyl, C₃-C₆-Cycloalkylcarbonyl, 5- bis 7-gliedriges Heterocyclylcarbonyl, Arylcarbonyl, 5- oder 6-gliedriges Heteroarylcarbonyl oder Alkylaminocarbonyl bedeutet,
wobei Alkyl, Cycloalkyl, Heterocyclyl, Aryl, Heteroaryl, Alkoxycarbonyl, Cyclo-alkylcarbonyl, Heterocyclylcarbonyl, Arylcarbonyl, Heteroarylcarbonyl und Alkylaminocarbonyl substituiert sein können mit 0, 1, 2 oder 3 Substituenten unabhängig voneinander ausgewählt aus der Gruppe bestehend aus Halogen, Hydroxy, Amino, Alkylamino und Phenyl,
und
wobei Alkylcarbonyl substituiert ist mit einem Substituenten Amino oder Alkyl-amino,
und
wobei Alkylcarbonyl substituiert sein kann mit weiteren 0, 1 oder 2 Substituenten unabhängig voneinander ausgewählt aus der Gruppe bestehend aus Halogen, Hydroxy, Trimethylsilyl, Alkoxy, Alkylthio, Benzyloxy, C₃-C₆-Cycloalkyl, Phenyl, Naphthyl, 5- bis 10-gliedriges Heteroaryl, Alkylcarbonylamino, Alkoxycarbonylamino, Arylcarbonylamino, Arylcarbonyloxy, Benzyloxycarbonyl und Benzyloxycarbonylamino,
worin Phenyl und Heteroaryl ihrerseits substituiert sein können mit 0, 1, 2 oder 3 Substituenten unabhängig voneinander ausgewählt aus der Gruppe bestehend aus Halogen, Hydroxy, Nitro, Alkyl, Alkoxy und Phenyl, oder zwei Substituenten an demselben Kohlenstoffatom im Alkylcarbonyl zusammen mit dem Kohlenstoffatom, an das sie gebunden sind, einen C₃-C₆-Cycloalkyl-Ring oder einen 5- bis 7-gliedrigen Heterocyclyl-Ring bilden,
wobei der Cycloalkyl-Ring und der Heterocyclyl-Ring substituiert sein können mit 0, 1, 2 oder 3 Substituenten unabhängig voneinander ausgewählt aus der Gruppe bestehend aus Trifluormethyl, Alkyl und Alkoxy,
oder
wobei der Cycloalkyl-Ring Benzo-anneliert sein kann,
R⁴ Wasserstoff, C₁-C₄-Alkyl, Cyclopropyl oder Cyclopropylmethyl bedeutet,
oder
R³ und R⁴ zusammen mit dem Stickstoffatom, an das sie gebunden sind, einen 5- bis 7-gliedrigen Heterocyclyl-Ring bilden, wobei der Heterocyclyl-Ring substituiert sein kann mit 0, 1, 2 oder 3 Substituenten unabhängig voneinander ausgewählt aus der Gruppe bestehend aus Halogen, Hydroxy, Amino, Cyano, Alkyl, Alkoxy und Alkylamino,
und
R⁵ Wasserstoff oder Methyl bedeutet,
oder eines ihrer Salze, ihrer Solvate und der Solvate ihrer Salze,
mit der Maßgabe, dass für den Fall, dass R¹ Wasserstoff, R² 2-Methylprop-1-yl, R⁴ Wasserstoff und R⁵ Methyl bedeutet und das Kohlenstoffatom, an das R¹ und R² gebunden sind, (S)-Konfiguration hat oder dass R¹ 2-Methylprop-1-yl, R² Wasserstoff, R⁴ Wasserstoff und R⁵ Methyl bedeutet und das Kohlenstoffatom, an das R¹ und R² gebunden sind, (S)-Konfiguration hat, R³ nicht Glycyl, D-Alanyl, L-Alanyl oder D-Leucyl bedeutet, und
mit der Maßgabe, dass für den Fall, dass R¹ Wasserstoff, R² 2-Methylprop-1-yl, R⁴ Wasserstoff und R⁵ Wasserstoff bedeutet und das Kohlenstoffatom, an das R¹ und R² gebunden sind, (S)-Konfiguration hat oder dass R¹ 2-Methylprop-1-yl, R² Wasserstoff, R⁴, Wasserstoff und R⁵ Wasserstoff bedeutet und das Kohlenstoffatom, an das R¹ und R² gebunden sind, (*S*)-Konfiguration hat, R³ nicht D-Leucyl bedeutet.

2. Verbindung nach Anspruch 1, **dadurch gekennzeichnet, dass** sie der Formel 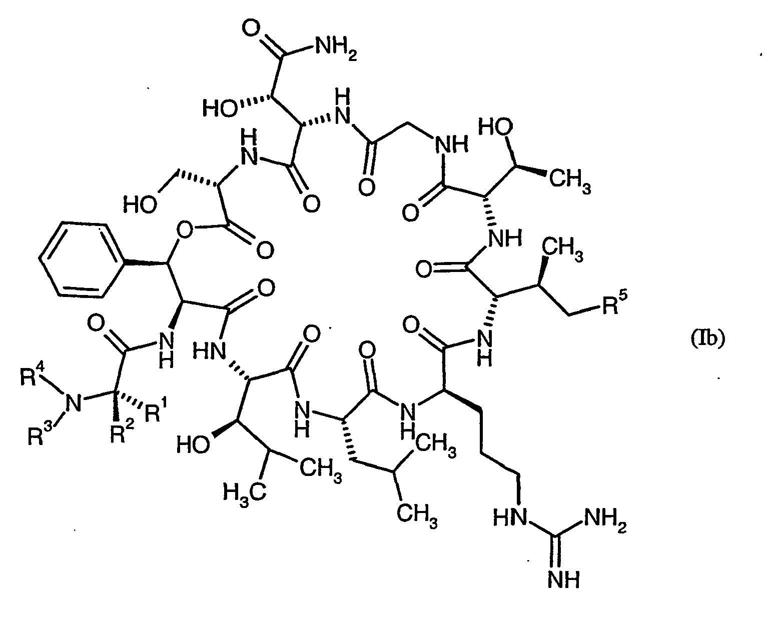 entspricht, in welcher
R¹, R², R³, R⁴ und R⁵ die in Anspruch 1 angegebene Bedeutung haben,
oder eines ihrer Salze, ihrer Solvate und der Solvate ihrer Salze.

3. Verbindung nach Anspruch 2, **dadurch gekennzeichnet, dass**
R¹ 2-Methylprop-1-yl bedeutet,
R² Wasserstoff oder C₁-C₄-Alkyl bedeutet,
R³ Alkyl, C₃-C₆-Cycloalkyl, 5- bis 7-gliedriges Heterocyclyl, Aryl, 5- oder 6-gliedriges Heteroaryl, Alkylcarbonyl, Alkoxycarbonyl, C₃-C₆-Cycloalkylcarbonyl, 5- bis 7-gliedriges Heterocyclylcarbonyl, Arylcarbonyl, 5- oder 6-gliedriges Heteroarylcarbonyl oder Alkylaminocarbonyl bedeutet,
wobei Alkyl, Cycloalkyl, Heterocyclyl, Aryl, Heteroaryl, Alkoxycarbonyl, Cyclo-alkylcarbonyl, Heterocyclylcarbonyl, Arylcarbonyl, Heteroarylcarbonyl und Alkylaminocarbonyl substituiert sein können mit 0, 1, 2 oder 3 Substituenten unabhängig voneinander ausgewählt aus der Gruppe bestehend aus Halogen, Hydroxy, Amino, Alkylamino und Phenyl,
und
wobei Alkylcarbonyl substituiert ist mit einem Substituenten Amino oder Alkyl-amino,
und
wobei Alkylcarbonyl substituiert sein kann mit weiteren 0, 1 oder 2 Substituenten unabhängig voneinander ausgewählt aus der Gruppe bestehend aus Halogen, Hydroxy, Trimethylsilyl, Alkoxy, Alkyithio, Benzyloxy, C₃-C₆-Cycloalkyl, Phenyl, Naphthyl, 5- bis 10-gliedriges Heteroaryl, Alkylcarbonylamino, Alkoxycarbonylamino, Arylcarbonylamino, Arylcarbonyloxy, Benzyloxycarbonyl und Benzyloxycarbonylamino,
worin Phenyl und Heteroaryl ihrerseits substituiert sein können mit 0, 1, 2 oder 3 Substituenten unabhängig voneinander ausgewählt aus der Gruppe bestehend aus Halogen, Hydroxy, Nitro, Alkyl, Alkoxy und Phenyl,
oder zwei Substituenten an demselben Kohlenstoffatom im Alkylcarbonyl zusammen mit dem Kohlenstoffatom, an das sie gebunden sind, einen C₃-C₆-Cycloalkyl-Ring oder einen 5- bis 7-gliedrigen Heterocyclyl-Ring bilden,
wobei der Cycloalkyl-Ring und der Heterocyclyl-Ring substituiert sein können mit 0, 1, 2 oder 3 Substituenten unabhängig voneinander ausgewählt aus der Gruppe bestehend aus Trifluormethyl, Alkyl und Alkoxy,
oder
wobei der Cycloalkyl-Ring Benzo-anneliert sein kann,
R⁴ Wasserstoff, C₁-C₄-Alkyl, Cyclopropyl oder Cyclopropylmethyl bedeutet,
oder
R³ und R⁴ zusammen mit dem Stickstoffatom, an das sie gebunden sind, einen 5- bis 7-gliedrigen Heterocyclyl-Ring bilden, wobei der Heterocyclyl-Ring substituiert sein kann mit 0, 1, 2 oder 3 Substituenten unabhängig voneinander ausgewählt aus der
Gruppe bestehend aus Halogen, Hydroxy, Amino, Cyano, Alkyl, Alkoxy und Alkylamino,
und
R⁵ Wasserstoff oder Methyl bedeutet.

4. Verbindung nach Anspruch 3, **dadurch gekennzeichnet, dass**
R¹ 2-Methylprop-1-yl bedeutet,
R² Wasserstoff bedeutet,
R³ C₁-C₆-Alkylcarbonyl bedeutet,
wobei Alkylcarbonyl substituiert ist mit einem Substituenten Amino,
und
wobei Alkylcarbonyl substituiert sein kann mit weiteren 0, 1 oder 2 Substituenten unabhängig voneinander ausgewählt aus der Gruppe bestehend aus Trimethylsilyl, C₁-C₄-Alkoxy, Methylhio, Benzyloxy, C₃-C₆-Cycloalkyl, Phenyl, Naphthyl, Thienyl, Pyridyl, Indolyl, C₁-C₄-Alkoxycarbonylamino, Benzyloxycarbonyl und Benzyloxycarbonylamino,
worin Phenyl seinerseits substituiert sein kann mit 0, 1, 2 oder 3 Substituenten unabhängig voneinander ausgewählt aus der Gruppe bestehend aus Halogen, C₁-C₄-Alkyl, C₁-C₄-Alkoxy und Phenyl,
oder zwei Substituenten an demselben Kohlenstoffatom im Alkylcarbonyl zusammen mit dem Kohlenstoffatom, an das sie gebunden sind, einen C₃-C₆-Cycloalkyl-Ring bilden,
wobei der Cycloalkyl-Ring Benzo-anneliert sein kann,
R⁴ Wasserstoff bedeutet,
und
R⁵ Methyl bedeutet.

5. Verbindung nach Anspruch 2, **dadurch gekennzeichnet, dass**
R¹ Wasserstoff, C₃-C₆-Cycloalkyl, C₅-C₆-Cycloalkenyl, C₃-C₆-Cycloalkylmethyl, 5-bis 7-gliedriges Heterocyclylmethyl, Methyl, Ethyl, n-Propyl, iso-Propyl, 1-Methylprop-1-yl, 2,2-Dimethylprop-1-yl, 1,1-Dimethylprop-1-yl, 1-Ethyl-prop-1-yl, 1 Ethyl-1-methylprop-1-yl, n-Butyl, 2-Methylbut-1-yl, 3-Methylbut-1-yl, 1-Ethylbut-1-yl, tert-Butyl, 4-Methylpent-1-yl, n-Hexyl, Alkenyl oder Aryl bedeutet,
wobei R¹ substituiert sein kann mit 0, 1, 2 oder 3 Substituenten unabhängig voneinander ausgewählt aus der Gruppe bestehend aus Halogen, Hydroxy, Amino, Cyano, Trimethylsilyl, Alkyl, Alkoxy, Benzyloxy, C₃-C₆-Cycloalkyl, Aryl, 5- bis 10-gliedriges Heteroaryl, Alkylamino, Arylamino, Alkylcarbonylamino, Arylcarbonylamino, Alkylcarbonyl, Alkoxycarbonyl, Arylcarbonyl und Benzyloxycarbonylamino,
worin Aryl und Heteroaryl ihrerseits substituiert sein können mit 0, 1, 2 oder 3 Substituenten unabhängig voneinander ausgewählt aus der Gruppe bestehend aus Halogen, Hydroxy, Amino, Cyano, Nitro, Alkyl, Alkoxy und Phenyl,
R² Wasserstoff oder C₁-C₄-Alkyl bedeutet,
oder
R¹ und R² zusammen mit dem Kohlenstoffatom, an das sie gebunden sind, einen C₃-C₆-Cycloalkyl-Ring oder einen 5- bis 7-gliedrigen Heterocyclyl-Ring bilden, wobei der Cycloalkyl-Ring und der Heterocyclyl-Ring substituiert sein können mit 0, 1, 2 oder 3 Substituenten unabhängig voneinander ausgewählt aus der Gruppe bestehend aus Trifluormethyl, Alkyl, Alkoxy und Alkylcarbonyl,
R³ Alkyl, C₃-C₆-Cycloalkyl, 5- bis 7-gliedriges Heterocyclyl, Aryl, 5- oder 6-gliedriges Heteroaryl, Alkylcarbonyl, Alkoxycarbonyl, C₃-C₆-Cycloalkylcarbonyl, 5- bis 7-gliedriges Heterocyclylcarbonyl, Arylcarbonyl, 5- oder 6-gliedriges Heteroarylcarbonyl oder Alkylaminocarbonyl bedeutet,
wobei Alkyl, Cycloalkyl, Heterocyclyl, Aryl, Heteroaryl, Alkoxycarbonyl, Cyclo-alkylcarbonyl, Heterocyclylcarbonyl, Arylcarbonyl, Heteroarylcarbonyl und Alkylaminocarbonyl substituiert sein können mit 0, 1, 2 oder 3 Substituenten unabhängig voneinander ausgewählt aus der Gruppe bestehend aus Halogen, Hydroxy, Amino, Alkylamino und Phenyl,
und
wobei Alkylcarbonyl substituiert ist mit einem Substituenten Amino oder Alkyl-amino,
und
wobei Alkylcarbonyl substituiert sein kann mit weiteren 0, 1 oder 2 Substituenten unabhängig voneinander ausgewählt aus der Gruppe bestehend aus Halogen, Hydroxy, Trimethylsilyl, Alkoxy, Alkylthio, Benzyloxy, C₃-C₆-Cycloalkyl, Phenyl, Naphthyl, 5- bis 10-gliedriges Heteroaryl, Alkylcarbonylamino, Alkoxy-carbonylamino, Arylcarbonylamino, Arylcarbonyloxy, Benzyloxycarbonyl und Benzyloxycarbonylamino,
worin Phenyl und Heteroaryl ihrerseits substituiert sein können mit 0, 1, 2 oder 3 Substituenten unabhängig voneinander ausgewählt aus der Gruppe bestehend aus Halogen, Hydroxy, Nitro, Alkyl, Alkoxy und Phenyl,
oder zwei Substituenten an demselben Kohlenstoffatom im Alkylcarbonyl zusammen mit dem Kohlenstoffatom, an das sie gebunden sind, einen C₃-C₆-Cycloalkyl-Ring oder einen 5- bis 7-gliedrigen Heterocyclyl-Ring bilden,
wobei der Cycloalkyl-Ring und der Heterocyclyl-Ring substituiert sein können mit 0, 1, 2 oder 3 Substituenten unabhängig voneinander ausgewählt aus der Gruppe bestehend aus Trifluormethyl, Alkyl und Alkoxy,
oder
wobei der Cycloalkyl-Ring Benzo-anneliert sein kann,
R⁴ Wasserstoff, C₁-C₄-Alkyl, Cyclopropyl oder Cyclopropylmethyl bedeutet,
oder
R³ und R⁴ zusammen mit dem Stickstoffatom, an das sie gebunden sind, einen 5- bis 7-gliedrigen Heterocyclyl-Ring bilden, wobei der Heterocyclyl-Ring substituiert sein kann mit 0, 1, 2 oder 3 Substituenten unabhängig voneinander ausgewählt aus der Gruppe bestehend aus Halogen, Hydroxy, Amino, Cyano, Alkyl, Alkoxy und Alkylamino,
und
R⁵ Wasserstoff oder Methyl bedeutet.

6. Verbindung nach Anspruch 5, **dadurch gekennzeichnet, dass**
R¹ Methyl, Ethyl, n-Propyl, iso-Propyl, 1-Methylprop-1-yl, 2,2-Dimethylprop-1-yl, 1,1-Dimethylprop-1-yl, 1-Ethyl-prop-1-yl, 1-Ethyl-1-methylprop-1-yl, n-Butyl, 2-Methylbut-1-yl, 3-Methylbut-1-yl, 1-Ethylbut-1-yl, *tert*-Butyl, 4-Methylpent-1-yl oder n-Hexyl bedeutet,
wobei R¹ substituiert sein kann mit 0 oder 1 Substituenten ausgewählt aus der Gruppe bestehend aus Trimethylsilyl, C₁-C₄-Alkoxy, Benzyloxy, C₃-C₆-Cycloalkyl, Phenyl, Naphthyl, Pyridyl, Indolyl, C₁-C₄-Alkoxycarbonyl und Benzyloxy-carbonylamino,
worin Phenyl und Pyridyl ihrerseits substituiert sein können mit 0, 1, 2 oder 3 Substituenten unabhängig voneinander ausgewählt aus der Gruppe bestehend aus Halogen, Hydroxy, Nitro, C₁-C₄-Alkyl, C₁-C₄-Alkoxy und Phenyl,
R² Wasserstoff bedeutet,
oder
R¹ und R² zusammen mit dem Kohlenstoffatom, an das sie gebunden sind, einen C₃-C₆-Cycloalkyl-Ring bilden, wobei der Cycloalkyl-Ring substituiert sein kann mit 0 oder 1 Substituenten ausgewählt aus der Gruppe bestehend aus Trifluormethyl und C₁-C₄-Alkoxy,
R³ C₁-C₆-Alkylcarbonyl bedeutet,
wobei Alkylcarbonyl substituiert ist mit einem Substituenten Amino,
und
wobei Alkylcarbonyl substituiert sein kann mit weiteren 0, 1 oder 2 Substituenten unabhängig voneinander ausgewählt aus der Gruppe bestehend aus Trimethylsilyl, C₁-C₄-Alkoxy, Methylhio, Benzyloxy, C₃-C₆-Cycloalkyl, Phenyl, Naphthyl, Thienyl, Pyridyl, Indolyl, C₁-C₄-Alkoxycarbonylamino, Benzyloxycarbonyl und Benzyloxycarbonylamino,
worin Phenyl seinerseits substituiert sein kann mit 0, 1, 2 oder 3 Substituenten unabhängig voneinander ausgewählt aus der Gruppe bestehend aus Halogen, C₁-C₄-Alkyl, C₁-C₄-Alkoxy und Phenyl,
oder zwei Substituenten an demselben Kohlenstoffatom im Alkylcarbonyl zusammen mit dem Kohlenstoffatom, an das sie gebunden sind, einen C₃-C₆-Cycloalkyl-Ring bilden,
wobei der Cycloalkyl-Ring Benzo-anneliert sein kann,
R⁴ Wasserstoff bedeutet,
und
R⁵ Methyl bedeutet.

7. Verfahren zur Herstellung einer Verbindung der Formel (I) nach Anspruch 1, **dadurch gekennzeichnet, dass** eine Verbindung der Formel 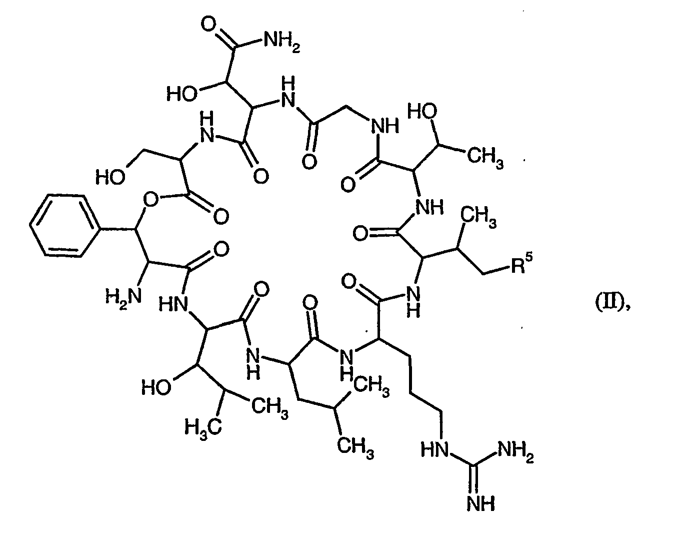 in welcher
R⁵ die in Anspruch 1 angegebene Bedeutung hat,
mit einer Verbindung der Formel 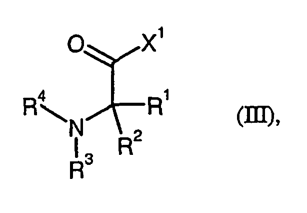 in welcher
R¹, R², R³ und R⁴ die in Anspruch 1 angegebene Bedeutung haben und
X¹ Halogen oder Hydroxy bedeutet,
umgesetzt wird.

8. Verbindung nach einem der Ansprüche 1 bis 6 zur Behandlung und/oder Prophylaxe von Krankheiten.

9. Verwendung einer Verbindung nach einem der Ansprüche 1 bis 6 zur Herstellung eines Arzneimittels zur Behandlung und/oder Prophylaxe von Krankheiten.

10. Verwendung einer Verbindung nach einem der Ansprüche 1 bis 6 zur Herstellung eines Arzneimittels zur Behandlung und/oder Prophylaxe von bakteriellen Infektionen.

11. Arzneimittel enthaltend eine Verbindung nach einem der Ansprüche 1 bis 6 in Kombination mit einem inerten, nichttoxischen, pharmazeutisch geeigneten Hilfsstoff.

12. Arzneimittel nach Anspruch 11 zur Behandlung und/oder Prophylaxe von bakteriellen Infektionen.

## Claims

1. Compound of formula 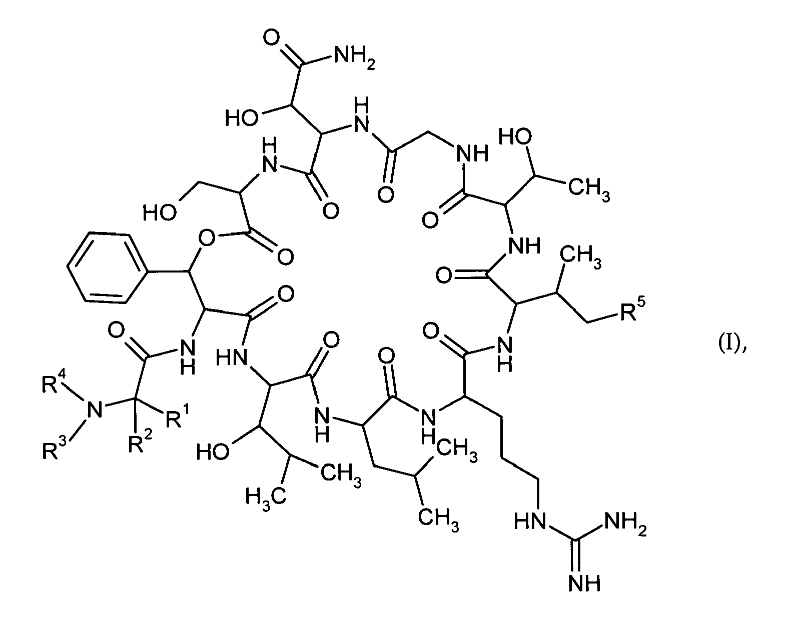 in which
R¹ represents hydrogen, C₃-C₆-cycloalkyl, C₅-C₆-cycloalkenyl, C₃-C₆-cyclo-alkylmethyl, 5- to 7-membered heterocyclylmethyl, methyl, ethyl, n-propyl, isopropyl, 1-methylprop-1-yl, 2-methylprop-1-yl, 2,2-dimethyl-prop-1-yl, 1,1-dimethylprop-1-yl, 1-ethylprop-1-yl, 1-ethyl-1-methyl-prop-1-yl, n-butyl, 2-methylbut-1-yl, 3-methylbut-1-yl, 1-ethylbut-1-yl, tert-butyl, 4-methylpent-1-yl, n-hexyl, alkenyl or aryl,
whereby R¹ may be substituted with 0, 1, 2 or 3 substituents selected independently of one another from the group consisting of halogen, hydroxy, amino, cyano, trimethylsilyl, alkyl, alkoxy, benzyloxy, C₃-C₆-cycloalkyl, aryl, 5- to 10-membered heteroaryl, alkylamino, arylamino, alkylcarbonylamino, arylcarbonylamino, alkylcarbonyl, alkoxycar-bonyl, arylcarbonyl and benzyloxycarbonylamino,
wherein aryl and heteroaryl in turn may be substituted with 0, 1, 2 or 3 substituents selected independently of one another from the group consisting of halogen, hydroxy, amino, cyano, nitro, alkyl, alkoxy and phenyl,
R² represents hydrogen or C₁-C₄-alkyl,
or
R¹ and R² together with the carbon atom to which they are bonded form a C₃-C₆-cycloalkyl ring or a 5- to 7-membered heterocyclyl ring, whereby the cycloalkyl ring and the heterocyclyl ring may be substituted with 0, 1, 2 or 3 substituents selected independently of one another from the group consisting of trifluoromethyl, alkyl, alkoxy and alkylcarbonyl,
R³ represents alkyl, C₃-C₆-cycloalkyl, 5- to 7-membered heterocyclyl, aryl, 5- or 6-membered heteroaryl, alkylcarbonyl, alkoxycarbonyl, C₃-C₆-cycloalkylcarbonyl, 5- to 7-membered heterocyclylcarbonyl, arylcarb-onyl, 5- or 6-membered heteroarylcarbonyl or alkylaminocarbonyl,
whereby alkyl, cycloalkyl, heterocyclyl, aryl, heteroaryl, alkoxycar-bonyl, cycloalkylcarbonyl, heterocyclylcarbonyl, arylcarbonyl, het-eroarylcarbonyl and alkylaminocarbonyl may be substituted with 0, 1, 2 or 3 substituents selected independently of one another from the group consisting of halogen, hydroxy, amino, alkylamino and phenyl,
and
whereby alkylcarbonyl is substituted with one amino or alkylamino
substituent,
and
whereby alkylcarbonyl may be substituted with a further 0, 1 or 2 sub-stituents selected independently of one another from the group consist-ing of halogen, hydroxy, trimethylsilyl, alkoxy, alkylthio, benzyloxy, C₃-C₆-cycloalkyl, phenyl, naphthyl, 5- to 10-membered heteroaryl, al-kylcarbonylamino, alkoxycarbonylamino, arylcarbonylamino, arylcarb-onyloxy, benzyloxycarbonyl and benzyloxycarbonylamino,
wherein phenyl and heteroaryl in turn may be substituted with 0, 1, 2 or 3 substituents selected independently of one another from the group consisting of halogen, hydroxy, nitro, alkyl, alkoxy and phenyl,
or two substituents on the same carbon atom in the alkylcarbonyl together with the carbon atom to which they are bonded form a C₃-C₆-cycloalkyl ring or a 5- to 7-membered heterocyclyl ring,
whereby the cycloalkyl ring and the heterocyclyl ring may be substituted with 0, 1, 2 or 3 substituents selected independently of one another from the group consisting of trifluoromethyl, alkyl and alkoxy,
or
whereby the cycloalkyl ring may be benzo-fused,
R⁴ represents hydrogen, C₁-C₄-alkyl, cyclopropyl or cyclopropylmethyl,
or
R³ and R⁴ together with the nitrogen atom to which they are bonded form a 5-to 7-membered heterocyclyl ring, whereby the heterocyclyl ring may be substituted with 0, 1, 2 or 3 substituents selected independently of one
another from the group consisting of halogen, hydroxy, amino, cyano, alkyl, alkoxy and alkylamino,
and
R⁵ represents hydrogen or methyl,
or one of its salts, its solvates and the solvates of its salts,
with the proviso that in the case where R¹ represents hydrogen, R² represents 2-methylprop-1-yl, R⁴ represents hydrogen and R⁵ represents methyl, and the carbon atom to which R¹ and R¹ are bonded has the (S) configuration, or where R¹ represents 2-methylprop-1-yl, R² represents hydrogen, R⁴ represents hydrogen and R⁵ represents methyl, and the carbon atom to which R¹ and R² are bonded has the (S) configuration, R³ does not represent glycyl, D-alanyl, L-alanyl or D-leucyl, and
with the proviso that in the case where R¹ represents hydrogen, R² represents 2-methylprop-1-yl, R⁴ represents hydrogen and R⁵ represents hydrogen, and the carbon atom to which R¹ and R² are bonded has the (S) configuration, or where R¹ represents 2-methylprop-1-yl, R² represents hydrogen, R⁴ represents hydrogen and R⁵ represents hydrogen, and the carbon atom to which R¹ and R² are bonded has the (S) configuration, R³ does not represent D-leucyl.

2. Compound according to Claim 1, **characterized in that** it corresponds to formula 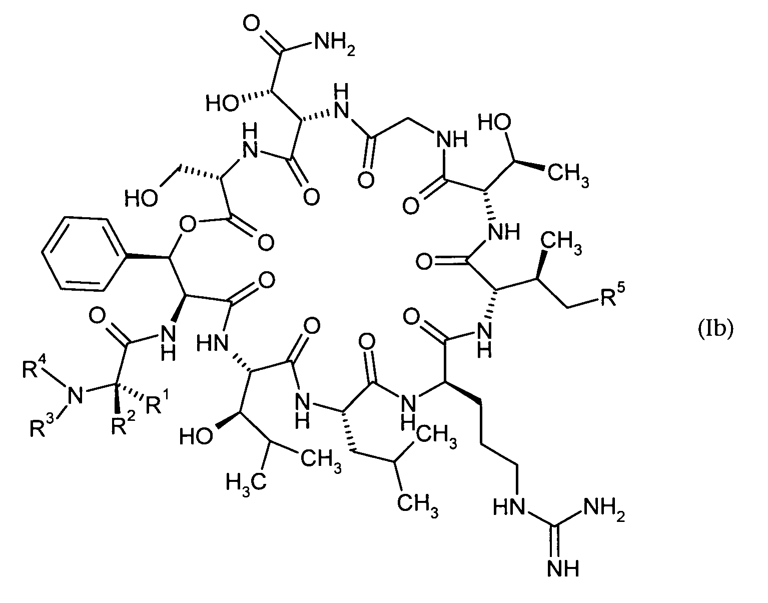 in which
R¹, R², R³, R⁴ and R⁵ have the meaning indicated in Claim 1,
or one of its salts, its solvates and the solvates of its salts.

3. Compound according to Claim 2, **characterized in that**
R¹ represents 2-methylprop-1-yl,
R² represents hydrogen or C₁-C₄-alkyl,
R³ represents alkyl, C₃-C₆-cycloalkyl, 5- to 7-membered heterocyclyl, aryl, 5- or 6-membered heteroaryl, alkylcarbonyl, alkoxycarbonyl, C₃-C₆-cycloalkylcarbonyl, 5- to 7-membered heterocyclylcarbonyl, arylcarb-onyl, 5- or 6-membered heteroarylcarbonyl or alkylaminocarbonyl,
whereby alkyl, cycloalkyl, heterocyclyl, aryl, heteroaryl, alkoxycar-bonyl, cycloalkylcarbonyl, heterocyclylcarbonyl, arylcarbonyl, het
eroarylcarbonyl and alkylaminocarbonyl may be substituted with 0, 1, 2 or 3 substituents selected independently of one another from the group consisting of halogen, hydroxy, amino, alkylamino and phenyl,
and
whereby alkycarbonyl is substituted with one amino or alkylamino substituent,
and
whereby alkylcarbonyl may be substituted with a further 0, 1 or 2 substituents selected independently of one another from the group consisting of halogen, hydroxy, trimethylsilyl, alkoxy, alkylthio, benzyloxy, C₃-C₆-cycloalkyl, phenyl, naphthyl, 5- to 10-membered heteroaryl, alk-ylcarbonylamino, alkoxycarbonylamino, arylcarbonylamino, arylcarb-onyloxy, benzyloxycarbonyl and benzyloxycarbonylamino,
wherein phenyl and heteroaryl in turn may be substituted with 0, 1, 2 or 3 substituents selected independently of one another from the group consisting of halogen, hydroxy, nitro, alkyl, alkoxy and phenyl,
or two substituents on the same carbon atom in the alkylcarbonyl together with the carbon atom to which they are bonded form a C₃-C₆-cycloalkyl ring or a 5- to 7-membered heterocyclyl ring,
whereby the cycloalkyl ring and the heterocyclyl ring may be substituted with 0, 1, 2 or 3 substituents selected independently of one another from the group consisting of trifluoromethyl, alkyl and alkoxy,
or
whereby the cycloalkyl ring may be benzo-fused,
R⁴ represents hydrogen, C₁-C₄-alkyl, cyclopropyl or cyclopropylmethyl,
or
R³ and R⁴ together with the nitrogen atom to which they are bonded form a 5-to 7-membered heterocyclyl ring, whereby the heterocyclyl ring may be substituted with 0, 1, 2 or 3 substituents selected independently of one another from the group consisting of halogen, hydroxy, amino, cyano, alkyl, alkoxy and alkylamino,
and
R⁵ represents hydrogen or methyl.

4. Compound according to Claim 3, **characterized in that**
R¹ represents 2-methylprop-1-yl,
R² represents hydrogen,
R³ represents C₁-C₆-alkylcarbonyl,
whereby alkylcarbonyl is substituted with one amino substituent,
and
whereby alkylcarbonyl may be substituted with a further 0, 1 or 2 substituents selected independently of one another from the group consisting of trimethylsilyl, C₁-C₄-alkoxy, methylthio, benzyloxy, C₃-C₆-cyclo-alkyl, phenyl, naphthyl, thienyl, pyridyl, indolyl, C₁-C₄-alkoxycarb-onylamino, benzyloxycarbonyl and benzyloxycarbonylamino,
wherein phenyl in turn may be substituted with 0, 1, 2 or 3 substituents selected independently of one another from the group consisting of halogen, C₁-C₄-alkyl, C₁-C₄-alkoxy and phenyl,
or two substituents on the same carbon atom in the alkylcarbonyl together with the carbon atom to which they are bonded form a C₃-C₆-cycloalkyl ring,
whereby the cycloalkyl ring may be benzo-fused,
R⁴ represents hydrogen,
and
R⁵ represents methyl.

5. Compound according to Claim 2, **characterized in that**
R¹ represents hydrogen, C₃-C₆-cycloalkyl, C₅-C₆-cycloalkenyl, C₃-C₆-cyclo-alkylmethyl, 5- to 7-membered heterocyclylmethyl, methyl, ethyl, n-propyl, isopropyl, 1-methylprop-1-yl, 2,2-dimethylprop-1-yl, 1,1-di-methylprop-1-yl, 1-ethylprop-1-yl, 1-ethyl-1-methylprop-1-yl, n-butyl, 2-methylbut-1-yl, 3-methylbut-1-yl, 1-ethylbut-1-yl, *tert*-butyl, 4-meth-ylpent-1-yl, n-hexyl, alkenyl or aryl,
whereby R¹ may be substituted with 0, 1, 2 or 3 substituents selected independently of one another from the group consisting of halogen, hydroxy, amino, cyano, trimethylsilyl, alkyl, alkoxy, benzyloxy, C₃-C₆-cycloalkyl, aryl, 5- to 10-membered heteroaryl, alkylamino, arylamino, alkylcarbonylamino, arylcarbonylamino, alkylcarbonyl, alkoxycarb-onyl, arylcarbonyl and benzyloxycarbonylamino,
wherein aryl and heteroaryl in turn may be substituted with 0, 1, 2 or 3 substituents selected independently of one another from the group consisting of halogen, hydroxy, amino, cyano, nitro, alkyl, alkoxy and phenyl,
R² represents hydrogen or C₁-C₄-alkyl,
or
R¹ and R² together with the carbon atom to which they are bonded form a C₃-C₆-cycloalkyl ring or a 5- to 7-membered heterocyclyl ring, whereby the cycloalkyl ring and the heterocyclyl ring may be substituted with 0, 1, 2 or 3 substituents selected independently of one another from the group consisting of trifluoromethyl, alkyl, alkoxy and alkylcarbonyl,
R³ represents alkyl, C₃-C₆-cycloalkyl, 5- to 7-membered heterocyclyl, aryl, 5- or 6-membered heteroaryl, alkylcarbonyl, alkoxycarbonyl, C₃-C₆-cycloalkylcarbonyl, 5- to 7-membered heterocyclylcarbonyl, arylcarb-onyl, 5- or 6-membered heteroarylcarbonyl or alkylaminocarbonyl,
whereby alkyl, cycloalkyl, heterocyclyl, aryl, heteroaryl, alkoxycarb-onyl, cycloalkylcarbonyl, heterocyclylcarbonyl, arylcarbonyl, hetero-arylcarbonyl and alkylaminocarbonyl may be substituted with 0, 1, 2 or 3 substituents selected independently of one another from the group consisting of halogen, hydroxy, amino, alkylamino and phenyl,
and
whereby alkylcarbonyl is substituted with one amino or alkylamino substituent,
and
whereby alkylcarbonyl may be substituted with a further 0, 1 or 2 sub-stituents selected independently of one another from the group consist-ing of halogen, hydroxy, trimethylsilyl, alkoxy, alkylthio, benzyloxy, C₃-C₆-cycloalkyl, phenyl, naphthyl, 5- to 10-membered heteroaryl, alk-ylcarbonylamino, alkoxycarbonylamino, arylcarbonylamino, arylcarb-onyloxy, benzyloxycarbonyl and benzyloxycarbonylamino,
wherein phenyl and heteroaryl in turn may be substituted with 0, 1, 2 or 3 substituents selected independently of one another from the group consisting of halogen, hydroxy, nitro, alkyl, alkoxy and phenyl,
or two substituents on the same carbon atom in the alkylcarbonyl together with the carbon atom to which they are bonded form a C₃-C₆-cycloalkyl ring or a 5- to 7-membered heterocyclyl ring,
whereby the cycloalkyl ring and the heterocyclyl ring may be substituted with 0, 1, 2 or 3 substituents selected independently of one another from the group consisting of trifluoromethyl, al-kyl and alkoxy,
or
whereby the cycloalkyl ring may be benzo-fused,
R⁴ represents hydrogen, C₁-C₄-alkyl, cyclopropyl or cyclopropylmethyl,
or
R³ and R⁴ together with the nitrogen atom to which they are bonded form a 5-to 7-membered heterocyclyl ring, whereby the heterocyclyl ring may be substituted with 0, 1, 2 or 3 substituents selected independently of one another from the group consisting of halogen, hydroxy, amino, cyano, alkyl, alkoxy and alkylamino,
and
R⁵ represents hydrogen or methyl.

6. Compound according to Claim 5, **characterized in that**
R¹ represents methyl, ethyl, n-propyl, isopropyl, 1-methylprop-1-yl, 2,2-dimethylprop-1-yl, 1,1-dimethylprop-1-yl, 1-ethylprop-1-yl, 1-ethyl-1-methylprop-1-yl, n-butyl, 2-methylbut-1-yl, 3-methylbut-1-yl, 1-ethyl-but-1-yl, *tert*-butyl, 4-methylpent-1-yl or n-hexyl,
whereby R¹ may be substituted with 0 or 1 substituent selected from the group consisting of trimethylsilyl, C₁-C₄-alkoxy, benzyloxy, C₃-C₆-cycloalkyl, phenyl, naphthyl, pyridyl, indolyl, C₁-C₄-alkoxycarbonyl and benzyloxycarbonylamino,
wherein phenyl and pyridyl in turn may be substituted with 0, 1, 2 or 3 substituents selected independently of one another from the group consisting of halogen, hydroxy, nitro, C₁-C₄-alkyl, C₁-C₄-alkoxy and phenyl,
R² represents hydrogen,
or
R¹ and R² together with the carbon atom to which they are bonded form a C₃-C₆-cycloalkyl ring, whereby the cycloalkyl ring may be substituted with 0 or 1 substituent selected from the group consisting of trifluoromethyl and C₁-C₄-alkoxy,
R³ represents C₁-C₆-alkylcarbonyl,
whereby alkylcarbonyl is substituted with one amino substituent,
and
whereby alkylcarbonyl may be substituted with a further 0, 1 or 2 substituents selected independently of one another from the group consisting of trimethylsilyl, C₁-C₄-alkoxy, methylthio, benzyloxy, C₃-C₆-cycloalkyl, phenyl, naphthyl, thienyl, pyridyl, indolyl, C₁-C₄-alkoxycarbonylamino, benzyloxycarbonyl and benzyloxycarbonyl-amino,
wherein phenyl in turn may be substituted with 0, 1, 2 or 3 sub-stituents selected independently of one another from the group consisting of halogen, C₁-C₄-alkyl, C₁-C₄-alkoxy and phenyl,
or two substituents on the same carbon atom in the alkylcarbonyl together with the carbon atom to which they are bonded form a C₃-C₆
cycloalkyl ring,
whereby the cycloalkyl ring may be benzo-fused,
R⁴ represents hydrogen,
and
R⁵ represents methyl.

7. Method for preparing a compound of formula (I) according to Claim 1, **characterized in that** a compound of formula 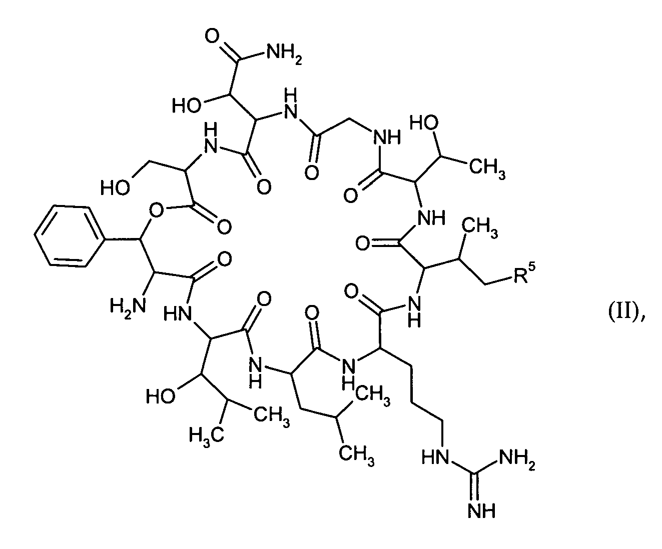 in which
R⁵ has the meaning indicated in Claim 1,
is reacted with a compound of formula 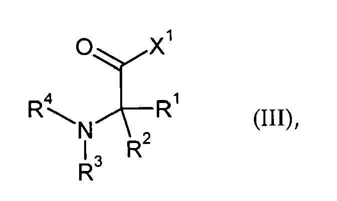 in which
R¹, R², R³ and R⁴ have the meaning indicated in Claim 1, and
X¹ represents halogen or hydroxy.

8. Compound according to any one of Claims 1 to 6 for the treatment and/or prophylaxis of diseases.

9. Use of a compound according to any one of Claims 1 to 6 for producing a medicament for the treatment and/or prophylaxis of diseases.

10. Use of a compound according to any one of Claims 1 to 6 for producing a medicament for the treatment and/or prophylaxis of bacterial infections.

11. Medicament comprising a compound according to any one of Claims 1 to 6 in combination with an inert, nontoxic, pharmaceutically suitable excipient.

12. Medicament according to Claim 11 for the treatment and/or prophylaxis of bacterial infections.

## Revendications

1. Composé de formule : 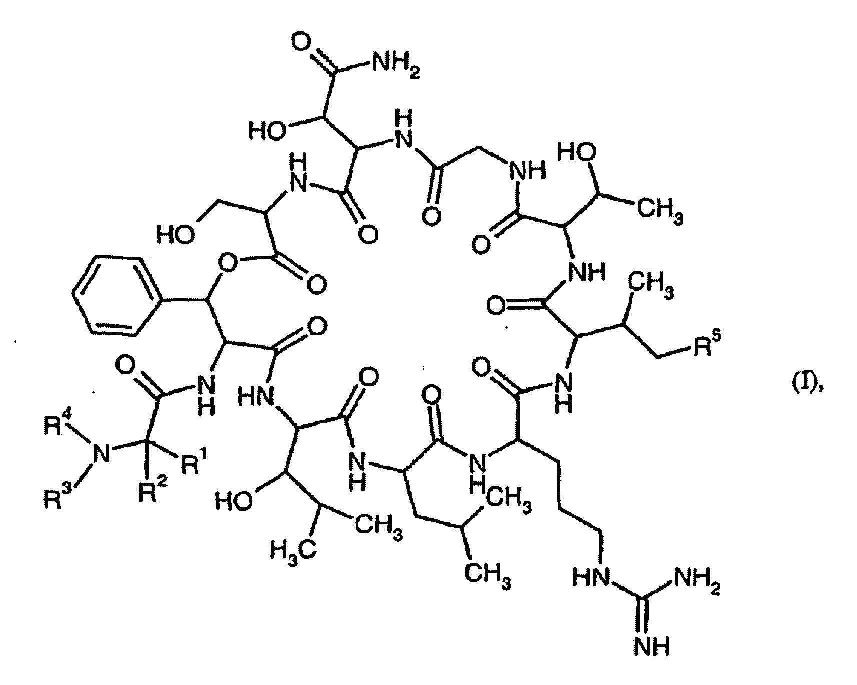 dans laquelle :
R¹ représente hydrogène, cycloalkyle en C₃-C₆, cycloalcényle en C₅-C₆, (cycloalkyl en C₃-C₆)méthyle, (hétérocyclyl de 5 à 7 membres)méthyle, méthyle, éthyle, n-propyle, isopropyle, 1-méthylprop-1-yle, 2-méthylprop-1-yle, 2,2-di-méthylprop-1-yle, 1,1-diméthylprop-1-yle, 1-éthylprop-1-yle, 1-éthyl-1-méthyl-prop-1-yle, n-butyle, 2-méthylbut-1-yle, 3-méthylbut-1-yle, 1-éthylbut-1-yle, t-butyle, 4-méthylpent-1-yle, n-hexyle, alcényle ou aryle,
où R¹ peut être substitué avec 0, 1, 2 ou 3 substituants choisis indépendamment l'un de l'autre parmi le groupe consistant en halogène, hydroxyle, amino, cyano, triméthylsilyle, alkyle, alcoxy, benzyloxy, cycloalkyle en C₃-C₆, aryle, hétéroaryle ayant 5 à 10 membres, alkylamino, arylamino, alkylcarbonylamino, arylcarbonylamino, alkylcarbonyle, alcoxycarbonyle, arylcarbonyle et benzyloxycarbonylamino,
où aryle et hétéroaryle peuvent être, à leur tour, substitués avec 0, 1, 2 ou 3 substituants choisis indépendamment l'un de l'autre parmi le groupe consistant en halogène, hydroxyle, amino, cyano, nitro, alkyle, alcoxy et phényle,
R² représente hydrogène ou alkyle en C₁-C₄,
ou
R¹ et R² avec l'atome de carbone sur lequel ils sont liés, forment un cycloalkyle en C₃-C₆ ou un hétérocycle ayant 5 à 7 membres, où le cycloalkyle et l'hétérocyclyle peuvent être substitués avec 0, 1, 2 ou 3 substituants choisis indépendamment l'un de l'autre parmi le groupe consistant en trifluorométhyle, alkyle, alcoxy et alkylcarbonyle,
R³ représente alkyle, cycloalkyle en C₃-C₆, hétérocycle ayant 5 à 7 membres, aryle, hétéroaryle ayant 5 ou 6 membres, alkylcarbonyle, alcoxycarbonyle, (cycloalkyl en C₃-C₆)carbonyle, (hétérocyclyl ayant 5 à 7 membres)carbonyle, arylcarbonyle, (hétéroaryl ayant 5 ou 6 membres)carbonyle ou alkylaminocarbonyle,
où alkyle, cycloalkyle, hétérocyclyle, aryle, hétéroaryle, alcoxycarbonyle, cycloalkylcarbonyle, hétérocyclylcarbonyle, arylcarbonyle, hétéroarylcarbonyle et alkylaminocarbonyle peuvent être substitués avec 0, 1, 2 ou 3 substituants choisis indépendamment l'un de l'autre parmi le groupe consistant en halogène, hydroxyle, amino, alkylamino et phényle, et
où alkylcarbonyle est substitué avec un substituant amino ou alkylamino, et
où alkylcarbonyle peut être substitué d'autre part, avec 0, 1, 2 substituants choisis indépendamment l'un de l'autre parmi le groupe consistant en halogène, hydroxyle, triméthylsilyle, alcoxy, alkylthio, benzyloxy, cycloalkyle en C₃-C₆, phényle, naphtyle, hétéroaryle ayant 5 à 10 membres, alkylcarbonylamino, alcoxycarbonylamino, arylcarbonylamino, arylcarbonyloxy, benzyloxycarbonyle et benzyloxycarbonylamino,
où phényle et hétéroaryle peuvent être, à leur tour, substitués avec 0, 1, 2 ou 3 substituants choisis indépendamment l'un de l'autre parmi le groupe consistant en halogène, hydroxyle, nitro, alkyle, alcoxy et phényle,
ou deux substituants sur le même atome de carbone dans le radical alkylcarbonyle, avec l'atome de carbone sur lequel ils sont liés, forment un cycloalkyle en C₃-C₆ ou un hétérocycle ayant 5 à 7 membres,
où le cycloalkyle et l'hétérocyclyle peuvent être substitués avec 0, 1, 2
ou 3 substituants choisis indépendamment l'un de l'autre parmi le groupe consistant en trifluorométhyle, alkyle et alcoxy,
ou
où le cycloalkyle peut être condensé à un cycle benzénique,
R⁴ représente hydrogène, alkyle en C₁-C₄, cyclopropyle ou cyclopropylméthyle,
ou
R³ et R⁴ forment avec l'atome d'azote sur lequel ils sont liés, un hétérocycle ayant 5 à 7 membres, où l'hétérocyclyle peut être substitué avec 0, 1, 2 ou 3 substituants choisis indépendamment l'un de l'autre parmi le groupe consistant en halogène, hydroxyle, amino, cyano, alkyle, alcoxy et alkylamino,
et
R⁵ représente hydrogène ou méthyle,
ou un de leurs sels, leurs solvates et les solvates de leurs sels, avec la condition que dans le cas où R¹ est hydrogène, R² est 2-méthyl-prop-1-yle, R⁴ est hydrogène et R⁵ est méthyle, et où l'atome de carbone sur lequel R¹ et R² sont liés, a la configuration (S), ou où R¹ est 2-méthylprop-1-yle, R² est hydrogène, R⁴ est hydrogène et R⁵ est méthyle, et où l'atome de carbone sur lequel R¹ et R² sont liés, a la configuration (S), R³ ne représente pas glycyle, D-alanyle, L-alanyle ou D-leucyle,
et
avec la condition que dans le cas où R¹ est hydrogène, R² est 2-méthyl-prop-1-yle, R⁴ est hydrogène et R⁵ est hydrogène, et où l'atome de carbone sur lequel R¹ et R² sont liés, a la configuration (S), ou où R¹ est 2-méthylprop-1-yle, R² est hydrogène, R⁴ est hydrogène et R⁵ est hydrogène, et où l'atome de carbone sur lequel R¹ et R² sont lié, à la configuration (S), R³ ne représente pas D-leucyle.

2. Composé selon la revendication 1, **caractérisé en ce qu'**il correspond
à la formule : 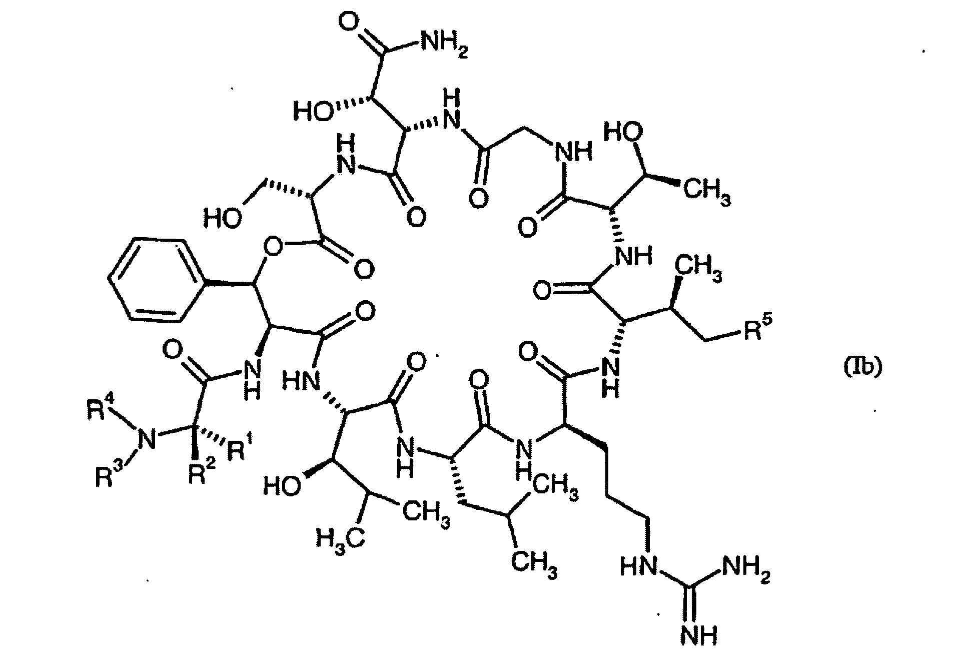 dans laquelle
R¹, R², R³, R⁴ et R⁵ ont la signification donnée à la revendication 1,
ou un de leurs sels, leurs solvates et les solvates de leurs sels.

3. Composé selon la revendication 2, **caractérisé en ce que**
R¹ représente 2-méthylprop-1-yle,
R² représente hydrogène ou alkyle en C₁-C₄,
R³ représente alkyle, cycloalkyle en C₃-C₆, hétérocycle ayant 5 à 7 membres, aryle, hétéroaryle ayant 5 ou 6 membres, alkylcarbonyle, alcoxycarbonyle, (cycloalkyl en C₃-C₆)carbonyle, (hétérocyclyl ayant 5 à 7 membres)carbonyle, arylcarbonyle, (hétéroaryl ayant 5 ou 6 membres)carbonyle ou alkylaminocarbonyle,
où alkyle, cycloalkyle, hétérocyclyle, aryle, hétéroaryle, alcoxycarbonyle, cycloalkylcarbonyle, hétérocyclylcarbonyle, arylcarbonyle, hétéroarylcarbonyle et alkylaminocarbonyle peuvent être substitués avec 0, 1, 2 ou 3 substituants choisis indépendamment l'un de l'autre parmi le groupe consistant en halogène, hydroxyle, amino, alkylamino et phényle,
et
où alkylcarbonyle est substitué avec un substituant amino ou alkylamino,
et
où alkylcarbonyle peut être substitué d'autre part, avec 0, 1 ou 2 substituants choisis indépendamment l'un de l'autre parmi le groupe consistant en halogène, hydroxyle, triméthylsilyle, alcoxy, alkylthio, benzyloxy, cycloalkyle en C₃-C₆, phényle, naphtyle, hétéroaryle ayant 5 à 10 membres, alkylcarbonylamino, alcoxycarbonylamino, arylcarbonylamino, arylcarbonyloxy, benzyloxycarbonyle et benzyloxycarbonylamino,
où phényle et hétéroaryle peuvent être, à leur tour, substitués avec 0, 1, 2 ou 3 substituants choisis indépendamment l'un de l'autre parmi le groupe consistant en halogène, hydroxyle, nitro, alkyle, alcoxy et phényle,
ou deux substituants sur le même atome de carbone dans le radical alkylcarbonyle, avec l'atome de carbone sur lequel ils sont liés, forment un cycloalkyle en C₃-C₆ ou un hétérocycle ayant 5 à 7 membres,
où le cycloalkyle et l'hétérocyclyle peuvent être substitués avec 0, 1, 2
ou 3 substituants choisis indépendamment l'un de l'autre parmi le groupe consistant en trifluorométhyle, alkyle et alcoxy,
ou
où le cycloalkyle peut être condensé à un cycle benzénique,
R⁴ représente hydrogène, alkyle en C₁-C₄, cyclopropyle ou cyclopropylméthyle,
ou
R³ et R⁴ forment avec l'atome d'azote sur lequel ils sont liés, un hétérocycle ayant 5 à 7 membres, où l'hétérocyclyle peut être substitué avec 0, 1, 2 ou 3 substituants choisis indépendamment l'un de l'autre parmi le groupe consistant en halogène, hydroxyle, amino, cyano, alkyle, alcoxy et alkylamino,
et
R⁵ représente hydrogène ou méthyle.

4. Composé selon la revendication 3, **caractérisé en ce que**
R¹ représente 2-méthylprop-1-yle,
R² représente hydrogène, R³ représente (alkyl en C₁-C₆)carbonyle,
où alkylcarbonyle est substitué avec un substituant amino,
et
où alkylcarbonyle peut être substitué d'autre part, avec 0, 1 ou 2 substituants choisis indépendamment l'un de l'autre parmi le groupe consistant en triméthylsilyle, alcoxy en C₁-C₄, méthylthio, benzyloxy, cycloalkyle en C₃-C₆, phényle, naphtyle, thiényle, pyridyle, indolyle, (alcoxy en C₁-C₄)carbonylamino, benzyloxycarbonyle et benzyloxycarbonylamino,
où phényle peut être, à son tour, substitué avec 0, 1, 2 ou 3 substituants choisis indépendamment l'un de l'autre parmi le groupe consistant en halogène, alkyle en C₁-C₄, alcoxy en C₁-C₄, et phényle,
ou deux substituants sur le même atome de carbone dans le radical alkylcarbonyle, avec l'atome de carbone sur lequel ils sont liés, forment un cycloalkyle en C₃-C₆,
où le cycloalkyle peut être condensé à un cycle benzénique, R⁴ représente hydrogène,
et
R⁵ représente méthyle.

5. Composé selon la revendication 2, **caractérisé en ce que**
R¹ représente hydrogène, cycloalkyle en C₃-C₆, cycloalcényle en C₅-C₆, (cycloalkyl en C₃-C₆)méthyle, (hétérocyclyl de 5 à 7 membres)méthyle, méthyle, éthyle, n-propyle, isopropyle, 1-méthylprop-1-yle, 2,2-diméthylprop-1-yle, 1,1-diméthylprop-1-yle, 1-éthylprop-1-yle, 1-éthyl-1-méthylprop-1-yle, n-butyle, 2-méthylbut-1-yle, 3-méthylbut-1-yle, 1-éthylbut-1-yle, t-butyle, 4-méthylpent-1-yle, n-hexyle, alcényle ou aryle,
où R¹ peut être substitué avec 0, 1, 2 ou 3 substituants choisis indépendamment l'un de l'autre parmi le groupe consistant en halogène, hydroxyle, amino, cyano, triméthylsilyle, alkyle, alcoxy, benzyloxy, cycloalkyle en C₃-C₆, aryle, hétéroaryle ayant 5 à 10 membres, alkylamino, arylamino, alkylcarbonylamino, arylcarbonylamino, alkylcarbonyle, alcoxycarbonyle, arylcarbonyle et benzyloxycarbonylamino,
où aryle et hétéroaryle peuvent être, à leur tour, substitués avec 0, 1, 2
ou 3 substituants choisis indépendamment l'un de l'autre parmi le groupe consistant en halogène, hydroxyle, amino, cyano, nitro, alkyle, alcoxy et phényle,
R² représente hydrogène ou alkyle en C₁-C₄,
ou
R¹ et R², avec l'atome de carbone sur lequel ils sont liés, forment un cycloalkyle en C₃-C₆ ou un hétérocycle ayant 5 à 7 membres, où le cycloalkyle et l'hétérocyclyle peuvent être substitués avec 0, 1, 2 ou 3 substituants choisis indépendamment l'un de l'autre parmi le groupe consistant en trifluorométhyle, alkyle, alcoxy et alkylcarbonyle,
R³ représente alkyle, cycloalkyle en C₃-C₆, hétérocycle ayant 5 à 7 membres, aryle, hétéroaryle ayant 5 ou 6 membres, alkylcarbonyle, alcoxycarbonyle, (cycloalkyl en C₃-C₆)carbonyle, (hétérocyclyl ayant 5 à 7 membres)carbonyle, arylcarbonyle, (hétéroaryl ayant 5 ou 6 membres)carbonyle ou alkylaminocarbonyle,
où alkyle, cycloalkyle, hétérocyclyle, aryle, hétéroaryle, alcoxycarbonyle, cycloalkylcarbonyle, hétérocyclylcarbonyle, arylcarbonyle, hétéroarylcarbonyle et alkylaminocarbonyle peuvent être substitués avec 0, 1, 2 ou 3 substituants choisis indépendamment l'un de l'autre parmi le groupe consistant en halogène, hydroxyle, amino, alkylamino et phényle,
et
où alkylcarbonyle est substitué avec un substituant amino ou alkylamino, et
où alkylcarbonyle peut être substitué d'autre part, avec 0, 1 ou 2 substituants choisis indépendamment l'un de l'autre parmi le groupe consistant en halogène, hydroxyle, triméthylsilyle, alcoxy, alkylthio, benzyloxy, cycloalkyle en C₃-C₆, phényle, naphtyle, hétéroaryle ayant 5 à 10 membres, alkylcarbonylamino, alcoxycarbonylamino, arylcarbonylamino, arylcarbonyloxy, benzyloxycarbonyle et benzyloxycarbonylamino,
où phényle et hétéroaryle peuvent être, à leur tour, substitués avec 0, 1, 2 ou 3 substituants choisis indépendamment l'un de l'autre parmi le groupe consistant en halogène, hydroxyle, nitro, alkyle, alcoxy et phényle,
ou deux substituants sur le même atome de carbone dans le radical alkylcarbonyle, avec l'atome de carbone sur lequel ils sont liés, forment un cycloalkyle en C₃-C₆ ou un hétérocycle ayant 5 à 7 membres,
où le cycloalkyle et l'hétérocyclyle peuvent être substitués avec 0, 1, 2
ou 3 substituants choisis indépendamment l'un de l'autre parmi le groupe consistant en trifluorométhyle, alkyle et alcoxy,
ou
où le cycloalkyle peut être condensé à un cycle benzénique,
R⁴ représente hydrogène, alkyle en C₁-C₄, cyclopropyle ou cyclopropylméthyle,
ou
R³ et R⁴ forment avec l'atome d'azote sur lequel ils sont liés, un hétérocycle ayant 5 à 7 membres, où l'hétérocyclyle peut être substitué avec 0, 1, 2 ou 3 substituants choisis indépendamment l'un de l'autre parmi le groupe consistant en halogène, hydroxyle, amino, cyano, alkyle, alcoxy et alkylamino,
et
R⁵ représente hydrogène ou méthyle.

6. Composé selon la revendication 5, **caractérisé en ce que**
R¹ représente méthyle, éthyle, n-propyle, isopropyle, 1-méthylprop-1-yle, 2,2-diméthylprop-1-yle, 1,1-diméthylprop-1-yle, 1-éthylprop-1-yle, 1-éthyl-1-méthylprop-1-yle, n-butyle, 2-méthylbut-1-yle, 3-méthylbut-1-yle, 1-éthylbut-1-yle, t-butyle, 4-méthylpent-1-yle ou n-hexyle,
où R¹ peut être substitué avec 0 ou 1 substituant choisi parmi le groupe consistant en triméthylsilyle, alcoxy en C₁-C₄, benzyloxy, cycloalkyle en C₃-C₆, phényle, naphtyle, pyridyle, indolyle, (alcoxy en C₁-C₄,)carbonyle, et benzyloxycarbonylamino,
où phényle et pyridyle peuvent être, à leur tour, substitués avec 0, 1, 2
ou 3 substituants choisis indépendamment l'un de l'autre parmi le groupe consistant en halogène, hydroxyle, nitro, alkyle en C₁-C₄, alcoxy en C₁-C₄ et phényle,
R² représente hydrogène,
ou
R¹ et R², avec l'atome de carbone sur lequel ils sont liés, forment un cycloalkyle en C₃-C₆, où le cycloalkyle peut être substitué avec 0 ou 1 substituant choisi parmi le groupe consistant en trifluorométhyle et alcoxy en C₁-C₄,
R³ représente (alkyl en C₁-C₆)carbonyle,
où alkylcarbonyle est substitué avec un substituant amino,
et
où alkylcarbonyle peut être substitué d'autre part, avec 0, 1, 2 substituants choisis indépendamment l'un de l'autre parmi le groupe consistant en, triméthylsilyle, alcoxy en C₁-C₄, méthylthio, benzyloxy, cycloalkyle en C₃-C₆, phényle, naphtyle, thiényle, pyridyle, indolyle, (alcoxy en C₁-C₄)carbonylamino, benzyloxycarbonyle et benzyloxycarbonylamino,
où phényle peut être, à son tour, substitué avec 0, 1, 2 ou 3 substituants choisis indépendamment l'un de l'autre parmi le groupe consistant en halogène, alkyle en C₁-C₄, alcoxy en C₁-C₄, et phényle,
ou deux substituants sur le même atome de carbone dans le radical alkylcarbonyle, avec l'atome de carbone sur lequel ils sont liés, forment un cycloalkyle en C₃-C₆,
où le cycloalkyle peut être condensé à un cycle benzénique, R⁴ représente hydrogène,
et
R⁵ représente méthyle.

7. Procédé de préparation d'un composé de la formule (I) selon la revendication 1, **caractérisé en ce qu'**un composé de formule : 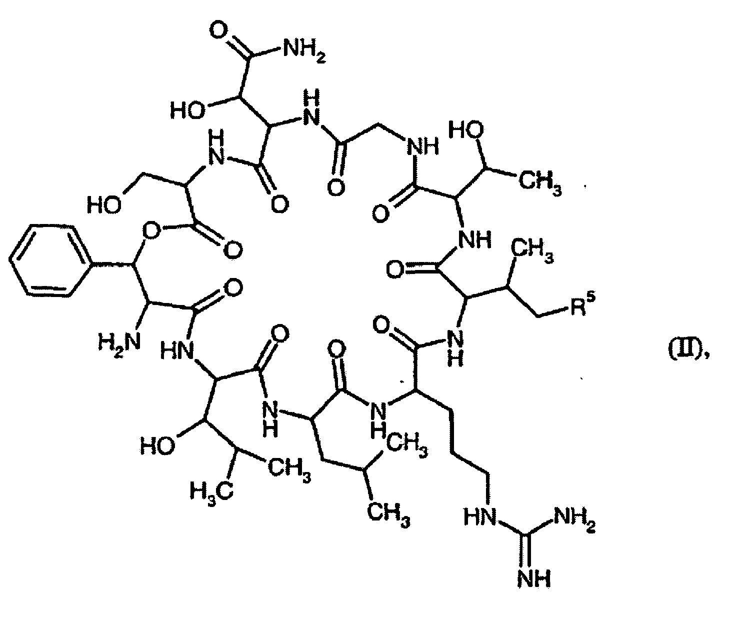 dans laquelle
R⁵ a la signification donnée à la revendication 1,
est mis à réagir
avec un composé de formule : 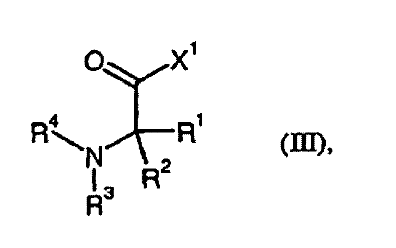 dans laquelle
R¹, R², R³ et R⁴ ont la signification donnée à la revendication 1, et
X¹ représente halogène ou hydroxyle.

8. Composé selon l'une des revendications 1 à 6, pour le traitement et/ou la prophylaxie de maladies.

9. Utilisation d'un composé selon l'une des revendications 1 à 6, pour la préparation d'un agent pharmaceutique pour le traitement et/ou la prophylaxie de maladies.

10. Utilisation d'un composé selon l'une des revendications 1 à 6, pour la préparation d'un agent pharmaceutique pour le traitement et/ou la prophylaxie d'infections bactériennes.

11. Agent pharmaceutique contenant un composé selon l'une des revendications 1 à 6, en combinaison avec un auxiliaire pharmaceutiquement approprié, inerte, non toxique.

12. Agent pharmaceutique selon la revendication 11, pour le traitement et/ou la prophylaxie d'infections bactériennes.
